# EUROPÄISCHE PATENTSCHRIFT

(11) **EP 2 150 530 B1**
(45) Veröffentlichungstag und Bekanntmachung des Hinweises auf die Patenterteilung: **26.10.2011**
(21) Anmeldenummer: 08749188.2
(22) Anmeldetag: 28.04.2008
(51) Int. Cl.: C07D 211/26, C07D 211/96, C07D 295/135, A61K 31/445, A61P 25/00

(54) **SUBSTITUIERTE SULFONAMID-DERIVATE**
SUBSTITUTED SULFONAMIDE DERIVATIVES
DÉRIVÉS DE SULFONAMIDE SUBSTITUÉS

(30) Priorität: 30.04.2007 DE 102007020492
(43) Veröffentlichungstag der Anmeldung: 10.02.2010
(73) Patentinhaber: Grünenthal GmbH, 52078 Aachen (DE)
(72) Erfinder: MERLA, Beatrix, 52078 Aachen (DE); OBERBÖRSCH, Stefan, 52074 Aachen (DE); REICH, Melanie, 52072 Aachen (DE); SUNDERMANN, Bernd, 61381 Friedrichsdorf (DE); ENGLBERGER, Werner, 52223 Stolberg (DE); STRÜNKER, Timo, 60937 Köln (DE); JOSTOCK, Ruth, 52223 Stolberg (DE); HEES, Sabine, 52076 Aachen (DE); BIJSTERVELD, Edward, NL-6546 XL Nijmegen (NL); THEIL, Fritz, 12247 Berlin (DE); GRAUBAUM, Heinz, 12557 Berlin (DE)
(86) Internationale Anmeldenummer: PCT/EP2008/003417
(87) Internationale Veröffentlichungsnummer: WO 2008/131947

(56) Entgegenhaltungen:
- WO-A-2004/092164
- WO-A-2007/115821

## Beschreibung

Die vorliegende Erfindung betrifft substituierte Sulfonamid-Derivate, Verfahren zu deren Herstellung, Arzneimittel enthaltend diese Verbindungen und die Verwendung von substituierten Sulfonamid-Derivaten zur Herstellung von Arzneimitteln.

Im Gegensatz zur konstitutiven Expression des Bradykinin 2 Rezeptors (B2R) wird der Bradykinin 1 Rezeptor (B1R) in den meisten Geweben nicht oder nur schwach exprimiert. Allerdings ist die Expression des B1R auf verschiedenen Zellen induzierbar. Beispielsweise erfolgt im Verlauf von Entzündungsreaktionen eine rasche und ausgeprägte Induktion des B1 R auf neuronalen Zellen aber auch verschiedenen peripheren Zellen wie Fibroblasten, Endothelzellen, Granulozyten, Makrophagen und Lymphozyten. Somit kommt es im Verlauf von Entzündungsreaktionen zu einem switch von einer B2R zu einer B1 R Dominanz auf den beteiligten Zellen. Wesentlich an dieser B1 R Heraufregulation beteiligt sind die Zytokine Interleukin-1 (IL:1) und Tumor Nekrose Faktor alpha (TNFα) (Passos et al. J. Immunol. 2004, 172, 1839-1847). Nach Aktivierung mit spezifischen Liganden können B1 R-exprimierende Zellen anschließend selbst entzündungsfördemde Zytokine wie IL-6 und IL-8 sezernieren (Hayashi et al., Eur. Respir. J. 2000, 16, 452-458). Dies führt zur Einwanderung weiterer Entzündungszellen, z.B. neutrophiler Granulozyten (Pesquero et al., PNAS 2000, 97, 8140-8145). Über diese Mechanismen kann das Bradykinin-B1 R-System zur Chronifizierung von Erkrankungen beitragen. Dies belegt eine Vielzahl tierexperimenteller Untersuchungen (Übersichten in Leeb-Lundberg et al., Pharmacol Rev. 2005, 57, 27-77 und Pesquero et al., Biol. Chem. 2006, 387, 119-126). Auch am Menschen zeigt sich eine verstärkte Expression des B1R, z.B. auf Enterozyten und Makrophagen im betroffenen Gewebe von Patienten mit entzündlichen Darmerkrankungen (Stadnicki et al., Am. J. Physiol. Gastrointest. Liver Physiol. 2005, 289, G361-366) bzw. auf T-Lymphozyten von Patienten mit Multipler Sklerose (Prat 1999) oder eine Aktivierung des Bradykinin-B2R-B1R Systems im Verlauf von Infektionen mit Staphyloccocus aureus (Bengtson et al., Blood 2006, 108, 2055-2063). Infektionen mit Staphyloccocus aureus sind verantwortlich für Krankheitsbilder wie oberflächliche Infektionen der Haut bis hin zu septischem Schock.

Aufgrund der dargestellten pathophysiologischen Zusammenhänge ergibt sich für die Anwendung von B1R-Antagonisten ein großes therapeutisches Potential bei akuten und insbesondere chronisch-entzündlichen Erkrankungen. Hierzu gehören Erkrankungen der Atemwege (Asthma bronchiale, Allergien, COPD/chronicobstruktive pulmonary disease, zystische Fibrose usw.), entzündliche Darmerkrankungen (Ulcerative Colitis, CD/Crohn's disease usw.), neurologische Erkrankungen (Multiple Sklerose, Neurodegeneration usw.), Entzündungen der Haut (atopische Dermatitis, Psoriasis, bakterielle Infektionen usw.) und Schleimhäute (M. Behcet, Pelvitis, Prostatitis usw.), rheumatische Erkrankungen (rheumatoide Arthritis, Osteoarthritis usw.), septischen Schock und Reperfusionssyndrom (nach Herzinfarkt, Schlaganfall).

Darüber hinaus ist das Bradykinin(Rezeptor)-System auch an der Regulation der Angiogenese beteiligt (Potential als Angiogenese-Inhibitor bei Krebs sowie Makula-Degeneration am Auge) und B1 R-knockout Mäuse sind geschützt vor der Induktion von Übergewicht durch eine besonders fettreiche Ernährung (Pesquero et al., Biol. Chem. 2006, 387, 119-126). B1R-Antagonisten eignen sich daher auch zur Behandlung von Fettleibigkeit.

B1 R-Antagonisten sind insbesondere geeignet zur Behandlung von Schmerz, insbesondere Entzündungsschmerz und neuropathischem Schmerz (Calixto et al., Br. J. Pharmacol 2004, 1-16), hier insbesondere von diabetischer Neuropathie (Gabra et al., Biol. Chem. 2006, 387, 127-143).

Verbindungen, die an den µ-Opioid-Rezeptor binden, eignen sich besonders zur Behandlung von Schmerz, aber auch zur Behandlung von Depressionen, Pruritus, Antriebslosigkeit, Diarrhöe und zur Anxiolyse.

WO 2004/092164 beschreibt cyclische Aminderivate zur Behandlung Bradykinin modulierter entzündlicher Erkrankungen.

WO 2007/115821 beschreibt die Verwendung verschiedenster Verbindungen, u.a. auch Sulfonamidverbindungen, als DPP-IV Inhibitoren.

Eine der Erfindung zugrundeliegende Aufgabe bestand darin, neue Verbindungen zur Verfügung zu stellen, die sich vorzugsweise als pharmakologische Wirkstoffe in Arzneimitteln eignen, insbesondere analgetisch wirksame Substanzen, die sich zur Schmerztherapie - insbesondere auch zur Therapie von Entzündungeschmerz und neuropathischem Schmerzen - eignen.

Gegenstand der Erfindung sind daher substituierte Sulfonamid-Derivate der allgemeinen Formel I. 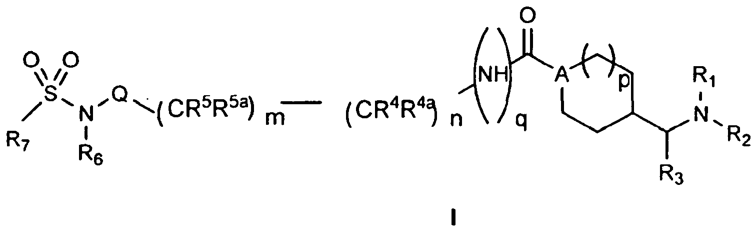 worin
m für 0, 1 oder 2 steht
n für 0, 1 oder 2 steht
p für 0, 1 oder 2 steht
q für 0 oder 1 steht

A für N, CH-NH-, CH-CH₂NH-, CH-CH₂-CH₂-NH oder CH-CH₂-CH₂-CH₂-NH steht, wobei einzelne H-Atome auch durch C₁₋₅-Alkyl ersetzt sein können,
R¹ und R² unabhängig voneinander H; C₁₋₆-Alkyl, jeweils gesättigt oder ungesättigt, verzweigt oder unverzweigt, unsubstituiert oder einfach oder mehrfach substituiert; oder über eine C₁₋₃-Alkylkette verknüpftes Aryl, unsubstituiert oder einfach oder mehrfach substituiert, bedeuten, wobei R¹ und R² nicht gleichzeitig H bedeuten,
oder die Reste R¹ und R² zusammen CH₂CH₂OCH₂CH₂, CH₂CH₂NR⁸CH₂CH₂ oder (CH₂)₃₋₆ bedeuten,
wobei R⁸ H; C₁₋₆-Alkyl, jeweils gesättigt oder ungesättigt, verzweigt oder unverzweigt, unsubstituiert oder einfach oder mehrfach substituiert; Aryl, Hetereoaryl, jeweils unsubstituiert oder einfach oder mehrfach substituiert, oder über eine C₁₋₃-Alkylkette verknüpftes Aryl oder Heteroaryl, jeweils unsubstituiert oder einfach oder mehrfach substituiert, bedeutet;
R³ für C₁₋₈-Alkyl jeweils gesättigt oder ungesättigt, verzweigt oder unverzweigt, unsubstituiert oder einfach oder mehrfach substituiert; Aryl oder Heteroaryl, jeweils unsubstituiert oder einfach oder mehrfach substituiert; oder über eine C₁₋₃-Alkylkette verknüpftes Aryl oder Heteroaryl, jeweils unsubstituiert oder einfach oder mehrfach substituiert, steht;
R⁴ und R^{4a} unabhängig voneinander für H, C₁₋₆-Alkyl, jeweils gesättigt oder ungesättigt, verzweigt oder unverzweigt, unsubstituiert oder einfach oder mehrfach substituiert; F; Cl; Aryl jeweils unsubstituiert oder einfach oder mehrfach substituiert; oder über eine C₁₋₃-Alkylkette verknüpftes Aryl, jeweils unsubstituiert oder einfach oder mehrfach substituiert, steht;
R⁵ und R^{5a} unabhängig voneinander für H; oder C₁₋₆-Alkyl, jeweils gesättigt oder ungesättigt, verzweigt oder unverzweigt, unsubstituiert oder einfach oder mehrfach substituiert; OH, OC₁₋₆-Alkyl, F, Cl, Phenoxy oder Benzyloxy; stehen;
Q eine Einfachbindung, -CH₂-, -CH₂-CH₂-, oder 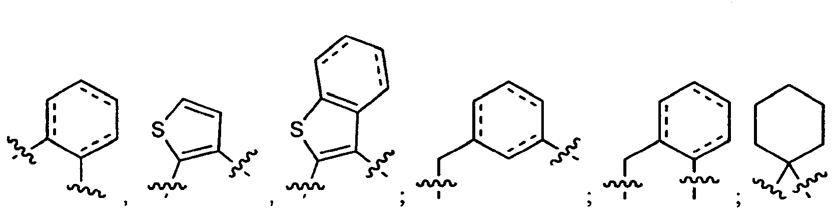 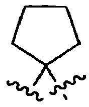 bedeutet,
wobei -̅ -̅ -̅ -̅ für eine Einfachbindung oder für eine Doppelbindung steht;
R⁶ für H; C₁₋₆-Alkyl, jeweils gesättigt oder ungesättigt, verzweigt oder unverzweigt, unsubstituiert oder einfach oder mehrfach substituiert; C₃₋₈-Cycloalkyl, gesättigt oder ungesättigt, unsubstituiert oder einfach oder mehrfach substituiert; Aryl oder Heteroaryl, unsubstituiert oder einfach oder mehrfach substituiert; über eine C₁₋₃-Alkylkette verknüpftes Aryl oder C₃₋₈-Cycloalkyl;
oder R⁶ mit Q zusammen unter Einschluss des benachbarten Stickstoffs einen vier-, fünf-, sechs- oder siebengliedrigen Carbocyclus bildet, der gesättigt oder ungesättigt sein und ein weiteres Heteroatom O, S oder N enthalten kann, an den ein weiterer fünf- oder sechsgliedriger Ring, gesättigt oder ungesättigt, ankondensiert sein kann; wobei im Fall des gemeinsamen Ringschlusses Q für 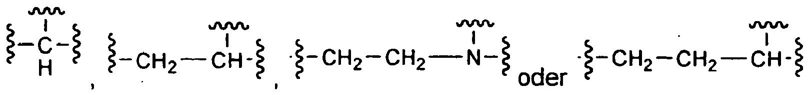 steht, und der Ring in jeder Position mit Phenyl, =O, OH, OC₁₋₆-Alkyl, F, Cl, CF₃, oder C₁₋₆-Alkyl substituiert sein kann;
und R⁷ für Aryl oder Heteroaryl, jeweils unsubstituiert oder einfach oder mehrfach substituiert; über eine C₁₋₃-Alkylgruppe verknüpftes Aryl oder Heteroaryl jeweils unsubstituiert oder einfach oder mehrfach substituiert, steht;

in Form des Razemats; der Enantiomere, Diastereomere, Mischungen der Enantiomere oder Diastereomere oder eines einzelnen Enantiomers oder Diastereomers; der Basen und/oder Salze physiologisch verträglicher Säuren.

Die Verbindungen weisen eine Affinität zum humanen B1-Rezeptor auf. Darüber hinaus weisen sie vorzugsweise eine Affinität zum µ-Opioid-Rezeptor auf.

Wenn die Gruppe A in den Verbindungen der allgemeinen Formel I für CH-NH-, CH-CH₂-NH-, CH-CH₂-CH₂-NH oder CH-CH₂-CH₂-CH₂-NH- steht, ist immer C-Kettenende in den Ring eingebunden und das N-Kettenende mit der Carbonylgruppe verknüpft.

Die Ausdrücke "C₁₋₃-Alkyl", C₁₋₆-Alkyl" und "C₁₋₈-Alkyl" umfassen im Sinne dieser Erfindung acyclische gesättigte Kohlenwasserstoffreste, die verzweigt- oder geradkettig sowie unsubstituiert oder ein- oder mehrfach substituiert sein können, mit 1 bis 3 C-Atomen bzw. 1 bis 6 C-Atomen bzw. 1 bis 8 C-Atome, d.h. C₁₋₃-Alkanyle, bzw. C₁₋₆-Alkanyle, bzw. C₁₋₈-Alkanyle. Dabei weisen Alkenyle mindestens eine C-C-Doppelbindung und Alkinyle mindestens eine C-C-Dreifachbindung auf. Vorteilhaft ist Alkyl aus der Gruppe ausgewählt, die Methyl, Ethyl, n-Propyl, 2-Propyl, n-Butyl, iso-Butyl, sec.-Butyl, tert.-Butyl, n-Pentyl, iso-Pentyl, neo-Pentyl, Hexyl, Heptyl, Octyl, umfasst. Besonders vorteihaft sind Methyl, Ethyl und n-Propyl.

Der Ausdruck "Aryl" bedeutet im Sinne dieser Erfindung aromatische Kohlenwasserstoffe, u.a. Phenyle und Naphthyle. Die Aryl-Reste können auch mit weiteren gesättigten, (partiell) ungesättigten oder aromatischen Ringsystemen kondensiert sein. Jeder Aryl-Rest kann unsubstituiert oder einfach oder mehrfach substituiert vorliegen, wobei die Aryl-Substituenten gleich oder verschieden und in jeder beliebigen und möglichen Position des Aryls sein können. Vorteilhafterweise ist Aryl aus der Gruppe ausgewählt, die Phenyl, 1-Naphthyl, 2-Naphthyl, welche jeweils unsubstituiert oder ein- oder mehrfach substituiert sein können, enthält. Besonders vorteilhaft ist der Phenyl-Rest.

Der Ausdruch "Heteroaryl" steht für einen 5-, 6- oder 7-gliedrigen cyclischen aromatischen Rest, der mindestens 1, ggf. auch 2, 3, 4 oder 5 Heteroatome, enthält, wobei die Heteroatome gleich oder verschieden sind und der Heterocyclus unsubstituiert oder ein- oder mehrfach substituiert sein kann; im Falle der Substitution am Heterocyclus können die Substituenten gleich oder verschieden sein und in jeder beliebigen und möglichen Position des Heteroaryls sein. Der Heterocyclus kann auch Teil eines bi- oder polycyclischen Systems sein. Bevorzugte Heteroatome sind Stickstoff, Sauerstoff und Schwefel. Es ist bevorzugt, daß der Heteroaryl-Rest ausgewählt ist aus der Gruppe, die Pyrrolyl, Indolyl, Furyl (Furanyl), Benzofuranyl, Thienyl (Thiophenyl), Benzothienyl, Benzothiadiazolyl, Benzothiazolyl, Benzotriazolyl, Benzodioxolanyl, Benzodioxanyl, Phtalazinyl, Pyrazolyl, Imidazolyl, Thiazolyl, Oxadiazolyl, Isoxazoyl, Pyridyl, Pyridazinyl, Pyrimidinyl, Pyrazinyl, Pyranyl, Indazolyl, Purinyl, Indolizinyl, Chinolinyl, Isochinolinyl, Chinazolinyl, Carbazolyl, Phenazinyl, Phenothiazinyl oder Oxadiazolyl enthält, wobei die Bindung an die Verbindungen der allgemeinen Struktur I über jedes beliebige und mögliche Ringglied des Heteroaryl-Restes erfolgen kann. Besonders bevorzugt sind Thienyl, Furyl, Benzothiadiazolyl, Oxadiazolyl und Pyridyl.

Der Ausdruck "über C₁₋₃-Alkyl gebundenes Aryl oder Heteroaryl" bedeuten für die Zwecke der vorliegenden Erfindung, daß C₁₋₃-Alkyl und Aryl bzw. Heteroaryl die oben definierten Bedeutungen haben und der Aryl- bzw. Heteroaryl-Rest über eine C₁₋₃-Alkyl-Gruppe an die Verbindung der allgemeinen Struktur I gebunden ist. Besonders vorteilhaft im Sinne dieser Erfindung ist Phenyl, Benzyl und Phenethyl.

Im Zusammenhang mit "Alkyl" und Cycloalkyl" versteht man unter dem Begriff "substituiert" im Sinne dieser Erfindung die Substitution eines Wasserstoffrestes durch F, Cl, Br, I, -CN, NH₂, NH-C₁₋₆-Alkyl, NH-C₁₋₆Alkyl-OH, C₁₋₆-Alkyl, N(C₁₋₆-Alkyl)₂, N(C₁₋₆-Alkyl-OH)₂, NO₂, SH, S-C₁₋₆-Alkyl, S-Benzyl, O-C₁₋₆-Alkyl, OH, O-C₁₋₆-Alkyl-OH, =O, O-Benzyl, C(=O)C₁₋₆-Alkyl, CO₂H, CO₂-C₁₋₆-Alkyl, Phenyl oder Benzyl, wobei unter mehrfach substituierten Resten solche Reste zu verstehen sind, die entweder an verschiedenen oder an gleichen Atomen mehrfach, z.B. zwei- oder dreifach, substituiert sind, beispielsweise dreifach am gleichen C-Atom wie im Falle von CF₃ oder -CH₂CF₃ oder an verschiedenen Stellen wie im Falle von -CH(OH)-CH=CH-CHCl₂. Die Mehrfachsubstitution kann mit dem gleichen oder mit verschiedenen Substituenten erfolgen. Bevorzugte Reste im Zusammenhang mit "Alkyl" und "Cycloalkyl" sind F, Cl, -CN, NH₂, OCH₃, OH, CO₂-CH₃, CO₂-C₂H₅, =O und SCH₃.

In Bezug auf "Aryl" und "Heteroaryl" versteht man im Sinne dieser Erfindung unter "ein- oder mehrfach substituiert" die ein- oder mehrfache, z.B. zwei-, drei- oder vierfache, Substitution eines oder mehrerer Wasserstoffatome des Ringsystems durch F, Cl, Br, I, CN, NH₂, NH-C₁₋₆-Alkyl, NH- C₁₋₆-Alkyl-OH, N(C₁₋₆Alkyl)₂, N(C₁₋₆-Alkyl-OH)₂, NO₂, SH, S-C₁₋₆-Alkyl, OH, O-C₁₋₆-Alkyl, O-C₁₋₆Alkyl-OH, C(=O)C₁₋₆-Alkyl, CO₂H, CH₂SO₂-Phenyl, CO₂-C₁₋₆-Alkyl, OCF₃, CF₃, 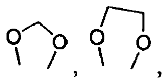 C₁₋₆-Alkyl, Phenyl, Pyridyl, Thienyl oder Furyl; an einem oder ggf. verschiedenen Atomen, wobei ein Substituent ggf. seinerseits substituiert sein kann. Die Mehrfachsubstitution erfolgt dabei mit dem gleichen oder mit unterschiedlichen Substituenten. Für "Aryl" sind dabei bevorzugte Substituenten -F, -Cl, *tert.*-Butyl, CF₃, OCF₃, 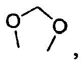 CH₃ oder OCH₃

Unter dem Begriff des mit einer physiologisch verträglichen Säure gebildeten Salzes versteht man im Sinne dieser Erfindung Salze des jeweiligen Wirkstoffes mit anorganischen bzw. organischen Säuren, die physiologisch - insbesondere bei Anwendung im Menschen - verträglich sind. Besonders bevorzugt ist das Hydrochlorid. Beispiele für physiologisch verträgliche Säuren sind: Salzsäure, Bromwasserstoffsäure, Schwefelsäure, Methansulfonsäure, Ameisensäure, Essigsäure, Oxalsäure, Bernsteinsäure, Weinsäure, Mandelsäure, Fumarsäure, Maleinsäure, Milchsäure, Zitronensäure, Glutaminsäure, 1,1-Dioxo-1,2-dihydro1λ⁶-benzo[d]isothiazol-3-on (Saccharinsäure), Monomethylsebacinsäure, 5-Oxo-prolin, Hexan-1-sulfonsäure, Nicotinsäure, 2-, 3- oder 4-Aminobenzoesäure, 2,4,6-Trimethyl-benzoesäure, α-Liponsäure, Acetylglycin, Hippursäure, Phosphorsäure und Asparaginsäure. Besonders bevorzugt sind die Zitronensäure und die Salzsäure.

Unter dem Begriff (CH₂)₃₋₆ bzw. (CH₂)₄₋₅ ist -CH₂-CH₂-CH₂-, -CH₂-CH₂-CH₂-CH₂-,-CH₂-CH₂-CH₂-CH₂-CH₂- und CH₂-CH₂-CH₂-CH₂-CH₂-CH₂- bzw -CH₂-CH₂-CH₂-CH₂-, -CH₂-CH₂-CH₂-CH₂-CH₂ zu verstehen.

Bevorzugt sind substituierte Amid-Derivate der allgemeinen Formel I, worin die Reste oder Gruppen R¹-R⁸, R^{H}, R^{J}, A, Z und Q sowie m, n, p und q die oben angegebene Bedeutung haben,
wobei
"Alkyl sustituiert" und "Cycloalkyl substituiert" die Substitution eines oder mehrerer Wasserstoffreste durch F, Cl, Br, I, -CN, NH₂, NH-C₁₋₆-Alkyl, NH-C₁₋₆-Alkyl-OH, C₁₋₆-Alkyl, N(C₁₋₆-Alkyl)₂, N(C₁₋₆-Alkyl-OH)₂, NO₂, SH, S-C₁₋₆-Alkyl, S-Benzyl, O-C₁₋₆-Alkyl, OH, O-C₁₋₆-Alkyl-OH, =O, O-Benzyl, C(=O)C₁₋₆-Alkyl, CO₂H, CO₂-C₁₋₆-Alkyl, Phenyl oder Benzyl,
"Aryl substituiert" und "Heteroaryl substituiert" die ein- oder mehrfache, z.B. zwei-, drei- oder vierfache, Substitution eines oder mehrerer Wasserstoffatome des Ringsystems durch F, Cl, Br, I, CN, NH₂, NH-C₁₋₆-Alkyl, NH- C₁₋₆-Alkyl-OH, N(C₁₋₆ Alkyl)₂, N(C₁₋₆-Alkyl-OH)₂, NO₂, SH, S-C₁₋₆-Alkyl, OH, O-C₁₋₆-Alkyl, O-C₁₋₆Alkyl-OH, C(=O)C₁₋₆-Alkyl, CO₂H, CH₂SO₂-Phenyl, CO₂-C₁₋₆-Alkyl, OCF₃, CF₃, 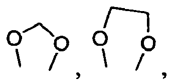 C₁₋₆-Alkyl, Phenyl, Pyridyl, Thienyl oder Furyl bedeutet,
in Form des Razemats; der Enantiomere, Diastereomere, Mischungen der Enantiomere oder Diastereomere oder eines einzelnen Enantiomers oder Diastereomers; der Basen und/oder Salze physiologisch verträglicher Säuren.

Die nachfolgend als bevorzugt beschriebenen Reste und Gruppen bzw. Substituenten können in den erfindungsgemäßen Verbindungen mit der breitesten Bedeutung der übrigen Reste, aber auch mit bevorzugten Bedeutungen anderer Reste und Gruppen bzw. Substituenten kombiniert werden.

Bevorzugt im Sinne dieser Erfindung sind substituierte Sulfonamid-Derivate, worin A für CH-NH-, CH-CH₂-NH-, CH-CH₂-CH₂-NH oder CH-CH₂-CH₂-CH₂-NH steht, wobei einzelne H-Atome auch durch C₁₋₅-Alkyl ersetzt sein können.

p steht vorzugsweise für 1.

Bevorzugt sind Verbindungen, bei denen q für 0 steht.

Bevorzugt sind auch substituierte Sulfonamid-Derivate, worin R¹ und R² unabhängig voneinander H; CH₃; C₂H₅; oder über eine C₁₋₃-Alkylkette verknüpftes Phenyl, bedeuten, wobei R¹ und R² nicht gleichzeitig H bedeuten,
oder die Reste R¹ und R² zusammen CH₂CH₂OCH₂CH₂, CH₂CH₂NR⁸CH₂CH₂ oder (CH₂)₃₋₅ bedeuten.

Besonders bevorzugt sind substituierte Sulfonamid-Derivate, worin R¹ und R² unabhängig voneinander H oder CH₃ bedeuten, insbesondere CH₃.

Bevorzugt im Sinne dieser Erfindung sind auch substituierte Sulfonamid-Derivate, worin R³ für Aryl oder Heteroaryl, jeweils unsubstituiert oder einfach oder mehrfach substituiert; oder über eine C₁₋₃-Alkylkette verknüpftes Aryl, jeweils unsubstituiert oder einfach oder mehrfach substituiert, steht.

Besonders bevorzugt sind substituierte Sulfonamid-Derivate, worin R³ für 2-Thienyl, 4-Fluorphenyl, 3-Fluorphenyl, 4-Chlorphenyl, Phenethyl, Phenyl oder Benzyl steht.

Bevorzugt sind auch substituierte Sulfonamid-Derivate, worin R⁴ und R^{4a} für H stehen.

Darüber hinaus sind substituierte Sulfonamid-Derivate bevorzugt, worin R⁵ und R^{5a} für H stehen.

Bevorzugt sind auch substituierte Sulfonamid-Derivate, worin R⁶ für Methyl, Ethyl oder Benzyl steht und Q eine Einfachbindung darstellt, wobei R⁶ insbesondere Methyl bedeutet.

Weiterhin bevorzugt sind substituierte Sulfonamid-Derivate, worin 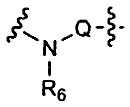 für, 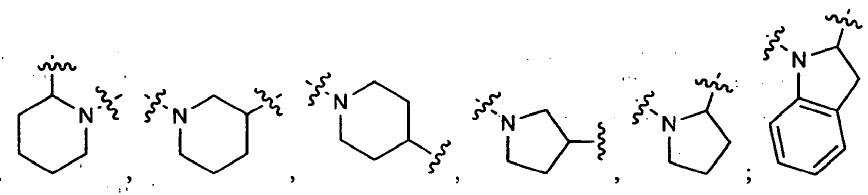 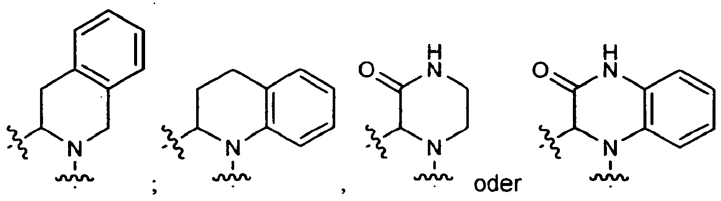 steht.

Bevorzugt sind auch substituierte Sulfonamid-Derivate, worin Q 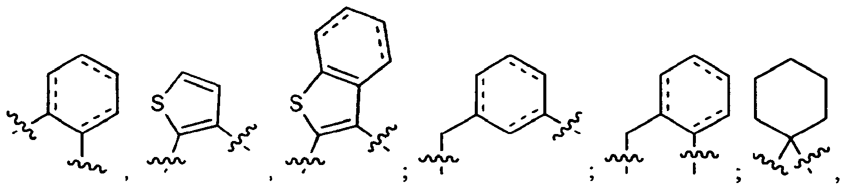 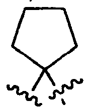 bedeutet.

Bevorzugt sind auch substituierte Sulfonamid-Derivate, worin R⁷ für Phenyl, oder Naphthyl, unsubstituiert oder einfach oder mehrfach substituiert, insbesondere Phenyl, steht.

Besonders bevorzugt sind substituierte Sulfonamid-Derivate, worin R⁷ 2,4-Dichlorphenyl, 2,4,6-Trichlorphenyl, 4-Methoxyphenyl, 3-Trifluormethylphenyl, 2,6-Dimethyl-4-methoxyphenyl, 3,4-Dichlorphenyl, 2,5-Dimethyl-4-Chlorphenyl oder Naphthyl steht.

Bevorzugt sind auch substituierte Sulfonamid-Derivate, worin die Gruppe 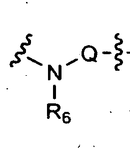 der allgemeinen Formel I für 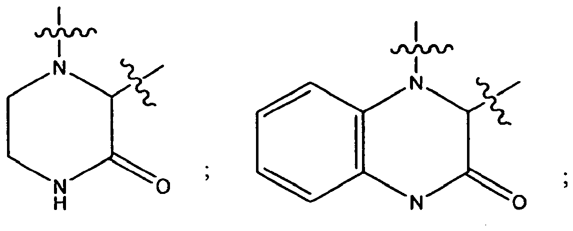 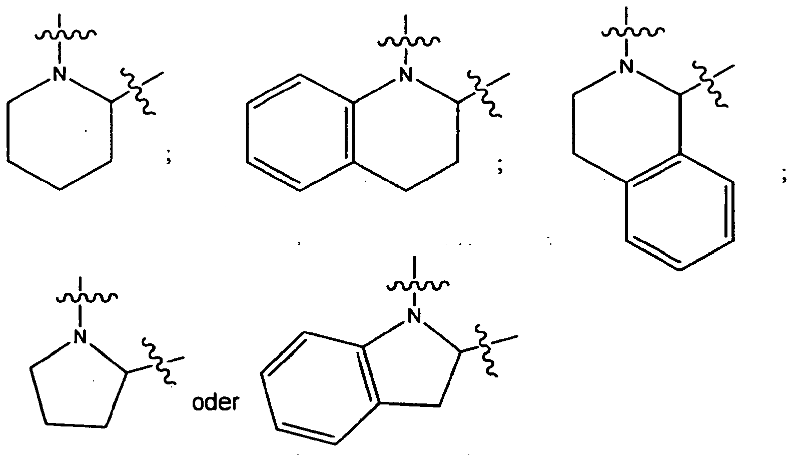 steht;
m für 0, 1 oder 2 steht,
n für 0 steht,
q für 0 steht; und
R⁵ und R^{5a} für H stehen;
oder
Q für eine Einfachbindung steht;
m für 0, 1 oder 2 steht;
n für 1 oder 2 steht;
q für 0 oder 1 steht;
R⁴ und R^{4a} jeweils unabhängig voneinander für H oder Aryl stehen;
R⁵ und R^{5a} für H stehen, und
R⁶ für H oder C₁₋₆-Alkyl steht;
oder
Q für 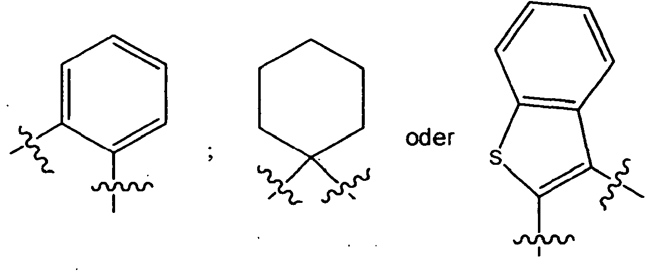 steht;
m für 0 oder 1, vorzugsweise 0, steht;
n und q für 0 stehen;
R⁵ und R^{5a} für H stehen; und
R⁶ für H oder C₁₋₆-Alkyl steht.

Auch bevorzugt sind substituierte Sulfonamid-Derivate, worin in der allgemeinen Formel I
A für N, NH-CH NH-CH₂-CH oder NH-CH₂-CH₂-CH steht, wobei einzelne H-Atome auch durch C₁₋₅-Alkyl ersetzt sein können;
R¹ und R² unabhängig voneinander für C₁₋₆-Alkyl, insbesondere Methyl, Ethyl, n-Propyl, iso-Propyl, n-Butyl, sec-Butyl, iso-Butyl und t-Butyl, stehen, oder zusammen mit dem N-Atom, an das sie gebunden sind, eine Gruppe ausgesucht aus 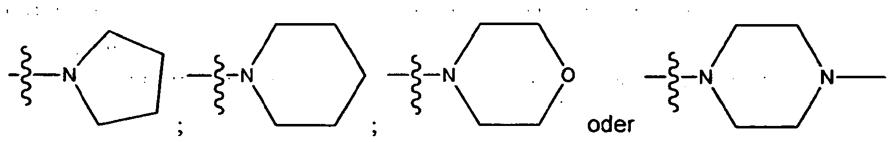 bilden; und
R³ für Aryl, insbesondere Phenyl oder Furanyl, das über eine C₁₋₃-Alkylgruppe verknüpft sein kann, steht, wobei das Aryl jeweils unsubstituiert oder einfach oder mehrfach, gleich oder verschieden mit Substituenten unabhängig ausgewählt aus der Gruppe bestehend aus Methyl, Ethyl, Methoxy, Ethoxy, F, Cl, Br, F, CN, CF₃, OCF₃ und OH substituiert ist.

Weiterhin bevorzugt sind substituierte Sulfonamid-Derivate, worin die Gruppe 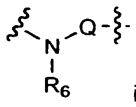 in der allgemeinen Formel I für 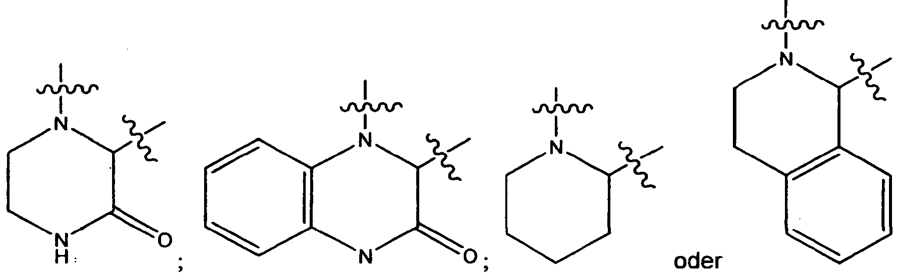 steht;
m für 1 oder 2, insbesondere 1, steht;
n und q für 0 stehen;
R⁵ und R^{5a} für H stehen;
A für N, NH-CH oder NH-CH₂-CH, insbesondere N oder NH-CH steht,
R¹ und R² unabhängig voneinander für C₁₋₆-Alkyl, insbesondere Methyl, Ethyl, n-Propyl, iso-Propyl, n-Butyl, sec-Butyl, iso-Butyl und t-Butyl, stehen, oder zusammen mit dem N-Atom, an das sie gebunden sind, eine Gruppe ausgesucht aus 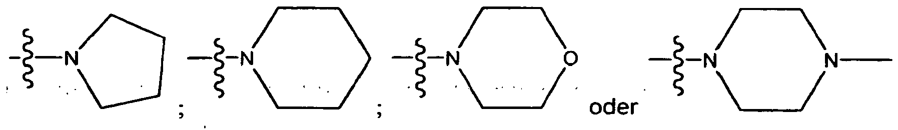 bilden; und
R₃ für Phenyl steht, das über eine C₁₋₃-Alkylgruppe verknüpft sein kann, wobei dass Phenyl jeweils unsubstituiert oder einfach oder mehrfach, gleich oder verschieden mit Substituenten unabhängig ausgewählt aus der Gruppe bestehend aus Methyl, Ethyl, Methoxy, Ethoxy, F, Cl, Br, F, CN, CF₃, OCF₃ und OH, insbesondere Methyl, Methosy, F, Cl, Br, CN, CF₃ und OH, substituiert ist.

Ganz besonders bevorzugt sind substituierte Sulfonamid-Derivate aus der Gruppe
(1) N-(3-{3-[4-(Dimethylaminophenylmethyl)cyclohexyl]ureido}propyl)-4-methoxy-2,3,6,N-tetramethylbenzolsulfonamid
(2) (*N*-(3-(3-((4-((dimethylamino)(phenyl)methyl)cyclohexyl)methyl)ureido)propyl)-4-methoxy-*N*,2,3,6-tetramethylbenzolsulfonamid
(3) 4-Methoxy-N,2,3,6-tetramethyl-N-(3-(3-(4-(phenyl(pyrrolidin-1-yl)methyl)cyclohexyl)ureido)propyl)benzolsulfonamid
(4) 4-(Dimethylamino-phenyl-methyl)-piperidin-1-carbonsäure-{3-[(4-methoxy-2,3,6-trimethyl-benzolsulfonyl)-methyl-amino]-propyl}-amid
(5) 5-[Methyl-(2,4,6-trichlor-benzolsulfonyl)-amino]-pentancarbonsäure-[4-(dimethylamino-phenyl-methyl)-cyclohexyl]-amid
(6) 5-[Methyl-(2,4,6-trichlor-benzolsulfonyl)-amino]-pentancarbonsäure-[4-(phenylpiperidin-1-yl-methyl)-cyclohexyl]-amid
(7) 5-[Methyl-(2,4,6-trichlor-benzolsulfonyl)-amino]-pentancarbonsäure-[4-(dimethylamino-phenyl-methyl)-cyclohexylmethyl]-amid
(8) N-[4-(Dimethylamino-phenyl-methyl)-cyclohexylmethyl]-2-[1-(3-trifluormethylbenzolsulfonyl)-piperidin-2-yl]-acetamid
(9) N-[4-(Dimethylamino-phenyl-methyl)-cyclohexyl]-2-[2-(4-methoxybenzolsulfonyl)-1,2,3,4-tetrahydro-isochinolin-1-yl]-acetamid
(10) 2-[2-(3,4-Dichlor-benzolsulfonyl)-1,2,3,4-tetrahydro-isochinolin-1-yl]-N-[4-(dimethylamino-phenyl-methyl)-cyclohexyl)-acetamid
(11) 2-[(3,4-Dichlor-benzolsulfonyl)-methyl-amino]-N-[4-(dimethylamino-phenylmethyl)-cyclohexyl]-benzamid
(12) 2-[(3,4-Dichlor-benzolsulfonyl)-methyl-amino]-4,5,6,7-tetrahydrobenzo[b]thiophen-3-carbonsäure-[4-(dimethylamino-phenyl-methyl)-cyclohexyl]-amid
(13) N-{4-[Dimethylamino-(3-fluor-phenyl)-methyl]-cyclohexyl}-2-[2-(4-methoxybenzolsulfonyl)-1,2,3,4-tetrahydro-isochinolin-1-yl]-acetamid
(14) N-{4-[Dimethylamino-(4-fluor-phenyl)-methyl]-cyclohexyl}-2-[2-(4-methoxybenzolsulfonyl)-1,2,3,4-tetrahydro-isochinolin-1-yl]-acetamid
(15) N-[4-(Dimethylamino-thiophen-2-yl-methyl)-cyclohexyl]-2-[2-(4-methoxybenzolsulfonyl)-1,2,3,4-tetrahydro-isochinolin-1-yl]-acetamid
(16) N-{4-[Dimethylamino-(3-fluor-phenyl)-methyl]-cyclohexylmethyl}-2-[2-(4-methoxy-benzolsulfonyl)-1,2,3,4-tetrahydro-isochinolin-1-yl]-acetamid
(17) N-[4-(Dimethylamino-phenyl-methyl)-cyclohexylmethyl]-2-[2-(4-methoxybenzolsulfonyl)-1,2,3,4-tetrahydro-isochinolin-1-yl]-acetamid
(18) N-[4-(1-Dimethylamino-3-phenyl-propyl)-cyclohexylmethyl]-2-[2-(4-methoxybenzolsulfonyl)-1,2,3,4-tetrahydro-isochinolin-1-yl]-acetamid
(19) N-{4-[Dimethylamino-(4-fluor-phenyl)-methyl]-cyclohexylmethyl}-2-[2-(4-methoxy-benzolsulfonyl)-1,2,3,4-tetrahydro-isochinolin-1-yl]-acetamid
(20) N-{4-[(4-Chlor-phenyl)-dimethylamino-methyl]-cyclohexylmethyl}-2-[2-(4-methoxy-benzolsulfonyl)-1,2,3,4-tetrahydro-isochinolin-1-yl]-acetamid
(21) N-[4-(Dimethylamino-thiophen-2-yl-methyl)-cyclohexylmethyl]-2-[2-(4-methoxybenzolsulfonyl)-1,2,3,4-tetrahydro-isochinolin-1-yl]-acetamid
(22) N-(2-{4-[(4-Chlor-phenyl)-dimethylamino-methyl]-cyclohexyl}-ethyl)-2-[2-(4-methoxy-benzolsulfonyl)-1,2,3,4-tetrahydro-isochinolin-1-yl]-acetamid
(23) N-(2-{4-[Dimethylamino-(3-fluor-phenyl)-methyl]-cyclohexyl}-ethyl)-2-[2-(4-methoxy-benzolsulfonyl)-1,2,3,4-tetrahydro-isochinolin-1-yl]-acetamid
(24) N-(2-{4-[Dimethylamino-(4-fluor-phenyl)-methyl]-cyclohexyl}-ethyl)-2-[2-(4-methoxy-benzolsulfonyl)-1,2,3,4-tetrahydro-isochinolin-1-yl]-acetamid
(25) N-{2-[4-(Dimethylamino-thiophen-2-yl-methyl)-cyclohexyl]-ethyl}-2-[2-(4-methoxy-benzolsulfonyl)-1,2,3,4-tetrahydro-isochinolin-1-yl]-acetamid
(26) 2-(2-(3,4-Dichlorphenylsulfonyl)-1,2,3,4-tetrahydroisochinolin-1-yl)-N-(4-((dimethylamino)(3-fluorphenyl)methyl)cyclohexyl)acetamid
(27) 2-(2-(3,4-Dichlorphenylsulfonyl)-1,2,3,4-tetrahydroisochinolin-1-yl)-N-(4-((dimethylamino)(4-fluorphenyl)methyl)cyclohexyl)acetamid
(28) 2-[2-(3,4-Dichlor-benzolsulfonyl)-1,2,3,4-tetrahydro-isochinolin-1-yl]-N-{4-[dimethylamino-(3-fluor-phenyl)-methyl]-cyclohexylmethyl}-acetamid
(29) 2-[2-(3,4-Dichlor-benzolsulfonyl)-1,2,3,4-tetrahydro-isochinolin-1-yl]-N-[4-(dimethylamino-phenyl-methyl)-cyclohexylmethyl]-acetamid
(30) 2-[2-(3,4-Dichlor-benzolsulfonyl)-1,2,3,4-tetrahydro-isochinolin-1-yl]-N-[4-(1-dimethylamino-3-phenyl-propyl)-cyclohexylmethyl]-acetamid
(31) 2-[2-(3,4-Dichlor-benzolsulfonyl)-1,2,3,4-tetrahydro-isochinolin-1-yl]-N-{4-[dimethylamino-(4-fluor-phenyl)-methyl]-cyclohexylmethyl}-acetamid
(32) N-{4-[(4-Chlor-phenyl)-dimethylamino-methyl]-cyclohexylmethyl}-2-[2-(3,4-dichlor-benzolsulfonyl)-1,2,3,4-tetrahydro-isochinolin-1-yl]-acetamid
(33) N-(2-{4-[(4-Chlor-phenyl)-dimethylamino-methyl]-cyclohexyl}-ethyl)-2-[2-(3,4-dichlor-benzolsulfonyl)-1,2,3,4-tetrahydro-isochinolin-1-yl]-acetamid
(34) 2-[2-(3,4-Dichlor-benzolsulfonyl)-1,2,3,4-tetrahydro-isochinolin-1-yl]-N-(2-{4-[dimethylamino-(3-fluor-phenyl)-methyl]-cyclohexyl}-ethyl)-acetamid
(35) 2-[2-(3,4-Dichlor-benzolsulfonyl)-1,2,3,4-tetrahydro-isochinolin-1-yl]-N-(2-{4-[dimethylamino-(4-fluor-phenyl)-methyl]-cyclohexyl}-ethyl)-acetamid
(36) 2-[(3,4-Dichlor-benzolsulfonyl)-methyl-amino]-N-{4-[dimethylamino-(3-fluorphenyl)-methyl]-cyclohexyl}-benzamid
(37) 2-[(3,4-Dichlor-benzolsulfonyl)-methyl-amino]-N-{4-[dimethylamino-(4-fluorphenyl)-methyl]-cyclohexyl}-benzamid
(38) 2-[(3,4-Dichlor-benzolsulfonyl)-methyl-amino]-N-[4-(dimethylamino-phenylmethyl)-cyclohexylmethyl]-benzamid
(39) 2-[(3,4-Dichlor-benzolsulfonyl)-methyl-amino]-N-[4-(1-dimethylamino-3-phenylpropyl)-cyclohexylmethyl]-benzamid
(40) 2-[(3,4-Dichlor-benzolsulfonyl)-methyl-amino]-N-{4-[dimethylamino-(4-fluorphenyl)-methyl]-cyclohexylmethyl}-benzamid
(41) N-{4-[(4-Chlor-phenyl)-dimethylamino-methyl]-cyclohexylmethyl}-2-[(3,4-dichlor-benzolsulfonyl)-methyl-amino]-benzamid
(42) 2-[(3,4-Dichlor-benzolsulfonyl)-methyl-amino]-N-[4-(dimethylamino-thiophen-2-yl-methyl)-cyclohexylmethyl]-benzamid
(43) N-(2-{4-[(4-Chlor-phenyl)-dimethylamino-methyl]-cyclohexyl}-ethyl)-2-[(3,4-dichlor-benzolsulfonyl)-methyl-amino]-benzamid
(44) 2-[(3,4-Dichlor-benzolsulfonyl)-methyl-amino]-N-(2-{4-[dimethylamino-(3-fluorphenyl)-methyl]-cyclohexyl}-ethyl)-benzamid
(45) 2-[(3,4-Dichlor-benzolsulfonyl)-methyl-amino]-N-(2-{4-[dimethylamino-(4-fluorphenyl)-methyl]-cyclohexyl}-ethyl)-benzamid
(46) 2-[(3,4-Dichlor-benzolsulfonyl)-methyl-amino]-4,5,6,7-tetrahydrobenzo[b]thiophen-3-carbonsäure-[4-(dimethylamino-phenyl-methyl)-cyclohexylmethyl]-amid
(47) 2-[(3,4-Dichlor-benzolsulfonyl)-methyl-amino]-4,5,6,7-tetrahydrobenzo[b]thiophen-3-carbonsäure-{4-[dimethylamino-(4-fluor-phenyl)-methyl]-cyclohexylmethyl}-amid
(48) 2-[(3,4-Dichlor-benzolsulfonyl)-methyl-amino]-4,5,6,7-tetrahydrobenzo[b]thiophen-3-carbonsäure-{4-[(4-chlor-phenyl)-dimethylamino-methyl]-cyclohexylmethyl}-amid
(49) 2-[(3,4-Dichlor-benzolsulfonyl)-methyl-amino]-4,5,6,7-tetrahydrobenzo[b]thiophen-3-carbonsäure-[4-(dimethylamino-thiophen-2-yl-methyl)-cyclohexylmethyl]-amid
(50) N-(2-(4-((4-chlorphenyl)(dimethylamino)methyl)cyclohexyl)ethyl)-2-(3,4-dichlor-N-methylphenylsulfonamido)-4,5,6,7-tetrahydrobenzo[b]thiophen-3-carboxamid
(51) 2-(3,4-Dichlor-N-methylphenylsulfonamido)-N-(2-(4-((dimethylamino)(3-fluorphenyl)methyl)cyclohexyl)ethyl)-4,5,6,7-tetrahydrobenzo[b]thiophen-3-carboxamid
(52) 2-(3,4-Dichlor-N-methylphenylsulfonamido)-N-(2-(4-((dimethylamino)(4-fluorphenyl)methyl)cyclohexyl)ethyl)-4,5,6,7-tetrahydrobenzo[b]thiophen-3-carboxamid
(53) 2-(3,4-Dichlor-N-methylphenylsulfonamido)-N-(2-(4-((dimethylamino)(thiophen-2-yl)methyl)cyclohexyl)ethyl)-4,5,6,7-tetrahydrobenzo[b]thiophen-3-carboxamid
(54) 2-[1-(3,4-Dichlor-benzolsulfonyl)-3-oxo-1,2,3,4-tetrahydro-chinoxalin-2-yl]-N-[4-(morpholin-4-yl-phenyl-methyl)-cyclohexyl]-acetamid
(55) 2-[1-(2,4-Dichlor-benzolsulfonyl)-3-oxo-piperazin-2-yl]-N-[4-(morpholin-4-yl-phenyl-methyl)-cyclohexylmethyl]-acetamid
(56) 2-[1-(2,4-Dichlor-benzolsulfonyl)-3-oxo-piperazin-2-yl]-N-[4-(1-morpholin-4-yl-3-phenyl-propyl)-cyclohexylmethyl]-acetamid
(57) 2-[1-(2,4-Dichlor-benzolsulfonyl)-3-oxo-piperazin-2-yl]-N-[4-(3-phenyl-1-pyrrolidin-1-yl-propyl)-cyclohexylmethyl]-acetamid
(58) 2-[1-(2,4-Dichlor-benzolsulfonyl)-3-oxo-piperazin-2-yl]-N-[4-(phenyl-pyrrolidin-1-yl-methyl)-cyclohexylmethyl]-acetamid
(59) 2-[1-(2,4-Dichlor-benzolsulfonyl)-3-oxo-piperazin-2-yl]-N-[4-(3-phenyl-1-piperidin-1-yl-propyl)-cyclohexyl]-acetamid
(60) 2-[1-(2,4-Dichlor-benzolsulfonyl)-3-oxo-piperazin-2-yl]-N-[4-(1-morpholin-4-yl-3-phenyl-propyl)-cyclohexyl]-acetamid
(61) 2-[1-(2,4-Dichlor-benzolsulfonyl)-3-oxo-piperazin-2-yl]-N-{4-[(4-methylpiperazin-1-yl)-phenyl-methyl]-cyclohexyl}-acetamid
(62) 4-(2,4-Dichlor-benzolsulfonyl)-3-{2-[4-(morpholin-4-yl-phenyl-methyl)-piperidin-1-yl]-2-oxo-ethyl}-piperazin-2-on
(63) 4-(2,4-dichlorphenylsulfonyl)-3-(2-(2-(1-morpholino-2-phenylethyl)piperidin-1-yl)-2-oxoethyl)piperazin-2-on
(64) 2-[1-(3,4-Dichlor-benzolsulfonyl)-3-oxo-1,2,3,4-tetrahydro-chinoxalin-2-yl]-N-[4-(morpholin-4-yl-phenyl-methyl)-cyclohexylmethyl]-acetamid
(65) 2-[1-(3,4-Dichlor-benzolsulfonyl)-3-oxo-1,2,3,4-tetrahydro-chinoxalin-2-yl]-N-[4-(1-morpholin-4-yl-3-phenyl-propyl)-cyclohexylmethyl]-acetamid
(66) 2-[1-(3,4-Dichlor-benzolsulfonyl)-3-oxo-1,2,3,4-tetrahydro-chinoxalin-2-yl]-N-[4-(3-phenyl-1-pyrrolidin-1-yl-propyl)-cyclohexylmethyl]-acetamid
(67) 2-[1-(3,4-Dichlor-benzolsulfonyl)-3-oxo-1,2,3,4-tetrahydro-chinoxalin-2-yl]-N-[4-(phenyl-pyrrolidin-1-yl-methyl)-cyclohexylmethyl]-acetamid
(68) 2-[1-(3,4-Dichlor-benzolsulfonyl)-3-oxo-1,2,3,4-tetrahydro-chinoxalin-2-yl]-N-[4-(3-phenyl-1-piperidin-1-yl-propyl)-cyclohexyl]-acetamid
(69) 2-[1-(3,4-Dichlor-benzolsulfonyl)-3-oxo-1,2,3,4-tetrahydro-chinoxalin-2-yl]-N-[4-(1-morpholin-4-yl-3-phenyl-propyl)-cyclohexyl]-acetamid
(70) 2-[1-(3,4-Dichlor-benzolsulfonyl)-3-oxo-1,2,3,4-tetrahydro-chinoxalin-2-yl]-N-{4-[(4-methyl-piperazin-1-yl)-phenyl-methyl]-cyclohexyl}-acetamid
(71) 4-(3,4-Dichlor-benzolsulfonyl)-3-{2-[4-(morpholin-4-yl-phenyl-methyl)-piperidin-1-yl]-2-oxo-ethyl}-3,4-dihydro-1H-chinoxalin-2-on
(72) 4-(3,4-dichlorphenylsulfonyl)-3-(2-(4-(1-morpholino-2-phenylethyl)piperidin-1-yl)-2-oxoethyl)-3,4-dihydrochinoxalin-2(1H)-on
(73) 2-[1-(3,4-Dichlor-benzolsulfonyl)-pyrrolidin-2-yl]-N-[4-(morpholin-4-yl-phenylmethyl)-cyclohexylmethyl]-acetamid
(74) 2-[1-(3,4-Dichlor-benzolsulfonyl)-pyrrolidin-2-yl]-N-[4-(1-morpholin-4-yl-3-phenyl-propyl)-cyclohexylmethyl]-acetamid
(75) 2-[1-(3,4-Dichlor-benzolsulfonyl)-pyrrolidin-2-yl]-N-[4-(3-phenyl-1-pyrrolidin-1-yl-propyl)-cyclohexylmethyl]-acetamid
(76) 2-[1-(3,4-Dichlor-benzolsulfonyl)-pyrrolidin-2-yl]-N-[4-(phenyl-pyrrolidin-1-yl-methyl)-cyclohexylmethyl]-acetamid
(77) 2-(1-(3,4-dichlorphenylsulfonyl)pyrrolidin-2-yl)-N-(4-(3-phenyl-1-(piperidin-1-yl)propyl)cyclohexyl)acetamid
(78) 2-(1-(3,4-dichlorphenylsulfonyl)pyrrolidin-2-yl)-N-(4-(1-morpholino-3-phenylpropyl)cyclohexyl)acetamid
(79) 2-[1-(3,4-Dichlor-benzolsulfonyl)-pyrrolidin-2-yl]-N-{4-[(4-methyl-piperazin-1-yl)-phenyl-methyl]-cyclohexyl}-acetamid
(80) 2-[1-(3,4-Dichlor-benzolsulfonyl)-pyrrolidin-2-yl]-1-[4-(morpholin-4-yl-phenylmethyl)-piperidin-1-yl]-ethanon
(81) 2-(1-(3,4-dichlorphenylsulfonyl)pyrrolidin-2-yl)-1-(4-(1-morpholino-2-phenylethyl)piperidin-1-yl)ethanon
(82) 2-[1-(3,4-Dichlor-benzolsulfonyl)-piperidin-2-yl]-N-[4-(morpholin-4-yl-phenylmethyl)-cyclohexylmethyl]-acetamid
(83) 2-[1-(3,4-Dichlor-benzolsulfonyl)-piperidin-2-yl]-N-[4-(1-morpholin-4-yl-3-phenyl-propyl)-cyclohexylmethyl]-acetamid
(84) 2-[1-(3,4-Dichlor-benzolsulfonyl)-piperidin-2-yl]-N-[4-(3-phenyl-1-pyrrolidin-1-yl-propyl)-cyclohexylmethyl]-acetamid
(85) 2-[1-(3,4-Dichlor-benzolsulfonyl)-piperidin-2-yl]-N-[4-(phenyl-pyrrolidin-1-yl-methyl)-cyclohexylmethyl]-acetamid
(86) 2-[1-(3,4-Dichlor-benzolsulfonyl)-piperidin-2-yl]-N-[4-(3-phenyl-1-piperidin-1-yl-propyl)-cyclohexyl]-acetamid
(87) 2-[1-(3,4-Dichlor-benzolsulfonyl)-piperidin-2-yl]-N-[4-(1-morpholin-4-yl-3-phenyl-propyl)-cyclohexyl]-acetamid
(88) 2-[1-(3,4-Dichlor-benzolsulfonyl)-piperidin-2-yl]-N-{4-[(4-methyl-piperazin-1-yl)-phenyl-methyl]-cyclohexyl}-acetamid
(89) 2-[1-(3,4-Dichlor-benzolsulfonyl)-piperidin-2-yl]-1-[4-(morpholin-4-yl-phenylmethyl)-piperidin-1-yl]-ethanon
(90) 2-(1-(3,4-Dichlorphenylsulfonyl)piperidin-2-yl)-1-(4-(1-morpholino-2-phenylethyl)piperidin-1-yl)ethanon
(91) 1-(3,4-Dichlorphenylsulfonyl)-N-(4-((dimethylamino)(phenyl)methyl)cyclohexyl) indolin-2-carboxamid
(92) 1-(3,4-Dichlorphenylsulfonyl)-N-(4-((dimethylamino)(3-fluorphenyl)methyl) cyclohexyl)indolin-2-carboxamid
(93) 1-(3,4-Dichlorphenylsulfonyl)-N-(4-(1-(dimethylamino)-3-phenylpropyl) cyclohexyl)indolin-2-carboxamid
(94) 1-(3,4-Dichlorphenylsulfonyl)-N-(4-((dimethylamino)(4-fluorphenyl)methyl)cyclohexyl)indolin-2-carboxamid
(95) 1-(3,4-Dichlorphenylsulfonyl)-N-((4-((dimethylamino)(phenyl) methyl)cyclohexyl)methyl)indolin-2-carboxamid
(96) 1-(3,4-Dichlorphenylsulfonyl)-N-((4-(1-(dimethylamino)-3-phenylpropyl)cyclohexyl)methyl)indolin-2-carboxamid
(97) 1-(3,4-Dichlorphenylsulfonyl)-N-(2-(4-(1-(dimethylamino)-3-phenylpropyl)cyclohexyl)ethyl)indolin-2-carboxamid
(98) 2-(2-(3,4-Dichlor-N-methylphenylsulfonamido)phenyl)-N-(2-(4-((dimethylamino)(phenyl)methyl)cyclohexyl)ethyl)acetamid
(99) 2-(2-(3,4-Dichlor-N-methylphenylsulfonamido)phenyl)-N-(2-(4-(1-(dimethylamino)-3-phenylpropyl)cyclohexyl)ethyl)acetamid
(100) N-(2-(4-((Dimethylamino)(phenyl)methyl)cyclohexyl)ethyl)-1-(4-methoxy-N-methylphenylsulfonamido)cyclohexancarboxamid
(101) 2-(1-(2,4-Dichlorphenylsulfonyl)-3-oxopiperazin-2-yl)-N-(4-(morpholino(phenyl)methyl)cyclohexyl)acetamid
(102) 2-(1-(2,4-Dichlorphenylsulfonyl)-3-oxopiperazin-2-yl)-N-(4-(phenyl(pyrrolidin-1-yl)methyl)cyclohexyl)acetamid
(103) 2-(1-(3,4-Dichlorphenylsulfonyl)-3-oxo-1,2,3,4-tetrahydrochinoxalin-2-yl)-N-(4-(phenyl(pyrrolidin-1-yl)methyl)cyclohexyl)acetamid
(104) 2-(1-(3,4-Dichlorphenylsulfonyl)pyrrolidin-2-yl)-N-(4-(morpholino(phenyl)methyl)cyclohexyl)acetamid
(105) 2-(1-(3,4-Dichlorphenylsulfonyl)pyrrolidin-2-yl)-N-(4-(phenyl(pyrrolidin-1-yl)methyl)cyclohexyl)acetamid
(106) 2-(1-(3,4-Dichlorphenylsulfonyl)piperidin-2-yl)-N-(4-(morpholino(phenyl)methyl)cyclohexyl)acetamid
(107) 2-(1-(3,4-Dichlorphenylsulfonyl)piperidin-2-yl)-N-(4-(phenyl(pyrrolidin-1-yl)methyl)cyclohexyl)acetamid
(108) 1-(4-((4-Methylpiperazin-1-yl)(phenyl)methyl)piperidin-1-yl)-2-(1-(3-(trifluormethyl)phenylsulfonyl)piperidin-2-yl)ethanon
(109) 1-(4-((4-Fluorphenyl)(4-methylpiperazin-1-yl)methyl)piperidin-1-yl)-2-(1-(3-(trifluormethyl)phenylsulfonyl)piperidin-2-yl)ethanon
(110) N-(3-(4-((3-Fluorphenyl)(4-methylpiperazin-1-yl)methyl)piperidin-1-yl)-3-oxo-1-phenylpropyl)naphthalin-2-sulfonamid
(111) 1-(4-(1-(4-Methylpiperazin-1-yl)-2-phenylethyl)piperidin-1-yl)-2-(1-(3-(trifluormethyl)phenylsulfonyl)piperidin-2-yl)ethanon
(112) 1-(4-(1-(4-Methylpiperazin-1-yl)-3-phenylpropyl)piperidin-1-yl)-2-(1-(3-(trifluormethyl)phenylsulfonyl)piperidin-2-yl)ethanon
(113) N-(3-(4-(1-(4-Methylpiperazin-1-yl)-3-phenylpropyl)piperidin-1-yl)-3-oxo-1-phenylpropyl)naphthalene-2-sulfonamid
(114) N-(3-(4-((4-Methylpiperazin-1-yl)(phenyl)methyl)piperidin-1-yl)-3-oxo-1-phenylpropyl)naphthalin-2-sulfonamid
(115) 1-(4-((3-Fluorphenyl)(4-methylpiperazin-1-yl)methyl)piperidin-1-yl)-2-(1-(3-(trifluormethyl)phenylsulfonyl)piperidin-2-yl)ethanon
(116) N-(3-(4-(1-(4-Methylpiperazin-1-yl)-2-phenylethyl)piperidin-1-yl)-3-oxo-1-phenylpropyl)naphthalin-2-sulfonamid
(117) N-(3-(4-((4-Fluorphenyl)(4-methylpiperazin-1-yl)methyl)piperidin-1-yl)-3-oxo-1-phenylpropyl)naphthalen-2-sulfonamid
(118) 3-(1-(4-Chlor-2,5-dimethylphenylsulfonyl)piperidin-2-yl)-1-(4-(1-(4-methylpiperazin-1-yl)-2-phenylethyl)piperidin-1-yl)propan-1-on
(119) 3-(1-(4-Chlor-2,5-dimethylphenylsulfonyl)piperidin-2-yl)-1-(4-(1-(4-methylpiperazin-1-yl)-3-phenylpropyl)piperidin-1-yl)propan-1-on
(120) 3-(1-(4-Chlor-2,5-dimethylphenylsulfonyl)piperidin-2-yl)-1-(4-((3-fluorphenyl)(4-methylpiperazin-1-yl)methyl)piperidin-1-yl)propan-1-on
(121) 3-(1-(4-Chlor-2,5-dimethylphenylsulfonyl)piperidin-2-yl)-1-(4-((4-fluorphenyl)(4-methylpiperazin-1-yl)methyl)piperidin-1-yl)propan-1-on
(122) 3-(1-(4-Chlor-2,5-dimethylphenylsulfonyl)piperidin-2-yl)-1-(4-((4-methylpiperazin-1-yl)(phenyl)methyl)piperidin-1-yl)propan-1-on
in Form des Razemats; der Enantiomere, Diastereomere, Mischungen der Enantiomere oder Diastereomere oder eines einzelnen Enantiomers oder Diastereomers; der Basen und/oder Salze physiologisch verträglicher Säuren.

Ein weiterer Gegenstand der Erfindung ist ein Verfahren zur Herstellung eines erfindungsgemäßen substituierten Sulfonamid-Derivates.

Zur Herstellung der Verbindungen der allgemeinen Formel **Ia**, worin q 0 bedeutet, werden Amine der allgemeinen Formel **II** mit Säuren der allgemeinen Formel **III** unter Zugabe eines Kupplungsreagenzes umgesetzt.

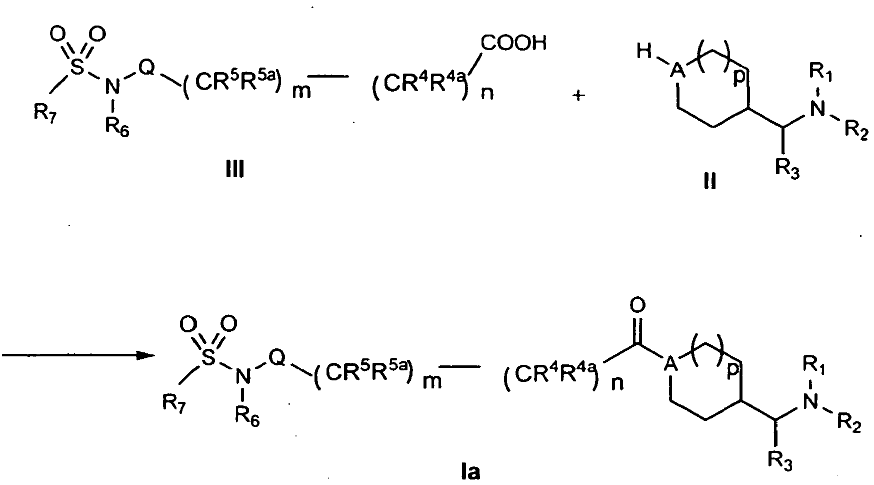

Dabei werden die Carbonsäuren **III** in einer Amidbildung unter Verwendung primärer oder sekundärer Amine der allgemeinen Formel **II** in Gegenwart wasserentziehender Mittel wie Natrium- oder Magnesiumsulfat, Phosphoroxid oder Reagenzien wie beispielsweise CDI, DCC (ggf. polymergebunden), TBTU, EDCI, PyBOP oder PFPTFA auch in Gegenwart von HOAt oder HOBt und einer organischen Base beispielsweise DIPEA oder Pyridin in einem organischen Lösungsmittel wie THF, Dichlormethan, Diethylether, Dioxan, DMF oder Acetonitril zu den Produkten der allgemeinen Formel **I** umgesetzt.

Zur Herstellung der Verbindungen der allgemeinen Formel **Ib**, worin q 1 bedeutet, werden Amine der allgemeinen Formel **II** mit Isocyanaten oder Carbamaten der Formel IV umgesetzt.

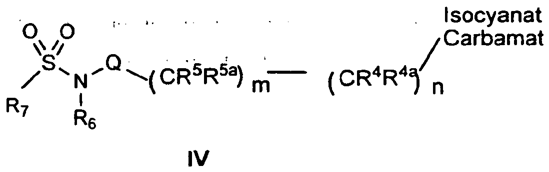

Dabei werden Carbamate mit Aminen der allgemeinen Formel II in einem organischen Lösungsmittel, beispielsweise 1,4-Dioxan, zu den Verbindungen der allgemeinen Formel Ib umgesetzt. Alternativ werden die Amine der allgemeinen Formel II in einem organischen Lösungsmittel mit Isocyanaten der allgemeinen Formel IV, ggf. in Gegenwart wenigstens einer Base, bevorzugt in Gegenwart wenigstens einer Base ausgewählt aus der Gruppe bestehend aus Triethylamin, 4,4-Dimethylaminopyridin und Diisopropylethylamin, zu den Verbindungen der allgemeinen Formel Ib umgesetzt und diese ggf. gereinigt und/oder isoliert wird.

Die verwendeten Aminbausteine der allgemeinen Formel II können beispielsweise nach folgenden Verfahren hergestellt werden. Nach dem im Folgenden Verfahren A bezeichneten Verfahren können Aminbausteine hergestellt werden, worin im Baustein der allgemeinen Formel II A für CHNH₂ oder (CH₂)ₙNH₂ steht.

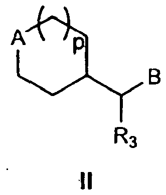

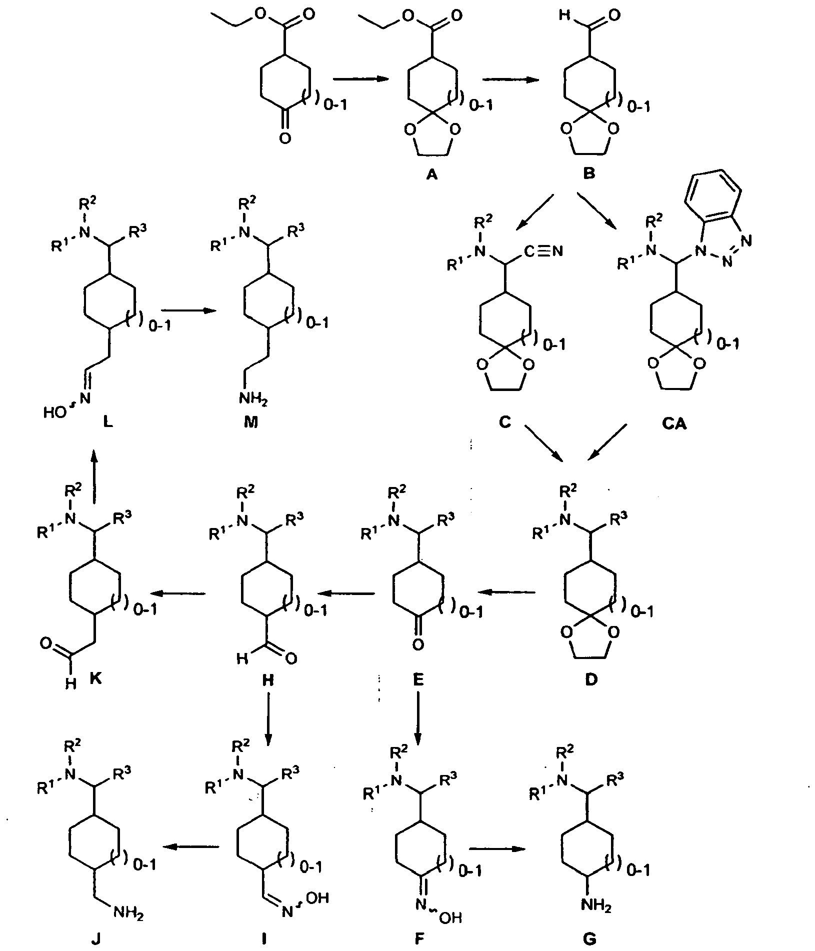

Dabei wird Ethyl 4-oxocyclohexancarboxylat oder Cyclopentanon-3-carbonsäureethylester in einer Acetalbildungsreaktion mit einem Diolderivat in einem organischen Lösungsmittel, wie Benzol, Toluol oder Xylol, Dichlormethan, Cyclohexan oder Ethanol, eventuell unter Zusatz katalytischer Mengen p-Toluolsulfonsäure, Camphersulfonsäure, Pyridiniumtosylat, oder Essigsäure möglicherweise auch in Gegenwart eines wasserentziehenden Reagenzes, wie Schwefelsäure, Natrium- oder Magnesiumsulfat, Molsieb oder Phosphorxiden, bei einer Temperatur von RT bis Rückflußtemperatur des jeweiligen organischen Lösungsmittels zu dem Acetal A umgesetzt.

Die vorherige Veresterung der Cyclopentanon-3-carbonsäure zum Cyclopentanon-3-carbonsäureethylester kann durch Umsetzung der Cyclopentanon-3-carbonsäure mit Ethanol unter Verwendung von Schwefelsäure oder Salzsäure oder durch Umsetzung der Cyclopentanon-3-carbonsäure mit Iodethan unter Verwendung von Natriumethanolat oder Cäsiumcarbonat in DMF erfolgen.

Das Acetal **A** kann in einer Reduktionsreaktion mit einem Reduktionsmittel, beispielsweise Diisobutylaluminiumhydrid, Natriumaluminiumhydrid oder Boran-THF-Komplex in Diethylether, Dichlormethan, THF, Hexan, Toluol oder einer Mischung aus den genannten Lösungsmitteln bei einer Temperatur von -95°C bis -20°C zum Aldehyd B reduziert werden.

Durch Zugabe eines Amins und einer Cyanidquelle kann der Aldehyd **B** zum Nitril **C** umgesetzt werden. Die Umsetzung kann ein - oder zweistufig erfolgen. Bei der zweistufigen Variante wird zunächst ein Nitrilalkohol gebildet und isoliert. Die Bildung des Nitrilalkohols kann durch Umsetzung des Aldehyds **B** mit HCN, KCN oder NaCN erfolgen. Geeignete Lösungsmittel sind Wasser, Methanol, Ethanol, THF, Piperidin, Diethylether oder eine Mischung dieser Lösungsmittel. Typischerweise wird bei der Verwendung von NaCN und KCN das erforderliche Cyanid durch die Zugabe von beispielsweise Natriumhydrogensulfit, Schwefelsäure, Essigsäure oder Salzsäure freigesetzt. Als Nitrilquelle ist ebenfalls beispielsweise Trimethylsilylcyanid geeignet. Die Freisetzung des Cyanids kann hierbei beispielsweise durch Bortrifluorid-Etherat, InF₃ oder HCl erfolgen. Typische Lösungsmittel sind hier Wasser oder Toluol. Als weitere Cyanidquelle ist beispielsweise (Cyano-C)diethylaluminium geeignet. Als Lösungsmittel kann THF, Toluol oder eine Mischung beider Lösungsmittel verwendet werden.

Die Reaktionstemperatur kann für alle Varianten zwischen - 78°C und +25°C liegen. Für die Umsetzung des Nitrilalkohols mit dem Amin eignen sich besonders Alkohole, wie Methanol oder Ethanol als Lösungsmittel. Die Reaktionstemperatur kann zwischen 0°C und +25°C liegen. Bei der einstufigen Variante wird der primär gebildete Nitrilalkohol *in situ* gebildet und mit dem Amin umgesetzt. In einer Variante der Reaktionsführung wird der Aldehyd **B** in einer Aminalbildungsreaktion mit einem Amin und 1H-Benzotriazol zum Benzotriazol-Aminal **CA** umgesetzt. Das Bezotriazolaminal kann im Gleichgewicht sowohl in der 1H- als auch in der 2H-Form vorliegen.

Als Lösungsmittel sind Benzol, Toluol, Ethanol, Diethylether oder THF geeignet. Eventuell ist die Verwendung eines Wasserabscheiders nach Dean-Stark, Molekularsieb oder andere wasserentziehende Mittel notwendig. Die Reaktionszeit liegt normalerweise zwischen 1 und 20 h bei einer Reaktionstemperatur von +20°C bis +110°C. Sowohl das Nitril **C** als auch das Benzotriazolaminal **CA** kann mit Metallorganylen, wie Magnesium-, Zink oder Lithiumorganyle in organischen Lösungsmitteln, beispielsweise Diethylether, Dioxan oder Tetrahydrofuran, zu Aminoacetalen **D** umgesetzt werden.

Die Aminketone **E** erhält man in einer Acetalspaltungsreaktion unter sauren Bedingungen. Als Säuren eignen sich sowohl anorganische Broenstedt oder LewisSäuren, wie Salzsäure, Schwefelsäure Ammoniumchlorid oder Hydrogensulfat , oder All₃, als auch organische Säuren, wie z. B. p-Toluolsulfonsäure, Essigsäure, Oxalsäure, Trifluormethansulfonsäure, Ameisensäure, Trifluoressigsäure oder Citronensäure. Die Reaktion kann in verschiedenen Lösungsmitteln, wie etwa Toluol, THF, Chlorform, DCM, Xylol, Acetonitril, Wasser, Dioxan, Aceton, Diethylether oder Ethylacetat bei Temperaturen von -10°C bis Raumtemperatur durchgeführt werden. Der Aldehyd **H** wird in einer Wittig-Reaktion unter Verwendung von Phosphoryliden und einer starken Base, beispielsweise Kalium-tert-butylat, n-Butyllithium, s-Buthyllithium, Phenyllithium, Lithiumdiisopropylamid oder Lithium-hexamethyldisilazid in organischen Lösungsmitteln, wie THF, Diethylether, Cyclohexan, Toluol oder einer Mischung der Lösungsmittel bei einher Temperatur von -78°C und +30°C, nach saurer Aufarbeitung der Reaktionsmischung aus dem Aminketon **E** erhalten.

Zur Synthese des Aldehyds **K** wird die Wittigreaktion unter gleichen Bedingungen mit dem Aldehyd **H** als Ausgangsverbindung wiederholt. Zur Synthese von Bausteinen, in denen A für (CH₂)ₙ NH₂ mit n > 1 steht, wird der Schritt n-mal wiederholt.

Das Keton **E** wird in einer Oximbildungsreaktion mit Hydroxylaminhydrochlorid,-Sulfat oder -Acetat in einem organischen Lösungsmittel, beispielsweise Ethanol, Methanol, 2-Propanol, 2-Methyl-propan-2-ol oder Acetonitril, unter Zugabe einer organischen Base, wie beispielsweise Pyridin, Natriumacetat, Triethylamin, 4-Dimethylaminopyridin oder Kalium-t-butylat oder einer wässrigen Lösung einer anorganischen Base, wie Natriumhydrogencarbonat, Natriumcarbonat, Kaliumcarbonat, Natriumhydroxid oder Kaliumhydroxid oder basischen Ionenaustauscher Amberlyst zu den Oximen **F** umgesetzt.

Unter den gleichen Bedingungen können ebenfalls die Aldehyde **H** und **K** zu den Oximen **I** bzw. **L** umgesetzt werden. Durch eine Reduktionsreaktion der Oxime **F** mit einem Reduktionsmittel, wie beispielsweise LiAlH₄, Natrium, Zink, Boran-Dimethylsulfid, Natriumborhydrid / Nickel(II)chlorid-Hexahydrat, in Ethanol, Methanol, Eisessig, THF, Diethylether oder Dioxan oder durch katalytische Hydrierung mit Palladium oder Platinoxid als heterogenem Katalysator unter Zugabe von HCl in einem Alkohol, wie Methanol oder Ethanol, können die Amine **G** erhalten werden. Unter den gleichen Bedingungen können aus den Oximen **I** bzw. **L** in die Amine **J** bzw. **M** hergestellt werden.

Aminbausteine der allgemeinen Formel **II**, worin A NH bedeutet, können nach folgendem Verfahren hergestellt werden: 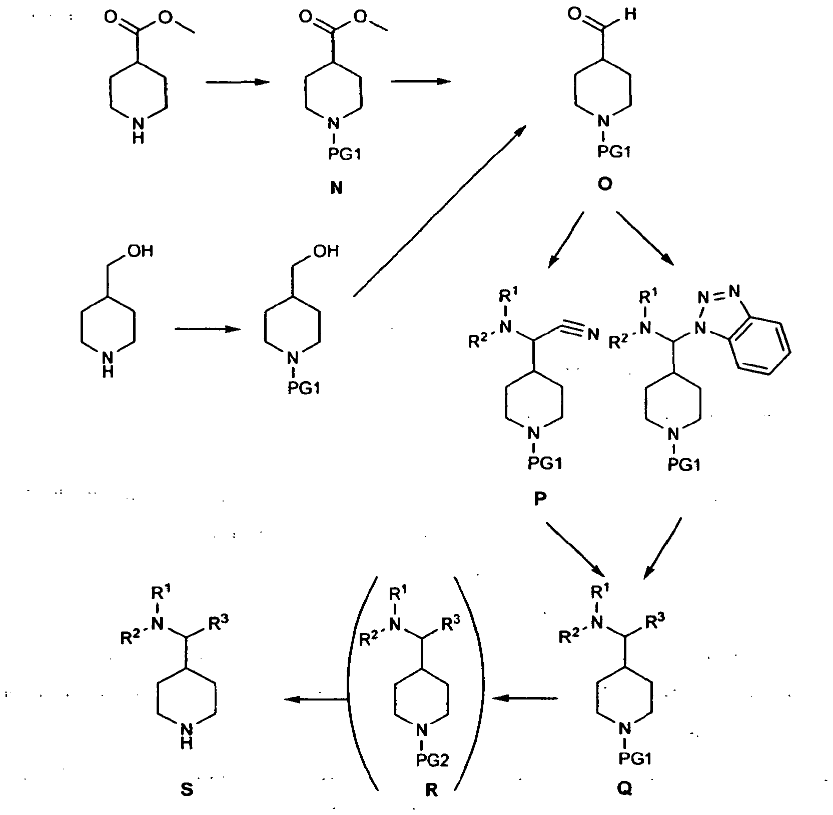 PG = Schutzgruppe

Isonipecotinsäuremethylester oder Piperidin-4-ylmethanol wird einer Alkylierungsreaktion unterzogen. Als Alkylierungsreagenz eignen sich insbesondere p-Methoxybenzylhalogenide. Die Umsetzung kann durch Reaktion des p-Methoxybenzylchlorids oder -bromids in THF, Benzol, Toluol, Dimethylformamid, Acetonitril, Dichlormethan, Ethanol oder Aceton unter Verwendung einer Base, wie beispielsweise Triethylamin, Diisopropylethylamin, Kaliumcarbonat oder Natriumcarbonat bei einer Temperatur von +20°C bis +80°C in 1-72 h erfolgen.

Als Alternative kann als Schutzgruppe auch durch Umsetzung mit Di-tert-Butyldicarbonat in einem organischen Lösungsmittel wie THF, Dichlormethan, Methanol, Dioxan, DMF, oder Diethylether eventuell unter Verwendung einer anorganischen Base, wie Natriumcarbonat, Natriumhydrogencarbonat, oder Natriumhydroxid oder einer organischen Base, wie Triethylamin, Diisopropylethylamin, oder n-Buthyllithium, bei einer Temperatur zwischen -78°C und Raumtemperatur eine BOC-Gruppe eingeführt werden.

Die Syntheseschritte zur Reduktion des Esters **N** zum Aldehyd **O**, Umsetzung des Aldehyds **O** zum Aminonitril **P** und Umsetzung mit einem Metallorganyl zum geschützten Amin **Q** erfolgt analog den Syntheseschritten, wie sie für die Verbindungen **A** → **B** → **C** → **D** beschrieben wurden.

Der geschützte Piperidin-4-ylmethanol läßt sich durch Verwendung von Reagenzien, wie PCC, Periodinan, IBX, TPAP, NMO, MnO₂ oder Oxalylchlorid, gegebenenfalls auch in Gegenwart von Molekularsieb oder einer Base, wie Triethylamin, in einem organischen Lösungsmittel wie Dichlormethan, DMSO, Methanol, Ethanol Diethylether, THF, DMF, DME, bei einer Temperatur von -78°C bis zur Rückflußtemperatur des jeweiligen organischen Lösungsmittels, zum Aldehyd **O** umsetzen.

Eine alternative Route für die Umsetzung der Verbindungen **Q** zur Verbindung **R** erfolgt analog den Syntheseschritten, wie sie für die Verbindungen **B** → **CA** → **D** beschrieben wurden.

Die Debenzylierung der Verbindungen **Q** zum Piperidinderivat **S** kann direkt mit Cerammoniumnitrat in Acetonitril bei Raumtemperatur innerhalb 0,5-2h erfolgen, oder indirekt durch Umsetzung der Verbindung **Q** mit Chlorameisensäurebenzylester in Dichlormethan bei Raumtemperatur zu den Verbindungen der allgemeinen Formel **R**.

Für die Entschützung der Verbindungen **R** sind verschiedene Methoden z.B. bei der Verwendung von Benzylcarbamat-Schutzgruppen bekannt, wie etwa die katalytische Hydrierung mit Pd oder Pd(OH)₂ als Katalysator in Lösungsmitteln wie Alkoholen, vorzugsweise Methanol oder Ethanol, THF, Dioxan, Ethylacetat, DMF oder Mischungen der genannten Lösungsmittel. Es können gegebenenfalls Hilfsreagenzien wie beispielsweise Essigsäure, Essigsäurechlorid, HCl, Ammoniumacetat, Ammoniumformat, Wasser, Kaliumcarbonat, Kaliumhydroxid, Cyclohexen oder 1,4-Cyclohexadien zugegeben werden. Ebenfalls bekannt ist die Entschützung mit Hilfe von Trimethylsilyliodid in organischen Lösungsmitteln, wie Chlorform, Dichlormethan oder Acetonitril. Weiterhin kann Methylsulfonsäure unter Zugabe von Anisol in Chlorform oder Dichlormethan verwendet werden oder auch HCl-Gas in Chlorform oder Dichlormethan bzw HBr in Eisessig.

BOC-Schutzgruppen können durch Umsetzung mit HCl in organischen Lösungsmitteln, wie Dioxan, Methanol, Ethanol, Acetonitril oder Ethylacetat oder durch Umsetzung mit Trifluoressigsäure oder Methansulfonsäure in Dichlormethan oder THF bei einer Temperatur von 0°C bis 110°C und einer Reaktionszeit von 0,5 - 20h abgespalten werden.

Die eingesetzten Säurebausteine können nach verschiedenen Methoden erhalten werden.

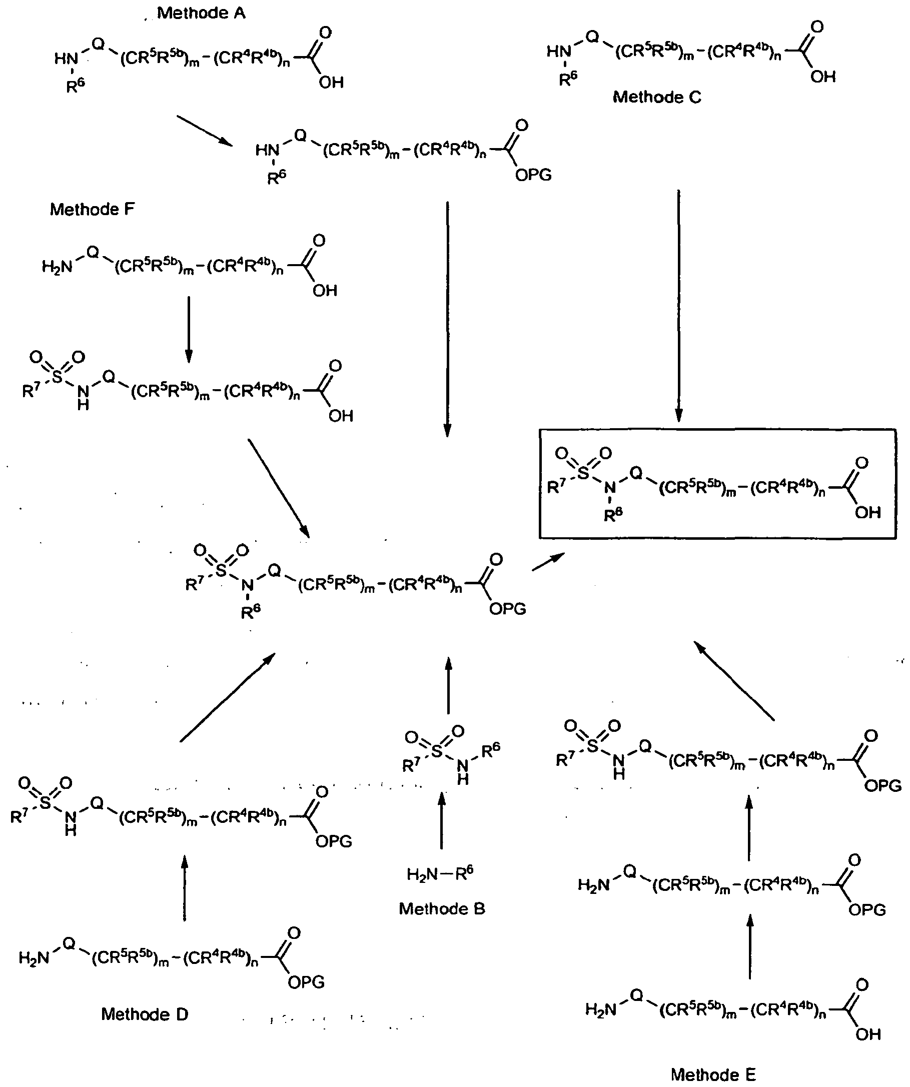

### Methode A

Die eingesetzten Aminosäuren werden unter Verwendung wasserentziehender Reagenzien beispielsweise anorganische Säuren wie H₂SO₄ oder Phosphoroxiden oder organischen Reagenzien wie Thionylchlorid, in organische Lösungsmitteln wie THF, Diethylether, Methanol, Ethanol oder DCM zu den Aminosäureestern verestert und anschließend in einer Sulfonylierung mit Sulfonylchloriden oder -bromiden oder - pentafluorphenolaten R₃SO₂X (X = Cl, Br, OPFP) ggf. in Gegenwart einer organischen oder anorganischen Base, beispielsweise Kaliumcarbonat, Natriumhydrogencarbonat, Diisopropylethylamin, Triethylamin, Pyridin, Diethylamin oder DBU, vorzugsweise in einem organischen Lösungsmittel beispielsweise Acetonitril, DCM oder THF zu den sulfonylierten Aminosäureestern umgesetzt. Die sulfonylierten Aminosäureester reagieren in einer Esterspaltung unter Verwendung von organischen Säuren, wie Trifluoressigsäure oder wässrigen anorganischen Säuren, wie Salzsäure oder Verwendung von wässrigen anorganischen Basen wie Lithiumhydroxid, Kaliumhydroxid, Natriumhydroxid, Natriumcarbonat, Natriumhydrogencarbonat, Kaliumcarbonat in organischen Lösungsmittel wie Methanol, Dioxan, DCM, THF, Diethylether oder diese Lösungsmittel als Gemische zu den sulfonylierten Aminosäuren ergeben.

### Methode B

Die eingesetzten primären Amine werden zunächst in einer Sulfonylierungsreaktion mit Sulfonylchloriden oder -bromiden oder -pentafluorphenolaten R₃SO₂X (X = Cl, Br, OPFP) ggf. in Gegenwart einer organischen oder anorganischen Base, beispielsweise Kaliumcarbonat, Natriumhydrogencarbonat, Diisopropylethylamin, Triethylamin, Pyridin, Diethylamin oder DBU, vorzugsweise in einem organischen Lösungsmittel beispielsweise Acetonitril, DCM oder THF zu den Sulfonamiden umgesetzt. Die Sulfonamide werden dann in einem zweiten Schritt durch eine Alkylierungsreaktion mit Halogenalkanen oder Alkoholen in Lösungsmitteln, wie Aceton, DMF, DCM, THF, Hexan, Toluol, Methanol oder Wasser unter Verwendung anorganischer oder organischer Basen, wie Kaliumcarbonat, n-Butyllithium, Litiumdiisopropylamid, Natriumhydrid, Natriumhydroxid, Natriummethanolat, Diisopropylethylamin und Triethylamin und gegebenflalls mit Hilfe von Reagenzien, wie Triphenylphosphin, Diisopropylazodicarboxylat, Diethylazodicarboxylat, (Cyanomethylen)trimethylphosphonat, bei einer Temperatur zwischen -78°C und +80°C zu den sulfonylierten Aminosäureestern umgesetzt.

Die sulfonylierten Aminosäureester reagieren in einer Esterspaltung unter Verwendung von organischen Säuren, wie Trifluoressigsäure oder wässrigen anorganischen Säuren, wie Salzsäure oder Verwendung von wässrigen anorganischen Basen wie Lithiumhydroxid, Kaliumhydroxid, Natriumhydroxid, Natriumcarbonat, Natriumhydrogencarbonat, Kaliumcarbonat in organischen Lösungsmittel wie Methanol, Dioxan, DCM, THF, Diethylether oder diese Lösungsmittel als Gemische zu den sulfonylierten Aminosäuren ergeben.

### Methode C

Die eingesetzten Aminosäuren werden in einer Sulfonylierungsreaktion mit Sulfonylchloriden oder -bromiden oder -pentafluorphenolaten R₃SO₂X (X = Cl, Br, OPFP) ggf. in Gegenwart einer organischen oder anorganischen Base, beispielsweise Kaliumcarbonat, Natriumhydrogencarbonat, Diisopropylethylamin, Triethylamin, Pyridin, Diethylamin oder DBU, vorzugsweise in einem organischen Lösungsmittel beispielsweise Acetonitril, DCM oder THF zu den sulfonylierten Aminosäuren umgesetzt.

### Methode D

Die eingesetzten Aminosäureester werden in einer Sulfonylierungsreaktion mit Sulfonylchloriden oder -bromiden oder -pentafluorphenolaten R₃SO₂X (X = Cl, Br, OPFP) ggf. in Gegenwart einer organischen oder anorganischen Base, beispielsweise Kaliumcarbonat, Natriumhydrogencarbonat, Diisopropylethylamin, Triethylamin, Pyridin, Diethylamin oder DBU, vorzugsweise in einem organischen Lösungsmittel beispielsweise Acetonitril, Dichlormethan oder Tetrahydrofuran zu den sulfonylierten Aminosäureestern umgesetzt.

Die sulfonylierten Aminosäureester werden dann in einem zweiten Schritt durch eine Alkylierungsreaktion mit Halogenalkanen oder Alkoholen in Lösungsmitteln, wie Aceton, DMF, DCM, THF, Hexan, Toluol, Methanol oder Wasser unter Verwendung anorganischer oder organischer Basen, wie Kaliumcarbonat, n-Butyllithium, Litiumdiisopropylamid, Natriumhydrid, Natriumhydroxid, Natriummethanolat, Diisopropylethylamin und Triethylamin und gegebenflalls mit Hilfe von Reagenzien, wie Triphenylphosphin, Diisopropylazodicarboxylat, Diethylazodicarboxylat, (Cyanomethylen)trimethylphosphonat, bei einer Temperatur zwischen -78°C und +80°C zu den sulfonylierten Aminosäureestern umgesetzt.

Die sulfonylierten Aminosäureester reagieren in einer Esterspaltung unter Verwendung von organischen Säuren, wie Trifluoressigsäure oder wässrigen anorganischen Säuren, wie Salzsäure oder Verwendung von wässrigen anorganischen Basen wie Lithiumhydroxid, Kaliumhydroxid, Natriumhydroxid, Natriumcarbonat, Natriumhydrogencarbonat, Kaliumcarbonat in organischen Lösungsmittel wie Methanol, Dioxan, Dichlormethan, THF, Diethylether oder diese Lösungsmittel als Gemische zu den sulfonylierten Aminosäuren ergeben.

### Methode E

Die eingesetzten Aminosäuren werden unter Verwendung wasserentziehender Reagenzien beispielsweise anorganische Säuren wie H₂SO₄ oder Phosphoroxiden oder organischen Reagenzien wie Thionylchlorid, in organische Lösungsmitteln wie THF, Diethylether, Methanol, Ethanol oder DCM zu den Aminosäureestern verestert und anschließend in einer Sulfonylierung mit Sulfonylchloriden oder -bromiden oder - pentafluorphenolaten R₃SO₂X (X = Cl, Br, OPFP) ggf. in Gegenwart einer organischen oder anorganischen Base, beispielsweise Kaliumcarbonat, Natriumhydrogencarbonat, Diisopropylethylamin, Triethylamin, Pyridin, Diethylamin oder DBU, vorzugsweise in einem organischen Lösungsmittel beispielsweise Acetonitril, DCM oder THF zu den sulfonylierten Aminosäureestern umgesetzt.

Die sulfonylierten Aminosäureester werden dann durch eine Alkylierungsreaktion mit Halogenalkanen oder Alkoholen in Lösungsmitteln, wie Aceton, DMF, DCM, THF, Hexan, Toluol, Methanol oder Wasser unter Verwendung anorganischer oder organischer Basen, wie kaliumcarbonat, n-Butyllithium, Litiumdiisopropylamid, Natriumhydrid, Natriumhydroxid, Natriummethanolat, Diisopropylethylamin und Triethylamin und gegebenflalls mit Hilfe von Reagenzien, wie Triphenylphosphin, Diisopropylazodicarboxylat, Diethylazodicarboxylat, (Cyanomethylen)trimethylphosphonat, bei einer Temperatur zwischen -78°C und +80°C zu den alkylierten, sulfonylierten Aminosäureestern umgesetzt.

Die sulfonylierten Aminosäureester reagieren in einer Esterspaltung unter Verwendung von organischen Säuren, wie Trifluoressigsäure oder wässrigen anorganischen Säuren, wie Salzsäure oder Verwendung von wässrigen anorganischen Basen wie Lithiumhydroxid, Kaliumhydroxid, Natriumhydroxid, Natriumcarbonat, Natriumhydrogencarbonat, Kaliumcarbonat in organischen Lösungsmittel wie Methanol, Dioxan, DCM, THF, Diethylether oder diese Lösungsmittel als Gemische zu den sulfonylierten Aminosäuren ergeben.

### Methode F

Die Aminosäuren werden zunächst in einer Sulfonylierungsreaktion mit Sulfonylchloriden oder -bromiden oder -pentafluorphenolaten R₃SO₂X (X = Cl, Br, OPFP) ggf. in Gegenwart einer organischen oder anorganischen Base, beispielsweise Kaliumcarbonat, Natriumhydrogencarbonat, Diisopropylethylamin, Triethylamin, Pyridin, Diethylamin oder DBU, vorzugsweise in einem organischen Lösungsmittel beispielsweise Acetonitril, DCM oder THF zu den sulfonylierten Aminosäuren umgesetzt.

Die sulfonylierten Aminosäuren werden dann durch eine Alkylierungsreaktion mit Halogenalkanen oder Alkoholen in Lösungsmitteln, wie Aceton, DMF, DCM, THF, Hexan, Toluol, Methanol oder Wasser unter Verwendung anorganischer oder organischer Basen, wie Kaliumcarbonat, n-Butyllithium, Litiumdiisopropylamid, Natriumhydrid, Natriumhydroxid, Natriummethanolat, Diisopropylethylamin und Triethylamin und gegebenflalls mit Hilfe von Reagenzien, wie Triphenylphosphin, Diisopropylazodicarboxylat, Diethylazodicarboxylat, (Cyanomethylen)trimethylphosphonat, bei einer Temperatur zwischen -78°C und +80°C zu den alkylierten, sulfonylierten Aminosäureestern umgesetzt.

Die sulfonylierten Aminosäureester reagieren in einer Esterspaltung unter Verwendung von organischen Säuren, wie Trifluoressigsäure oder wässrigen anorganischen Säuren, wie Salzsäure oder Verwendung von wässrigen anorganischen Basen wie Lithiumhydroxid, Kaliumhydroxid, Natriumhydroxid, Natriumcarbonat, Natriumhydrogencarbonat, Kaliumcarbonat in organischen Lösungsmittel wie Methanol, Dioxan, Dichlormethan, THF, Diethylether oder diese Lösungsmittel als Gemische zu den sulfonylierten Aminosäuren ergeben.

Sofern die erfindungsgemäßen substituierten Sulfonamid-Verbindungen nach ihrer Herstellung in Form einer Mischung ihrer Stereoisomeren, vorzugsweise in Form ihrer Racemate oder anderer Mischungen ihrer verschiedenen Enantiomeren und/oder Diastereomeren erhalten werden, können diese nach üblichen; dem Fachmann bekannten Verfahren getrennt und ggf. isoliert werden. Beispielhaft seien chromatographische Trennverfahren, insbesondere Flüssigkeitschromatographie-Verfahren unter Normaldruck oder unter erhöhtem Druck, bevorzugt MPLC- und HPLC-Verfahren, sowie Verfahren der fraktionierten Kristallisation genannt. Dabei können insbesondere einzelne Enantiomeren, z.B. mittels HPLC an chiraler stationärer Phase oder mittels Kristallisation mit chiralen Säuren, etwa (+)-Weinsäure, (-)-Weinsäure oder (+)-10-Camphersulfonsäure, gebildete diastereomere Salze voneinander getrennt werden.

Es hat sich gezeigt, dass die erfindungsgemäßen Substanzen B1-Rezeptor Antagonisten sind. Daher eignen sich die Verbindungen zur Behandlung von akutem, viszeralem, chronischem oder neuropathischem Schmerz und Entzündungsschmerz, aber auch zur Behandlung von Erkrankungen der Atemwege, Diabetes, Erkrankungen der Atemwege, entzündlichen Darmerkrankungen, neurologischen Erkrankungen, Entzündungen der Haut, rheumatischen Erkrankungen, septischem Schock, Reperfusionssyndrom, Fettleibigkeit und als Angiognese-Inhibitor.

Die erfindungsgemäßen Substanzen eignen sich als pharmazeutische Wirkstoffe in Arzneimitteln. Ein weiterer Gegenstand der Erfindung sind daher Arzneimittel enthaltend wenigstens ein erfindungsgemäßes substituiertes Sulfonamid-Derivat, sowie gegebenenfalls geeignete Zusatz- und/oder Hilfsstoffe und/oder gegebenenfalls weitere Wirkstoffe.

Die erfindungsgemäßen Arzneimittel enthalten neben mindestens einem erfindungsgemäßen substituierten substituierten Sulfonamid-Derivat gegebenenfalls geeignete Zusatz- und/oder Hilfsstoffe, so auch auch Trägermaterialien, Füllstoffe, Lösungsmittel, Verdünnungsmittet, Farbstoffe und/oder Bindemittel und können als flüssige Arzneiformen in Form von Injektionslösungen, Tropfen oder Säfte, als halbfeste Arzneiformen in Form von Granulaten, Tabletten, Pellets, Patches, Kapseln, Pflaster oder Aerosolen verabreicht werden. Die Auswahl der Hilfsstoffe etc. sowie die einzusetzenden Mengen derselben hängen davon ab, ob das Arzneimittel oral, peroral, parenteral, intravenös, intraperitoneal, intradermal, intramuskulär, intranasal, buccal, rektal oder örtlich, zum Beispiel auf die Haut, die Schleimhäute oder in die Augen, appliziert werden soll. Für die orale Applikation eignen sich Zubereitungen in Form von Tabletten, Dragees, Kapseln, Granulaten, Tropfen, Säften und Sirupen, für die parenterale, topische und inhalative Applikation Lösungen, Suspensionen, leicht rekonstituierbare Trockenzubereitungen sowie Sprays. Erfindungsgemäße substituierte Sulfonamid-Derivate in einem Depot, in gelöster Form oder in einem Pflaster, gegebenenfalls unter Zusatz von die Hautpenetration fördernden Mitteln, sind geeignete perkutane Applikationszubereitungen. Oral oder perkutan anwendbare Zubereitungsformen können die erfindungsgemäßen substituierten Sulfonamid-Derivate verzögert freisetzen. Prinzipiell können den erfindungsgemäßen Arzneimitteln andere dem Fachmann bekannte weitere Wirkstoffe zugesetzt werden.

Die an den Patienten zu verabreichende Wirkstoffmenge variiert in Abhängigkeit vom Gewicht des Patienten, von der Applikationsart, der Indikation und dem Schweregrad der Erkrankung. Üblicherweise werden 0,005 bis 20 mg/kg, bevorzugt 0,05 bis 5 mg/kg wenigstens eines erfindungsgemäßen Sulfonamid-Derivats appliziert.

Das Arzneimittel kann ein erfindungsgemäßes substituiertes Sulfonamid-Derivat als reines Diastereomer und/oder Enantiomer, als Razemat oder als nicht-äquimolare oder äquimolare Mischung der Diastereomere und/oder Enantiomere enthalten.

Ein weiterer Gegenstand der Erfindung ist die Verwendung eines erfindungsgemäßen substituierten Sulfonamid-Derivats zur Herstellung eines Arzneimittels zur Behandlung von Schmerz, insbesondere von akutem, viszeralem, chronischem oder neuropathischem Schmerz und Entzündungsschmerz.

Ein weiterer Gegenstand der Erfindung ist die Verwendung eines erfindungsgemäßen substituierten Sulfonamid-Derivats zur Herstellung eines Arzneimittels zur Behandlung von Erkrankungen der Atemwege.

Die substituierten substituierten Sulfonamid-Derivate der allgemeinen Formel I eignen sich auch zur Behandlung von von Depressionen, Harninkontinenz, Diarrhöe, Pruritus, Alkohol- und Drogenmissbrauch, Antriebslosigkeit, Migräne, Diabetes, entzündlichen Darmerkrankungen, neurologischen Erkrankungen, Entzündungen der Haut, rheumatischen Erkrankungen; septischem Schock, Reperfusionssyndrom, Fettleibigkeit, als Angiognese-Inhibitor und zur Anxiolyse.

Gegenstand der Erfindung ist daher auch die Verwendung eines substituierten substituierten Sulfonamid-Derivates der allgemeinen Formel I zur Herstellung eines Arzneimittels zur Behandlung von von Depressionen, Harninkontinenz, Diarrhöe, Pruritus, Alkohol- und Drogenmissbrauch, Antriebslosigkeit, Migräne, Diabetes, entzündlichen Darmerkrankungen, neurologischen Erkrankungen, Entzündungen der Haut, rheumatischen Erkrankungen, septischem Schock, Reperfusionssyndrom, Fettleibigkeit, als Angiognese-Inhibitor und zur Anxiolyse.

### Beispiele:

Die folgenden Beispiele sollen die Erfindung erläutern, schränken jedoch die Erfindung nicht ein.

Die Ausbeuten der hergestellten Verbindungen wurden nicht optimiert.

Alle Temperaturen sind unkorrigiert.

Dia Angabe "RT" bedeutet Raumtemperatur, "konz." konzentriert, "d" Tage, "min" Minuten, "h" Stunden, "*M*" ist eine Konzentrationsangabe in mol/l, "MeOH" Methanol, "THF" Tetrahydrofuran, "aq." wäßrig, "ges." gesättigt, "Lsg." Lösung, "EtOAc" Ethylacetat, "NaHCO₃-Lsg." Natriumhydrogencarbonat-Lsg., "DCM" Dichlormethan, "CHCl₃" Chlorform, "DMF" *N,N*-Dimethylformamid, "Et₂O" Diethylether, "Et₃N" Triethylamin, "i. Vak." im Vakuum, "Na₂SO₄" Natriumsulfat, "NH₄Cl-Lsg." ges. aq. AmmoniumchloridLsg.

Die eingesetzten Chemikalien und Lösungsmittel wurden kommerziell bei den herkömmlichen Anbietern (Acros, Avocado, Aldrich, Bachem, Fluka, Lancaster, Maybridge, Merck, Sigma, TCI, etc.) bezogen oder nach literaturbekannten Methoden synthetisiert.

Als stationäre Phase für die Säulenchromathographie wurde Kieselgel 60 (0.040 - 0.063 mm) der Firma E. Merck, Darmstadt, eingesetzt.

### Abkürzungen

TBTU = O-(Benzotriazol-1-yl)-N,N,N',N'-tetramethyluronium tetrafluorborat
CDI = 1,1'-Carbonyldiimidazol
DCC = Dicyclohexylcarbodiimid
EDCI = 1-ethyl-3-(3-dimethylaminopropyl)carbodiimid
HOAt = 1-Hydroxy-7-azabenzotriazol
DIPEA = N,N-Diisopropylamin
HOBt = 1-Hydroxybenzotriazol
EDCI = N-(3-Dimethylaminopropyl)-N'-ethylcarbodiimide hydrochloride
PyBOP = Benzotriazol-1-yl-oxy-tris-pyrrolidino-phosphonium hexafluorphosphat
PFPTFA = Pentafluorphenyltrifluoracetyl
OPFP = O-Pentafluorphenyl
DBU = 1,8-Diazabicyclo[5.4.0]undec-7-en
AcOH = Essigsäure
DIBAL-H = Diisobutylaluminiumhydrid
EtOH = Ethanol
HBt = 1H-Benzotriazol
KtOBu = Kalium-*tert*.-butylat
LAH = Lithiumaluminiumhydrid
PG = Schutzgruppe
TEA = Triethylamin
TFA = Trifluoressigsäure
p-TosOH = p-Toluolsulfonsäure

Die dünnschicht-chromatographischen Untersuchungen wurden mit HPTLC-Fertigplatten, Kieselgel 60 F 254, der Firma E. Merck, Darmstadt, durchgeführt.

Die Mischungsverhältnisse von Lösungsmitteln, Laufmitteln oder für chromatographische Untersuchungen sind stets in Volumen/Volumen angegeben.

### Verwendete Aminbausteine

| **Nr.** | **Name** |
|---|---|
| **A1** | 4-(Dimethylamino-phenyl-methyl)-cyclohexylamin |
| **A2** | 4-[Dimethylamino-(4-fluorphenyl)-methyl]-cyclohexylamin |
| **A3** | 4-[Dimethylamino-(3-fluorphenyl)-methyl]-cyclohexylamin |
| **A4** | 4-(Dimethylamino-thiophen-2-yl-methyl)-cyclohexylamin |
| **A5** | 4-(1-Dimethylamino-3-phenyl-propyl)-cyclohexylamin |
| **A6** | 4-(1-Morpholin-4-yl-3-phenyl-propyl)-cyclohexylamin |
| **A7** | 4-(3-Phenyl-1-piperidin-1-yl-propyl)-cyclohexylamin |
| **A8** | 4-[(4-Methyl-piperazin-1-yl)-phenyl-methyl]-cyclohexylamin |
| **A9** | 4-(Phenyl-pyrrolidin-1-yl-methyl)-cyclohexylamin |
| **A10** | 4-(Phenyl-piperidin-1-yl-methyl)-cyclohexylamin |
| **A11** | 4-(Morpholin-4-yl-phenyl-methyl)-cyclohexylamin |
| **A12** | [(4-Aminomethyl-cyclohexyl)-phenyl-methyl]-dimethylamin |
| **A13** | [(4-Aminomethyl-cyclohexyl)-(4-fluorphenyl)-methyl]-dimethylamin |
| **A14** | [(4-Aminomethyl-cyclohexyl)-(3-fluorphenyl)-methyl]-dimethylamin |
| **A15** | [(4-Aminomethyl-cyclohexyl)-(4-chlorphenyl)-methyl]-dimethylamin |
| **A16** | [(4-Aminomethyl-cydohexyl)-thiophen-2-yl-methyl]-dimethylamin |
| **A17** | [1-(4-Aminomethyl-cyclohexyl)-3-phenyl-propyl]-dimethylamin |
| **A18** | C-[4-(Morpholin-4-yl-phenyl-methyl)-cydohexyl]-methylamin |
| **A19** | C-[4-(1-Morpholin-4-yl-3-phenyl-propyl)-cyclohexyl]-methylamin |
| **A20** | C-[4-(Phenyl-pyrrolidin-1-yl-methyl)-cyclohexyl]-methylamin |
| **A21** | C-[4-(3-Phenyl-1-pyrrolidin-1-yl-propyl)-cyclohexyl]-methylamin |
| **A22** | 2-[4-(Dimethylamino-phenyl-methyl)-cyclohexyl]-ethylamin |
| **A23** | 2-{4-[Dimethylamino-(4-fluorphenyl)-methyl]-cyclohexyl}-ethylamin |
| **A24** | 2-{4-[Dimethylamino-(3-fluorphenyl)-methyl]-cyclohexyl}-ethylamin |
| **A25** | 2-{4-[(4-Chlorphenyl)-dimethylamino-methyl]-cyclohexyl}-ethylamin |
| **A26** | 2-[4-(Dimethylamino-thiophen-2-yl-methyl)-cyclohexyl]-ethylamin |
| **A27** | {1-[4-(2-Amino-ethyl)-cyclohexyl]-3-phenyl-propyl}-dimethylamin |
| **A28** | N,N-Dimethyl-1-phenyl-1-(piperidin-4-yl)methanamin |
| **A29** | 4-(Phenyl-piperidin-4-yl-methyl)-morpholin |
| **A30** | 4-(2-Phenyl-1-piperidin-4-yl-ethyl)-morpholin |
| **A31** | 1-((3-Fluorphenyl)(piperidin-4-yl)methyl)-4-methylpiperazin |
| **A32** | 1-((4-Fluorphenyl)(piperidin-4-yl)methyl)-4-methylpiperazin |
| **A33** | 1-Methyl-4-(phenyl(piperidin-4-yl)methyl)piperazin |
| **A34** | 1-Methyl-4-(2-phenyl-1-(piperidin-4-yl)ethyl)piperazin |
| **A35** | 1-Methyl-4-(3-phenyl-1-(piperidin-4-yl)propyl)piperazin |

### Synthese der Aminbausteine

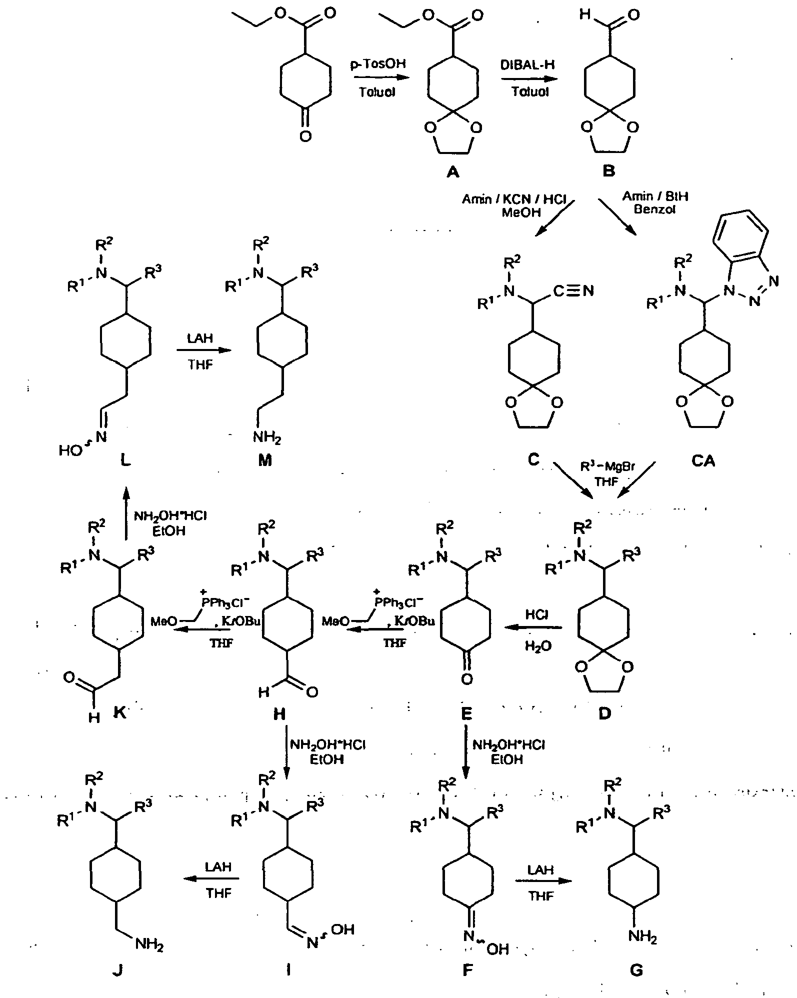

### a) Synthese der verwendeten Cyclohexanone

Die Ketone wurden in einer mehrstufigen Synthese aus dem kommerziell erhältlichen 4-Oxo-cyclohexancarbonsäure-ethylester erhalten. Die Ausbeuten der hergestellten Verbindungen sind nicht optimiert. Alle Temperaturen sind unkorrigiert.

### 1,4-Dioxa-spiro[4.5]decan-8-carbonsäure-ethylester

4-Oxo-cydohexancarbonsäure-ethylester (52,8 g, 0,31 mol, Merck, Bestell-Nr. 814249), Ethylenglykol (67,4 g, 1,08 mol) und p-Toluolsulfonsäure (0,7 g) in Toluol (160 ml) wurden 20 h bei RT gerührt, die Reaktionslösung in Diethylether (300 ml) gegossen und mit Wasser, Natriumhydrogencarbonat-Lösung und Natriumchlorid-Lösung gewaschen. Die Lösung wurde getrocknet (Natriumsulfat), i. Vak. eingeengt und die verbliebene farblose Flüssigkeit ohne Reinigung weiter verarbeitet. Ausbeute: 66,5 g (100 %)
¹H-NMR (CDCl₃): 1,24 (t, 3 H); 1,53 (m, 2 H); 1,76 (m, 4 H); 1,92 (m, 2 H); 2,31 (m, 1 H); 3,91 (s, 4 H); 4,11 (q, 2 H).
¹³C-NMR (CDCl₃): 14,28 (q); 26,32 (t); 33,76 (t); 41,59 (d); 60,14 (t); 64,21 (t); 107,90 (d); 174,77 (s).

### 1,4-Dioxa-spiro[4.5]decan-8-carbaldehyd

Eine Lösung aus 1,4-Dioxa-spiro[4.5]decan-8-carbonsäureethylester (32,13 g, 150 mmol) in absol.Toluol (160 ml) wurde bei -70 bis -65 °C unter Argon tropfenweise mit Diisobutylaluminiumhydrid (1,5 M Lösung in Toluol, 102 ml, 153 mmol) versetzt und 30 min gerührt. Anschließend wurde der Ansatz bei -70 bis -60 °C durch Zugabe von Methanol (80 ml) gequencht. Die Reaktionslösung wurde auf RT erwärmt, mit gesättigter Natriumchlorid-Lösung (100 ml) versetzt und die Rektionslösung über Kieselgur abgesaugt. Kiesegur wurde zweimal mit Ethylacetat gewaschen, die wäßrige Lösung abgetrennt und zweimal mit Ethylacetat extrahiert. Die vereinigten organischen Extrakte wurden mit gesättigter Natriumchlorid-Lösung gewaschen, über Natriumsulfat getrocknet und i. Vak. eingeengt.
Ausbeute: 24,01 g (94 %), gelbes Öl
¹H-NMR (CDCl₃): 1,54 (m, 2 H); 1,74 (m, 4 H); 1,91 (m, 2 H); 2,21 (m, 1 H); 3,91 (s, 4 H); 9,60 (s, 1 H).
¹³C-NMR (CDCl₃): 23,35 (t); 33,37 (t); 48,18 (d); 64,30 (t); 107,89 (d); 203,51 (s).

### Amino-(1,4-dioxa-spiro[4.5]dec-8-yl)-acetonitril

### Dimethylamino-(1,4-dioxa-spiro[4.5]dec-8-yl)-acetonitril (R¹ = Me, R² = Me)

Zu einem Gemisch aus 4 N Salzsäure (37 ml) und Methanol (22 ml) wurde unter Eiskühlung 40-proz. wäßrige Dimethylaminlösung (85 ml, 0,67 mol), 1,4-Dioxa-spiro-[4.5]decan-8-carbaldehyd (240 g, 0,141 mol) und Kaliumcyanid (22,05 g, 0,338 mol) addiert. Die Mischung wurde 4 d bei Raumtemperatur gerührt und anschließend nach Zugabe von Wasser (80 ml) mit Diethylether (4 x 100 ml) extrahiert. Die organische Phase wurde über Natriumsulfat getrocknet, i. Vak. eingeengt und das Produkt als weißer Feststoff erhalten.
Ausbeute: 25,2 g (81 %); Schmelzpunkt: 48-51 °C.
¹H-NMR (CDCl₃): 1,23 - 2,03 (m, 9 H); 2,28 (s, 6 H); 3,16 (d, 1H); 3,93 (m, 4 H).
¹³C-NMR (CDCl₃): 26,67 (t); 27,93 (t); 33,87 (t); 36,94 (d); 41.90 (q); 64.30 (t); 64.36 (t); 108.33 (d); 115.94 (s).

### Morpholino-(1,4-dioxa-spiro[4.5]dec-8-yl)-acetonitril (NR¹R² = Morpholin)

Zu einer Lösung von 1,4-Dioxa-spiro[4,5]decan-8-carboxaldehyd (0.141 mol) in einer Mischung aus Ethanol (141 ml) und Wasser (70 ml) wurde KCN (0.17 mol) und Morpholin (14,7g, 0.17 mol) hinzugegeben und für 72 h bei 25°C gerührt. Nach Zugabe von Ethylacetat (700 ml) wurde die organische Phase abgetrennt und nacheinander mit Wasser (4 x 150 ml) und wässriger FeSO₄-Lösung (4 x 150 ml) gewaschen. Die separierte organische Phase wurde über Na₂SO₄ getrocknet und anschließend abfiltriert. Das Lösungsmittel wurde unter Vakuum entfernt und das Produkt ohne weitere Aufreinigung in die nächste Stufe eingesetzt.

### Pyrrofidino-(1,4-dioxa-spiro[4.5]dec-8-yl)-acetonitril (NR¹R² = Pyrrolidin)

Zu einer Lösung von 1,4-Dioxa-spiro[4,5]decan-8-carboxaldehyd (0.141 mol) in einer Mischung aus Ethanol (141 ml) und Wasser (70 ml) wurde KCN (0.17 mol) und Pyrrolidin (12,07g, 0.17 mol) hinzugegeben und für 72 h bei 25°C gerührt. Nach Zugabe von Ethylacetat (700 ml) wurde die organische Phase abgetrennt und nacheinander mit Wasser (4 x 150 ml) und wässriger FeSO₄-Lösung (4 x 150 ml) gewaschen. Die separierte organische Phase wurde über Na₂SO₄ getrocknet und anschließend abfiltriert. Das Lösungsmittel wurde unter Vakuum entfernt und das Produkt ohne weitere Aufreinigung in die nächste Stufe eingesetzt.

### Piperidino-(1,4-dioxa-spiro[4.5]dec-8-yl)-acetonitril (NR¹R² = Piperidin)

Zu einer Lösung von 1,4-Dioxa-spiro[4,5]decan-8-carboxaldehyd (0.141 mol) in einer Mischung aus Ethanol (141 ml) und Wasser (70 ml) wurde KCN (0.17 mol) und Piperidin (14,45g, 0.17 mol) hinzugegeben und für 72 h bei 25°C gerührt. Nach Zugabe von Ethylacetat (700 ml) wurde die organische Phase abgetrennt und nacheinander mit Wasser (4 x 150 ml) und wässriger FeSO₄-Lösung (4 x 150 ml) gewaschen. Die separierte organische Phase wurde über Na₂SO₄ getrocknet und anschließend abfiltriert. Das Lösungsmittel wurde unter Vakuum entfernt und das Produkt ohne weitere Aufreinigung in die nächste Stufe eingesetzt.

### [(1,4-Dioxa-spiro(4.5)dec-8-yl)-phenyl-methyl]-dimethyl-amin (R¹ = Me, R² = Me, R³ = Phenyl)

Eine 25-proz. Lösung von Phenylmagnesiumchlorid (144 ml, 262.5 mmol) in THF wurde unter Argon und Eiskühlung tropfenweise mit einer Lösung des Aminonitrils (23,56 g, 105 mmol) in absol. THF (100 ml) versetzt und 20 h bei RT gerührt. Zur Aufarbeitung des Reaktionsgemisches wurde unter Eiskühlung gesättigte Ammoniumchlorid-Lösung (100 ml) und Wasser (100 ml) addiert und mit Diethylether (3 x 100 ml) extrahiert. Die organische Phase wurde mit Wasser und gesättigter Natriumchlorid-Lösung gewaschen, getrocknet (Natriumsulfat) und eingeengt.
Ausbeute: 28.9 g (100 %).
¹³C-NMR (CDCl₃): 27,05; 28,13; 34,48; 34,57; 36,94 (C₈); 41,64 (N(CH₃)₂); 64,15; 74,33 (CH); 109,02 (C₅); 126,70 (Cₐᵣₒₘ); 127,49 (Cₐᵣₒₘ); 129,12 (Cₐᵣₒₘ); 136,57 (Cₐᵣₒₘ).

### [(1,4-Dioxa-spiro[4.5jdec-8-yl)-4-fluorphenyl-methylj-dimethylamin (R¹ = Me, R² = Me, R³ = 4-Fluorphenyl)

Eine 1M Lösung von 4-Fluorphenylmagnesiumbromid in THF (220 ml, 220 mmol) wurde unter Argon und Eiskühlung tropfenweise mit einer Lösung des Aminonitrils (19,89 g, 88 mmol) in absol. THF (160 ml) versetzt und 20 h bei RT gerührt. Zur Aufarbeitung des Reaktionsgemisches wurde unter Eiskühlung gesättigte Ammoniumchlorid-Lösung (100 ml) und Wasser (100 ml) gegeben und mit Diethylether (3 x 100 ml) extrahiert. Die organische Phase wurde mit Wasser und gesättigter Natriumchlorid-Lösung gewaschen, getrocknet (Natriumsulfat) und eingeengt.
Ausbeute: 31 g (>100 %)
¹³C-NMR (CDCl₃): 26,68 (t); 28,11 (t); 34,43 (t); 34,55 (t); 37,37 (d); 41,68 (q); 64,12 (t); 73,65 (d); 108,88 (d); 114,23 (d); 114,44 (d); 130,27; 130,35; 132,43; 160,36 (s); 162,78 (s).

### [(1,4-Dioxa-spiro[4.5]dec-8-yl)-3-fluorphenyl-methyl]-dimethyl-amin (R¹ = Me, R² = Me, R³ = 3-Fluorphenyl)

Eine 1M Lösung von 3-Fluorphenylmagnesiumbromid in THF (208 ml, 208 mmol) wurde unter Argon und Eiskühlung tropfenweise mit einer Lösung des Aminonitrils (23,45 g, 104 mmol) in absol. THF (100 ml) versetzt und 20 h bei RT gerührt. Zur Aufarbeitung des Reaktionsgemisches wurde unter Eiskühlung gesättigte Ammoniumchlorid-Lösung (100 ml) und Wasser (100 ml) gegeben und mit Diethylether (3 x 100 ml) extrahiert. Die organische Phase wurde mit Wasser und gesättigter Natriumchlorid-Lösung gewaschen, getrocknet und eingeengt. Ausbeute: 30,33 g (99 %).
¹H-NMR (CDCl₃): 1,12 (m, 1 H); 1,26 (m, 1 H); 1,46 -1,81 (m, 7 H); 2,10 (s, 6 H); 3,10 (d, 1 H); 3,90 (m, 4 H); 6,85 (m, 3 H); 7,27 (m, 1 H).
¹³C-NMR (CDCl₃): 26,80 (t); 28,08 (t); 34,48 (t); 34,45 (t); 34,59 (t); 37,26 (d); 41,71 (q); 64,19 (t); 74,04 (t); 108,91 (d); 113,51 (d); 113,71 (d); 115,52 (d); 115,72 (d); 124,83 (d); 128,82 (d); 128,90 (d); 139,66 (s); 161,15 (s); 163,58 (s).

### [(4-Chlorphenyl)-(1,4-dioxa-spiro[4.5]dec-8-yl)-methyl]-dimethyl-amin (R¹ = Me, R² = Me, R³ = 4-Chlorphenyl)

Eine 1 M Lösung von 4-Chlorphenylmagnesiumbromid in Diethylether (200 ml, 200 mmol) wurde unter Argon und Eiskühlung tropfenweise mit einer Lösung des Aminonitrils (22,43 g, 100 mmol) in absol. Diethylether (100 ml) versetzt und 20 h bei RT gerührt. Zur Aufarbeitung des Reaktionsgemisches wurde unter Eiskühlung gesättigte Ammoniumchlorid-Lösung (100 ml) und Wasser (100 ml) gegeben und mit Diethylether (3 x 100 ml) extrahiert. Die organische Phase wurde mit Wasser und gesättigter Natriumchlorid-Lösung gewaschen, getrocknet und eingeengt.
Ausbeute: 30,9 g (100%)
¹³C-NMR (CDCl₃): 26,65 (t); 28.11 (t); 34,46 (t); 34,60 (t); 37,28 (d); 41,76 (q); 64,17 (t); 73,80 (d); 108,88 (s); 127,72 (d); 129,53 (d); 132,39 (d); 135,33 (d).

### [(1,4-Dioxa-spiro[4.5]dec-8-yl)-thiophen-2-yl-methyl]-dimethylamin (R¹ = Me, R² = Me, R³ = 2-Thienyl)

Eine 1 M Lösung von Thiophen-2-yl-magnesiumbromid in THF (20 ml, 20 mmol) wurde unter Argon und Eiskühlung tropfenweise mit einer Lösung des Aminonitrils (2,24 g, 10 mmol) in absol. THF (10 ml) versetzt und 20 h bei RT gerührt. Zur Aufarbeitung des Reaktionsgemisches wurde unter Eiskühlung gesättigte Ammoniumchlorid-Lösung (10 ml) und Wasser (10 ml) gegeben und mit Diethylether (3 x 10 ml) extrahiert. Die organische Phase wurde mit Wasser und gesättigter Natriumchlorid-Lösung gewaschen, getrocknet und eingeengt.
Ausbeute: 2,8 g (100 %)
¹³C-NMR (CDCl₃): 27,72 (t); 27,88 (t); 34,27 (t); 39,28 (d); 41,10 (q); 64,11 (t); 68,89 (d); 108,88 (s); 123,55 (d); 125,88 (d); 127,53 (d); 139,50 (s).

### [1-(1,4-Dioxa-spiro[4.5]dec-8-yl)-3-phenyl-propyl]-dimethylamin (R¹ = Me, R² = Me, R³ = Phenethyl)

Eine 1 M Lösung von Phenylethylmagnesiumchlorid in THF (242 ml, 242 mmol) wurde unter Argon und Eiskühlung tropfenweise mit einer Lösung des Aminonitrils (21,93 g, 97 mmol) in absol. THF (180 ml) versetzt und 20 h bei RT gerührt. Zur Aufarbeitung des Reaktionsgemisches wurde unter Eiskühlung gesättigte Ammoniumchlorid-Lösung (100 ml) und Wasser (100 ml) gegeben und mit Diethylether (3 x 100 ml) extrahiert. Die organische Phase wurde mit Wasser und gesättigter Natriumchlorid-Lösung gewaschen, getrocknet und eingeengt.
Ausbeute: 34 g (> 100 %).
¹³C-NMR (CDCl₃): 27,43 (t); 28,95 (t); 29,42 (t); 34,82 (t); 35,40 (t); 38,76 (d); 41,16 (q); 64,17 (t); 67,41 (d); 108,86 (s); 125,41 (d); 127,66 (d); 128,11 (d); 142,69 (s).

### [(1,4-Dioxa-spiro[4.5]dec-8-yl)-phenyl-methyl]-morpholin (NR¹R² = Morpholin, R³ = Phenyl)

Zu einer Lösung des Aminonitrils (105 mmol) in THF (100 ml) gab man tropfenweise unter Argon eine 25-proz. Lösung von Phenylmagnesiumchlorid (144 ml, 262.5 mmol) in THF bei 0°C hinzu und rührte anschließend für weitere 20 h bei 25°C. Nach Zugabe einer wässrigen gesättigten NH₄Cl-Lösung (200 ml) wurde die Reaktionsmischung mit Ethylacetat (3 x 100 ml) extrahiert. Die vereinigten organischen Phasen wurden anschließend mit Wasser und mit wässriger gesättigter NaCl-Lösung gewaschen. Die separierte organische Phase wurde über Na₂SO₄ getrocknet und abfiltriert. Das Lösungsmittel wurde unter Vakuum entfernt und das Produkt über Säulenchromatographie (2-5% Methanol/Dichlormethan) aufgereinigt.

### [1-(1,4-Dioxa-spiro[4.5]dec-8-yl)-3-phenyl-propyl]-morpholin (NR¹R² = Morpholin, R³ = Phenethyl

Zu einer Lösung des Aminonitrils (105 mmol) in THF (100 ml) gab man tropfenweise unter Argon eine 1 M Lösung von Phenylethylmagnesiumchlorid in THF (262 ml, 262 mmol) in THF bei 0°C hinzu und rührte anschließend für weitere 20 h bei 25°C. Nach Zugabe einer wässrigen gesättigten NH₄Cl-Lösung (200 ml) wurde die Reaktionsmischung mit Ethylacetat (3 x 100 ml) extrahiert. Die vereinigten organischen Phasen wurden anschließend mit Wasser und mit wässriger gesättigter NaCl-Lösung gewaschen. Die separierte organische Phase wurde über Na₂SO₄ getrocknet und abfiltriert. Das Lösungsmittel wurde unter Vakuum entfernt und das Produkt über Säulenchromatographie (2-5% Methanol/Dichlormethan) aufgereinigt.

### [(1,4-Dioxa-spiro[4.5]dec-8-yl)-phenyl-methyl]-pyrrolidin (NR¹R² = Pyrrolidin, R³ = Phenyl)

Zu einer Lösung des Aminonitrils (105 mmol) in THF (100 ml) gab man tropfenweise unter Argon eine 25-proz. Lösung von Phenylmagnesiumchlorid (144 ml, 262.5 mmol) in THF bei 0°C hinzu und rührte anschließend für weitere 20 h bei 25°C. Nach Zugabe einer wässrigen gesättigten NH₄Cl-Lösung (200 ml) wurde die Reaktionsmischung mit Ethylacetat (3 x 100 ml) extrahiert. Die vereinigten organischen Phasen wurden anschließen mit Wasser und mit wässriger gesättigter NaCl-Lösung gewaschen. Die separierte organische Phase wurde über Na₂SO₄ getrocknet und abfiltriert. Das Lösungsmittel wurde unter Vakuum entfernt und das Produkt über Säulenchromatographie (2-5% Methanol/Dichlormethan) aufgereinigt.

### [1-(1,4-Dioxa-spiro[4.5]dec-8-yl)-3-phenyl-propyl]-pyrrolidin (NR¹R² = Pyrrolidin, R³ = Phenethyl)

Zu einer Lösung des Aminonitrils (105 mmol) in THF (100 ml) gab man tropfenweise unter Argon eine 1M Lösung von Phenylethylmagnesiumchlorid in THF (262 ml, 262 mmol) in THF bei 0°C hinzu und rührte anschließend für weitere 20 h bei 25°C. Nach Zugabe einer wässrigen gesättigten NH₄Cl-Lösung (200 ml) wurde die Reaktionsmischung mit Ethylacetat (3 x 100 ml) extrahiert. Die vereinigten organischen Phasen wurden anschließend mit Wasser und mit wässriger gesättigter NaCl-Lösung gewaschen. Die separierte organische Phase wurde über Na₂SO₄ getrocknet und abfiltriert. Das Lösungsmittel wurde unter Vakuum entfernt und das Produkt über Säulenchromatographie (2-5% Methanol/Dichlormethan) aufgereinigt.

### [1-(1,4-Dioxa-spiro[4.5]dec-8-yl)-3-phenyl-propyl]-piperidin (NR¹R² = Piperidin, R³ = Phenethyl

Zu einer Lösung des Aminonitrils (105 mmol) in THF (100 ml) gab man tropfenweise unter Argon eine 1 M Lösung von Phenylethylmagnesiumchlorid in THF (262 ml, 262 mmol) in THF bei 0°C hinzu und rührte anschließend für weitere 20 h bei 25°C. Nach Zugabe einer wässrigen gesättigten NH₄Cl-Lösung (200 ml) wurde die Reaktionsmischung mit Ethylacetat (3 x 100 ml) extrahiert: Die vereinigten organischen Phasen wurden anschließend mit Wasser und mit wässriger gesättigter NaCl-Lösung gewaschen. Die separierte organische Phase wurde über Na₂SO₄ getrocknet und abfiltriert. Das Lösungsmittel wurde unter Vakuum entfernt und das Produkt über Säulenchromatographie (2-5% Methanol/Dichlormethan) aufgereinigt.

### 1-[1,4-Dioxaspiro[4.5]dec-8-yl)phenylmethyl]piperidin ( NR¹R² = Piperidin, R³ = Phenyl)

Eine 2 *M* Lösung von Phenylmagnesiumchlorid in Tetrahydrofuran (13 ml, 26.4 mmol) wurde unter Argon und Eiskühlung tropfenweise mit einer Lösung von Piperidino-(1,4-dioxa-spiro[4.5]dec-8-yl)-acetonitril (2.8 g, 10.6 mmol) in absolutem Tetrahydrofuran (10 ml) versetzt und 20 h bei Raumtemperatur gerührt. Zum Reaktionsgemisch wurde anschließend unter Eiskühlung gesättigte Ammoniumchloridlösung (10 ml) und Wasser (10 ml) getropft und die Lösung mit Diethylether (3 × 30 ml) extrahiert. Die vereinigten organischen Phasen wurden mit Wasser (30 ml) und gesättigter Ammoniumchloridlösung (30 ml) gewaschen, mit Natriumsulfat getrocknet und i. Vak. eingeengt.
Ausbeute: 3.20 g (96 %), gelber, klebriger Feststoff
Schmelzpunkt: nicht bestimmbar
¹H-NMR (DMSO-d₆), 0.08-2.30 (m, 19H); 3.12 (d, 1H, J = 9.9 Hz); 3.81 (s, 4H); 7.00-7.80 (m, 5H).

### 4-(Dimethylamino-phenyl-methyl)-cyclohexanon (R¹ = Me, R² = Me, R³ = Phenyl)

Das Ketal (28,9 g, 0,105 mol) wurde in Wasser (44 ml) gelöst, mit konz. Salzsäure (64 ml) versetzt und 20 h bei RT gerührt. Das Reaktionsgemisch wurde mit Diethylether (2 x 100 ml) ausgeschüttelt, die wässrige Phase unter Eiskühlung mit 5N NaOH alkalisch gestellt, mit DCM (3 x 100 ml) extrahiert, getrocknet und eingeengt. Das Keton wurde als farbloses Öl isoliert.
Ausbeute: 18.2 g (75 %)
¹H-NMR (CDCl₃): 1,20 (1 H, m); 1,33 (1 H, m); 1,74 (1 H, m); 2,17 (6 H, s, N(CH₃)₂); 2,70 (6 H, m); 3,10 (1 H, d, Ce-H); 7,07 (2 H, m, Cₐᵣₒₘ-H); 7,23 (3 H, m, Cₐᵣₒₘ-H).
¹³C-NMR (CDCl₃): 29,13; 30,56; 36,90 (C₄); 40,61; 40,82; 41,89 (N(CH₃)₂); 73,79 (CH); 127,05 (Cₐᵣₒₘ); 127,67 (Cₐᵣₒₘ); 129,00 (Cₐᵣₒₘ); 136,13 (Cₐᵣₒₘ); 211,79 (C=O).

### 4-[Dimethylarnino-(4-fluorphenyl)-methyl]-cyclohexanon (R¹ = Me, R² = Me, R³ = 4-Fluorphenyl) .

Das Rohprodukt des Ketals (26 g, 88 mmol) wurde in Wasser (40 ml) gelöst, mit konz. Salzsäure (59 ml) versetzt und 20 h bei RT gerührt. Das Reaktionsgemisch wurde mit Diethylether (2 x 100 ml) extrahiert, die wässrige Phase unter Eiskühlung mit 5N NaOH alkalisch gestellt, mit DCM (3 x 100 ml) extrahiert, getrocknet und eingeengt.
Ausbeute: 21,36 g (98%)
¹³C-NMR (CDCl₃): 28,90 (t); 30,48 (t); 37,00 (t); 40,49 (t); 40,72 (t); 41,79 (q); 72,98 (d); 114,42 (d); 114,62 (d); 130,20 (d); 130,28 (d); 131,88 (s); 160,50 (s); 162,93 (s); 211,44 (s).

### 4-[Dimethylamino-(3-fluorphenyl)-methyl]-cyclohexanon (R¹ = Me, R² = Me, R³ = 3-Fluorphenyl)

Das Ketal (30,3 g, 103 mmol) wurde in Wasser (44 ml) gelöst, mit konz. Salzsäure (64 ml) versetzt und 20 h bei RT gerührt. Das Reaktionsgemisch wurde mit Diethylether (2 x 100 ml) ausgeschüttelt, die wässrige Phase unter Eiskühlung mit 5N NaOH alkalisch gestellt, mit DCM (3 x 100 ml) extrahiert, getrocknet und eingeengt. Das Keton wurde als farbloser Feststoff isoliert.
Ausbeute: 22,4 g (87 %); Schmelzpunkt: 72-75 °C.
¹³C-NMR (CDCl₃): 28,97 (t); 30,44 (t); 36,90 (t); 40,52 (t); 40,75 (t); 41,82 (q); 73,37 (d); 113,84; 114,06; 115,42; 115,62; 124,71; 129,03; 129,11; 139,00; 139,06; 161,16; 163,60; 211,40 (s).

### 4-[(4-Chlor-phenyl)-dimethylamino-methyl]-cyclohexanon (R³ = 4-Chlorphenyl)

Das Ketal (30,98 g, 100 mmol) wurde in Wasser (44 ml) gelöst, mit konz. Salzsäure (64 ml) versetzt und 20 h bei RT gerührt. Das Reaktionsgemisch wurde mit Diethylether (2 x 100 ml) ausgeschüttelt, die wässrige Phase unter Eiskühlung mit 5N NaOH alkalisch gestellt, mit DCM (3 x 100 ml) extrahiert, getrocknet und eingeengt Das Keton wurde als Öl isoliert.
Ausbeute: 21,9 g (82 %)
¹³C-NMR (CDCl₃): 28,88 (t); 30,45 (t); 36,89 (t); 40,49 (t); 40,74 (t); 41,83 (q); 73,12 (d); 127,87 (d); 130,16 (d); 132,75 (d); 13470 (s); 211,35 (s).

### 4-(Dimethylamino-thiophen-2-yl-methyl)-cyclohexanon (R¹ = Me, R² = Me, R³ = 2-Thienyl)

Das Ketal (2,80 g, 10 mmol) wurde in Wasser (4,4 ml) gelöst, mit konz. Salzsäure (6,4 ml) versetzt und 20 h bei RT gerührt. Das Reaktionsgemisch wurde mit Diethylether (2 x 10 ml) ausgeschüttelt, die wässrige Phase unter Eiskühlung mit 5N NaOH alkalisch gestellt, mit DCM (3 x 10 ml) extrahiert, getrocknet und eingeengt. Das Keton wurde als Öl isoliert.
Ausbeute: 1,79 g (75 %)
¹³C-NMR (CDCl₃): 30,02 (t); 30,18 (t); 38,84 (t); 40,29 (t); 39,28 (d); 41,17 (q); 68,24 (d); 123,88 (d); 126,01 (d); 126,34 (d); 138,77 (d); 211,49 (s).

### 4-(1-Dimethylamino-3-phenyl-propyl)-cyclohexanon (R¹ = Me, R² = Me, R³ = Phenethyl)

Das Rohprodukt des Ketals (29,6 g, 97 mmol) wurde in Wasser (44 ml) gelöst, mit konz. Salzsäure (64 ml) versetzt und 20 h bei RT gerührt. Das Reaktionsgemisch wurde mit Diethylether (2 x 100 ml) ausgeschüttelt, die wässrige Phase unter Eiskühlung mit 5N NaOH alkalisch gestellt, mit DCM (3 x 100 ml) extrahiert, getrocknet und eingeengt. Das Keton wurde als farbloses Öl isoliert.
Ausbeute: 16.9 g (58 %)
¹³C-NMR (CDCl₃): 29,40 (t); 30,02 (t); 30,97 (t); 35,34 (t); 38,71 (t); 40,79 (t); 41,01 (t); 41,23 (q); 66,65 (d); 125,66 (d); 128,12 (d); 128,19 (d); 142,27 (s); 211,70 (s).

### 4-(Morpholino(phenyl)methyl)cyclohexanon (NR¹R² = Morpholin, R³ = Phenyl)

Zum Grignard-Addukt (105 mmol) wurde langsam konz. HCl und Wasser (1:1, 88 ml) bei 0°C hinzugegeben und für 20 h bei 25°C gerührt. Anschließend wurde die Reaktionslösung mit Ethylacetat (2 x 100 ml) extrahiert. Nach Zugabe von 5N Natronlauge, zur Einstellung eines basischen pH-Wertes, wurde mit Dichlormthan (3 x 100 ml) extrahiert. Die vereinigten organischen Phasen wurden über Na₂SO₄ getrocknet und abfiltriert. Das Lösungsmittel wurde unter Vakuum entfernt und das Produkt ohne weitere Aufreinigung in der nächsten Stufe verwendet.

### 4-(1-Morpholino-3-phenylpropyl)cyclohexanon (NR¹R² = Morpholin, R³ = Phenethyl)

Zum Grignard-Addukt (105 mmol) wurde langsam konz. HCl und Wasser (1.1, 88 ml) bei 0°C hinzugegeben und für 20 h bei 25°C gerührt. Anschließend wurde die Reaktionslösung mit Ethylacetat (2 x 100 ml) extrahiert. Nach Zugabe von 5N Natronlauge, zur Einstellung eines basischen pH-Wertes, wurde mit Dichlormthan (3 x 100 ml) extrahiert. Die vereinigten organischen Phasen wurden über Na₂SO₄ getrocknet und abfiltriert. Das Lösungsmittel wurde unter Vakuum entfernt und das Produkt ohne weitere Aufreinigung in der nächsten Stufe verwendet.

### 4-(Phenyl(pyrrolidin-1-yl)methyl)cyclohexanon (NR¹R² = Pyrrolidin, R³ = Phenyl)

Zum Grignard-Addukt (105 mmol) wurde langsam konz. HCl und Wasser (1.1, 88 ml) bei 0°C hinzugegeben und für 20 h bei 25°C gerührt. Anschließend wurde die Reaktionslösung mit Ethylacetat (2 x 100 ml) extrahiert. Nach Zugabe von 5N Natronlauge, zur Einstellung eines basischen pH-Wertes, wurde mit Dichlormthan (3 x 100 ml) extrahiert. Die vereinigten organischen Phasen wurden über Na₂SO₄ getrocknet und abfiltriert. Das Lösungsmittel wurde unter Vakuum entfernt und das Produkt ohne weitere Aufreinigung in der nächsten Stufe verwendet.

### 4-(3-Phenyl-1-(pyrrolidin-1-yl)propyl)cyclohexanon (NR¹R² = Pyrrolidin, R³ = Phenethyl)

Zum Grignard-Addukt (105 mmol) wurde langsam konz. HCl und Wasser (1.1, 88 ml) bei 0°C hinzugegeben und für 20 h bei 25°C gerührt. Anschließend wurde die Reaktionslösung mit Ethylacetat (2 x 100 ml) extrahiert. Nach Zugabe von 5N Natronlauge, zur Einstellung eines basischen pH-Wertes, wurde mit Dichlormthan (3 x 100 ml) extrahiert. Die vereinigten organischen Phasen wurden über Na₂SO₄ getrocknet und abfiltriert. Das Lösungsmittel wurde unter Vakuum entfernt und das Produkt ohne weitere Aufreinigung in der nächsten Stufe verwendet.

### 4-(3-Phenyl-1-(piperidin-1-yl)propyl)cyclohexanon (NR¹R² = Piperidin, R³ = Phenethyl)

Zum Grignard-Addukt (105 mmol) wurde langsam konz. HCl und Wasser (1.1, 88 ml) bei 0°C hinzugegeben und für 20 h bei 25°C gerührt. Anschließend wurde die Reaktionslösung mit Ethylacetat (2 x 100 ml) extrahiert. Nach Zugabe von 5N Natronlauge, zur Einstellung eines basischen pH-Wertes, wurde mit Dichlormthan (3 x 100 ml) extrahiert. Die vereinigten organischen Phasen wurden über Na₂SO₄ getrocknet und abfiltriert. Das Lösungsmittel wurde unter Vakuum entfernt und das Produkt ohne weitere Aufreinigung in der nächsten Stufe verwendet.

### 4-(Phenylpiperidin-1-yl-methyl)cyclohexanon (NR¹R² = Piperidin, R³ = Phenyl)

Eine Lösung von 1-[1,4-Dioxaspiro[4.5]dec-8-yl)phenylmethyl]piperidin (3,10 g, 9,8 mmol) in Wasser (8 ml) und konzentrierter Salzsäure (12 ml) wurde 20 h bei Raumtemperatur gerührt. Das Reaktionsgemisch wurde mit Diethylether (2 × 20 ml) gewaschen, die wässrige Phase unter Eiskühlung mit 5 N Natronlauge auf pH 12 eingestellt und mit Dichlormethan (3 × 40 ml) extrahiert. Die vereinigten organischen Phasen wurden mit Natriumsulfat getrocknet und i. Vak. eingeengt.
Ausbeute: 2,00 g (76 %), gelblicher Feststoff
Schmelzpunkt: 88-90 °C
¹H-NMR (DMSO-d₆): 1,00-1,56 (m, 10H); 2,02-2,50 (m, 9H); 3,28 (d, 1H, J = 10,7 Hz); 7,14-7,37 (m, 5H).
¹³C-NMR (DMSO-d₆): 24,4; 26,09; 29,5; 29,7; 34,5; 49,9 (2 C); 72,7; 126,7; 127,6 (2 C); 128,8 (2 C); 136,4; 211,3. 2 C-Signale sind vom DMSO-Signal überlagert.

### Synthese der Amino-, Aminomethyl- und Aminoethylcyclohexyle

Aus den Cyclohexanonderivaten wurde die entsprechenden Amine erhalten.

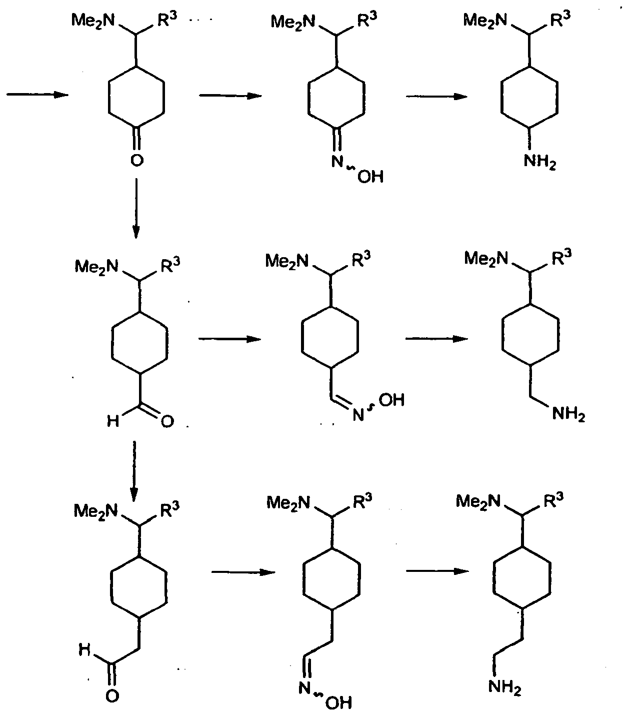

### Synthese der Aminocyclohexane

Die Aminocyclohexane wurden durch zweistufige Reaktionen aus den entsprechend substituierten Cyclohexanonen mit Hydroxylamin Hydrochlorid und anschließender Spaltung mit Lithiumaluminiumhydrid dargestellt.

### Synthese des 4-(Dimethylamino-phenyl-methyl)-cyclohexylamin (R¹ = Me, R² = Me, R³ = Phenyl) A1

### 4-(Dimethylamino-phenyl-methyl)-cyclohexanon-oxim (R¹ = Me, R² = Me, R³ = Phenyl)

Das Keton (9,25 g, 40 mmol) und Hydroxylamin Hydrochlorid (4,17 g, 60 mmol) wurden in absol. Ethanol (150 ml) gelöst, mit basischem lonenaustauscher Amberlyst A21 (28 g) versetzt und bei RT über Nacht gerührt. Der lonenaustauscher wurde abfiltriert und mit Ethanol (2 x 50 ml) gewaschen. Die Lösung wurde eingeengt und der Rückstand mit 5N NaOH auf pH 11 eingestellt. Die wässrige Phase wurde mit Essigsäureethylester (3 x 50 ml) extrahiert, die organische Phase über Natriumsulfat getrocknet und i. Vak. eingeengt.
Ausbeute: 9,54 g (97 %); Schmelzpunkt: 110-115 °C, (farblose Kristalle)
¹³C-NMR (CDCl₃): 23,53; 23,70; 27,87; 29,04; 29,48; 30,70; 31,26; 31,40; 37,89 (C₄); 42,02 (N(CH₃)₂); 74,36 (CH); 126,87 (Cₐᵣₒₘ); 127,56 (Cₐᵣₒₘ); 129,09 (Cₐᵣₒₘ); 136,57 (Cₐᵣₒₘ); 160,12 (C=N-O).

### 4-(Dimethylamino-phenyl-methyl)-cyclohexylamin (R¹ = Me, R² = Me, R³ = Phenyl) A1

Absolutes THF (400 ml) wurde unter Argon mit LiAlH₄ (2,92 g, 77 mmol) versetzt, auf 60 °C erwärmt und tropfenweise das Oxim (9,5 g, 38,5 mmol), gelöst in THF (90 ml), zugegeben. Nach 4-stündigem Rühren bei 60 °C wurde unter Eisbadkühlung (10 °C) Wasser (100 ml) zugetropft und die Lösung über Kieselgur abgesaugt. Der Filterrückstand wurde mit THF gewaschen. Das THF wurde i. Vak. entfernt, der Rückstand mit 5N NaOH auf pH 11 eingestellt und mit Ethylacetat (4 x 40 ml) extrahiert. Die organische Phase wurde über Natriumsulfat getrocknet, eingedampft und der Rückstand über eine Kieselgelsäule (300 g) mit Acetonitril/Methanol/0,5 M NH₄Cl (9:1:1) gereinigt.

Die einzelnen Fraktionen wurden in Wasser und DCM gelöst, mit Ammoniak alkalisch gestellt und die wässrige Phase mit DCM (zweimal) extrahiert.
Gesamtausbeute: 6,33 g (71 %), Öl
¹³C-NMR (CDCl₃): 24,22; 24,80; 28,24; 29,96; 32,39; 32,45; 36,03; 36,58; 36,79; 37,93 (C₄); 41,33; 41,89 (N(CH₃)₂); 47,42; 50,85; 71,95; 75,22 (CH); 126,52 (Cₐᵣₒₘ); 127,29 (Cₐᵣₒₘ); 127,33 (Cₐᵣₒₘ); 129,04 (Cₐᵣₒₘ); 129,11 (Cₐᵣₒₘ); 136,22 (Cₐᵣₒₘ); 137,03 (Cₐᵣₒₘ).

### Synthese des 4-[Dimethylamino-(4-fluorphenyl)-methyl]-cyclohexylamin (R¹ = Me, R² = Me R³ = 4-Fluorphenyl) A2

### 4-[Dimethylamino-(4-fluorphenyl)-methyl]-cyclohexanon-oxim (R¹ = Me, R² = Me R³ = 4-Fluorphenyl)

Das Keton (10,68 g, 43 mmol) und Hydroxylamin Hydrochlorid (4,52 g, 65 mmol) wurden in absol. Ethanol (160 ml) gelöst, mit basischem lonenaustauscher Amberlyst A21 (30 g) versetzt und bei RT über Nacht gerührt. Der Ionenaustauscher wurde abfiltriert und mit Ethanol (2 x 50 ml) gewaschen. Die Lösung wurde eingeengt, der Rückstand mit 5N NaOH auf pH 11 eingestellt, die wässrige Phase mit Ethylacetat (3 x 50 ml) extrahiert, die organische Phase über Natriumsulfat getrocknet und i. Vak. eingeengt.
Ausbeute: 10,49 g (93 %)
¹³C-NMR (CDCl₃): 23,76; 23,66; 27,69; 28,87; 29,50; 30,73; 31,22; 31,38; 38,06 (C₄); 42,01 (N(CH₃)₂); 73,66 (CH); 114,36 (Cₐᵣₒₘ); 114,57 (Cₐᵣₒₘ); 130,32 (Cₐᵣₒₘ); 130,40 (Cₐᵣₒₘ); 132,40 (Cₐᵣₒₘ); 160,03 (C=N-O); 160,49 (Cₐᵣₒₘ); 162,93 (Cₐᵣₒₘ).

### 4-[Dimethylamino-(4-fluorphenyl)-methyl]-cyclohexylamin (R¹ = Me, R² = Me R³ = 4-Fluorphenyl) A2

Absolutes THF (435 ml) wurde unter Argon mit LiAlH₄ (3,04 g, 82 mmol) versetzt, auf 60 °C erwärmt und tropfenweise das Oxim (10,49 g, 40 mmol), gelöst in THF (90 ml), zugegeben. Nach 4-stündigem Rühren bei 60 °C wurde unter Eisbadkühlung (10 °C) Wasser (100 ml) zugetropft und über Kieselgur abgesaugt. Der Filterrückstand wurde mit THF gewaschen. Das THF i. Vak. entfernt, der Rückstand mit 5N NaOH auf pH 11 eingestellt und mit Ethylacetat (4 x 50 ml) extrahiert. Die organische Phase wurde über Natriumsulfat getrocknet, eingedampft und der Rückstand durch Flashchromatographie mit Acetonitril/Methanol/0,5M NH₄Cl (9:1:1) gereinigt.

Die einzelnen Fraktionen wurden in Wasser und DCM gelöst, mit Ammoniak alkalisch gestellt und die wässrige Phase zweimal mit DCM extrahiert.
Ausbeute: 6,95 g (70 %), Öl
¹³C-NMR (CDCl₃): 24,01; 24,76; 27,99; 29,92; 32,32; 36,26; 36,51; 36,73; 38,07; 41,26 (C₄); 41,85 (N(CH₃)₂); 47,31; 50,81; 71,25; 74,44 (CH); 114,01(Cₐᵣₒₘ); 114,08 (Cₐᵣₒₘ); 130,20 (Cₐᵣₒₘ); 130,27 (Cₐᵣₒₘ); 132,02 (Cₐᵣₒₘ); 132,85 (Cₐᵣₒₘ); 160,22 (Cₐᵣₒₘ); 162.64 (Cₐᵣₒₘ).

### Synthese des 4-[Dimethylamino-(3-fluorphenyl)-methyl]-cyclohexylamins (R¹ = Me, R² = Me, R³ = 3-Fluorphenyl) A3

### 4-[Dimethylamino-(3-fluorphenyl)-methyl]-cyclohexanon-oxim (R¹ = Me, R² = Me, R³ = 3-Fluorphenyl)

Das Keton (10 g, 40 mmol) und Hydroxylamin Hydrochlorid (4,17 g, 60 mmol) wurden in absol. Ethanol (150 ml) gelöst, mit basischem lonenaustauscher Amberlyst A21 (28 g) versetzt und bei RT über Nacht gerührt. Der Ionenaustauscher wurde abfiltriert und mit Ethanol (2 x 50 ml) gewaschen. Die Lösung wurde eingeengt, der Rückstand mit 5N NaOH auf pH 11 eingestellt, die wässrige Phase mit Ethylacetat (3x 50 ml) extrahiert, die organische Phase über Natriumsulfat getrocknet und i. Vak. eingeengt. Ausbeute: 10,05 g (95 %)
¹³C-NMR (CDCl₃): 23,48; 23,66; 27,69; 28,87; 29,39; 30,61; 31,18; 31,33; 37,91 (C₄); 41,99 (N(CH₃)₂); 74,00 (CH); 113,70 (Cₐᵣₒₘ); 113,90 (Cₐᵣₒₘ); 115,51 (Cₐᵣₒₘ); 124,80 (Cₐᵣₒₘ); 128,90 (Cₐᵣₒₘ); 128,98 (Cₐᵣₒₘ); 139,48 (Cₐᵣₒₘ); 139,54 (Cₐᵣₒₘ); 159,89 (C=N-O); 161,13 (Cₐᵣₒₘ); 163,57(Cₐᵣₒₘ).

### 4-(Dimethylamino-(3-fluorphenyl)-methyll-cyclohexylamin (R¹ = Me, R² = Me, R³ = 3-Fluorphenyl) A3

Absolutes THF (400 ml) wurde unter Argon mit LiAlH₄ (2,83 g, 75 mmol) versetzt, auf 60 °C erwärmt und tropfenweise das Oxim (9,86 g, 37,3 mmol), gelöst in THF (90 ml), zugegeben. Nach 4-stündigem Rühren bei 60 °C wurde unter Eisbadkühlung (10 °C) Wasser (100 ml) zugetropft und über Kieselgur abgesaugt. Der Filterrückstand wurde mit THF gewaschen. Das THF i. Vak. entfernt, der Rückstand mit 5N NaOH auf pH 11 eingestellt und mit Ethylacetat (4 x 40 ml) extrahiert. Die organische Phase wurde über Natriumsulfat getrocknet, eingedampft und der Rückstand über eine Kieselgelsäule (300 g) mit Acetonitril/Methanol/0,5 M NH₄Cl (9:1:1) gereinigt.

Die einzelnen Fraktionen wurden in Wasser und DCM gelöst, mit Ammoniak alkalisch gestellt und die wässrige Phase zweimal mit DCM extrahiert.
Ausbeute: 6,81 g (73 %), Öl
¹³C-NMR (CDCl₃): 24,08; 24,69; 28,05; 29,84; 32,33; 32,37; 36,10; 36,48; 36,69; 37,95; 41,27 (C₄); 41,85 (N(CH₃)₂), 47,32; 50,81; 71,63; 74,81 (CH); 113,29 (Cₐᵣₒₘ); 115,43 (Cₐᵣₒₘ); 124,74 (Cₐᵣₒₘ); 128,58 (Cₐᵣₒₘ); 139,19 (Cₐᵣₒₘ); 139,99 (Cₐᵣₒₘ); 160,97 (Cₐᵣₒₘ); 163,41 (Cₐᵣₒₘ).

### Synthese des 4-(Dimethylamino-thiophen-2-yl-methyl)-cyclohexylamins (R¹ = Me, R² = Me, R³ = 2-Thiophen) A4

### 4-(Dimethylamino-thiophen-2-yl-methyl)-cyclohexanonoxim (R¹ = Me, R² = Me, R³ = 2-Thiophen)

Das Keton (9,49 g, 40 mmol) und Hydroxylamin Hydrochlorid (4,17 g, 60 mmol) wurden in absol. Ethanol (150 ml) gelöst, mit basischem lonenaustauscher Amberlyst A21 (28 g) versetzt und bei RT über Nacht gerührt. Der Ionenaustauscher wurde abfiltriert und mit Ethanol (2 x 50 ml) gewaschen. Die Lösung wurde eingeengt und der Rückstand mit 5N NaOH auf pH 11 eingestellt. Die wässrige Phase wurde mit Ethylacetat (3 x 50 ml) extrahiert, die organische Phase über Natriumsulfat getrocknet und i. Vak. eingeengt.
Ausbeute: 9,21 g (91 %)
Schmelzpunkt: 118-121 °C, gelbe Kristalle

### 4-(Dimethylamino-thiophen-2-yl-methyl)-cyclohexylamin (R¹ = Me, R² = Me, R³ = 2-Thiophen) A4

Absolutes THF (300 ml) wurde unter Argon mit LiAlH₄ (2,73 g, 72 mmol) versetzt, auf 60 °C erwärmt und tropfenweise das Oxim (9,08 g, 35,9 mmol), gelöst in THF (80 ml), zugegeben. Nach 4-stündigem Rühren bei 60 °C wurde unter Eisbadkühlung (10 °C) Wasser (80 ml) zugetropft und die Lösung über Kieselgur abgesaugt. Der Filterrückstand wurde mit THF gewaschen. Das THF i. Vak. entfernt, der Rückstand mit 5N NaOH auf pH 11 eingestellt und mit Ethylacetat (3 x 50 ml) extrahiert. Die organische Phase wurde über Natriumsulfat getrocknet, eingedampft und der Rückstand über eine Kieselgelsäule (300 g) mit Acetonitril/Methanol/0,5M NH₄Cl (8:2:1) gereinigt.

Die einzelnen Fraktionen wurden in Wasser und DCM gelöst, mit Ammoniak alkalisch gestellt und die wässrige Phase zweimal mit DCM extrahiert.
Gesamtausbeute: 5,66 g (66 %), Öl
¹³C-NMR (CDCl₃): 24,81; 24,96; 29,26; 29,76; 32,18; 32,22; 36,46; 36,58; 38,10; 39,99; 40,86; 41,20 (N(CH₃)₂); 47,66; 50,80; 64,27; 69,82; 123,43; 125,71; 125,75; 125,95; 126,07; 139,34; 139,79.

### Synthese des 4-(1-Dimethylamino-3-phenyl-propyl)-cyclohexylamins (R¹ = Me, R² = Me, R³ = Phenethyl) A5

### 4-(1-Dimethylamino-3-phenyl-propyl)-cyclohexanon oxim (R¹ = Me, R² = Me, R³ = Phenethyl)

Das Keton (10,2 g, 40 mmol) und Hydroxylamin Hydrochlorid (4,17 g, 60 mmol) wurden in absol. Ethanol (150 ml) gelöst, mit basischem Ionenaustauscher Amberlyst A21 (28 g) versetzt und bei RT über Nacht gerührt. Der Ionenaustauscher wurde abfiltriert, mit Ethanol (2 x 50 ml) gewaschen, die Lösung eingeengt und der Rückstand mit 5N NaOH auf pH 11 eingestellt. Die wässrige Phase wurde mit Ethylacetat (3 x 50 ml) extrahiert, die organische Phase über Natriumsulfat getrocknet und i. Vak. eingeengt.
Ausbeute: 10,8 g (100 %), Öl
¹³C-NMR (CDCl₃): 23,80; 23,96; 28,80; 29,27; 30,00; 31,21; 31,49; 31,58; 35,89 (C₄); 39,29; 41,26 (N(CH₃)₂); 67,24 (CH); 125,58 (Cₐᵣₒₘ); 128,13 (Cₐᵣₒₘ); 142,40 (Cₐᵣₒₘ); 159,99: 160,04 (C=N-O).

### 4-(1-Dimethylamino-3-phenyl-propyl)-cyclohexylamin (R¹ = Me, R² = Me, R³ = Phenethyl) A5

Absolutes THF (435 ml) wurde unter Argon mit LiAlH₄ (3,04 g, 82 mmol) versetzt, auf 60°C erwärmt und tropfenweise das Oxim (11,14 g, 40 mmol), gelöst in THF (90 ml), zugegeben. Nach 4-stündigem Rühren bei 60 °C wurde unter Eisbadkühlung (10 °C) Wasser (100 ml) zugetropft und über Kieselgur abgesaugt. Der Filterrückstand wurde mit THF gewaschen. Das THF i. Vak. entfernt, der Rückstand mit 5N NaOH auf pH 11 eingestellt und mit Ethylacetat (4 x 50 ml) extrahiert. Die organische Phase wurde über Natriumsulfat getrocknet, eingedampft und der Rückstand über eine Kieselgelsäule (300 g) mit Acetonitril/Methanol/0,5M NH₄Cl (9:1:1) und (9:4:1) gereinigt.

Die einzelnen Fraktionen wurden in Wasser und DCM gelöst, mit Ammoniak alkalisch gestellt und die wässrige Phase zweimal mit DCM extrahiert.
Ausbeute: 5,02 g (50 %), Öl
¹³C-NMR (CDCl₃): 24,70; 25,36; 29,22; 29,35; 30,42; 32,98; 35,46; 35,72; 36,95; 37,07; 38,89 (C₄); 39,32; 41,04; 41,26 (N(CH₃)₂); 46,98; 50,85; 66,01; 68,05 (CH); 125,49 (Cₐᵣₒₘ); 128,11 (Cₐᵣₒₘ); 128,14 (Cₐᵣₒₘ); 142,75(Cₐᵣₒₘ).

### Synthese des 4-(1-Morpholin-4-yl-3-phenyl-propyl)-cyclohexylamins (NR¹R² = Morpholin, R³ = Phenethyl) A6

### 4-(1-Morpholino-3-phenylpropyl)cyclohexanonoxim (NR¹R² = Morpholin, R³ = Phenethyl)

Zu einer Lösung des Ketons (40 mmol) und Hydroxylamin Hydrochlorid (4,17 g, 60 mmol) in absolutem Ethanol (200 ml) wurde Amberlyst A21 (40 g) hinzugegeben und für 20 h bei 25°C gerührt. Nach Filtration und waschen mit Ethanol (2 x 200 ml) wurde das Lösungsmittel unter Vakuum entfernt. Das Produkt wurde ohne weitere Aufreinigung in die nächste Stufe eingesetzt.

### 4-(1-Morpholin-4-yl-3-phenyl-propyl)-cyclohexylamin (NR¹R² = Morpholin, R³ = Phenethyl) A6

Zu einer Reaktionsmischung von Lithiumaluminiumhydrid (77 mmol) in absolutem THF (400 ml) gab man bei 60°C das Oxim (38.5 mmol) in THF (90 ml) tropfenweise hinzu und rührte für 4 h bei 60°C. Anschließend wurde bei 10°C Wasser (100 ml) langsam hinzugegeben und die Reaktionsmischung über Silica abfiltriert. Der Filterrückstand wurde mit Ethylacetat gewaschen und das Lösungsmittel der vereinigten organischen Phasen unter Vakuum entfernt. Das Produkt wurde über Säulenchromatographie (5-10% Methanol/Dichlormethan) aufgereinigt.

### Synthese des 4-(3-Phenyl-1-piperidin-1-yl-propyl)-cyclohexylamins (NR¹R² = Piperidin, R³ = Phenethyl) A7

### 4-(3-Phenyl-1-(piperidin-1-yl)propyl)cyclohexanonoxim (NR¹R² = Piperidin, R³ = Phenethyl)

Zu einer Lösung des Ketons (40 mmol) und Hydroxylamin Hydrochlorid (4,17 g, 60 mmol) in absolutem Ethanol (200 ml) wurde Amberlyst A21 (40 g) hinzugegeben und für 20 h bei 25°C gerührt. Nach Filtration und waschen mit Ethanol (2 x 200 ml) wurde das Lösungsmittel unter Vakuum entfernt. Das Produkt wurde ohne weitere Aufreinigung in die nächste Stufe eingesetzt.

### 4-(3-Phenyl-1-piperidin-1-yl-propyl)-cyclohexyamin (NR¹R² = Piperidin, R³ = Phenethyl) A7

Zu einer Reaktionsmischung von Lithiumaluminiumhydrid (77 mmol) in absolutem THF (400 ml) gab man bei 60°C das Oxim (38.5 mmol) in THF (90 ml) tropfenweise hinzu und rührte für 4 h bei 60°C. Anschließend wurde bei 10°C Wasser (100 ml) langsam hinzugegeben und die Reaktionsmischung über Silica abfiltriert. Der Filterrückstand wurde mit Ethylacetat gewaschen und das Lösungsmittel der vereinigten organischen Phasen unter Vakuum entfernt. Das Produkt wurde über Säulenchromatographie (5-10% Methanol/Dichlormethan) aufgereinigt.

### Synthese des 4-(Phenylpyrrolidin-1-yl-methyl)cyclohexylamin (NR¹R² = Pyrrolidin, R³ = Phenyl) A9

### 4-(Phenylpyrrolidin-1-yl-methyl)cyclohexanonoxim (NR¹R² = Pyrrolidin, R³ = Phenyl)

Eine Lösung von 4-(Phenylpyrrolidin-1-yl-methyl)cyclohexanon (1,55 g, 5,7 mmol) und Hydroxylamin-Hydrochlorid (594 mg, 8,55 mmol) in wasserfreiem Ethanol (30 ml) wurde mit basischem Ionenaustauscher Amberlyst A21 (4 g) versetzt und 3 d bei Raumtemperatur gerührt. Der Ionenaustauscher wurde abfiltriert und mit Ethanol (2 × 5 ml) gewaschen. Das Filtrat wurde i. Vak. eingeengt, der Rückstand mit Wasser (5 ml) versetzt, mit 5 *N* Natronlauge auf pH 11 eingestellt und mit Ethylacetat (3 × 15 ml) extrahiert. Die vereinigten organischen Phasen wurden mit Natriumsulfat getrocknet und i. Vak. eingeengt.
Ausbeute: 1,45 g (93 %), weißer Feststoff; Schmelzpunkt: 49-54 °C
¹H-NMR (DMSO-d₆): 0,50-0,90 (m, 2H); 1,54-2,48 (m, 14H); 3,02-3,18 (m, 1H); 7,10-7,37 (m, 5H); 10,07 (s, 1H).

### 4-(Phenylpyrrolidin-1-yl-methyl)cyclohexylamin (NR¹R² = Pyrrolidin, R³ = Phenyl) A9

Zu einer Aufschlämmung von Lithiumaluminiumhydrid (398 mg, 10,5 mmol) in absolutem Tetrahydrofuran (50 ml) wurde unter Argon bei 60 °C eine Lösung von - (Phenylpyrrolidin-1-yl-methyl)cyclohexanonoxim (1,43 g, 5,25 mmol) in absolutem Tetrahydrofuran (14 ml) getropft und 4,5 h bei dieser Temperatur gerührt, Nach dem Abkühlen wurden Wasser (2 ml) und 4 *N* Natronlauge (500 µl) zugetropft. Die Aufschlämmung wurde filtriert, das Filtrat mit Natriumsulfat getrocknet und i. Vak. eingeengt.
Ausbeute: 1,19 g (88 %), gelbes Öl
¹H-NMR (DMSO-d₆): 0,40-3,25 (m, 21H); 7,10-7,30 (m, 5H).
¹³C-NMR (DMSO-d₆): 21,7; 22,6; 22,7; 24,4; 25,7; 29,6; 32,3; 32,5; 36,2; 36,4; 45,9; 49,9; 50,7; 50,8; 126,4; 126,5; 127,3; 127,4; 128,9; 129,0; 138,7, 139,4.

Es handelt sich um ein *cis*/*trans*-Isomerengemisch.

### Synthese des 4-(Phenylpiperidin-1-yl-methyl)cyclohexylamin (NR¹R² = Piperidin, R³ = Phenyl) A10

### 4-(Phenylpiperidin-1-yl-methyl)cyclohexanonoxim (NR¹R² = Piperidin, R³ = Phenyl)

Eine Lösung von 4-(Phenylpiperidin-1-yl-methyl)cyclohexanon (1.85 g, 6.8 mmol) und Hydroxylamin-Hydrochlorid (709 mg, 10.2 mmol) in wasserfreiem Ethanol (40 ml) wurde mit basischem Ionenaustauscher Amberlyst A21 (4.80 g) versetzt und 3 d bei Raumtemperatur gerührt. Der Ionenaustauscher wurde abfiltriert und mit Ethanol (2 × 10 ml) gewaschen. Das Filtrat wurde i. Vak. eingeengt, der Rückstand mit Wasser (15 ml) versetzt, mit 5 *N* Natronlauge auf pH 11 eingestellt und mit Ethylacetat (3 × 25 ml) extrahiert. Die vereinigten organischen Phasen wurden mit Natriumsulfat getrocknet und i. Vak. eingeengt.
Ausbeute: 1.70 g (87 %), weißer Feststoff; Schmelzpunkt: 131-137 °C
¹H-NMR (DMSO-d₆): 0.70-2.40 (m, 18H); 2.82-3.17 (m, 1H); 3.18 (d, 1H, J = 9.8 Hz); 7.10-7.37 (m, 5H); 10.08 und 10.11 (2 s, 1H).

Es handelt sich um ein E/Z-Isomerengemisch.

### 4-(Phenylpiperidin-1-yl-methyl)cyclohexylamin (NR¹R² = Piperidin, R³ = Phenyl) A10

Zu einer Aufschlämmung von Lithiumaluminiumhydrid (425 mg, 11.2 mmol) in absolutem Tetrahydrofuran (50 ml) wurde unter Argon bei 60 °C eine Lösung von 4-(Phenylpiperidin-1-yl-methyl)cyclohexanonoxim (1.60 g, 5.6 mmol) in absolutem Tetrahydrofuran (20 ml) getropft und 4 h bei dieser Temperatur gerührt. Nach dem Abkühlen wurden Wasser (2 ml) und 4 *N* Natronlauge (0.5 ml) zugetropft. Die Aufschlämmung wurde filtriert, das Filtrat mit Natriumsulfat getrocknet und i. Vak. eingeengt.
Ausbeute: 1.50 g (98 %), gelbliches Öl
¹H-NMR (DMSO-d₆): 0.60-2.23 (m, 20H); 2.23-2.42 (m, 0.5H); 2.73-2.84 (m, 0.5H); 3.05 (d, 0.5H, J = 10.7 Hz); 3.35 (d, 0.5H, J = 10.7 Hz); 3.56-3.63 (m, 1H) 7.00-7.40 (m, 5H).
Es handelt sich um ein *cis*/*trans*-Isomerengemisch.

### Synthese des 4-(Morpholin-4-yl-phenylmethyl)cyclohexylamins (NR¹R² = Morpholin, R³ = Phenyl) A11

### 4-(Morpholin-4-yl-phenylmethyl)cyclohexanonoxim (NR¹R² = Morpholin, R³ = Phenyl)

Eine Lösung von 4-(Morpholin-4-yl-phenylmethyl)cyctohexanon (1,20 g, 4,4 mmol) und Hydroxylamin-Hydrochlorid (459 mg, 6,6 mmol) in wasserfreiem Ethanol (30 ml) wurde mit basischem Ionenaustauscher Amberlyst A21 (3,1 g) versetzt und 20 h bei Raumtemperatur gerührt. Der Ionenaustauscher wurde abfiltriert und mit Ethanol gewaschen. Das Filtrat wurde i. Vak. eingeengt, der Rückstand mit Wasser (10 ml) versetzt, mit 5 *N* Natronlauge auf pH 11 eingestellt und mit Ethylacetat (3 × 20 ml) extrahiert. Die vereinigten organischen Phasen wurden mit Natriumsulfat getrocknet und i. Vak. eingeengt.
Ausbeute: 1,20 g (95 %), weißer Feststoff
Schmelzpunkt: 82-87 °C.
¹H-NMR (DMSO-d₆): 0,66-2,50 (m, 12H); 2,90-3,14 (m, 1H); 3.18-3,48 (m, 1H); 3,48-3,66 (m, 4H); 7,15-7,38 (m, 5H); 10,09 und 10,11 (2 s, 1H).
Es handelt sich um ein E/Z-Isomerengemisch.

### 4-(Morpholin-4-yl-phenylmethyl)cyclohexylamin (NR¹R² = Morpholin, R³ = Phenyl) A11

Zu einer Aufschlämmung von Lithiumaluminiumhydrid (316 mg, 8.32 mmol) in absolutem Tetrahydrofuran (40 ml) wurde unter Argon bei 60 °C eine Lösung von 4-(Morpholin-4-yl-phenylmethyl)cyclohexanonoxim (1.20 g, 4.16 mmol) in absolutem Tetrahydrofuran (12 ml) getropft und 4 h bei dieser Temperatur gerührt. Nach dem Abkühlen wurden Wasser (1.5 ml) und 4 *N* Natronlauge (0.4 ml) zugetropft. Die Aufschlämmung wurde filtriert, das Filtrat mit Natriumsulfat getrocknet und i. Vak eingeengt.
Ausbeute: 1.12 g (98 %), gelbliches Öl
¹H-NMR (DMSO-d₆): 0.60-3.65 (m, 21H); 7.10-7.40 (m, 5H).
Es handelt sich um ein *cis*/*trans*-Isomerengemisch.

Das 4-Methylpiparazin-Derivat wurde über das Benzotriazolaminal nach folgender Syntheseroute hergestellt:

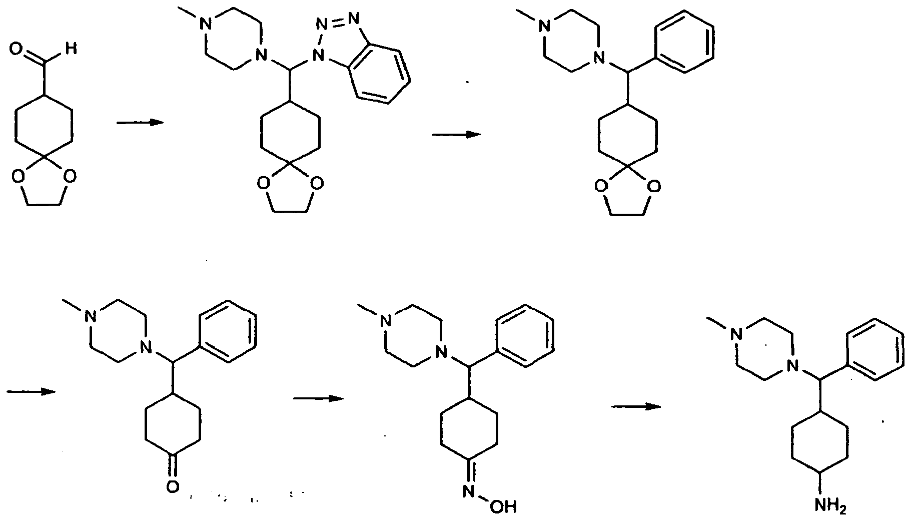

### 11-(1-(1,4-Dioxa-spiro[4.5]dec-8-yl)(4-methylpiperazin-1-yl)methyl)-1H-benzo[d][1,2,3]triazol

Der Aldehyd (23.4 mmol), N-Methylpiperazin (23.4 mmol) und 1H-Benzotriazol (23.4 mmol) wurden in Benzol (60 ml) für 16 h unter Rückfluß erhitzt und das entstehende Reaktionswasser über einen Wasserabscheider abgeführt. Das Benzol wurde unter Vakuum entfernt und der Rückstand unmittelbar in die nächste Stufe eingesetzt.

### 4-Methyl-[1-(1,4-Dioxa-spiro[4.5]dec-8-yl)-3-phenyl-propyl]-piparazin

Zu einer Lösung von Phenylmagnesiumchlorid (47.1 mmol) in THF wurde eine Lösung des Benzotriazol-Addukts (23.5 mmol) in THF tropfenweise hinzugegeben und für 16 h bei 25°C gerührt. Die Reaktionslösung wurde auf 0°C abgekühlt und mit wässriger gesättigter NH₄Cl-Lösung versetzt und anschließend mit Ethylacetat (2 x 300 ml) extrahiert. Die organische Phase wurde mit Wasser und wässriger gesättigter NaCl-Lösung gewaschen. Nach Trocknung der organischen Phase über Na₂SO₄ und Filtration wurde das Lösungsmittel unter Vakuum entfernt. Das Produkt wurde über Säulenchromatographie (2-5% Methanol/Dichlormethan) aufgereinigt.

### 4-((4-Methylpiperazin-1-yl)(phenyl)methyl)cyclohexanon

Zum Grignard-Addukt (105 mmol) wurde langsam konz. HCl und Wasser (1.1, 88 ml) bei 0°C hinzugegeben und für 20 h bei 25°C gerührt. Anschließend wurde die Reaktionslösung mit Ethylacetat (2 x 100 ml) extrahiert. Nach Zugabe von 5N Natronlauge, zur Einstellung eines basischen pH-Wertes, wurde mit Dichlormthan (3 x 100 ml) extrahiert. Die vereinigten organischen Phasen wurden über Na₂SO₄ getrocknet und abfiltriert. Das Lösungsmittel wurde unter Vakuum entfernt und das Produkt ohne weitere Aufreinigung in der nächsten Stufe verwendet.

### 4-((4-Methylpiperazin-1-yl)(phenyl)methyl)cyclohexanonoxim

Zu einer Lösung des Ketons (40 mmol) und Hydroxylamin Hydrochlorid (4,17 g, 60 mmol) in absolutem Ethanol (200 ml) wurde Amberlyst A21 (40 g) hinzugegeben und für 20 h bei 25°C gerührt. Nach Filtration und waschen mit Ethanol (2 x 200 ml) wurde das Lösungsmittel unter Vakuum entfernt. Das Produkt wurde ohne weitere Aufreinigung in die nächste Stufe eingesetzt.

### 4-[(4-Methyl-piperazin-1-yl)-phenyl-methyl]-cyclohexylamin A8

Zu einer Reaktionsmischung von Lithiumaluminiumhydrid (77 mmol) in absolutem THF (400 ml) gab man bei 60°C das Oxim (38.5 mmol) in THF (90 ml) tropfenweise hinzu und rührte für 4 h bei 60°C. Anschließend wurde bei 10°C Wasser (100 ml) langsam hinzugegeben und die Reaktionsmischung über Silica abfiltriert. Der Filterrückstand wurde mit Ethylacetat gewaschen und das Lösungsmittel der vereinigten organischen Phasen unter Vakuum entfernt. Das Produkt wurde über Säulenchromatographie (5-10% Methanol/Dichlormethan) aufgereinigt.

### Synthese der Aminomethylcyclohexane

Die Aminomethylcyclohexane wurden durch dreistufige Reaktionen aus den entsprechend substituierten Cyclohexanonen über die Stufe der Cyclohexylaldehyde durch Umstzung mit Hydroxylamin Hydrochlorid und anschließender Spaltung mit Lithiumaluminiumhydrid dargestellt.

### 4-(Dimethylamino-phenyl-methyl)-cyclohexan-carbaldehyd (R¹ = Me, R² = Me, R³ = Phenyl)

(Methoxymethyl)triphenylphosphoniumchlorid (31,5 g, 0,092 mol) wurde in absol. THF (150 ml) unter Argon suspendiert, bei 0 °C tropfenweise mit Kalium-*tert*-butylat (10, 38 g, 0,092 mol), gelöst in absol. THF (100 ml), versetzt und anschließend 15 min bei 0 °C nachgerührt.

Bei RT wurde dann das Keton (14,2 g, 0,061 mol), gelöst in absol. THF (100 ml), zur obigen Lösung zugetropft und über Nacht bei RT gerührt. Unter Eiswasserkühlung wurde tropfenweise mit Wasser (50 ml) und 6N HCl (150 ml) hydrolysiert. Nach 1 h Rühren bei RT wurde mit Diethylether (10 x 50 ml) extrahiert, die wässrige Phase mit 5N NaOH auf pH 11 gebracht, mit Ethylacetat (3 x 50 ml) ausgeschüttelt, über Natriumsulfat getrocknet und i. Vak. eingeengt. Das Rohprodukt wurde über eine Kieselgelsäule (300 g) mit Ethylacetat/Cyclohexan (1:1) gereinigt.
Ausbeute: 12.2 g (82 %)
¹³C-NMR (CDCl₃): 24,01; 24,22; 25,90; 26,06; 26,40; 27,33; 28,21; 29,92; 37,00; 38,19 (C₄); 41,51; 41,98; (N(CH₃)₂); 47,45; 50,60; 73,37; 75,24 (CH); 126,72 (Cₐᵣₒₘ); 126,76 (Cₐᵣₒₘ); 127,48 (Cₐᵣₒₘ); 129,13 (Cₐᵣₒₘ); 136,14 (Cₐᵣₒₘ); 136,79(Cₐᵣₒₘ); 204,22; 205.05 (CHO).

### 4-(Dimethylamino-phenyl-methyl)-cyclohexan-carbaldehyd-oxim (R¹ = Me, R² = Me, R³ = Phenyl)

Der Carbaldehyd (7,36 g, 30 mmol) und Hydroxylamin Hydrochlorid (3,12 g, 45 mmol) wurden in absol. Ethanol (100 ml) gelöst, mit.basischem Ionenaustauscher Amberlyst A21 (21 g) versetzt und bei RT über Nacht gerührt. Der Ionenaustauscher wurde abfiltriert und mit Ethanol (2 x 50 ml) gewaschen. Die Lösung wurde eingeengt und der Rückstand mit 5N NaOH auf pH 11 eingestellt. Die wässrige Phase wurde mit Ethylacetat (3 x 50 ml) extrahiert, die organische Phase über Natriumsulfat getrocknet und i. Vak. eingeengt.
Ausbeute: 7,81 g (100 %)
¹³C-NMR (CDCl₃): 25,83; 26,34; 27,10; 27,55; 28,25; 29,41; 30,12; 30,32; 34,20;, 36,45; 36,74; 37,00; 38,19 (C₄); 41,37; 41,03; (N(CH₃)₂); 72,28; 75,59 (CH); 126,77 (Cₐᵣₒₘ); 127,50 (Cₐᵣₒₘ); 129,22 (Cₐᵣₒₘ); 136,14 (Cₐᵣₒₘ); 136,94 (Cₐᵣₒₘ); 137,05 (Cₐᵣₒₘ); 154,84; 155,55; 156,35.

### [(4-Aminomethyl-cyclohexyl)-phenyl-methyl]-dimethylamin (R¹ = Me, R² = Me, R³ = Phenyl) A12

Absolutes THF (300 ml) wurde unter Argon mit LiAlH₄ (2,27 g, 60 mmol) versetzt, auf 60 °C erwärmt und tropfenweise das Oxim (7,81 g, 30 mmol), gelöst in THF (60 ml), zugegeben. Nach 4-stündigem Rühren bei 60 °C wurde unter Eisbadkühlung (10 °C) Wasser (70 ml) zugetropft und die Reaktionslösung über Kieselgur abgesaugt. Der Filterrückstand wurde mit THF gewaschen. Die vereinigten organischen Phasen wurden i. Vak. eingeengt, der Rückstand mit 5N NaOH auf pH 11 eingestellt und mit Ethylacetat (4 x 40 ml) extrahiert. Die organische Phase wurde über Natriumsulfat getrocknet und eingeengt.
Ausbeute: 6,4 g (87 %), Öl
¹³C-NMR (CDCl₃): 25,53; 26,03; 26,64; 26,68; 29,06; 30,37; 30,51; 30,67; 30,74; 36,01; 38,83; 38,93; (C₄); 41,50; 41,94; (N(CH₃)₂); 72,28; 75,59 (CH); 126,77 (Cₐᵣₒₘ); 127,50 (Cₐᵣₒₘ); 129,22 (Cₐᵣₒₘ); 136,14 (Cₐᵣₒₘ); 136,94 (Cₐᵣₒₘ); 137,05 (Cₐᵣₒₘ); 154,84; 155,55; 156,35.

### 4-[Dimethylamino-(4-fluorphenyl)-methyl]-cyclohexan-carbaldehyd (R¹ = Me, R² = Me, R³ = 4-Fluorphenyl)

(Methoxymethyl)triphenylphosphoniumchlorid (25,7 g, 75 mmol) wurde in absol. THF (100 ml) unter Argon suspendiert, bei 0 °C tropfenweise mit Kalium-tert-butylat (8,42 g, 75 mmol), gelöst in absol. THF (70 ml) versetzt und anschließend 15 min bei 0 °C nachgerührt.

Bei RT wurde dann das Keton (12,44 g, 50 mmol), gelöst in absol. THF (75 ml), zur obigen Lösung zugetropft und über Nacht bei RT gerührt. Unter Eiswasserkühlung wurde tropfenweise mit Wasser (38 ml) und 6N HCl (112 ml) hydrolysiert. Nach 1 h Rühren bei RT wurde mit Diethylether (10 x 50 ml) extrahiert, die wässrige Phase mit 5N NaOH auf pH 11 gebracht, mit Ethylacetat (3 x 50 ml) ausgeschüttelt, über Natriumsulfat getrocknet und i. Vak. eingeengt. Das Rohprodukt wurde durch Flashchromatographie mit Ethylacetat/Cyclohexan (1:1) gereinigt.
Ausbeute: 9,13g (70 %).
¹H-NMR (DMSO, 600 MHz, ausgesuchte Signale): δ = 1,97 (s, 3 H, Unterschussdiastereomer); 1,99 (s, 3 H, Überschussdiastereomer); 3,08 (d, 1 H, *J* = 9.06 Hz, Überschussdiastereomer); 3,14 (d, 1 H, *J* = 9,82 Hz, Unterschussdiastereomer); 9,53 (s, 1 H, Überschussdiastereomer); 9,56 (s, 1 H, Unterschussdiastereomer).
¹³C-NMR (CDCl₃, beide diastereomere): δ = 23,97; 24,21; 25,85; 26,02; 26,17; 27,35; 28,00; 29,90; 37,26; 38,34 ; 41,50; 41,95; 47,36; 50,55; 72,75; 75,84; 114,25; 114,45; 130,33; 130,40; 132,61; 160,41; 162,83; 204,10; 204,93.

### 4-[Dimethylamino-(4-fluorphenyl)-methyl]-cyclohexanecarbaldehydoxim (R¹ = Me, R² = Me, R³ = 4-Fluorphenyl)

Der Aldehyd (6,50 g, 25 mmol) und Hydroxylamin Hydrochlorid (2,6 g, 37,5 mmol) wurden in absol. Ethanol (80 ml) gelöst, mit basischem Ionenaustauscher Amberlyst A21 (16,5 g) versetzt und bei RT über Nacht gerührt. Der Ionenaustauscher wurde abfiltriert und mit Ethanol (2 x 50 ml) gewaschen. Die Lösung wurde eingeengt und der Rückstand mit 5N NaOH auf pH 11 eingestellt. Die wässrige Phase wurde mit Ethylacetat (3 x 50 ml) extrahiert, die organischen Phase über Natriumsulfat getrocknet und i. Vak. eingeengt.
Ausbeute: 6,9 g (99 %)

### [(4-Aminomethyl-cyclohexyl)-(4-fluorphenyl)-methyl]-dimethylamin (R¹ = Me, R² = Me, R³ = 4-Fluorphenyl) A13

Absolutes THF (360 ml) wurde unter Argon mit LiAlH₄ (1,9 g, 50 mmol) versetzt, auf 60 °C erwärmt und tropfenweise das Oxim (6,9 g, 25 mmol), gelöst in THF (60 ml), zugegeben. Nach 4-stündigem Rühren bei 60 °C wurde unter Eisbadkühlung (10 °C) Wasser (93 ml) zugetropft und die Reaktionslösung über Kieselgur abgesaugt. Der Filterrückstand wurde mit THF gewaschen. Die vereinigten organischen Phasen wurden i. Vak. eingeengt, der Rückstand mit 5N NaOH auf pH 11 eingestellt und dreimal mit Ethylacetat (je 100 ml) extrahiert. Die organische Phase wurde über Natriumsulfat getrocknet und eingeengt.
Ausbeute: 5,4 g (82 %), Öl
¹³C-NMR (CDCl₃): 25,25; 25,93; 26,60; 28,75; 30,30; 30,40; 30,67; 36,20; 38,78; 38,93; (C₄); 41,24; 41,43 (N(CH₃)₂); 48,71; 70,62; 74;69 (CH); 113,97 (Cₐᵣₒₘ); 114,04 (Cₐᵣₒₘ); 130,24 (Cₐᵣₒₘ); 130,31 (Cₐᵣₒₘ); 132,94 (Cₐᵣₒₘ); 160,19; 162,62; (Cₐᵣₒₘ).

### 4-[Dimethylamino-(3-fluorphenyl)-methyl]-cyclohexan-carbaldehyd (R¹ = Me, R² = Me, R³ = 3-Fluorphenyl)

(Methoxymethyl)triphenylphosphoniumchlorid (15,42 g, 45 mmol) wurde in absol. THF (50 ml) unter Argon suspendiert, bei 0 °C tropfenweise mit Kalium-*tert*-butylat (5,05 g, 45 mmol), gelöst in absol. THF (50 ml), versetzt und anschließend 15 min bei 0 °C nachgerührt.

Bei RT wurde dann das Keton (7,48 g, 0.30 mmol), gelöst in absol.THF (50 ml), zur obigen Lösung zugetropft und über Nacht bei RT gerührt. Unter Eiswasserkühlung wurde tropfenweise mit Wasser (25 ml) und 6N HCl (75 ml) hydrolysiert. Nach 1 h Rühren bei RT wurde mit Diethylether (10 x 50 ml) extrahiert, die wässrige Phase mit 5N NaOH auf pH 11 gebracht, mit Ethylacetat (3 x 50 ml) extrahiert, über Natriumsulfat getrocknet und i. Vak. eingeengt. Das Rohprodukt wurde über durch Flashchromatographie mit Ethylacetat/Cyclohexan (1:1) gereinigt. Ausbeute: 6.55 g (83 %); Schmelzpunkt: 40-43 °C.
¹H-NMR (DMSO, 600 MHz, ausgesuchte Signale): δ = 1,99 (s, 3 H); 2,01 (s, 3 H); 3,10 (d, 1 H, *J* = 9,06 Hz); 3,18 (d, 1 H, *J* = 9,82 Hz); 9,54 (s, 1 H); 9,56 (s, 1 H). ¹³C-NMR (CDCl₃): 23,93; 24,12; 25,79; 25,95; 26,19; 27,19; 27,99; 29,77; 37,05; 38,16; 41,45; 41,91; 47,30; 50,49; 71,50; 74,78; 113,50; 115,37; 124,78; 128,24; 130,59; 131,24; 131,67; 139,14; 139,76; 160,06; 163,50; 204,01; 204,85.

### 4-[Dimethylamino-(3-fluorphenyl)-methyl]-cyclohexan-carbaldehyd-oxim (R¹ = Me, R² = Me, R³ = 3-Fluorphenyl)

Der Carbaldehyd (6,32 g, 24 mmol) und Hydroxylamin Hydrochlorid (2,5 g, 36 mmol) wurden in absol. Ethanol (90 ml) gelöst, mit basischem Ionenaustauscher Amberlyst A21 (17 g) versetzt und 3,5 h bei RT gerührt. Der Ionenaustauscher wurde abfiltriert und mit Ethanol (2 x 50 ml) gewaschen. Die Lösung wurde eingeengt und der Rückstand mit 5N NaOH auf pH 11 eingestellt. Die wässrige Phase wurde mit Ethylacetat (3 x 50 ml) extrahiert, die organische Phase über Natriumsulfat getrocknet und im Vakuum eingeengt.
Ausbeute: 6,68 g (100 %)
¹³C-NMR (CDCl₃): 25,59; 26,21; 27,38; 28,02; 28,36; 29,27; 29,45; 30,00; 34,14; 35,58; 36,56; 38,19 (C₄); 41, 33; 41, 99; (N(CH₃)₂); 172,02; 75,05; 75,19 (CH); 113,55 (Cₐᵣₒₘ); 115,62 (Cₐᵣₒₘ): 124,88 (Cₐᵣₒₘ); 128,78 (Cₐᵣₒₘ); 128,86 (Cₐᵣₒₘ); 139,84 (Cₐᵣₒₘ); 139,90 (Cₐᵣₒₘ); 154, 38; 155,13; 161,10 (Cₐᵣₒₘ); 163, 54 (Cₐᵣₒₘ).

### [(4-Aminomethyl-cyclohexyl)-(3-fluorphenyl)-methyl]-dimethylamin (R¹ = Me, R² = Me, R³ = 3-Fluorphenyl) A14

Absolutes THF (300 ml) wurde unter Argon mit LiAlH₄ (1,82 g, 48 mmol) versetzt, auf 60 °C erwärmt und tropfenweise das Oxim (6,68 g, 24 mmol), gelöst in THF (60 ml), dazu gegeben. Nach 4-stündigem Rühren bei 60 °C wurde unter Eisbadkühlung (10 °C) Wasser (70 ml) zugetropft und die Reaktionslösung über Kieselgur filtriert. Der Filterrückstand wurde mit THF gewaschen, die organischen Phasen vereinigt, das THF i. Vak. entfernt, der Rückstand mit 5N NaOH auf pH 11 eingestellt und mit Ethylacetat (4 x 40 ml) extrahiert. Die organische Phase wurde über Natriumsulfat getrocknet und eingeengt.
Ausbeute: 5,7 g (90 %), Öl
¹³C-NMR (CDCl₃): 25,38; 25,93; 26,44; 28,89; 30,36; 30,45; 30,65; 36,10; 38,87; (C₄); 41,33; 41,49; 41,93 (N(CH₃)₂); 71,05; 75,11 (CH); 113,94 (Cₐᵣₒₘ); 115,53 (Cₐᵣₒₘ); 124,86 (Cₐᵣₒₘ); 128,59 (Cₐᵣₒₘ); 128.67 (Cₐᵣₒₘ); 140,14 (Cₐᵣₒₘ); 141,21 (Cₐᵣₒₘ); 161,03 (Cₐᵣₒₘ); 163,46 (Cₐᵣₒₘ).

### 4-[(4-Chlor-phenyl)-dimethylamino-methyl]-cyclohexancarbaldehyd (R¹ = Me, R² = Me, R³ = 4-Chlorphenyl)

(Methoxymethyl)triphenylphosphoniumchlorid (68,55 g, 200 mmol) wurde in absol. THF (200 ml) unter Argon suspendiert, bei 0 °C tropfenweise mit Kalium-*tert*-butylat (22,44 g, 200 mmol), gelöst in absol. THF (300 ml), versetzt und anschließend 15 min bei 0 °C nachgerührt.

Bei RT wurde dann das Keton (38 g, 143 mmol), gelöst in absol. THF (200 ml), zur obigen Lösung getropft und über Nacht bei RT gerührt. Unter Eiswasserkühlung wurde tropfenweise mit Wasser (150 ml) und 6N HCl (450 ml) hydrolysiert. Nach 1 h Rühren bei RT wurde mit Diethylether (10 x 100 ml) extrahiert, die wässrige Phase mit 5N NaOH auf pH 11 gebracht, mit Ethylacetat (3 x 100 ml) ausgeschüttelt, über Natriumsulfat getrocknet und i. Vak. eingeengt. Das Rohprodukt wurde über zwei Kieselgelsäulen (400 g) mit Ethylacetat/Cyclohexan (1:1) gereinigt.
Ausbeute: 32.17 g (80 %).
¹H-NMR (DMSO, 600 MHz, ausgesuchte Signale): δ = 1,97 (s, 3 H); 1,99 (s, 3 H); 3,07 (d, 1 H, *J* = 9,07 Hz); 3,14 (d, 1 H, *J* = 9,82 Hz); 9,53 (s, 1 H,; 9,55 (s, 1 H). ¹³C-NMR (CDCl₃): δ = 23,92; 24,16; 25,80; 25,97; 26,13; 27,25; 27,90; 29,81; 37,08; 38,19; 41,47; 41,96; 47,29; 50,48; 72,81; 74,54 127,65 130,28; 132,40; 134,78; 135,43; 203.98; 204.82.

### 4-[(4-Chlorphenyl)-dimethylamino-methyl]-cyclohexanecarbaldehydoxim (R¹ = Me, R² = Me, R³ = 4-Chlorphenyl)

Der Carbaldehyd (7,55 g, 27 mmol) und Hydroxylamin Hydrochlorid (2,81 g, 40 mmol) wurden in absol. Ethanol (100 ml) gelöst, mit basischem Ionenaustauscher Amberlyst A21 (19 g) versetzt und 3,5 h bei RT gerührt. Der Ionenaustauscher wurde abfiltriert und mit Ethanol (2 x 50 ml) gewaschen. Die Lösung wurde eingeengt und der Rückstand mit 5N NaOH auf pH 11 eingestellt. Die wässrige Phase wurde mit Ethylacetat (3 x 50 ml) extrahiert, die organische Phase über Natriumsulfat getrocknet und im Vakuum eingeengt.
Ausbeute: 7,57 g (96 %)

### [(4-Aminomethyl-cyclohexyl)-(4-chlorphenyl)-methyl]-dimethylamin (R¹ = Me, R² = Me, R³ = 4-Chlorphenyl) A15

Absolutes THF (300 ml) wurde unter Argon mit LiAlH₄ (1,89 g, 50 mmol) versetzt, auf 60 °C erwärmt und tropfenweise das Oxim (7,5 g, 25 mmol), gelöst in THF (60 ml), dazu gegeben. Nach 4-stündigem Rühren bei 60 °C wurde unter Eisbadkühlung (10 °C) Wasser (70 ml) zugetropft und die Reaktionslösung über Kieselgur filtriert. Der Filterrückstand wurde mit THF gewaschen, die organischen Phasen gereinigt, das THF i. Vak. entfernt, der Rückstand mit 5N NaOH auf pH 11 eingestellt und mit Ethylacetat (4 x 40 ml) extrahiert. Die organische Phase wurde über Natriumsulfat getrocknet und eingeengt.
Ausbeute: 6,3 g (90 %), Öl
¹³C-NMR (CDCl₃): 25,22; 25,87; 26,58; 28,70; 30,36; 30,53; 30,59; 36,02; 38,76 (C₄); 41,29; 41,39; 41,91 (N(CH₃)₂); 45,64; 48,72; 70,72; 74,77 (CH); 127,46 (Cₐᵣₒₘ); 127,52 (Cₐᵣₒₘ); 130,27 (Cₐᵣₒₘ); 132,11 (Cₐᵣₒₘ); 132,15 (Cₐᵣₒₘ); 134,80 (Cₐᵣₒₘ); 135,72 (Cₐᵣₒₘ).

### 4-(Dimethylamino-thiophen-2-yl-methyl)-cyclohexancarbaldehyd (R¹ = Me, R² = Me, R³ = 2-Thienyl)

(Methoxymethyl)triphenylphosphoniumchlorid (20,56 g, 60 mmol) wurde in absol. THF (70 ml) unter Argon suspendiert, bei 0 °C tropfenweise mit Kalium-*tert*-butylat (6,73 g, 60 mmol), gelöst in absol. THF (70 ml), versetzt und anschließend 15 min bei 0 °C nachgerührt Bei RT wurde dann das Keton (9,4 g, 40 mmol), gelöst in absol. THF (70 ml), zur obigen Lösung zugetropft und über Nacht bei RT gerührt. Unter Eiswasserkühlung wurde tropfenweise mit Wasser (60 ml) und 6 N HCl (180 ml) hydrolysiert. Nach 1 h Rühren bei RT wurde mit Diethylether (5 x 50 ml) extrahiert, die wässrige Phase mit 5 N NaOH auf pH 11 gebracht, mit Ethylacetat (3 x 50 ml) ausgeschüttelt, über Natriumsulfat getrocknet und i. Vak. eingeengt. Das Rohprodukt wurde durch Flashchromatographie mit Ethylacetat/Cyclohexan (1:1) gereinigt. Ausbeute: 7,66 g (77 %).
¹H-NMR (DMSO, 600 MHz, ausgesuchte Signale): δ = 2,03 (s, 3 H); 2,05 (s, 3 H); 3,44 (d, 1 H, *J* = 9,82 Hz); 3,52 (d, 1 H, *J* = 10,58 Hz); 9,54 (s, 1 H); 9,58 (s, 1 H).
¹³C-NMR (CDCl₃): δ = 23,74; 23,83; 25,80; 25,84; 26,98; 27,09; 29,15; 29,68; 39,13; 40,20; 40,98; 41,29 (N(CH₃)₂); 47,48; 50,49; 67,81; 69,79; 123,61; 123,70; 125,89; 126,20; 126,24; 139,14; 139,48; 204,07; 204,82.

**4-(Dimethylamino-thiophen-2-yl-methyl)-cyclohexanecarbaldehydoxim** (R¹ = Me, R² = Me, R³ = 2-Thiophen)

Der Carbaldehyd (7,54 g, 30 mmol) und Hydroxylamin Hydrochlorid (3,12 g, 45 mmol) wurden in absol. Ethanol (100 ml) gelöst, mit basischem Ionenaustauscher Amberlyst A21 (21 g) versetzt und bei RT über Nacht gerührt. Der Ionenaustauscher wurde abfiltriert und mit Ethanol (2 x 50 ml) gewaschen. Die Lösung wurde eingeengt und der Rückstand mit 5N NaOH auf pH 11 eingestellt. Die wässrige Phase wurde mit Ethylacetat (3 x 50 ml) extrahiert, die organische Phase über Natriumsulfat getrocknet und im Vakuum eingeengt.
Ausbeute: 7,99 g (100 %)

### [(4-Aminomethyl-cyclohexyl)-thiophen-2-yl-methyl]-dimethylamin (R¹ = Me, R² = Me, R³ = 2-Thiophen) A16

Absolutes THF (300 ml) wurde unter Argon mit LiAlH₄ (2,27 g, 60 mmol) versetzt, auf 60 °C erwärmt und tropfenweise das Oxim (7,99 g, 30 mmol), gelöst in THF (60 ml), dazu gegeben. Nach 4-stündigem Rühren bei 60 °C wurde unter Eisbadkühlung (10 °C) Wasser (70 ml) zugetropft und die Reaktionslösung über Kieselgur filtriert. Der Filterrückstand wurde mit THF gewaschen, die organischen Phasen vereinigt, das THF i. Vak. entfernt, der Rückstand mit 5N NaOH auf pH 11 eingestellt und mit Ethylacetat (3 x 50 ml) extrahiert. Die organische Phase wurde über Natriumsulfat getrocknet und eingeengt.
Ausbeute: 6,72 g (89 %), Öl
¹³C-NMR (CDCl₃): 25,93; 26,11; 26,24; 26,30; 29,97, 30,34; 30,42; 38,03; 40,65; 40,82; 41,18; 41,34 (N(CH₃)₂); 46,19; 48,67; 65,58; 70,06; 123,61; 125,88; 126,23; 140,08.

### 4-(1-Dimethylamino-3-phenyl-propyl)-cyclohexan-carbaldehyd (R¹ = Me, R² = Me, R³ = Phenethyl)

(Methoxymethyl)triphenylphosphoniumchlorid (20,56 g, 60 mmol) wurde in absol. THF (85 ml) unter Argon suspendiert, bei 0 °C tropfenweise mit Kalium-*tert*-butylat (6,73 g, 60 mmol), gelöst in absol. THF (70 ml) versetzt und anschließend 15 min bei 0 °C nachgerührt.

Bei RT wurde dann das Keton (10,2 g, 40 mmol), gelöst in absol. THF (60 ml), zur obigen Lösung getropft und über Nacht bei RT gerührt. Unter Eiswasserkühlung wurde tropfenweise mit Wasser (35 ml) und 6N HCl (90 ml) hydrolysiert. Nach 1 h Rühren bei RT wurde mit Diethylether (10 x 50 ml) extrahiert, die wässrige Phase mit 5N NaOH auf pH 11 gebracht, mit Ethylacetat (3 x 50 ml) extrahiert, über Natriumsulfat getrocknet und i. Vak. eingeengt. Das Rohprodukt wurde durch Flashchromatographie mit Ethylacetat / Cyclohexan (1:1) gereinigt.
Ausbeute: 6.73 g (63 %).
¹H-NMR (DMSO, 600 MHz, ausgesuchte Signale): δ = 2,18 (s, 3 H); 2,20 (s, 3 H); 9,54 (s, 1 H); 9,61 (s, 1 H).
¹³C-NMR (CDCl₃): δ = 24,35; 24,49; 26,00; 26,09; 26,85; 27,79; 29,07; 29,13; 35,27; 39,02; 40,98; 41,19; 46,99; 50,33; 66,85; 67,85; 70,54; 71,42; 125,40; 125,44; 128,02; 128,13; 131,15; 131,17; 142,45; 204,10; 205,01.

### 4-(1-Dimethylamino-3-phenyl-propyl)-cyclohexancarbaldehydoxim (R¹ = Me, R² = Me, R³ = Phenethyl)

Der Aldehyd (6,55 g, 24 mmol) und Hydroxylamin Hydrochlorid (2,5 g, 36 mmol) wurden in absol. Ethanol (90 ml) gelöst, mit basischem Ionenaustauscher Amberlyst A21 (15,6 g) versetzt und über Nacht bei RT gerührt. Der Ionenaustauscher wurde abfiltriert und zweimal mit Ethanol (je 50 ml) gewaschen. Die Lösung wurde eingeengt und der Rückstand mit 5N NaOH auf pH 11 eingestellt. Die wässrige Phase wurde dreimal mit Ethylacetat (je 50 ml) extrahiert, die organische Phase über Natriumsulfat getrocknet und im Vakuum eingeengt.
Ausbeute: 6,90 g (100 %)

### [1-(4-Aminomethyl-cyclohexyl)-3-phenyl-propyl]-dimethylamin (R¹ = Me, R² = Me, R³ = Phenethyl) A17

Absolutes THF (360 ml) wurde unter Argon mit LiAlH₄ (1,82 g, 48 mmol) versetzt, auf 60 °C erwärmt und tropfenweise das Oxim (6,90 g, 24 mmol), gelöst in THF (60 ml), dazu gegeben. Nach 4-stündigem Rühren bei 60 °C wurde unter Eisbadkühlung (10 °C) Wasser (90 ml) zugetropft und die Reaktionslösung über Kieselgur filtriert. Der Filterrückstand wurde mit THF gewaschen, die organischen Phasen vereinigt, das THF i. Vak. entfernt, der Rückstand mit 5N NaOH auf pH 11 eingestellt und mit Ethylacetat (4 x 40 ml) extrahiert. Die organische Phase wurde über Natriumsulfat getrocknet und eingeengt.
Ausbeute: 5,6 g (85 %), Öl
¹³C-NMR (CDCl₃): 25,93; 26,58; 27,09; 29,21; 29,90; 30,32; 30,73; 30,77; 35,38; 35,66; 38,73; (C₄); 40,06; 40,90; 41,19 (N(CH₃)₂); 48,78; 65,15; 68,22 (CH); 125, 36; 127,99; 128,05; 142,69.

### 4-(Morpholino(phenyl)methyl)cyclohexancarbaldehyd (NR¹R² = Morpholin, R³ = Phenyl)

Zu einer Suspension von (Methoxytriphenyl)phosphoniumchlorid (0.1 mol) in absolutem THF (150 ml) gab man unter Argon eine Lösung von Kalium-tert.-butylat (0.1 mol) in THF (100 ml) tropfenweise bei 0°C hinzu und rührte für 15 min. Anschließend wurde das Keton (0.06 mol) in absolutem THF (100 ml) tropfenweise bei 25°C hinzugegeben und für 16 h gerührt. Nach Zugabe von 6 N HCl bei 0-5°C und Rühren für 1 h wurde mit Ethylacetat (10 x 50 ml) extrahiert. Anschließend wurde die wässrige Phase mit 5 N Natronlauge auf pH 11 eingestellt und mit Ethylacetat (3 x 100 ml) extrahiert. Die vereinigten organischen Phasen wurden über Na₂SO₄ getrocknet und abfiltriert. Das Lösungsmittel wurde unter Vakuum entfernt und das Produkt ohne weitere Aufreinigung in der nächsten Stufe verwendet.

### 4-(Morpholino(phenyl)methyl)cyclohexanecarbaldehydoxim (NR¹R² = Morpholin, R³ = Phenyl)

Zu einer Lösung des Ketons (40 mmol) und Hydroxylamin Hydrochlorid (4,17 g, 60 mmol) in absolutem Ethanol (200 ml) wurde Amberlyst A21 (40 g) hinzugegeben und für 20 h bei 25°C gerührt. Nach Filtration und waschen mit Ethanol (2 x 200 ml) wurde das Lösungsmittel unter Vakuum entfernt. Das Produkt wurde ohne weitere Aufreinigung in die nächste Stufe eingesetzt.

### C-[4-(Morpholin-4-yl-phenyl-methyl)-cyclohexyl]-methylamin NR¹R² = Morpholin, R³ = Phenyl) A18

Zu einer Reaktionsmischung von Lithiumaluminiumhydrid (77 mmol) in absolutem THF (400 ml) gab man bei 60°C das Oxim (38.5 mmol) in THF (90 ml) tropfenweise hinzu und rührte für 4 h bei 60°C. Anschließend wurde bei 10°C Wasser (100 ml) langsam hinzugegeben und die Reaktionsmischung über Silica abfiltriert. Der Filterrückstand wurde mit Ethylacetat gewaschen und das Lösungsmittel der vereinigten organischen Phasen unter Vakuum entfernt. Das Produkt wurde über Säulenchromatographie (5-10% Methanol/Dichlormethan) aufgereinigt.

### 4-(1-Morpholino-3-phenylpropyl)cyclohexancarbaldehyd (NR¹R²=Morpholin, R³ = Phenethyl)

Zu einer Suspension von (Methoxytriphenyl)phosphoniumchlorid (0.1 mol) in absolutem THF (150 ml) gab man unter Argon eine Lösung von Kalium-tert.-butylat (0.1 mol) in THF (100 ml) tropfenweise bei 0°C hinzu und rührte für 15 min. Anschließend wurde das Keton (0.06 mol) in absolutem THF (100 ml) tropfenweise bei 25°C hinzugegeben und für 16 h gerührt. Nach Zugabe von 6 N HCl bei 0-5°C und Rühren für 1 h wurde mit Ethylacetat (10 x 50 ml) extrahiert. Anschließend wurde die wässrige Phase mit 5 N Natronlauge auf pH 11 eingestellt und mit Ethylacetat (3 x 100 ml) extrahiert. Die vereinigten organischen Phasen wurden über Na₂SO₄ getrocknet und abfiltriert. Das Lösungsmittel wurde unter Vakuum entfernt und das Produkt ohne weitere Aufreinigung in der nächsten Stufe verwendet.

### 4-(1-Morpholino-3-phenylpropyl)cyclohexanecarbaldehydoxim (NR¹R² =

### Morpholin, R³ = Phenethyl)

Zu einer Lösung des Ketons (40 mmol) und Hydroxylamin Hydrochlorid (4,17 g, 60 mmol) in absolutem Ethanol (200 ml) wurde Amberlyst A21 (40 g) hinzugegeben und für 20 h bei 25°C gerührt. Nach Filtration und waschen mit Ethanol (2 x 200 ml) wurde das Lösungsmittel unter Vakuum entfernt. Das Produkt wurde ohne weitere Aufreinigung in die nächste Stufe eingesetzt.

### C-[4-(1-Morpholin-4-yl-3-phenyl-propyl)-cyclohexyl]-methylamin (NR¹R² = Morpholin, R³ = Phenethyl) A19

Zu einer Reaktionsmischung von Lithiumaluminiumhydrid (77 mmol) in absolutem THF (400 ml) gab man bei 60°C das Oxim (38.5 mmol) in THF (90 ml) tropfenweise hinzu und rührte für 4 h bei 60°C. Anschließend wurde bei 10°C Wasser (100 ml) langsam hinzugegeben und die Reaktionsmischung über Silica abfiltriert. Der Filterrückstand wurde mit Ethylacetat gewaschen und das Lösungsmittel der vereinigten organischen Phasen unter Vakuum entfernt. Das Produkt wurde über Säulenchromatographie (5-10% Methanol/Dichlormethan) aufgereinigt.

### 4-(Phenyl(pyrrolidin-1-yl)methyl)cyclohexancarbaldehyd (NR¹R² = Pyrrolidin, R³ = Phenethyl)

Zu einer Suspension von (Methoxytriphenyl)phosphoniumchlorid (0.1 mol) in absolutem THF (150 ml) gab man unter Argon eine Lösung von Kalium-tert.-butylat (0.1 mol) in THF (100 ml) tropfenweise bei 0°C hinzu und rührte für 15 min. Anschließend wurde das Keton (0.06 mol) in absolutem THF (100 ml) tropfenweise bei 25°C hinzugegeben und für 16 h gerührt. Nach Zugabe von 6 N HCl bei 0-5°C und Rühren für 1 h wurde mit Ethylacetat (10 x 50 ml) extrahiert. Anschließend wurde die wässrige Phase mit 5 N Natronlauge auf pH 11 eingestellt und mit Ethylacetat (3 x 100 ml) extrahiert. Die vereinigten organischen Phasen wurden über Na₂SO₄ getrocknet und abfiltriert. Das Lösungsmittel wurde unter Vakuum entfernt und das Produkt ohne weitere Aufreinigung in der nächsten Stufe verwendet

### 4-(Phenyl(pyrrolidin-1-yl)methyl)cyclohexancarbaldehydoxim (NR¹R² = Pyrrolidin, R³ = Phenethyl)

Zu einer Lösung des Ketons (40 mmol) und Hydroxylamin Hydrochlorid (4,17 g, 60 mmol) in absolutem Ethanol (200 ml) wurde Amberlyst A21 (40 g) hinzugegeben und für 20 h bei 25°C gerührt. Nach Filtration und waschen mit Ethanol (2 x 200 ml) wurde das Lösungsmittel unter Vakuum entfernt. Das Produkt wurde ohne weitere Aufreinigung in die nächste Stufe eingesetzt.

### C-[4-(Phenyl-pyrrolidin-1-yl-methyl)-cyclohexyl]-methylamin (NR¹R² = Pyrrolidin, R³ = Phenethyl) A20

Zu einer Reaktionsmischung von Lithiumaluminiumhydrid (77 mmol) in absolutem THF (400 ml) gab man bei 60°C das Oxim (38.5 mmol) in THF (90 ml) tropfenweise hinzu und rührte für 4 h bei 60°C. Anschließend wurde bei 10°C Wasser (100 ml) langsam hinzugegeben und die Reaktionsmischung über Silica abfiltriert. Der Filterrückstand wurde mit Ethylacetat gewaschen und das Lösungsmittel der vereinigten organischen Phasen unter Vakuum entfernt. Das Produkt wurde über Säulenchromatographie (5-10% Methanol/Dichlormethan) aufgereinigt.

### 4-(3-Phenyl-1-(pyrrolidin-1-yl)propyl)cyclohexancarbaldehyd (NR¹R² = Pyrrolidin, R³ = Phenyl)

Zu einer Suspension von (Methoxytriphenyl)phosphoniumchlorid (0.1 mol) in absolutem THF (150 ml) gab man unter Argon eine Lösung von Kalium-tert.-butylat (0.1 mol) in THF (100 ml) tropfenweise bei 0°C hinzu und rührte für 15 min. Anschließend wurde das Keton (0.06 mol) in absolutem THF (100 ml) tropfenweise bei 25°C hinzugegeben und für 16 h gerührt. Nach Zugabe von 6 N HCl bei 0-5°C und Rühren für 1 h wurde mit Ethylacetat (10 x 50 ml) extrahiert. Anschließend wurde die wässrige Phase mit 5 N Natronlauge auf pH 11 eingestellt und mit Ethylacetat (3 x 100 ml) extrahiert. Die vereinigten organischen Phasen wurden über Na₂SO₄ getrocknet und abfiltriert. Das Lösungsmittel wurde unter Vakuum entfernt und das Produkt ohne weitere Aufreinigung in der nächsten Stufe verwendet.

### 4-(3-Phenyl-1-(pyrrolidin-1-yl)propyl)cyclohexanecarbaldehydoxim (NR¹R² = Pyrrolidin, R³ = Phenyl)

Zu einer Lösung des Ketons (40 mmol) und Hydroxylamin Hydrochlorid (4,17 g, 60 mmol) in absolutem Ethanol (200 ml) wurde Amberlyst A21 (40 g) hinzugegeben und für 20 h bei 25°C gerührt. Nach Filtration und waschen mit Ethanol (2 x 200 ml) wurde das Lösungsmittel unter Vakuum entfernt. Das Produkt wurde ohne weitere Aufreinigung in die nächste Stufe eingesetzt.

### C-[4-(3-Phenyl-1-pyrrolidin-1-yl-propyl)-cyclohexyl]-methylamin (NR¹R² = Pyrrolidin, R³ = Phenyl) A21

Zu einer Reaktionsmischung von Lithiumaluminiumhydrid (77 mmol) in absolutem THF (400 ml) gab man bei 60°C das Oxim (38.5 mmol) in THF (90 ml) tropfenweise hinzu und rührte für 4 h bei 60°C. Anschließend wurde bei 10°C Wasser (100 ml) langsam hinzugegeben und die Reaktionsmischung über Silica abfiltriert. Der Filterrückstand wurde mit Ethylacetat gewaschen und das Lösungsmittel der vereinigten organischen Phasen unter Vakuum entfernt. Das Produkt wurde über Säulenchromatographie (5-10% Methanol/Dichlormethan) aufgereinigt.

### Synthese der Aminoethylcyclohexane

Die Aminoethylcyclohexane wurden durch dreistufige Reaktionen aus den entsprechend substituierten Cyclohexylaldehyde durch Kettenverlängerung (Wittig) und Umsetzung mit Hydroxylamin Hydrochlorid und anschließender Spaltung mit Lithiumaluminiumhydrid dargestellt.

### [4-(Dimethylamino-phenyl-methyl)-cyclohexyl]-acetaldehyd (R³ = Phenyl)

(Methoxymethyl)triphenylphosphoniumchlorid (38,39 g, 0,112 mol) wurde in absol THF (150 ml) unter Argon suspendiert, bei 0 °C tropfenweise mit Kalium-tert-butylat (12,56 g, 0,112 mol), gelöst in absol.THF (120 ml), versetzt und anschließend 15 min bei 0 °C nachgerührt (die Lösung färbte sich tieforange).

Bei RT wurde dann der Aldehyd (18,4 g, 0,075 mol), gelöst in absol.THF (120 ml), zugetropft und über Nacht bei RT gerührt. Unter Eiswasserkühlung wurde tropfenweise mit Wasser (50 ml) und 6N HCl (150 ml) hydrolysiert. Nach 1 h Rühren bei RT wurde mit Diethylether (10 x 100 ml) extrahiert. Die wässrige Phase wurde mit 5N NaOH auf pH 11 gebracht, mit Ethylacetat (3 x 80 ml) ausgeschüttelt, über Natriumsulfat getrocknet und im Vakuum eingeengt. Das Rohprodukt wurde durch Flashchromatographie mit Ethylacetat/Cyclohexan (1:1) gereinigt.
Ausbeute: 16,31 g (84 %), Öl
¹³C-NMR (CDCl₃): 25,30; 25,92; 29,04; 29,19; 29,74; 30,86; 32,99; 33,02; 35,98; 38,31 (C₄); 41,42; 42,06; (N(CH₃)₂); 48,04; 51,24; 71,82; 75,47 (CH); 126,64 (Cₐᵣₒₘ); 126,68 (Cₐᵣₒₘ); 127,39 (Cₐᵣₒₘ); 127,46 (Carom); 129,15 (Cₐᵣₒₘ); 136,20 (Cₐᵣₒₘ); 137,11 (Cₐᵣₒₘ); 202,27; 202,37 (CHO).

### [4-(Dimethylamino-phenyl-methyl)-cyclohexyl]-acetaldehyd-oxim (R³ = Phenyl)

Der Carbaldehyd (11,04 g, 42,5 mmol) und Hydroxylamin Hydrochlorid (4,44 g, 64 mmol) wurden in absol. Ethanol (150 ml) gelöst, mit basischem Ionenaustauscher Amberlyst A21 (30 g) versetzt und 4 h bei RT gerührt. Der Ionenaustauscher wurde abfiltriert und mit Ethanol (2 x 50 ml) gewaschen. Die Lösung wurde eingeengt, der Rückstand mit 5N NaOH auf pH 11 eingestellt, die wässrige Phase mit Ethylacetat (3 x 50 ml) extrahiert, die organische Phase über Natriumsulfat getrocknet und i. Vak. eingeengt.
Ausbeute: 11,66 (100 %)
¹³C-NMR (CDCl₃): 25,41; 25,57; 28,87; 29,11; 30,92; 30,97; 32,33; 32,99; 33,67; 35,99; 36,10; 38,59 (C₄); 41,31; 41,40; 42,11; 42,14 (N(CH₃)₂): 71,74; 75,63 (CH); 126,71 (Cₐᵣₒₘ); 127,46 (Cₐᵣₒₘ); 129,26 (Cₐᵣₒₘ); 137,26 (Cₐᵣₒₘ); 150,95; 151,37; 151,56 (C=N-O).

### 2-[4-Dimethylamino-phenyl-methyl)-cyclohexyl]-ethylamin (R³ = Phenyl) A22

Absolutes THF (400 ml) wurde unter Argon mit LiAlH₄ (3,22 g, 85 mmol) versetzt, auf 60 °C erwärmt und tropfenweise das Oxim (11,66 g, 42,5 mmol), gelöst in THF (80 ml), addiert. Nach 4-stündigem Rühren bei 60 °C wurde unter Eisbadkühlung (10 °C) Wasser (100 ml) zugetropft, die Reaktionslösung über Kieselgur abgesaugt und das Kieselgur mit THF gewaschen. Die vereinigten THF-Lösungen wurden i. Vak. eingeengt, der Rückstand mit 5N NaOH auf pH 11 eingestellt und mit Ethylacetat (4 x 50 ml) extrahiert. Die organische Phase wurde über Natriumsulfat getrocknet und eingedampft.
Ausbeute: 9,15 g (83 %), Öl
¹³C-NMR (CDCl₃): 25,58; 26,08; 29,16; 29,21; 30,39; 31,10; 32,49; 33,16; 33,33; 35,54; 36,22; 38,80 (C₄); 40,32; 41,36; 41,50; 42,11; (N(CH₃)₂); 71,77; 75,66 (CH); 126,52 (Cₐᵣₒₘ); 127,31 (Cₐᵣₒₘ); 127,38 (Cₐᵣₒₘ); 129,18 (Cₐᵣₒₘ); 139,39 (Cₐᵣₒₘ); 137,41 (Cₐᵣₒₘ).

### {4-[Dimethylamino-(4-fluorphenyl)-methyl]-cyclohexyl}-acetaldehyd (R³ = 4-Fluorphenyl)

(Methoxymethyl)triphenylphosphoniumchlorid (43,53 g, 127 mmol) wurde in absol. THF (200 ml) unter Argon suspendiert, bei 0 °C tropfenweise mit Kalium-tert-butylat (14,25 g, 127 mmol), gelöst in absol. THF (130 ml), versetzt und anschließend 15 min bei 0 °C nachgerührt.

Bei RT wurde dann der Aldehyd (22,3 g, 85 mmol), gelöst in absol. THF (130 ml), zugetropft und über Nacht bei RT gerührt. Unter Eiswasserkühlung wurde tropfenweise mit Wasser (80 ml) und 6N HCl (200 ml) hydrolysiert. Nach 1 h Rühren bei RT wurde zehnmal mit Diethylether (je 100 ml) extrahiert. Die wässrige Phase wurde mit 5N NaOH auf pH 11 gebracht, dreimal mit Ethylacetat (je 100 ml) ausgeschüttelt, über Natriumsulfat getrocknet und i. Vak. eingeengt. Das Rohprodukt wurde durch Flashchromatographie mit Ethylacetat/Cyclohexan (1:1) gereinigt.
Ausbeute: 15,8 g (67 %)
¹³C-NMR (CDCl₃): δ = 25,08; 25,87; 28,80; 29,10; 29,13; 29,62; 30,82; 32,90; 33,08; 36,19; 38,43; 41,36; 42,01; 47,94; 51,17; 71,11; 74,69; 114,11; 114,20; 114,32; 130,32; 130,40; 132,00; 132,92; 160,31; 162,74; 202,15; 202,23.

### {4-[Dimethylamino-(4-fluorphenyl)-methyl]-cyclohexyl}-acetaldehydoxim (R³ = 4-Fluorphenyl)

Der Carbaldehyd (5,30 g, 20,0 mmol) und Hydroxylamin Hydrochlorid (2,08 g, 30 mmol) wurden in absol. Ethanol (90 ml) gelöst, mit basischem Ionenaustauscher Amberlyst A21 (14,8 g) versetzt und über Nacht bei RT gerührt. Der Ionenaustauscher wurde abfiltriert und mit Ethanol (2 x 50 ml) gewaschen. Die Lösung wurde eingeengt, der Rückstand mit 5N NaOH auf pH 11 eingestellt, die wässrige Phase mit Ethylacetat (3 x 100 ml) extrahiert, die organische Phase über Natriumsulfat getrocknet und i. Vak. eingeengt. Der Rückstand wurde durch Flashchromatographie mit EE/Cyclohexan (2:1) gereinigt.
Ausbeute: 3,50 (60 %)

### 2-{4-[Dimethylamino-(4-fluorphenyl)-methyl]-cyclohexyl}-ethylamin A23 (R³ = 4-Fluorphenyl)

Absolutes THF (450 ml) wurde unter Argon mit LiAlH₄ (2,35 g, 62 mmol) versetzt, auf 60 °C erwärmt und tropfenweise das Oxim (9,10 g, 31,0 mmol), gelöst in THF (75 ml), addiert. Nach 4-stündigem Rühren bei 60 °C wurde unter Eisbadkühlung (10 °C) Wasser (116 ml) zugetropft, die Reaktionslösung über Kieselgur abgesaugt und das Kieselgur mit THF gewaschen. Die vereinigten THF-Lösungen wurden i. Vak. eingeengt, der Rückstand mit 5N NaOH auf pH 11 eingestellt und mit Ethylacetat (4 x 50 ml) extrahiert. Die organische Phase wurde über Natriumsulfat getrocknet und i. Vak. eingeengt.
Ausbeute: 6,80 g (79 %), Öl
¹³C-NMR (CDCl₃): 25,32; 26,03; 28,94; 29,08; 30,37; 31,06; 32,39; 32,90; 33,07; 33,26; 35,50; 37,81; 38,80 39,78 (C₄); 41,33; 41,42; 42,09 (N(CH₃)₂); 71,11; 74,89 (CH); 114,03; 114,11; 130,32; 130,40; 132,19; 133,18; 133,21; 160,27; 162,69.

### {4-[Dimethylamino-(3-fluorphenyl)-methyl]-cyclohexyl}-acetaldehyd (R³ = 3-Fluorphenyl)

(Methoxymethyl)triphenylphosphoniumchlorid (26,73 g, 78 mmol) wurde in absol. THF (90 ml) unter Argon suspendiert, bei 0 °C tropfenweise mit Kalium-*tert*-butylat (8,75 g, 78 mmol), gelöst in absol. THF (90 ml), versetzt und anschließend 15 min bei 0 °C nachgerührt .

Bei RT wurde dann der Aldehyd (13,69 g, 52 mmol), gelöst in absol. THF (90 ml), zugetropft und über Nacht bei RT gerührt. Unter Eiswasserkühlung wurde tropfenweise mit Wasser (50 ml) und 6N HCl (150 ml) hydrolysiert. Nach 1 h Rühren bei RT wurde zehnmal mit Diethylether (je 50 ml) extrahiert. Die wässrige Phase wurde mit 5N NaOH auf pH 11 gebracht, dreimal mit Ethylacetat (je 100 ml) ausgeschüttelt, über Natriumsulfat getrocknet und i. Vak. eingeengt. Das Rohprodukt wurde durch Flashchromatographie mit Ethylacetat/Cyclohexan (1:1) gereinigt. Ausbeute: 12,61 g (87 %)
¹³C-NMR (CDCl₃): δ = 25,19; 25,83; 28,90; 29,06; 29,14; 29,68; 30,77; 32,92; 32,98; 33,10; 36,05; 38,36; 41,39; 42,04; 48,02; 51,20; 71,48; 75,07; 113,43; 113,49; 113,64; 113,69; 115,55; 115,76; 124,89; 128,70; 128,78; 128,88; 139,24; 140,08; 140,14; 161,09; 163,52; 202,19; 202,27.

### {4-[Dimethylamino-(3-fluorphenyl)-methyl]-cyclohexyl}-acetaldehydoxim (R³ = 3-Fluorphenyl)

Der Carbaldehyd (7,18 g, 25,8 mmol) und Hydroxylamin Hydrochlorid (2,71 g, 39 mmol) wurden in absol. Ethanol (90 ml) gelöst, mit basischem Ionenaustauscher Amberlyst A21 (20 g) versetzt und 20 h bei RT gerührt. Der Ionenaustauscher wurde abfiltriert und mit Ethanol (2 x 50 ml) gewaschen. Die Lösung wurde eingeengt, der Rückstand mit 5N NaOH auf pH 11 eingestellt, die wässrige Phase mit Ethylacetat (3 x 50 ml) extrahiert, die organische Phase über Natriumsulfat getrocknet und i. Vak. eingeengt.
Ausbeute: 7,54 (100 %)

### 2-{4-[Dimethylamino-(3-fluorphenyl)-methyl]-cyclohexyl}-ethylamin (R³ = 3-Fluorphenyl) A24

Absolutes THF (300 ml) wurde unter Argon mit LiAlH₄ (1,97 g, 52 mmol) versetzt, auf 60 °C erwärmt und tropfenweise das Oxim (7,54 g, 25,8 mmol), gelöst in THF (70 ml), addiert. Nach 4-stündigem Rühren bei 60 °C wurde unter Eisbadkühlung (10 °C) Wasser (100 ml) zugetropft, die Reaktionslösung über Kieselgur abgesaugt und das Kieselgur mit THF gewaschen. Die vereinigten THF-Lösungen wurden i. Vak. eingeengt, der Rückstand mit 5N NaOH auf pH 11 eingestellt und mit Ethylacetat (3 x 50 ml) extrahiert. Die organische Phase wurde über Natriumsulfat getrocknet und i. Vak. eingeengt.
Ausbeute: 6,3 g (88 %), Öl
¹³C-NMR (CDCl₃): 25,28; 25,84; 28,87; 28,98; 30,28; 32,30; 32,93; 33,13; 35,38; 36,16; 37,81; 38,69 (C₄); 39,69; 41,20; 41,37; 41,94 (N(CH₃)₂); 71,29; 75,11 (CH); 113,14; 113,18; 113,38; 115,41; 115,62; 124,73; 128,44; 128,53; 139,25; 140,27; 140,33; 160,91; 163,34.

### {4-[(4-Chlorphenyl)-dimethylamino-methyl]-cyclohexyl}-acetaldehyd (R³ = 4-Chlorphenyl)

Methoxymethyl)triphenylphosphoniumchlorid (25,02 g, 73 mmol) wurde in absol. THF (90 ml) unter Argon suspendiert, bei 0 °C tropfenweise mit Kalium-*tert*-butylat (8,19 g, 73 mmol), gelöst in absol. THF (90 ml), versetzt und anschließend 15 min bei 0 °C nachgerührt.

Bei RT wurde dann der Aldehyd (13,86 g, 49 mmol), gelöst in absol. THF (90 ml), zugetropft und über Nacht bei RT gerührt. Unter Eiswasserkühlung wurde tropfenweise mit Wasser (50 ml) und 6N HCl (150 ml) hydrolysiert. Nach 1 h Rühren bei RT wurde zehnmal mit Diethylether (je 50 ml) extrahiert. Die wässrige Phase wurde mit 5N NaOH auf pH 11 gebracht, dreimal mit Ethylacetat (je 100 ml) ausgeschüttelt, über Natriumsulfat getrocknet und i. Vak. eingeengt. Das Rohprodukt wurde durch Flashchromatographie mit Ethylacetat/Cyclohexan (1:1) gereinigt.
Ausbeute: 12,07 g (84%).
¹³C-NMR (CDCl₃): δ = 25,06; 25,82; 28,74; 29,00; 29,13; 29,60; 30,77; 32,87; 32,94; 33,07; 36,06; 38,32; 41,38; 42,05; 47,95; 51,17; 71,23; 74,80; 127,58; 127,66; 130,31; 132,28; 132,34; 134,81; 135,77; 202,12; 202,20.

### {4-[Dimethylamino-(4-chlorphenyl)-methyl)-cyclohexyl)-acetaldehydoxim (R³ = 4-Chlorphenyl)

Der Carbaldehyd (6,72 g, 22,8 mmol) und Hydroxylamin Hydrochlorid (2,36 g, 34 mmol) wurden in absol. Ethanol (90 ml) gelöst, mit basischem Ionenaustauscher Amberlyst A21 (16 g) versetzt und 20 h bei RT gerührt. Der Ionenaustauscher wurde abfiltriert und mit Ethanol (2 x 50 ml) gewaschen. Die Lösung wurde eingeengt, der Rückstand mit 5N NaOH auf pH 11 eingestellt, die wässrige Phase mit Ethylacetat (3 x 50 ml) extrahiert, die organische Phase über Natriumsulfat getrocknet und i. Vak. eingeengt.
Ausbeute: 7,04 (100 %)

### 2-{4-[Dimethylamino-(4-chlorphenyl)-methyl]-cyclohexyl}-ethylamin (R³ = 4-Chlorphenyl) A25

Absolutes THF (300 ml) wurde unter Argon mit LiAlH₄ (1,73 g, 45,6 mmol) versetzt, auf 60 °C erwärmt und tropfenweise das Oxim (7,04 g, 22,8 mmol), gelöst in THF (60 ml), addiert. Nach 4-stündigem Rühren bei 60 °C wurde unter Eisbadkühlung (10 °C) Wasser (100 ml) zugetropft, die Reaktionslösung über Kieselgur abgesaugt und das Kieselgur mit THF gewaschen. Die vereinigten THF-Lösungen wurden i. Vak. eingeengt, der Rückstand mit 5N NaOH auf pH 11 eingestellt und mit Ethylacetat (3 x 50 ml) extrahiert. Die organische Phase wurde über Natriumsulfat getrocknet und i. Vak. eingeengt.
Ausbeute: 5,76 g (86 %), Öl
¹³C-NMR (CDCl₃): 25,67; 26,35; 29,23; 29,44; 30,74, 31,39; 33,41; 33,61; 35,86; 36,71; 38,20; 39,18; 40,17; 40,67; 41,72; 41,81; 42,50 (N(CH₃)₂); 71,59; 75,37; 127,86; 127,95; 130,70; 132,52; 135,38; 136,45.

### {4-[Dimethylamino-thiophen-2-yl-methyl]-cyclohexyl)-acetaldehyd (R³ = 2-Thienyl)

(Methoxymethyl)triphenylphosphoniumchlorid (28,79 g, 84 mmol) wurde in absol. THF (100 ml) unter Argon suspendiert, bei 0 °C tropfenweise mit Kalium-tert-butylat (9,42 g, 84 mmol), gelöst in absol. THF (100 ml), versetzt und anschließend 15 min bei 0 °C nachgerührt.

Bei RT wurde dann der Aldehyd (14,08 g, 56 mmol), gelöst in absol. THF (100 ml), zugetropft und über Nacht bei RT gerührt. Unter Eiswasserkühlung wurde tropfenweise mit Wasser (50 ml) und 6N HCl (150 ml) hydrolysiert. Nach 1 h Rühren bei RT wurde zehnmal mit Diethylether (je 50 ml) extrahiert. Die wässrige Phase wurde mit 5N NaOH auf pH 11 gebracht, dreimal mit Ethylacetat (je 100 ml) ausgeschüttelt, über Natriumsulfat getrocknet und i. Vak. eingeengt. Das Rohprodukt wurde durch Flashchromatographie mit Ethylacetat/Cyclohexan (1:2) gereinigt. Ausbeute: 11,48 g (77 %).
¹³C-NMR (CDCl₃): δ = 25,80; 25,88; 28,73; 29,95; 30,49, 32,23; 32,76; 37,89; 40,21; 40,88; 41,23; 48,36; 51,09; 66,02; 69,97; 123,19; 123,72; 125,95; 126,31; 139,42; 139,91; 202,61.

### [4-(Dimethylamino-thiophen-2-yl-methyl)-cyclohexyl]-acetaldehydoxim (R³ = 2-Thiophen)

Der Carbaldehyd (6,3 g, 23,7 mmol) und Hydroxylamin Hydrochlorid (2,5 g, 36 mmol) wurden in absol. Ethanol (90 ml) gelöst, mit basischem Ionenaustauscher Amberlyst A21 (20 g) versetzt und 20 h bei RT gerührt. Der Ionenaustauscher wurde abfiltriert und mit Ethanol (2 x 50 ml) gewaschen. Die Lösung wurde eingeengt, der Rückstand mit 5N NaOH auf pH 11 eingestellt, die wässrige Phase mit Ethylacetat (3 x 50 ml) extrahiert, die organische Phase über Natriumsulfat getrocknet und i. Vak. eingeengt. Ausbeute: 6,64 (100 %)

### 2-[4-(Dimethylamino-thiophen-2-yl-methyl)-cyclohexyl]-ethylamin (R³ = 2-Thiophen) A26

Absolutes THF (250 ml) wurde unter Argon mit LiAlH₄ (1,78 g, 47 mmol) versetzt, auf 60 °C erwärmt und tropfenweise das Oxim (6,64 g, 23,7 mmol), gelöst in THF (60 ml), addiert. Nach 4-stündigem Rühren bei 60 °C wurde unter Eisbadkühlung (10 °C) Wasser (100 ml) zugetropft, die Reaktionslösung über Kieselgur abgesaugt und das Kieselgur mit THF gewaschen. Die vereinigten THF-Lösungen wurden i. Vak. eingeengt, der Rückstand mit 5N NaOH auf pH 11 eingestellt und mit Ethylacetat (3 x 50 ml) extrahiert. Die organische Phase wurde über Natriumsulfat getrocknet und i. Vak. eingeengt.
Ausbeute: 5,62 g (89 %), Öl
¹³C-NMR (CDCl₃): 25,97; 26,13; 28,72; 28,79; 30,15, 30,23; 30,74; 32,61; 32,90; 35,32; 38,22; 39,70; 40,09; 40,69; 40,84; 41,26 (N(CH₃)₂); 70,14; 123,56; 123,60; 125,86; 126,21; 126,23; 139,70; 140,24.

### [4-(1-Dimethylamino-3-phenyl-propyl)-cyclohexyl]-acetaldehyd (R³ = Phenethyl)

(Methoxymethyl)triphenylphosphoniumchlorid (50,3 g, 147 mmol) wurde in absol. THF (150 ml) unter Argon suspendiert, bei 0 °C tropfenweise mit Kalium-tert-butylat (16,5 g, 147 mmol), gelöst in absol. THF (140 ml), versetzt und anschließend 15 min bei 0 °C nachgerührt.

Bei RT wurde dann der Aldehyd (27,0 g, 98 mmol), gelöst in absol. THF (150 ml), zugetropft und über Nacht bei RT gerührt. Unter Eiswasserkühlung wurde tropfenweise mit Wasser (102 ml) und 6N HCl (240 ml) hydrolysiert. Nach 1 h Rühren bei RT wurde fünfmal mit Diethylether (je 200 ml) extrahiert. Die wässrige Phase wurde unter Eiskühlung mit 5 N NaOH auf pH 11 gebracht, dreimal mit Ethylacetat (je 200 ml) ausgeschüttelt, über Natriumsulfat getrocknet und i. Vak. eingeengt. Das Rohprodukt wurde durch Flashchromatographie mit Ethylacetat/Cyclohexan (1:1) gereinigt. Ausbeute: 18,1 g (64 %)
¹³C-NMR (CDCl₃): δ = 25,55; 26,19; 29,04; 29,15; 29,35; 29,85; 31,00; 32,87; 32,68; 33,04; 35,33; 38,49; 40,86; 41,13; 47,51; 51,15; 65,48; 68,09.

### [4-(1-Dimethylamino-3-phenyl-propyl)-cyclohexyl]-acetaldehydeoxim (R³ = Phenethyl)

Der Carbaldehyd (12,6 g, 44,0 mmol) und Hydroxylamin Hydrochlorid (4,60 g, 66,0 mmol) wurden in absol. Ethanol (200 ml) gelöst, mit basischem Ionenaustauscher Amberlyst A21 (32 g) versetzt und über Nacht bei RT gerührt. Der Ionenaustauscher wurde abfiltriert und mit Ethanol (2 x 50 ml) gewaschen. Die Lösung wurde eingeengt, der Rückstand mit 5N NaOH auf pH 11 eingestellt, die wässrige Phase mit Ethylacetat (3 x 50 ml) extrahiert, die organische Phase über Natriumsulfat getrocknet und i. Vak. eingeengt.
Ausbeute: 13,3 (100%)

### {1-[4-(2-Amino-ethyl)-cyclohexyl]-3-phenyl-propyl}-dimethylamin (R³ = Phenethyl) A27

Absolutes THF (600 ml) wurde unter Argon mit LiAlH₄ (4,25 g, 112 mmol) versetzt, auf 60 °C erwärmt und tropfenweise das Oxim 71 (17,1 g, 56,0 mmol), gelöst in THF (150 ml), addiert. Nach 4-stündigem Rühren bei 60 °C wurde unter Eisbadkühlung (10 °C) Wasser (360 ml) zugetropft, die Reaktionslösung über Kieselgur abgesaugt und das Kieselgur mit THF gewaschen. Die vereinigten THF-Lösungen wurden i. Vak. eingeengt, der Rückstand mit 5N NaOH auf pH 11 eingestellt und mit Ethylacetat (5 x 100 ml) extrahiert. Die organische Phase wurde über Natriumsulfat getrocknet und i. Vak. eingeengt.
Ausbeute: 16,2 g (100 %), Öl
¹³C-NMR (CDCl₃): 25,67; 26,44; 29,07; 29,16; 30,05, 30,22; 31,32; 31,80; 33,30; 35,24; 35,37; 37,26; 39,77; 40,30; 40,85; 41,15; 41,40 (N(CH₃)₂); 65,61; 68,29; 125,53; 127,68; 128,16; 128,200; 142,91.

### Synthese der Piperidin-Derivate

Für die Piperidin-Derivate wurde vom kommerziell erhältlichen Isonipecotinsäuremethylester ausgegangen, der zunächst N-geschützt wurde, um anschließend die Esterfunktion zum Aldehyd zu reduzieren. Die Einführung des N,N-dimethyl(aryl)methanamin-Restes erfolgte analog zu den Cyclohexylamin-Derivaten. Nach dem Entschützen erhielt man das gewünschte Produkt.

### Methyl 1-(4-methoxybenzyl)piperidin-4-carboxylat

Zu einer Lösung von Isonipecotinsäuremethylester (1,00 g, 6,98 mmol) und Triethylamin (1,40 g, 14 mmol) in THF (30 ml) wurde 4-Methoxybenzylchlorid (1,10 g, 6,98 mmol) getropft und 72 h bei 60 °C gerührt. Das Reaktionsgemisch wurde anschließend mit 5 % Natriumhydrogencarbonat-Lösung (50 ml) versetzt und mit Ethylacetat (3 x 50 ml) extrahiert. Die vereinigten organischen Phasen wurden mit Natriumsulfat getrocknet und i. Vak. eingeengt. Der Rückstand wurde durch Flashchromatographie mit Cyclohexan / Ethylacetat (2: 1) gereinigt.
Ausbeute: 1,23 g (67 %) Methyl 1-(4-methoxybenzyl)piperidin-4-carboxylat
¹H-NMR (DMSO-d₆): 1,53 (dq, 2H); 1,77 (dd, 2H); 1,93 (dt, 2H); 2,28 (tt, 1H); 2,71 (td, 2H); 3,35 (s, 2H); 3,58 (s, 3H); 3,72 (s, 3H); 6,86 (d, 2H); 7,17 (d, 2H).

### 1-(4-Methoxybenzyl)piperidin-4-carbaldehyd

Zu einer Lösung von Methyl 1-(4-methoxybenzyl)piperidin-4-carboxylat (1,23 g, 4,68 mmol) in Toluol (30 ml) wurde unter Argon bei -78 °C eine 1,5 M Lösung von Diisobutylaluminiumhydrid in Toluol (3,12 ml, 4,68 mmol) innerhalb von 30 min getropft und anschließend 30 min bei dieser Temperatur gerührt. Danach wurde Methanol (15 ml) so zugetropft, dass die Innentemperatur bei -78 °C blieb, bevor dann langsam auf Raumtemperatur erwärmt wurde. Das Reaktionsgemisch wurde mit gesättigter Natriumchlorid-Lösung (20 ml) versetzt und die Suspension durch Seesand filtriert. Die organische Phase wurde mit Natriumsulfat getrocknet und i. Vak. eingeengt. Der Rückstand wurde durch Flashchromatographie mit Cyclohexan /Ethylacetat (1:3) gereinigt.
Ausbeute: 750 mg (69 %) 1-(4-Methoxybenzyl)piperidin-4-carbaldehyd
¹H-NMR (DMSO-d₆): 1,47 (dtd, 2H); 1,78 (m, 2H); 2,00 (dt, 2H); 2,27 (m, 1H); 2,66 (td, 2H); 3,36 (s, 2H); 3,73 (s, 3H); 6,86 (d, 2H); 7,18 (d, 2H); 9,57 (s, 1 H).

### 2-(Dimethylamino)-2-(1-(4-methoxybenzyl)piperidin-4-yl)acetonitril (R¹, R² = Methyl)

Zu einem Gemisch aus 4 N Salzsäure (1,2 ml) und Methanol (5 ml) wurde unter Eiskühlung 40 % wässrige Dimethylamin-Lösung (2,66 ml, 21 mmol), 1-(4-Methoxybenzyl)piperidin-4-carbaldehyd (750 mg, 3,2 mmol) und Kaliumcyanid (688 mg, 10,6 mmol) hinzugefügt. Das Reaktionsgemisch wurde 5 d bei Raumtemperatur gerührt, dann mit Wasser (50 ml) versetzt und mit Ethylacetat (3 x 50 ml) extrahiert. Die vereinigten organischen Phasen wurden mit Natriumsulfat getrocknet und i. Vak. eingeengt.
Ausbeute: 875 mg (95 %) 2-(Dimethylamino)-2-(1-(4-methoxybenzyl)piperidin-4-yl)acetonitril
¹H-NMR (DMSO-d₆): 1,22-1,29 (m, 2H); 1,57 (ddt, 1H); 1,76-1,97 (m, 4H); 2,18 (s, 6H); 2,73-2,88 (m, 3H); 3,36 (s, 3H); 3,73 (s, 2H); 6,85 (d, 2H); 7,16 (d, 2H).

### (1-(4-Methoxybenzyl)piperidin-4-yl)-N,N-dimethyl(phenyl)methanamin (R¹, R² = Methyl, R³ = Phenyl)

Zu einer eisgekühlten Lösung von 2-(Dimethylamino)-2-(1-(4-methoxybenzyl)-piperidin-4-yl)acetonitril (875 mg, 3,0 mmol) in THF (20 ml) wurde eine 2 M Phenylmagnesiumchlorid-Lösung in THF (3,75 ml, 7,5 mmol) getropft, das Reaktionsgemisch anschließend langsam auf Raumtemperatur erwärmt und 16 h gerührt. Die Reaktionslösung wurde danach mit gesättigter Ammoniumchlorid-Lösung (50 ml) versetzt, mit Ethylacetat (3 x 50 ml) extrahiert, die vereinigten organischen Phasen mit Natriumsulfat getrocknet, i. Vak. eingeengt und der Rückstand durch Flashchromatographie mit Chlorform / Methanol / Triethylamin (9:1:0,1) gereinigt.
Ausbeute: 832 mg (82 %) 1-(1-(4-Methoxybenzyl)piperidin-4-yl)-N,N-dimethyl-1-phenylmethanamin
¹H-NMR (DMSO-d₆): 0,84-1,18 (m, 3H); 1,74-1,89 (m, 4H); 1,99 (s, 6H); 2,68 (d, 1 H); 2,80 (d, 1 H); 3,07 (d, 1H, J = 8,9 Hz); 3,32 (s, 2H); 3,71 (s, 3H); 6,84 (d, 2H); 7,15 (q, 4H); 7,21-7,33 (m, 3H).

### Benzyl 4-((dimethylamino)(phenyl)methyl)piperidin-1-carboxylat (R¹, R² = Methyl, R³ = Phenyl)

Eine Lösung von 1-(1-(4-Methoxybenzyl)piperidin-4-yl)-*N,N*-dimethyl-1-phenylmethanamin (3,00 g, 8,86 mmol) in DCM (50 ml) wurde mit Chlorameisensäurebenzylester (1,50 g, 1,25 ml, 8,86 mmol) versetzt und 30 min bei Raumtemperatur gerührt. Das Reaktionsgemisch wurde anschließend mit Natriumhydrogencarbonat-Lösung (40 ml) versetzt, die Phasen wurden getrennt und die wässrige Phase mit DCM (2 × 30 ml) extrahiert. Die vereinigten organischen Phasen wurden mit Natriumsulfat getrocknet, i. Vak. eingeengt und der Rückstand durch Flashchromatographie mit Chlorform / Methanol / Triethylamin (100:5:1). gereinigt.
Ausbeute: 2,32 g (74 %), Benzyl 4-((dimethylamino)(phenyl)methyl)piperidin-1-carboxylat
¹H-NMR (DMSO-d₆): 0,82 (ddd, 2H); 0,97 (ddd, 2H); 1,27 (d, 1H); 2,00 (s, 6H); 2,69-2,84 (m, 2H); 3,11 (d, 1 H); 3,89 (d, 1H); 4,03 (d, 1H); 5,03 (s, 2H); 7,14 (m, 2H); 7,20-7,35 (m, 8H).

### N,N-Dimethyl-1-phenyl-1-(piperidin-4-yl)methanamin (R¹, R² = Methyl, R³ = Phenyl) A28

Eine Lösung von Benzyl 4-((dimethylamino)(phenyl)methyl)piperidin-1-carboxylat (2,32 g, 6,58 mmol) in Essigsäure (20 ml) wurde mit 33 % Bromwasserstoff in Eisessig (20 ml) versetzt und 1,5 h bei Raumtemperatur gerührt. Durch Zugabe von Diethylether fiel ein Feststoff aus. Die überstehende Lösung wurde dekantiert, zum Rückstand wurde wiederholt Diethylether gegeben und jeweils wieder dekantiert. Der Rückstand wurde i. Vak. getrocknet, in Methanol (20 ml) gelöst, mit stark basischem Ionenaustauscher Dowex 1 x2-200 versetzt und 1 h bei Raumtemperatur gerührt. Anschließend wurde das Gemisch filtriert, der Filterrückstand mit Methanol gewaschen und das Filtrat i. Vak. eingeengt.
Ausbeute: 1,09 g (76 %) N,N-Dimethyl-1-phenyl-1-(piperidin-4-yl)methanamin
1 H-NMR (DMSO-d₆): 1,06 (ddd, 1H); 1,23 (td, 2H); 1,72 (br s, 1 H); 1,99 (s, 6H); 2,20 (dd, 1H); 2,66 (dt, 1H); 2,77 (dt, 1H); 3,03 (d, 1H); 3,10 (d, 1 H); 3,17 (d, 1H); 7,15 (d, 2H); 7,25 (m, 1H); 7,34 (m, 2H).

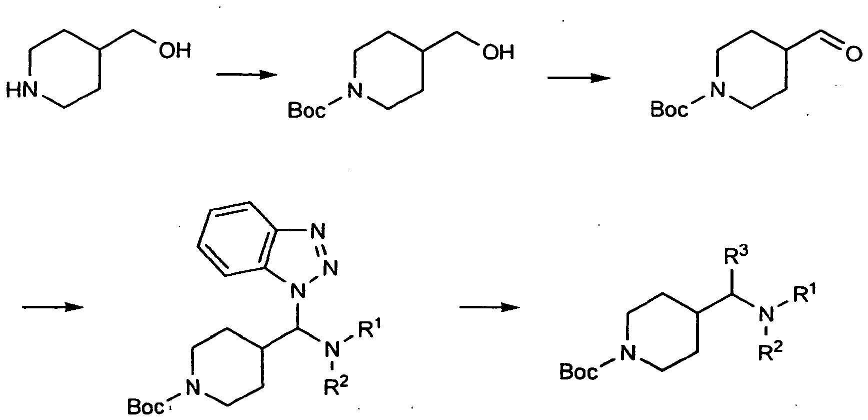

### tert-Butyl 4-(hydroxymethyl)piperidin-1-carboxylat

Zu einer Lösung von 4-Hydroxymethylpiperidin in DCM gab man unter Inertgasatmosphäre Triethylamin (1.5 Äquivalente) und Boc-Anhydrid (1.2 Äquivalente) bei 0°C hinzu und rührte für 1 h bei 25°C. Nach Zugabe von DCM wurde die organische Phase separiert und mit Wasser und wässriger gesättigter NaCl-Lösung gewaschen. Nach Trocknung der organischen Phase über Na₂SO₄ und Filtration wurde das Lösungsmittel unter Vakuum entfernt. Das Produkt wurde über Säulenchromatographie (30% Ethylacetat/Hexan) aufgereinigt.

### tert-Butyl 4-formylpiperidin-1-carboxylat

Zu einer Lösung von Oxalylchlorid (1.1 Äquivalente) in trockenem CH₂Cl₂ gab man unter Inertgasatmosphäre DMSO (2.2 Äquivalente) bei -78°C hinzu und rührte für 1 h. Eine Lösung von N-Boc-4-piperidinmethanol (1 Äquivalent) in trockenem DCM wurde tropfenweise bei -70°C zur Reaktionsmischung gegeben und für 2 h gerührt. Nach Zugabe von Triethylamin (2.5 Äquivalente) ließ man die Reaktionslösung bis auf RT erwärmen, versetzte mit wässriger gesättigter NH₄Cl-Lösung und mit DCM. Die abgetrennte organische Phase wurde mit Wasser und wässriger gesättigter NaCl-Lösung gewaschen. Nach Trocknung der organischen Phase über Na₂SO₄ und Filtration wurde das Lösungsmittel unter Vakuum entfernt. Das Produkt wurde ohne weitere Aufreinigung in der nächsten Stufe verwendet.

### tert-Butyl 4-((1H-benzo[d][1,2,3]triazol-1-yl)(morpholino)methyl)piperidin-1-carboxylat

Der Aldehyd (23.4 mmol), Morpholin (23.4 mmol) und 1H-Benzotriazol (23.4 mmol) wurden in Benzol (60 ml) für 16 h unter Rückfluß erhitzt und das entstehende Reaktionswasser über einen Wasserabscheider abgeführt. Das Benzol wurde unter Vakuum entfernt und der Rückstand unmittelbar in die nächste Stufe eingesetzt.

### tert-Butyl 4-(morpholino(phenyl)methyl)piperidin-1-carboxylat (NR¹R² = Morpholin, R³ = Phenyl) X1

Zu einer Lösung des Grignard-Reagenzes (47.1 mmol) in THF wurde eine Lösung des Benzotriazol-Addukts (23.5 mmol) in THF tropfenweise hinzugegeben und für 16 h bei 25°C gerührt. Die Reaktionslösung wurde auf 0°C abgekühlt und mit wässriger gesättigter NH₄Cl-Lösung versetzt und anschließend mit Ethylacetat (2 x 300 ml) extrahiert. Die organische Phase wurde mit Wasser und wässriger gesättigter NaCl-Lösung gewaschen. Nach Trocknung der organischen Phase über Na₂SO₄ und Filtration wurde das Lösungsmittel unter Vakuum entfernt. Das Produkt wurde über Säulenchromatographie (2-5% Methanol/Dichlormethan) aufgereinigt.

### tert-Butyl 4-(morpholino(benzyll)methyl)piperidin-1-carboxylat carboxylat (NR¹R² = Morpholin, R³ = benzyl) X2

Zu einer Lösung des Grignard-Reagenzes (47.1 mmol) in THF wurde eine Lösung des Benzotriazol-Addukts (23.5 mmol) in THF tropfenweise hinzugegeben und für 16 h bei 25°C gerührt. Die Reaktionslösung wurde auf 0°C abgekühlt und mit wässriger gesättigter NH₄Cl-Lösung versetzt und anschließend mit Ethylacetat (2 x 300 ml) extrahiert. Die organische Phase wurde mit Wasser und wässriger gesättigter NaCl-Lösung gewaschen. Nach Trocknung der organischen Phase über Na₂SO₄ und Filtration wurde das Lösungsmittel unter Vakuum entfernt. Das Produkt wurde über Säulenchromatographie (2-5% Methanol/Dichlormethan) aufgereinigt.

### Durch saure Abspaltung der Boc-Gruppe wurden die Amine A29 und A30 aus X1 und X2 erhalten.

### Synthese der Aminbausteine A31 - A35

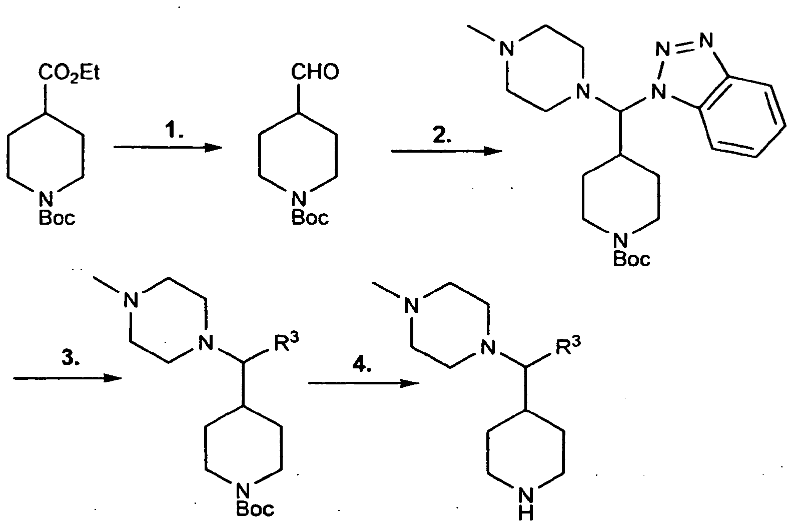

Stufe 1. Zu einer Lösung Piperidin-1,4-dicarbonsäure-1-tert.-butylester-4-ethylester (15 mmol) in trockenem Toluol (20 ml) wurde tropfenweise unter Argon-Atmossphäre bei -70°C Diisobutylaluminiumhydrid (15.3 mmol, 1.5 M Lösung in Toluol) gegeben und 2 h bei dieser Tempewratur gerührt. Nach vollständiger Reaktion (DC-Kontrolle) wurde Methanol (20 ml) bei -70°C zugegeben und der Reaktionsansatz auf RT erwärmt. Es wurde eine gesättigte Natriumchlorid-Lösung zugegeben (30 ml) und die Mischung über Kieselgel filtriert. Es wurde mit Ethylacetat nachgewaschen, die wäßrige Phase abgetrennt und nocheinmal mit Ethylacetat extrahiert. Die vereinigten organischen Phasen wurden mit gesättigter Natriumchlorid-Lösung gewaschen, anschließend über Natriumsulfat getrocknet und eingeengt. Das Rohprodukt wurde ohne weitere Aufreinigung weiter eingesetzt.

Stufe 2. tert-Butyl 4-formylpiperidin-1-carboxylat (15 mmol), N-Methylpiperazin (15 mmol) und Benzotriazol (15 mmol) wurden in Benzol (60 ml) wurde am Wasserabscheider nach Dean-Stark unter Rückfluss erhitzt. Das Lösungsmittel wurde anschließend unter vermindertem Druck abgezogen. Das erhaltene Rohprodukt wurde ohne weitere Aufreinigung weiter verwendet.

Stufe 3. Zu einer Lösung des entsprechenden Gringnard-Reagenzes in THF (60 mmol) wurde tert-Butyl-4-((1H-benzo[d][1,2,3]triazol-1-yl)(4-methylpiperazin-1-yl)methyl)piperidin-1-carboxylat (12 mmol) in THF tropfenweise bei 0°C zugegeben. Die Reaktionsmischung wurde auf 25 °C erwärmt und bei dieser Temperatur 16 h gerührt (DC-Kontrolle). Anschließend wurde auf 0°C abgekühlt, mit gesättigter Ammoniumchlorid-Lösung versetzt und mit Ethylacetat extrahiert. Die organische Phase wurde nacheinander mit Wasser und gesättigter Natriumchlorid-Lösung gewaschen und über Natriumsulfat getrocknet. Das Lösungsmittel wurde entfernt und das erhaltene Rohprodukt säulenchromatographisch aufgereinigt (Kieselgel, DCM /Methanol, 98 : 2 → 95 : 5)

Stufe 4. Die Boc-geschützte Verbindung wurde bei 0°C mit TFA (20% in DCM, 5 ml/mmol) versetzt und anschließend bei Raumtemperatur 3 h gerührt (DC-Kontrolle). Das Lösungsmittel wurde vollständig entfernt und das Rohprodukt (TFA-Salz) wurde ohne weitere Aufreinigung weiter verwendet.

Nach dem oben beschreibenen Verfahren wurden die folgenden Amin-Bausteine hergestellt:

| | Name | R³ |
|---|---|---|
| **A31** | 1-((3-Fluorophenyl)(piperidin-4-yl)methyl)-4-methylpiperazin | 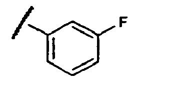 |
| **A32** | 1-((4-Fluorophenyl)(piperidin-4-yl)methyl)-4-methylpiperazin | 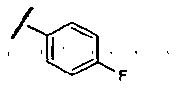 |
| **A33** | 1-Methyl-4-(phenyl(piperidin-4-yl)methyl)piperazin | 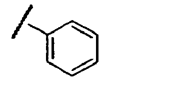 |
| **A34** | 1-Methyl-4-(2-phenyl-1-(piperidin-4-yl)ethyl)piperazin | 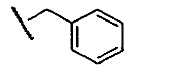 |
| **A35** | 1-Methyl-4-(3-phenyl-1-(piperidin-4-yl)propyl)piperazin | 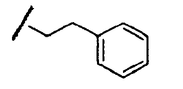 |

### b) Herstellung der Säurebausteine

### Beispiele der Säurebausteine (Tabelle 2)

- **S1**: 2-(2-(3,4-Dichlorphenylsulfonyl)-1,2,3,4-tetrahydroisochinolin-1-yl)essigsäure
- **S2**: 2-(2-(3,4-Dichlor-N-methylphenylsulfonamido)phenyl)essigsäure
- **S3**: 3-((3,4-Dichlor-N-methylphenylsulfonamido)methyl)benzoesäure
- **S4**: 2-(3,4-Dichlor-N-methylphenylsulfonamido)benzoesäure
- **S5**: 2-(3,4-Dichlor-N-methylphenylsulfonamido)-4,5,6,7-tetrahydrobenzo [b]thiophen-3-carbonsäure
- **S6**: 1-(3,4-Dichlorphenylsulfonyl)indolin-2-carbonsäure
- **S7**: 1-(4-Methoxyphenylsulfonyl)indoline-2-carbonsäure
- **S8**: 2-(1-(3-(Trifluormethyl)phenylsulfonyl)piperidin-2-yl)essigsäure
- **S9**: 1-(4-Methoxy-N-methylphenylsulfonamido)cyclohexanecarbonsäure
- **S10**: 5 -(2,4,6-Trichlor-*N*-methylphenylsulfonamido)pentansäure
- **S11**: 2-(1-(2,4-Dichlorphenylsulfonyl)-3-oxopiperazin-2-yl)essigsäure
- **S12**: 2-(1-(3,4-Dichlorphenylsulfonyl)-3-oxo-1,2,3,4-tetrahydrochinoxalin-2-yl)essigsäure
- **S13**: 2-(1-(3,4-Dichlorphenylsulfonyl)pyrrolidin-2-yl)essigsäure
- **S14**: 2-(1-(3,4-Dichlorphenylsulfonyl)piperidin-2-yl)essigsäure
- **S15**: 2-(2-(4-Methoxyphenylsulfonyl)-1,2,3,4-tetrahydroisochinolin-1-yl)essigsäure
- **S16**: 3-(Naphthalin-2-sulfonamido)-3-phenylpropionsäure
- **S17**: 3-(1-(4-Chlor-2,5-dimethylphenylsulfonyl)piperidin-2-yl)propionsäure

### Synthese der 2-(2-(3,4-dichlorphenylsulfonyl)-1,2,3,4-tetrahydroisochinolin-1-yl)-essigsäure S1

Zu einer gerührten Lösung 1,2,3,4-Tetrahydroisochinolin (20,80 g, 156 mmol) in DCM (400 ml) wurde N-Bromosuccinimid (30,6 g, 172 mmol) portionsweise über 15 min. zugegeben. Die Reaktionslösung wurde so lange gerührt, bis das Edukt vollständig abreagiert war (DC-Kontrolle). Anschließend wurde NaOH (100 ml einer 30% wäßrigen Lösung) zugegeben und 1 h bei Raumtemperatur gerührt. Die Phasen wurden getrennt und die organische Phase mit Wasser gewaschen (200 ml). Das Produkt wurde mit HCl (10% wässrige Lösung, 2 x 200 ml) extrahiert, und die vereinigten sauren wässrigen Lösungen mit DCM gewaschen. Anschließend wurde mit Ammoniak basisch gestellt (pH 9) und das abgeschiedene Öl mit DCM (3 x 200 ml) extrahiert. Nach der Trocknung über Natriumsulfat wurde eingeengt und ein gelbliches Öl erhalten.
Ausbeute: (20,0 g, 98%)

### 2-(1,2,3,4-Tetrahydroisochinolin-1-yl)essigsäure

3,4-Dihydroisochinolin (20,0 g, 152 mmol) und Malonsäure (15,9 g, 152 mmol) wurden bei Raumtemperatur gut durchmischt. Die Mischung wurde in ein auf 120°C vorgeheiztes Ölbad getaucht und manuell weiter durchmischt. Nach 30 min war keine Gasentwicklung mehr zu beobachten und die Mischung wurde auf Raumtemperatur abgekühlt. Der feste Rückstand (29,0 g) wurde direkt aus wässrigem 2-Propanol umkristallisiert.
Ausbeute: 16,2 g, 56%

### 2-(1,2,3,4-Tetrahydroisochinolin-1-yl)essigsäuremethylester

2-(1,2,3,4-Tetrahydroisochinolin-1-yl)essigsäure (7,90 g, 41,3 mmol) wurde in Methanol (200 ml) gelöst und unter Stickstoff-Atmosphäre mit H₂SO₄ (4,4 ml, 82,6 mmol) versetzt. Die Reaktionslösung wurde unter Rückfluß erhitzt und nach beendeter Reaktion über Nacht bei Raumtemperatur gerührt. Das Methanol wurde im Vakuum entfernt und der entstandene Rückstand in Ethylacetat (200 ml) aufgenommen. Die Lösung wurde mit gesättigter NaHCO₃ -Lösung (150 ml) gewaschen, getrocknet (Natriumsulfat) und eingeengt.
Ausbeute: 7,50 g (88%)

### Methyl 2-(2-(3,4-dichlorphenylsulfonyl)-1,2,3,4-tetrahydroiso-chinolin-1-yl)-acetat

2-(1,2,3,4-Tetrahydroisochinolin-1-yl)essigsäuremethylester (7,49 g, 36,5 mmol) wurde in DCM gelöst (200 ml) und mit Triethylamin (11,7 ml, 83 mmol) versetzt. Die Reaktionslösung wurde 0°C abgekühlt und eine Lösung 3,4-Dichlorbenzolsulfonylchlorid (8,14 g, 33,2 mmol) in DCM (100 ml) tropfenweise zugegeben. Nach 3 h wurde 0,5 M HCl (100 ml) zugegeben. Nach Phasentrennung wurde die organische Phase mit Wasser gewaschen, getrocknet (Natriumsulfat) und eingeengt. Die Aufreinigung erfogte säulenchromatographisch an Kieselgel (Heptan /Ethylacetat 4:1)
Ausbeute: 14,84 g 2-(2-(3,4-Dichlorphenylsulfonyl)-1,2,3,4-tetrahydroisochinolin-1-yl)-essigsäure S1

Zu einer Mischung aus Methyl 2-(2-(3,4-Dichlorphenylsulfonyl)-1,2,3,4-tetrahydroisochinolin-1-yl)-acetat (14,84 g, 33,2 mmol), THF (200 ml) und Wasser (120 ml) wurde eine und bei Raumtemperatur gerührt. Nach 4 h wurde erneut wäßrige NaOH-Lösung (6 M, 60 ml) gegeben und über Nacht bei Raumtemperatur gerührt. Die Reaktionslösung wurde bei vermindertem Druck eingeengt. Es wurde 6 M HCl-Lösung (125 ml) und DCM (400 ml) zugegeben. Nach Phasentrennung wurde die organische Phase mit gesättigter NaCl-Lösung gewaschen, getrocknet (Natriumsulfat), und eingeengt. Zur Aufreinigung wurde aus 2-Propanol umkristallisiert.
Ausbeute: 11,30 g

### 2-(2-(3,4-Dichlor-N-methylphenylsulfonamido)phenyl)-essigsäure S2

Zu einer Lösung 2-Aminophenylessigsäure (7,2 g, 47,6 mmol) in Methanol (150 ml) wurde unter rühren und Eiskühlung tropfenweise Thionylchlorid (5,2 ml, 71,4 mmol) zugegeben. Der Reaktionsansatz wurde über Nacht bei Raumtemperatur gerührt. Die Reaktionslösung wurde aufkonzentriert und die Reste des Thionylchlorids durch ausschleppen mit Toluol und DCM entfernt. Es wurden 10,3 g (verunreinigt mit Edukt) des als brauner Feststoff erhalten. Das Rohprodukt wurde ohne weitere Aufreinigung weiterverwendet.

### 2-(2-(3,4-Dichlorphenylsulfonamido)phenyl)essigsäuremethylester

Der 2-Aminophenylessigsäuremethylester (10,2 g, 39,45 mmol) wurde in DCM gelöst (200 ml). Anschließend wurden zunächst Pyridin (12,4 ml, 151,71 mmol) und 3,4-Dichlorbenzolsulfonylchlorid (11,8 ml, 75,87 mmol) in DCM (50 ml) zugegeben. Die Reaktionslösung wurde über Nacht gerührt und anschließend mit DCM verdünnt und nacheinander mit 0,5 M KHSO₄-Lösung, gesättigter NaHCO₃-Lösung und gesättigter NaCl-Lösung gewaschen, über Natriumsulfat getrocknet und eingeengt. Die Aufreinigung erfolgte säulenchromatographisch an Kieselgel (DCM).
Ausbeute: 14,3 g (96%)

### 2-(2-(3,4-Dichlor-N-methylphenylsulfonamido)phenyl)-essigsäuremethylester

Der 2-(2-(3,4-Dichlorphenylsulfonamido)phenyl)essigsäure-methylester (14,3 g, 38,21 mmol) und Methyliodid (8,2 ml, 132,25 mmol) wurden in Aceton (300 ml) gelöst, mit K₂CO₃ (7,3 g, 52,90 mmol) versetzt und über Nacht bei 40°C in einem geschlossenen Kolben gerührt. Die Suspension wurde nach dem Abkühlen filtriert, aufkonzentriert, über Kieselgel filtriert (Eluent DCM) und aufkonzentriert.
Ausbeute: 15,3 g

### 2-(2-(3,4-Dichlor-N-methylphenylsulfonamido)phenyl)-essigsäure S2

Der 2-(2-(3,4-Dichlor-N-methylphenylsulfonamido)phenyl)essigsäuremethylester (13,7 g, 34,26 mmol) wurde in einer Mischung aus Methanol / Dioxan / 4 M NaOH (15/4/1) (266 ml, 52,92 mmol NaOH) gelöst und anschließend weitere 4 M NaOH (39,7 ml, 158,76 mmol) zugegeben. Die entstandene klare Lösung wurde über Nacht bei Raumtemperatur gerührt und anschließend im Vakuum aufkonzentriert. Der Rückstand wurde in Ethylacetat aufgenommen und mit 0,5 M KHSO₄ gewaschen. Die wässrige Phase wurde noch mehrfach mit Ethylacetat extrahiert. Die vereinigten organischen Phasen wurden mit gesättigter, NaCl-Lösung gewaschen, über Natriumsulfat getrocknet in aufkonzentriert.
Ausbeute: 13,0 g

### Synthese der 3-((3,4-Dichlor-N-methylphenylsulfonamido)methyl)benzoesäure S3

### 3,4-Dichlor-N-methylbenzolsulfonamid

Methylamin Hydrochlorid (5,5 g, 81,46 mmol) wurde in DCM (300 ml) gelöst und mit Et₃N (40 ml, 285,11 mmol) versetzt. Anschließend wurde die Reaktionslösung auf 0°C abgekühlt und tropfenweise 3,4-Dichlorbenzolsulfonylchlorid (20,00g, 81,46 mmol) gelöst in DCM (50 ml) zugegeben und bei Raumtemperatur über Nacht gerührt (DC-Kontrolle, Kieselgel, Ethylacetat). Nach beendeter Reaktion wurde 0,5 N HCl zugegeben, die Phasen getrennt und die organische Phase mit Wasser gewaschen und getrocknet (Natriumsulfat). Das Lösungsmittel wurde am Rotationsverdampfer entfernt. Die Aufreinigung erfolgte säulenchromatographisch an Kieselgel (Gradient Heptan / Ethylacetat 4 : 1 bis 2 : 1)
Ausbeute: 13,0 g (66%)

### Methyl 3((3,4-dichlor-N-methylphenylsulfonamido)methyl)-benzoat

Das 3,4-Dichlor-N-methylbenzolsulfonamid (12,9 g, 53,72 mmol) wurde in Aceton (200 ml) gelöst und mit K₂CO₃ (14,8 g, 107,44 mmol) versetzt. Anschließend wurde Methyl (3-bromomethyl)benzoat (24,6 g, 107,44 mmol) zugegeben und die Suspension über Nacht auf 40 °C erhitzt. Nach dem Abkühlen auf Raumtemperatur wurde der Feststoff abfiltriert und das Filtrat am Rotationsverdampfer eingeengt.
Die Aufreinigung erfolgte säulenchromatographisch an Kieselgel (Heptan /Ethylacetat 4:1).
Ausbeute: 19,9 g (95%)

### 3-((3,4-Dichlor-N-methylphenylsulfonamido)methyl)-benzoesäure S3

Das Methyl 3-((3,4-dichtor-N-methylphenylsulfonamido)methyl)benzoat (19,9 g, 51,25 mmol) wurde in einer Mischung aus Methanol / Dioxan / 4M NaOH (15/4/1, (384 ml, 76,88 mmol NaOH, 1,5 eq), gelöst und mit weiterer 4 M NaOH (57,7 ml, 230,63mmol, 4,5 eq) versetzt. Nach dem Rühren über Nacht bei Raumtemperatur wurde am Rotationsverdampfer aufkonzentriert. Der Rückstand wurde mit Ethylacetat (300 ml) versetzt und mit 0,5 M KHSO₄ gewaschen. Die wässrige Phase wurde noch einmal mit Ethylacetat (200 ml) extrahiert Die vereinigten organischen Phasen wurden getrocknet (Natriumsulfat) und im Vakuum aufkonzentriert.
Es wurde erneut Ethylacetat zugegeben, die Suspension filtriert und eingeengt.
Ausbeute: 11,6 g (60 %)

### Synthese der 2-(3,4-Dichlor-N-methylphenylsulfonamido)benzoesäure S4

### Methyl2-(3,4-dichlorphenylsulfonamido)benzoat

Methyl 2-Aminobenzoat (10 g, 66,2 mmol) wurde in DCM (100 ml) gelöst und mit Pyridin (8,1 ml, 99,2 mmol, 1,5 eq) versetzt. Anschließend wurde eine Lösung des 3,4-Dichlorbenzolsulfonylchlorids (24,4 g, 99,2 mmol, 1,5 eq) in DCM (150 ml) tropfenweise bei 0 °C zugegeben und die Reaktionsmischung über Nacht bei Raumtemperatur gerührt. Die DC-Kontrolle (Kieselgel, DCM) ergab vollständigen Umsatz. Nach beendeter Reaktion wurde die Reaktionslösung mit DCM (250 ml) verdünnt und mit 0,5 M KHSO₄ (500 ml), gesättigter NaHCO₃ (500 ml) und gesättigter NaCl-Lösung (500 ml) gewaschen. Die organische Phase wurde über Natriumsulfat getrocknet und im Vakuum aufkonzentriert. Zur Aufreinigung wurde über eine dünne Schicht Kieselgel filtriert (Gradient: Heptan / DCM (3:1) bis DCM) Ausbeute: 21,9 g (91%)

### Methyl 2-(3,4-dichlor-N-methylphenylsulfonamido)benzoat

Das Methyl 2-(3,4-dichlorphenylsulfonamido)benzoat (21,3 g, 59,1 mmol) wurde in Aceton (300 ml) gelöst und mit K₂CO₃ (16,3 g, 118,3 mmol, 2 eq) versetzt. Anschließend wurde Methyliodid (7,4 ml, 118,3 mmol, 2 eq) zugegeben und die Suspension über Nacht auf 40 °C erhitzt. Die Feststoffe wurde abfiltriert und das Filtrat am Rotationsverdampfer eingeengt. Die Aufreinigung erfolgte durch Filtration über Kieselgel (DCM)
Ausbeute: 21,8 g (98%) Produkt wurde direkt weiter eingesetzt.

### 2-(3,4-Dichlor-N-methylphenylsulfonamido)benzoesäure S4

Das Methyl 2-(3,4-dichlor-N-methylphenylsulfonamido)benzoat (21,0 g, 56,1 mmol) wurde in einer Mischung aus Methanol / Dioxan / 4 M NaOH (15/4/1. 420 ml, 84 mmol NaOH, 1,5 eq), gelöst und mit weiterer 4 M NaOH (63 ml, 252 mmol, 4,5 eq) versetzt. Die Lösung wurde bei Raumtemperatur über Nacht gerührt und anschließend am Rotationsverdampfer aufkonzentriert. Der Rückstand wurde mit Ethylacetat (800 ml) versetzt und mit 0,5 M KHSO₄ (1000 ml) gewaschen. Die wässrige Phase wurde noch dreimal mit Ethylacetat (je 350 ml) extrahiert und die vereinigten organischen Phasen mit gesättigter NaCl-Lösung (500 ml) gewaschen. Die organische Phase wurde getrocknet (Natriumsulfat) und im Vakuum aufkonzentriert
Der Rückstand wurde mit Diisopropylether gewaschen und über Nacht bei 40°C im Trockenschrank getrocknet.
Ausbeute: 18,83 g (90%)

### Synthese der 2-(3,4-Dichlor-N-methylphenylsulfonamido)-4,5,6,7-tetrahydrobenzo[b]thiophen-3-carbonsäure S5

### tert-Butyl-2-amino-4,5,6,7-tetrahydrobenzo[b]thiophen-3-carboxylat

Zu einer Mischung aus Cyclohexanon (5,00 g, 50,9 mmol), *t*-Butylcyanoacetat (7,91 g, 56,0 mmol) und Schwefel (1,80 g, 56,0 mmol) in Ethanol (abs., 150 ml) wurde Morpholin (6,7 ml, 76 mmol) gegeben. Die Reaktionslösung wurde bei 50°C über Nacht gerührt und anschließend auf Raumtemperatur abgekühlt. Nach Filtration wurde des Filtrat aufkonzentriert und der Rückstand in Ethylacetat (100 ml) aufgenommen, mit Wasser (2x 50 ml), gesättigter NaCl-Lösung (2x 50 ml), gewaschen, getrocknet (Natriumsulfat) und aufkonzentriert. Die Aufreinigung erfolgte säulenchromatographisch an Kieselgel (Heptan / Ethylacetat =10/1)
Ausbeute: 12,73 g (99%)

### tert-Butyl 2-(3,4-dichlorphenylsulfonamido)-4,5,6,7-tetrahydrobenzo[b]thiophen-3-carboxylat

Das tert-Butyl-2-amino-4,5,6,7-tetrahydrobenzo[b]thiophen-3-carboxylat (12,26 g, 48,4 mmol) wurde in DCM (150 ml) gelöst und mit Pyridin (6,0 ml, 74 mmol) versetzt. Anschließend wurde eine Lösung des 3,4-Dichlorbenzolsulfonylchlorids (8,4 ml, 54 mmol) zugegeben, 17 h unter Rückfluß erhitzt und anschließend auf Raumtemperatur abgekühlt. Es wurde Wasser zugegeben (100 ml) und über Nacht bei Raumtemperatur gerührt. Anschließend wurden die Phasen getrennt (pH organische Phase = 2), mit gesättigter NaHCO₃ -Lösung gewaschen, getrocknet (Natriumsulfat) und im Vakuum aufkonzentriert.
Ausbeute: (21,64 g, 97%)

### tert-Butyl2-(3,4-dichlor-N-methylphenylsulfonamido)-4,5,6,7-tetrahydrobenzo[b] thiophen-3-carboxylat

Das tert-Butyl 2-(3,4-dichlorphenylsulfonamido)-4,5,6,7-tetrahydrobenzo[b]thiophen-3-carboxylat (21,64 g, 46,8 mmol) wurde in Aceton (200 ml) gelöst und mit K₂CO₃ (12,9 g, 93,6 mmol) versetzt. Anschließend wurde Methyliodid (5,83 ml, 93,6 mmol) zugegeben und die Suspension über Nacht auf 40°C erhitzt. Nach dem Abkühlen auf Raumtemperatur wurde Wasser und gesättigte NaCl-Lösung zugegeben und das Produkt mit Ethylacetat extrahiert. Die organische Phase wurde getrocknet (Natriumsulfat) und das Produkt durch Kristallisation aus Methanol aufgereinigt.
Ausbeute: (16,47 g, 74%)
Die Mutterlauge wurde eingeengt und weiteres Produkt durch Kristallisation aus Methanol erhalten.
Ausbeute: 1,57g, 7%
Gesamtausbeute: 18,04g (81%)

### 2-(3,4-Dichlor-N-methylphenylsulfonamido)-4,5,6,7-tetrahydrobenzo[b]thiophen-3-carbonsäure S5

Zu einer Lösung aus tert-Butyl 2-(3,4-dichlor-N-methylphenylsulfonamido)-4,5,6,7-tetrahydrobenzo[b]thiophen-3-carboxylat (8,00 g, 16,8 mmol) in DCM (100 ml) wurde TFA (30 ml, 404 mmol) gegeben und 1 h bei Raumtemperatur gerührt. Der Reaktionsansatz wurde aufkonzentriert und die verbliebene TFA mit Toluol, DCM und Diethylether ausgeschleppt. Der Rückstand wurde zweimal mit Diisopropylether gewaschen.
Ausbeute: 6,49 g, 92%

### Synthese der 1-(3,4-Dichlorphenylsulfonyl)indolin-2-carbonsäure S6

### Indolin-2-carbonsäuremethylester

Zu einer Lösung Indolin-2-carbonsäure (24,8 g, 152,0 mmol) in Methanol (500 ml) wurde unter Rühren und Eiskühlung tropfenweise Thionylchlorid (12,1 ml, 167,2 mmol)) zugegeben. Der Reaktionsansatz wurde über Nacht bei 40°C gerührt. Die Reaktionslösung wurde aufkonzentriert und die Reste des Thionylchlorids durch ausschleppen mit Diethylether (3 mal) entfernt
Ausbeute: 33,5 g

### Methyl1-(3,4-dichlorphenylsulfonyl)indolin-2-carboxylat

Indolin-2-carbonsäuremethylester (33,0 g, 149,7 mmol) wurde in DCM (400 ml) gelöst und mit Pyridin (37,8 ml, 463,2 mmol) versetzt. Anschließend wurde eine Lösung des 3,4-Dichlorbenzolsulfonylchlorids (24,1 ml, 154,4 mmol) gelöst in DCM (100 ml) zugegeben, über Nacht unter Rückfluß erhitzt und anschließend auf Raumtemperatur abgekühlt. Es wurde mit DCM verdünnt und nacheinander mit 0,5 M KHSO₄, gesättigter NaHCO₃-Lösung und gesättigter NaCl-Lösung gewaschen. Die organische Phase wurde getrocknet (Natriumsulfat) und im Vakuum aufkonzentriert Der Rückstand wurde säulenchromatographisch an Kieselgel (Heptan / Ethylacetat = 2:1) aufgereinigt.
Ausbeute: 50,4 g (87%)

### 1-(3,4-Dichlorphenylsulfonyl)indolin-2-carbonsäure 56

Das Methyl 1-(3,4-dichlorphenylsulfonyl)indolin-2-carboxylat (49,8 g, 128,92 mmol) wurde in einer Mischung aus Methanol / Dioxan / 4 M NaOH (15/4/1, (960 ml, 193,38 mmol NaOH, 1,5 eq), gelöst und mit weiterer 4 M NaOH (145 ml, 580,14 mmol, 4,5 eq) versetzt. Die Lösung wurde bei Raumtemperatur über Nacht gerührt und anschließend am Rotationsverdampfer aufkonzentriert. Der Rückstand wurde mit Ethylacetat versetzt und mit 0,5 M KHSO₄ gewaschen. Die wässrige Phase wurde noch dreimal mit Ethylacetat extrahiert und die vereinigten organischen Phasen mit gesättigter NaCl-Lösung (500 ml) gewaschen, getrocknet (Natriumsulfat) und im Vakuum aufkonzentriert
Der Rückstand wurde mit Diethylether gewaschen, filtriert und getrocknet.
Ausbeute: 22,3 g (46%)

### Synthese der 1-(4-Methoxyphenylsulfonyl)indolin-2-carbonsäure S7 Methyl 1-(4-methoxyphenylsulfonyl)indolin-2-carboxylat

Indolin-2-carbonsäuremethylester (32,7 g, 151,8 mmol) wurde in DCM (400 ml) gelöst und mit Pyridin (37,4 ml, 459,0 mmol) versetzt. Anschließend wurde eine Lösung des 4-Methoxybenzolsulfonylchlorids (31,6 g, 153 mmol) gelöst in DCM (100 ml) zugegeben und über Nacht bei Raumtemperatur gerührt. Es wurde mit DCM verdünnt und nacheinander mit 0,5 M KHSO₄, gesättigter NaHCO₃-Lösung und gesättigter NaCl-Lösung gewaschen. Die organische Phase wurde getrocknet (Natriumsulfat) und im Vakuum aufkonzentriert.

Der Rückstand wurde säulenchromatographisch an Kieselgel (Heptan / Ethylacetat, 2:1) aufgereinigt.
Ausbeute: 47,1 g (89%)

### 1-(4-Methoxyphenylsulfonyl)indolin-2-carbonsäure S7

Das Methyl 1-(4-methoxyphenylsulfonyl)indolin-2-carboxylat (47,1 g, 135,6 mmol) wurde in einer Mischung aus Methanol / Dioxan / 4 M NaOH (15/4/1, 1020 ml, 203,4 mmol NaOH, 1,5 eq), gelöst und mit weiterer 4 M NaOH (153 ml, 610,2 mmol, 4,5 eq) versetzt. Die Lösung wurde bei Raumtemperatur über Nacht gerührt und anschließend am Rotationsverdampfer aufkonzentriert. Der Rückstand wurde mit Ethylacetat versetzt und mit 0,5 M KHSO₄ gewaschen. Die wässrige Phase wurde noch einmal mit Ethylacetat extrahiert und die vereinigten organischen Phasen mit gesättigter NaCl-Lösung gewaschen, getrocknet (Natriumsulfat) und im Vakuum aufkonzentriert. Der Rückstand wurde mit Diethylether gewaschen, filtriert und getrocknet.
Ausbeute: 38,1 g (84%)

### Synthese der 2-(1-(3-(Trifluormethyl)phenylsulfonyl)piperidin-2-yl)essigsäure S8

### 2-(Piperidin-2-yl)essigsäure

Zu einer Lösung von 2-Piperidinethanol (10,0 g, 10,1 ml, 77,4 mmol) in Wasser (10 ml) wurde unter Eiskühlung innerhalb von 1,5 h eine Lösung von Chromtrioxid (20 g, 200 mmol) in Wasser (300 ml) und konzentrierter Schwefelsäure (40 g, 73,6 ml) getropft. Das Reaktionsgemisch wurde 3 h bei Raumtemperatur gerührt. Die Lösung wurde anschließend mit gesättigter, wässriger Bariumhydroxidlösung alkalisch gestellt. Danach wurde in die Lösung Kohlenstoffdioxid eingeleitet und die entstandene Suspension durch Celite filtriert. Das Filtrat wurde i. Vak. eingeengt. Ausbeute: 9,26 g (83 %)
¹H-NMR (D₂O): 1,50-1,70 (m, 3H); 1,80-1,93 (m, 2H); 1,93-2,02 (m, 1H); 2,76 (d, 2H); 3,04 (dt, 1 H); 3,38-3,45 (m, 1H); 3,45-3,57 (m, 1H).

### 2-(1-(3-(Trifluormethyl)phenylsulfonyl)piperidin-2-yl)essigsäure S8

Eine Lösung von 2-(Piperidin-2-yl)essigsäure (1,43 g, 10 mmol) in 1,4-Dioxan (5 ml) und 1 *N* Natronlauge (22 ml) wurde innerhalb 1 h langsam mit einer Lösung von 3-Trifluormethylbenzolsulfonsäurechlorid (2,70 g, 11 mmol) in 1,4-Dioxan (10 ml) versetzt. Das Gemisch wurde anschließend 4,5 h bei Raumtemperatur gerührt, dann mit 1 *N* Salzsäure angesäuert und danach mit DCM (3 × 60 ml) extrahiert. Die vereinigten organischen Phasen wurden mit Natriumsulfat getrocknet und i. Vak. eingeengt.
Ausbeute: 917 mg (26 %)
¹H-NMR (DMSO-d₆): 1,05-1,55 (m, 6H); 2,25 (dd, 1 H,); 2,66 (dd, 1 H); 2,99 (dt, 1 H); 3,69 (br dd, 1H); 4,41 (1H, m); 7,87 (1H, t); 8,03-8,08 (m, 2H) 8,13 (d, 1H), 12,33 (br s, 1H).

### Synthese der 1-(4-Methoxy-N-methylphenylsulfonamido)carbonsäure S9

### 1-Amino-cyclohexancarbonsäuremethylester Hydrochlorid

Zu einer auf 0°C abgekühlten Lösung Thionylchlorid (49,6 ml, 684 mmol) in Methanol (750 ml) wurde 1-Amino-cyclohexancarbonsäure (49 g, 342 mmol) gegeben. Der Reaktionsansatz wurde unter Rückfluß 4 h und anschließend bei Raumtemperatur über Nacht gerührt. Anschließend wurde noch einmal 3 h unter Rückfluß erhitzt und abermals über Nacht bei Raumtemperatur gerührt. Die Reaktionslösung wurde aufkonzentriert und die Reste des Thionylchlorids durch ausschleppen mit Methanol und Diethylether entfernt.
Ausbeute: 66,64 g

### Methyl 1-(4-methoxyphenylsulfonamido)-cyclohexancarboxylat

Das 1-Amino-cyclohexancarbonsäuremethylester Hydrochlorid (32 g, 165 mmol) wurde in DCM (1000 ml) suspendiert und mit Diisopropylethylamin (84,9 ml, 496 mmol) versetzt. Bei 0 °C wurde eine Lösung des 4-Methoxybenzolsulfonylchlorids (51,2 g, 248 mmol) gelöst in DCM (150 ml) tropfenweise zugegeben und über Nacht bei Raumtemperatur gerührt. Wässrige 1 M HCl (150 ml) und Wasser (50 ml) wurden zugegeben und die Phasen getrennt. Die organische Phase wurde über Natriumsulfat getrocknet und zur Trockne eingeengt. Das Produkt wurde säulenchromatographisch an Kieselgel aufgereinigt (DCM / Methanol 99 : 1)

Die Mischfraktionen wurden erneut säulenchromatographisch an Kieselgel gereinigt (Gradient: DCM / Heptan 5 : 1 bis DCM / Methanol 99 : 1)
Gesamtausbeute: 37,8 g (70%)

### Methyl 1-(4-methoxy-N-methylphenylsulfonamido)cyclohexanecarboxylat

Das Methyl 1-(4-methoxyphenylsulfonamido)cyclohexancarboxylat (27 g, 82,5 mmol) wurde in Aceton (450 ml) gelöst und mit K₂CO₃ (22,8 g, 165 mmol) versetzt. Anschließend wurde Methyliodid (10,3 ml, 165 mmol) zugegeben und die Suspension über Nacht auf 40 °C in einem verschlossenen Kolben erhitzt. Da die Reaktion noch nicht abgeschlossen war (DC-Kontrolle, (Heptane / Ethylacetat 2:1), wurde erneut Methyliodid (7,7 ml, 124 mmol) zugegeben und über das Wochende bei Raumtemperatur gerührt. Es wurde erneut Methyliodid (2,6 ml, 41,8 mmol) zugegeben und über Nacht bei 40°C gerührt. Nach dem Abkühlen wurden die Feststoffe abfiltriert und das Filtrat zur Trockne eingeengt. Das Rohprodukt wurde ohne weitere Aufreinigung weiterverwendet.

### 1-(4-Methoxy-N-methylphenylsulfonamido)carbonsäure S9

Das Methyl 1-(4-methoxy-N-methylphenylsulfonamido)cyclohexancarboxylat (28,2 g, 82,5 mmol) wurde in einer Mischung aus Methanol / Dioxan / 4 M NaOH (15/4/1, (620 ml, 124 mmol NaOH, 1,5 eq), gelöst und mit weiterer 4 M NaOH (93 ml, 372 mmol, 4,5 eq) versetzt. Da nach dem Rühren über Nacht bei Raumtemperatur keine Reaktion festgestellt werden konnte, wurde zunächst für 8 h auf 68°C erhitzt, über das Wochenende bei Raumtemperatur und über Nacht erneut refluxiert. Anschließend wurde am Rotationsverdampfer aufkonzentriert. Der Rückstand wurde mit Ethylacetat (500 ml) versetzt und mit 0,5 M KHSO₄ (500 ml) gewaschen. Die wässrige Phase wurde noch einmal mit Ethylacetat (200 ml) extrahiert, getrocknet (Natriumsulfat) und im Vakuum aufkonzentriert. Die wässrige Phase wurde mit HCl (2 M, 300 ml) angesäuert und dreimal mit DCM (je 300 ml) extrahiert. Gesamtausbeute: 26,5 g

### Synthese der 5-(2,4,6-Trichlor-N-methylphenylsulfonamido)pentansäure S10

### 5-(2,4,6-Trichlor-phenylsulfonamido)pentansäure

Zu einer Lösung von 5-Aminovaleriansäure-Hydrochlorid (1,50 g, 10 mmol) in 1 *N* Natriumhydroxidlösung (30 ml) wurde eine Lösung von 2,4,6-Trichlorbenzolsulfonsäurechlorid (3,00 g, 11 mmol) in 1,4-Dioxan (50 ml) getropft. Das Reaktionsgemisch wurde 18 h bei Raumtemperatur gerührt und 1,4-Dioxan anschließend i. Vak. abdestilliert. Die wässrige Phase wurde mit Ethylacetat extrahiert (3 × 50 ml) und anschließend mit konzentrierter Salzsäure auf pH 1 eingestellt. Die saure wässrige Phase wurde mit Ethylacetat (3 × 60 ml) extrahiert. Die vereinigten organischen Phasen wurden mit Natriumsulfat getrocknet und i. Vak. eingeengt.
Ausbeute: 2,28 g (64 %)
¹H-NMR (DMSO-d₆): 1,33-1,57 (m, 4H); 2,12 (t, 2H); 2,87 (q, 2H); 4,00 (sehr br s, 1 H); 7,85 (s, 1 H); 8,22 (t, 1 H).

### Methyl 5-(2,4,6-trichlor-N-methylphenylsulfonamido) pentanoat

Eine Lösung von-5-(2,4,6-Trichlorphenylsulfonamido)pentansäure (250 mg, 0,69 mmol) in einem 1:1 Gemisch aus *N,N*-Dimethylformamid / Aceton (10 ml) wurde mit Cäsiumcarbonat (449 mg, 1,38 mmol) und anschließend Methyliodid (487 mg, 213 µL, 3,45 mmol) versetzt und 4 h bei 50 °C gerührt. Das Reaktionsgemisch wurde danach i. Vak. eingeengt, der Rückstand wiederholt (3 ×) mit Toluol versetzt, jeweils wieder i. Vak. eingeengt, dann in 5 % Natriumhydrogencarbonat-Lösung aufgenommen und mit Ethylacetat (3 × 30 ml) extrahiert. Die vereinigten organischen Phasen wurden mit Natriumsulfat getrocknet und i. Vak. eingeengt.
Ausbeute: 188 mg (69 %),
¹H-NMR (DMSO-d₆): 1,40-1,60 (m, 4H); 2,30 (t, 2H); 2,82 (s, 3H); 3,32 (t, 2H); 3,57 (s, 3H); 7,89 (s, 2H).

### 5-(2,4,6-Trichlor-N-methylphenylsulfonamido)pentansäure S10

Eine Lösung von Methyl 5-(2,4,6-trichlor-*N*-methylphenylsulfonamido)pentanoat (178 mg, 0,45 mmol) in THF (5ml) wurde mit einer Lösung von Lithiumhydroxid (18,2 mg, 0,76 mmol) in Wasser (3 ml) versetzt und 18 h bei Raumtemperatur gerührt. Anschließend wurde THF i. Vak. eingeengt, der Rückstand mit Wasser versetzt und mit DCM (2 × 20 ml) extrahiert. Die wässrige Phase wurde mit konzentrierter Salzsäure auf pH 1-2 eingestellt und mit Ethylacetat (3 × 20 ml) extrahiert. Die vereinigten organischen Extrakte wurde mit Natriumsulfat getrocknet und i. Vak. eingeengt.
Ausbeute: 111 mg (64 %)
¹H-NMR (DMSO-d₆): 1,40-1,58 (m, 4H); 2,21 (t, 2H); 2,83 (s, 3H); 3,24 (t, 2H); 7,89 (s, 2H); 12,0 (s, 1H).

### Synthese 2-(1-(2,4-Dichlorphenylsulfonyl)-3-oxopiperazin-2-yl)essigsäure S11

### Ethyl 2-(3-oxopiperazin-2-yl)acetat

Ethylendiamin (1,17 ml, 17,42 mmol) und Diethylmaleat (3 g, 17,42 mmol) wurden 16h bei 55°C in Propanol (30 ml) gerührt. Das Lösungsmittel wurde im Vakuum abgezogen und der Rückstand im Vakuum getrocknet. Das Produkt wurde ohne weiter Aufreinigung in die näcste Stufe eingesetzt.
Ausbeute: 3,4 g (100%).

### Ethyl 2-(1-(2,4-dichlorphenylsulfonyl)-3-oxopiperazin-2-yl)acetat

Zu einer auf 0°C gekühlten Lösung des Piperazin-Derivats (2,5 g, 13,4 mmol) in DCM (55 ml) und Triethylamin (4,2 ml, 33,5 mmol) wurde eine 2,4-Dichlorbenzolsulfonylchlorid (3 g, 13,4 mmol) in DCM (25 ml) bei dieser Temperatur tropfenweise zugegeben und anschließend mit einer katalytischen Menge DMAP versetzt. Die Reaktionslösung wurde 16h bei Raumtemperatur rühren gelassen und anschließend mit DCM verdünnt. Der Reaktionsansatz wurde erst mit 0,5M HCl und anschließend mit Wasser und gesättigter NaCl-Lösung gewaschen. Die organische Phase wurde mit Natriumsulfat getrocknet und bis zur Trockne eingeengt. Das Produkt wurde säulenchromatographisch gereinigt.
Ausbeute: 2,85 g (58%)

### 2-(1-(2,4-Dichlorphenylsulfonyl)-3-oxopiperazin-2-yl)essigsäure S11

Zu einer gekühlten Lösung Ethyl 2-(1-(2,4-dichlorphenylsulfonyl)-3-oxopiperazin-2-yl)acetat (2,7 g, 7,3 mmol) in Methanol (25 ml) und Wasser (6 ml) wurde Lithiumhydroxid (0,92 mg, 21,9 mmol) gegeben und 16 h bei Raumtemperatur gerührt (DC-Kontrolle). Das Lösungsmittel wurde am Rotationsverdampfer abgezogen und der Rückstand in Wasser aufgenommen. Die wässrige Phase wurde mit Diethylether gewaschen, mit HCl angesäuert und das Produkt mit Ethylacetat extrahiert. Die organische Phase wurde anschließend mit gesättigter NaCl-Lösung gewaschen. Die organische Phase wurde mit Natriumsulfat getrocknet und bis zur Trockne eingeengt.
Ausbeute: 2,5 g (97%)

### 2-(1-(3,4-Dichlorphenylsulfonyl)-3-oxo-1,2,3,4-tetrahydrochinoxalin-2-yl)essigsäure S12

### Methyl 2-(3-oxo-1,2,3,4-tetrahydrochinoxalin-2-yl)acetat

Orthophenylendiamin (10 g, 92,4 mmol) und Diehylmaleat (45 g, 646,8 mmol) wurden 75 h in Propanol refluxiert. Das Lösungsmittel wurde am Rotationsverdampfer abgezogen und der Rückstand säulenchromatographisch gereinigt (Ethylacetat / Hexan 1 : 1)
Ausbeute: 4 g (18,5%)

### Methyl 2-(1-(3,4-dichlorphenylsulfonyl)-3-oxo-1,2,3,4-tetrahydrochinoxalin-2-yl)acetat

Zu einer Lösung (3 g, 12,8 mmol) in DCM (125 ml) und Pyridin (5,17 ml, 64 mmol) wurde eine Lösung 3,4-Dichlorbenzolsulphonylchlorid (4 ml, 25,6 mmol) in DCM (60 ml) tropfenweise zugegeben und anschließend mit einer katalytischen Menge DMAP versetzt. Die Reaktionslösung wurde 16 h bei Raumtemperatur rühren gelassen und anschließend mit DCM verdünnt. Der Reaktionsansatz wurde mit Kupfersulfat-Lösung, 1 M HCl und Natriumcarbonat-Lösung, Wasser und gesättigter NaCl-Lösung gewaschen. Die organische Phase wurde mit Natriumsulfat getrocknet und bis zur Trockne eingeengt. Das Produkt wurde säulenchromatographisch gereinigt (Ethylacetat / Hexan 4:6;. Ausbeute: 3,12 g (55%)

### 2-(1-(3,4-Dichlorphenylsulfonyl)-3-oxo-1,2,3,4-tetrahydrochinoxalin-2-yl)essigsäure S12

Zu einer gekühlten Lösung Methyl 2-(1-(3,4-dichlorphenylsulfonyl)-3-oxo-1,2,3,4-tetrahydrochinoxalin-2-yl)acetat (2,7 g, 7,3 mmol) in THF (45 ml) und Wasser (45 ml) wurde Lithiumhydroxid (0,041g, 10,95 mmol) gegeben und 48 h bei Raumtemperatur gerührt (DC-Kontrolle) Das Lösungsmittel wurde am Rotationsverdampfer abgezogen und der Rückstand in Wasser aufgenommen. Die wässrige Phase wurde mit Ethylacetat extrahiert, und anschließend mit gesättigter NaCl-Lösung gewaschen. Die organische Phase wurde mit Natriumsulfat getrocknet und bis zur Trockne eingeengt.
Ausbeute: 0,6 g (23%)

### 2-(1-(3,4-Dichlorphenylsulfonyl)pyrrolidin-2-yl)essigsäure. S13

### Pyrrolidin-2-ylessigsäuremethylester

Zu einer auf 0°C gekühlten Lösung Pyrrolidin-2-ylessigsäure Hydrochlorid (1,4 g, 8,4 mmol) in Methanol (35 ml) wurde Thionylchlorid (1,8 ml, 25,2 mmol) tropfenweise zugegeben und 16 h zum gelinden sieden erhitzt und anschließend auf Raumtemperatur abgekühlt. Das Lösungsmittel wurde azeotrop im Vakuum mit Benzol abgezogen. Ausbeute: 1,5 g (100 %).

### Methyl 2-(1-(3,4-dichlorphenylsulfonyl)pyrrolidin-2-yl)acetat

Zu einer auf 0°C gekühlte Lösung Pyrrolidin-2-ylessigsäuremethylester (1,5 g, 8,4 mmol) in DCM (33 ml) und Triethylamin wurde (2,66 ml, 21 mmol) wurde tropfenweise eine Lösung
3,4-Dichlor benzolsulfonylchlorid (1,88 g, 7,65 mmole) in DCM (15 ml) bei 0°C zugegeben und anschließend 90 Minuten bei Raumtemperatur gerührt. Anschließend wurde 0,5M HCl (20 ml) zugegeben und weitere 15 Minuten gerührt. Die organische Phase wurde abgetrennt, mit Wasser gewaschen und über Natriumsulfat getrocknet. Das Lösungsmittel wurde am Rotationsverdampfer entfernt und das Rohprodukt ohne weitere Aufreinigung in die nächste Stufe eingesetzt. Ausbeute: 2,1 g (77%)

### 2-(1-(3,4-Dichlorphenylsulfonyl)pyrrolidin-2-yl)essigsäure S13

Zu einer gekühlten Lösung Methyl 2-(1-(3,4-dichlorphenylsulfonyl)pyrrolidin-2-yl)acetat (2,1 g, 6 mmol) in Methanol (20 ml) und Wasser (20 ml) wurde Lithiumhydroxid (0,75 mg, 18 mmol) gegeben und 16 h bei Raumtemperatur gerührt (DC-Kontrolle) Das Lösungsmittel wurde am Rotationsverdampfer abgezogen und der Rückstand in Wasser aufgenommen. Die wässrige Phase wurde mit Ethylacetat gewaschen, mit 1 M HCl angesäuert, anschließend das Produkt mit Ethylacetat extrahiert und die organische Phase mit gesättigter NaCl-Lösung gewaschen. Die organische Phase wurde mit Natriumsulfat getrocknet und bis zur Trockne eingeengt. Ausbeute: 2,0 g (99%)

### 2-(1-(3,4-Dichlorphenylsulfonyl)piperidin-2-yl)essigsäure S14 Piperidin-2-ylessigsäuremethylester Hydrochlorid

Zu einer auf 0°C gekühlten Lösung Piperidin-2-ylessigsäure Hydrochlorid (1,5 g, 8,4 mmol) in Methanol (35 ml) wurde Thionylchlorid (1,8 ml, 25,2 mmol) tropfenweise zugegeben und 16h zum gelinden sieden erhitzt und anschließend auf Raumtemperatur abgekühlt. Das Lösungsmittel wurde azeotrop im Vakuum mit Benzol abgezogen. Ausbeute: 1,3 g (100 %)

### Methyl 2-(1-(3,4-dichlorphenylsulfonyl)piperidin-2-yl)acetat

Zu einer auf 0°C gekühlte Lösung Piperidin-2-ylessigsäuremethylester Hydrochlorid (1,3 g, 8,4 mmol) in DCM (33 ml) und Triethylamin (2,66 ml, 21 mmol) wurde wurde tropfenweise eine Lösung
3,4-Dichlorbenzolsulfonylchlorid (1,88 g, 7,65 mmol) in DCM (15 ml) bei 0°C zugegeben und anschließend 90 Minuten bei Raumtemperatur gerührt. Anschließend wurde 0,5M HCl (20 ml) zugegeben und weitere 15 Minuten gerührt. Die organische Phase wurde abgetrennt, mit Wasser gewaschen und über Natriumsulfat getrocknet. Das Lösungsmittel wurde am Rotationsverdampfer entfernt und das Rohprodukt ohne weitere Aufreinigung in die nächste Stufe eingesetzt. Ausbeute: 1.9 g (63%)

### 2-(1-(3,4-Dichlorphenylsulfonyl)piperidin-2-yl)essigsäure S14

Zu einer gekühlten Lösung Methyl 2-(1-(3,4-dichlorphenylsulfonyl)piperidin-2-yl)acetat (2,1 g, 6 mmol) in Methanol (20 ml) und Wasser (20 ml) wurde Lithiumhydroxid (0,75 mg, 18 mmol) gegeben und 16 h bei Raumtemperatur gerührt (DC-Kontrolle). Das Lösungsmittel wurde am Rotationsverdampfer abgezogen und der Rückstand in Wasser aufgenommen. Die wässrige Phase wurde mit Ethylacetat gewaschen und anschließend mit 1 M HCl angesäuert und das Produkt mit Ethylacetat extrahiert und die organische Phase anschließend mit gesättigter NaCl-Lösung gewaschen. Die organische Phase wurde mit Natriumsulfat getrocknet und bis zur Trockne eingeengt. Ausbeute: 2,9 g (98%)

### 2-(2-(4-Methoxyphenylsulfonyl)-1,2,3,4-tetrahydroisochinolin-1-yl)essigsäure S15

### Methyl 2-(2-(4-methoxyphenylsulfonyl)-1,2,3,4-tetrahydroisochinolin-1-yl)acetat

2-(1,2,3,4-Tetrahydroisochinolin-1-yl)essigsäuremethylester (9.55 g, 46,5 mmol) wurden in DCM (150 ml) gelöst und mit Triethylamin (14,9 ml, 106 mmol) versetzt. Die Reaktionsmischung wurde auf 0°C abgekühlt und mit einer Lösung Methoxybenzenesulfonylchlorid (8,74g, 42,3 mmol) in DCM (100 ml) tropfenweise versetzt. Dere Reaktionsansatz wurde bei Raumtemperatur über nacht gerührt. Zur Aufarbeitung wurden 100 ml einer 0,5 M HCl-Lösung zugegeben und anschließend die Phasen getrennt. Die organische Phase wurde erst mit wasser gewaschen und anschließend über natriumsulfat getrocknet. Das Rohprodukt wurde säulenchromatographisch an Kieselgel (Laufmittel: DCM) aufgereinigt.
Ausbeute: 15,22g (96%)

### 2-(2-(4-Methoxyphenylsulfonyl)-1,2,3,4-tetrahydroisochinolin-1-yl)essigsäure S15

Zu einer Mischung des Methyl 2-(2-(4-methoxyphenylsulfonyl)-1,2,3,4-tetrahydroisochinolin-1-yl)acetats (15,22g, 40,54 mmol) in THF (200ml) und Wasser (120 ml) wurde eine 6 M wäßrige NaOH-Lösung gegeben Und über Nacht bei raumtemperatur gerührt. Anschließend wurde dasd Lösungsmittel unter vermindertem Druck entfernt und mit einer 6 M wäßrigen HCl-Lösung (125 ml) und DCM (400 ml) versetzt. Nach der Phasentrennung wurde die organische Phase mit konzentrierten NaCl-Lösung gewaschen, über Natriumsulfat getrocknet und eingeengt. Ausbeute: 14,65g (100 %)

### 3-(Naphthalin-2-sulfonamido)-3-phenylpropionsäure S16

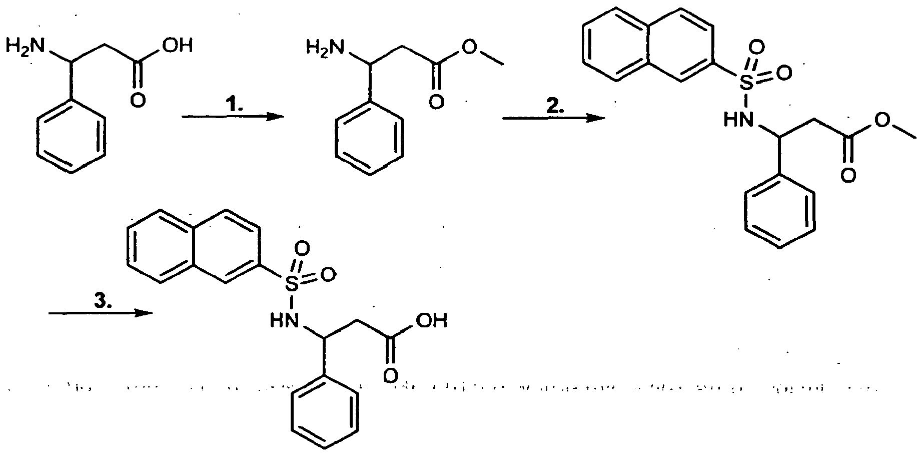

Stufe 1. Zu einer auf 0°C abgekühlten Lösung von 3-Amino-3-phenylpropionsäure (8.9 g, 54 mmol) in Methanol (150 ml) wurde Thionylchlorid (19.1g, 162 mmol) tropfenweise zugegeben. Anschließend wurde der Reaktionsansatz 12 h unter Rückfluss erhitzt (DC-Kontrolle). Das Lösungsmittel wurde komplett entfernt und der Rückstand im Vakuum getrocknet. Das Rohprodukt wurde ohne weitere Aufreinigung in die nächste Stufe eingesetzt.

Stufe 2. Der Aminoalkohol (1.1 Äq.) wurde in DCM (4 ml / mmol) gelöst und mit Triethylamin (2.2 Äq.) versetzt. Die Lösung wurde auf 0°C abgekühlt und tropfenweise mit einer Lösung des entsprechenden Sulfonsäurechlorids (1 Äq.) gelöst in DCM (2.3 ml / mmol) zugegeben und 1.5 h bei RT gerührt. Nach beendeter Reaktion wurde HCl (0.5 M, 2.3 ml / mmol) zugegeben, die Phasen getrennt, mit Wasser gewaschen, über Na₂SO₄ getrocknet und aufkonzentriert. Das Rohprodukt wurde säulenchromatographisch aufgereinigt (Kieselgel, Ethylacetat / Hexan, 3 : 7)

Stufe 3. Zu einer Lösung des Esters (1 Äq.) in einem Methanol / Wasser Gemisch (3 1, 10 ml / mmol) wurde bei einer Reaktionstemperatur von 0°C LiOH · H₂O (2 Äq.) gegeben. Der Reaktionsansatz wurde 16 h bei RT gerührt. Das Lösungsmittel wurde unter vermindertem Druck abgezogen, der Rückstand in Wasser aufgenommen und mit DCM gewaschen. Anschließend wurde die wässrige Phase vorsichtig mit HCl (1 N) angesäuert und mit Ethylacetat extrahiert. Die organische Phase wurde mit Wasser und ges. NaCl-Lösung gewaschen und über Na₂SO₄ getrocknet. Nach entfernen des Lösungsmittels wurde das Produkt in ausreichender Reinheit erhalten.

### 3-(1-(4-Chlor-2,5-dimethylphenylsulfonyl)piperidin-2-yl)propionsäure S17

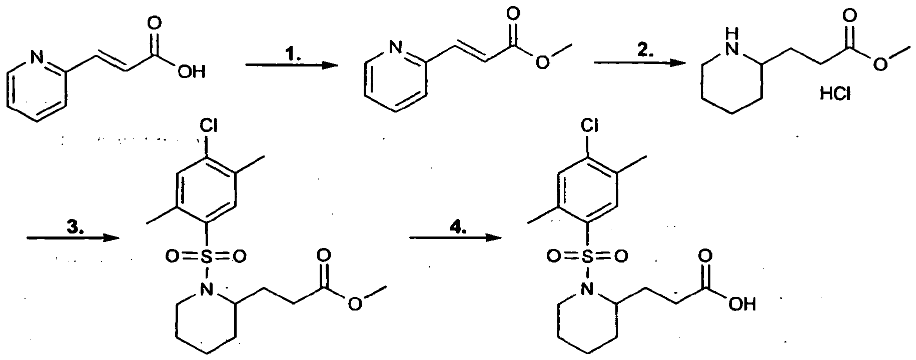

Stufe 1. Zu einer Lösung 3-(2-Pyridyl)-acrylsäure (23.88g, 160 mmol) in Methanol (750 ml) wurde H₂SO₄ (12.8 ml, 240 mmol) gegeben. Der Reaktionsansatz wurde über Nacht unter Rückfluss erhitzt und nach dem Abkühlen auf RT in gesättigte wässrige NaHCO₃-Lösung (1000 ml) gegossen. Das Methanol wurde am Rotationsverdampfer abgezogen und die wässrige Phase zweimal mit Ethylacetat (400 ml) extrahiert. Die organische Phase wurde mit gesättigter NaCl-Lösung (500 ml) gewaschen; über Na₂SO₄ getrocknet und aufkonzentriert. Das Rohprodukt des Methyl 3-(pyridin-2-yl)acrylats wurde ohne weitere Aufreinigung in die nächste Stufe eingesetzt.

Stufe 2. Methyl 3-(pyridin-2-yl)acrylat (22.15g, 136 mmol) wurde in THF (300 ml) und Chloroform (10.9 ml) gelöst und mit PtO₂ (3.08 g, 13.6 mmol, 0.1 Äq.) unter Stickstoffatmosphäre versetzt. Die Lösung wurde zunächst für 10 min. mit Stickstoff gespült und anschließend über Nacht unter H₂-Atmosphäre (8 bar) gerührt. Nach dem Abkühlen wurde zunächst erneut mit Stickstoff gespült, der Katalysator durch Filtrieren über Filtererde entfernt, mit DCM nachgespült und das Filtrat im Vakuum bis zur Trockne eingeengt. Das Methyl 3-(piperidin-2-yl)propionat Hydrochlorid wurde ohne weitere Aufreinigung in die nächste Stufe eingesetzt.

Stufe 3. Der Aminoalkohol (1.1 Äq.) wurde in DCM (4 ml / mmol) gelöst und mit Triethylamin (2.2 Äq.) versetzt. Die Lösung wurde auf 0°C abgekühlt und tropfenweise mit einer Lösung des entsprechenden Sulfonsäurechlorids (1 Äq.) gelöst in DCM (2.3 ml / mmol) zugegeben und 1.5 h bei RT gerührt. Nach beendeter Reaktion wurde HCl (0.5 M, 2.3 ml / mmol) zugegeben, die Phasen getrennt, mit Wasser gewaschen, über Na₂SO₄ getrocknet und aufkonzentriert. Das Rohprodukt wurde säulenchromatographisch an Kieselgel aufgereinigt (Kieselgel, Hetan /Ethylacetat, 6:1→3:1) -Stufe 4. Zu einer Lösung des Esters (1 Äq.) in THF (3 ml / mmol) wurde wässrige NaOH-Lösung (6 M, 3 ml / mmol) gegeben. Nach 1 h Reaktionszeit wurde das Lösungsmittel am Rotationsverdampfer entfernt und auf 0°C abgekühlt. Es wurde HCl (6 M, 3 ml / mmol) zugegeben und mit Ethylacetat extrahiert. Die organische Phase wurde über Na₂SO₄ getrocknet und aufkonzentriert.

### Synthese der Carbamate

### Synthese des Phenyl 3-(4-methoxy-N,2,3,6-tetramethylphenylsulfonamido)-propylcarbamat V1

### N-(2-Cyanoethyl)-4-methoxy-N,2,3,6-tetramethylbenzolsulfonamid

Eine Lösung von 3-Methylaminopropionitril (1,50 g, 18,3 mmol) und Triethylamin (5,50 g, 55 mmol) in THF (30 ml) wurde bei Raumtemperatur mit 4-Methoxy-2,3,6-trimethylbenzolsulfonylchlorid (5,00 g, 20 mmol) versetzt und 16 h gerührt. Das Reaktionsgemisch wurde anschließend mit Ethylacetat (50 ml) versetzt und mit Natriumhydrogencarbonat-Lösung (3 × 50 ml) gewaschen. Die organische Phase wurde mit Natriumsulfat getrocknet und i. Vak. eingeengt.
Ausbeute: 4,92 g (91%)
¹H-NMR (DMSO-d₆): 2,09 (s, 3H); 2,44 (s, 3H); 2,59 (s, 3H); 2,68 (s, 3H); 2,76 (t, 2H); 3,30 (t, 2H); 3,84 (s, 3H); 6,87 (s, 1H).

### N-(3-Aminopropyl)-4-methoxy-N,2,3,6-tetramethylbenzolsulfonamid

Eine Lösung von *N*-(2-Cyanoethyl)-4-methoxy-2,3,6,*N*-tetramethylbenzolsulfonamid (4,92 g, 16,6 mmol) und konzentrierter Schwefelsäure (3,20 g, 33,2 mmol) in Ethanol (100 ml) wurde mit Platin(IV)-oxid (400 mg) versetzt und 3 h unter einer WasserstoffAtmosphäre von 3 bar bei Raumtemperatur gerührt. Die Lösung wurde anschließend mit Natriumhydrogencarbonat versetzt, filtriert und i. Vak. eingeengt. Der Rückstand wurde in Natriumhydrogencarbonat-Lösung (50 ml) aufgenommen und mit DCM (3 × 50 ml) extrahiert. Die vereinigten organischen Phasen wurden mit Natriumsulfat getrocknet und i. Vak. eingeengt.
Ausbeute: 4,65 g (93 %)
¹H-NMR (DMSO-d₆): 1,53 (td, 2H); 2,09 (s, 3H); 2,43 (s, 3H); 2,50 (m, 2H); 2,58 (s, 3H); 2,61 (s, 3H); 3,06 (t, 2H); 3,21 (br s, 2H); 3,84 (s, 3H); 6,84 (s, 1 H).

### Phenyl 3-(4-methoxy-N,2,3,6-tetramethylphenylsulfon-amido)-propylcarbamat V1

Eine Lösung von *N*-(3-Aminopropyl)-4-methoxy-2,3,6,*N*-tetramethylbenzolsulfonamid (3,99 g, 13,3 mmol) und Triethylamin (4,0 g, 5,5 ml, 40 mmol) in THF (60 ml) wurde bei Raumtemperatur mit Chlorameisensäurephenylester (2,29 g, 14,6 mmol) versetzt und 16 h bei dieser Temperatur gerührt. Die Lösung wurde anschließend mit gesättigter Natriumhydrogencarbonat-Lösung (30 ml) versetzt und mit Ethylacetat (2 × 30 ml) extrahiert. Die vereinigten organischen Phasen wurden mit Natriumsulfat getrocknet und i. Vak. eingeengt und der Rückstand wurde durch Flashchromatographie mit Cyclohexan / Ethylacetat (3:1) gereinigt.
Ausbeute: 4,62 g (83%)
¹H-NMR (DMSO-d₆): 1,71 (td, 2H); 2,09 (s, 3H); 2,44 (s, 3H); 2,59 (s, 3H); 2,65 (s, 3H); 2,99 (dd, 2H); 3,08 (t, 2H); 3,83 (s, 3H); 6,85 (s, 1H); 7,06 (d, 2H); 7,19 (t, 1 H); 7,37 (t, 2H); 7,70 (t, 1H).

Es wurden die folgenden Harnstoffe hergestellt:

### N-(3-{3-[4-(Dimethylaminophenylmethyl)cyclohexyl]ureido}propyl)-4-methoxy-2,3,6,N-tetramethylbenzolsulfonamid (Beispiel 1)

Eine Lösung von 4-((Dimethylamino)(phenyl)methyl)cyclohexanamin **A1** (439 mg, 1,89 mmol) und Phenyl 3-(4-methoxy-*N*,2,3,6-tetramethylphenylsulfonamido)-propylcarbarnat **V1** (794 mg, 1,89 mmol) in 1,4-Dioxan (10 ml) wurde 1 d bei 110°C gerührt. Anschließend wurde das Lösungsmittel i. Vak. entfernt und der Rückstand durch Flashchromatographie mit Chlorform / Methanol / Triethylamin (100:5:1) gereinigt.
Ausbeute: 700 mg (66 %) 2 Isomere
¹H-NMR (DMSO-d₆): 0,94 (m, 5H); 1, 33 (m, 2H); 1,55 (m, 2H); 1,67 (d, 1H); 1,81 (d, 1H): 1,99 (s, 6H); 2,08 (s, 3H); 2,41 (s, 3H); 2,56 (s, 3H); 2,60 (s, 3H); 2,89 (m, 2H); 3,00 (m, 2H); 3,22 (d, 1 H); 3,59 (m, 1 H); 3,83 (s, 3H); 5,62 (m, 0,5H); 5,69 (m, 0,5H); 5,78 (d, 1H); 6,82 (s, 1H); 7,13 (d, 2H); 7,26 (dd, 1H); 7,33 (dt, 2H).

### (N-(3-(3-((4-((dimethylamino)(phenyl)methyl)cyclohexyl)methyl)ureido)propyl)-4-methoxy-N,2,3,6-tetramethylbenzolsulfonamid (Beispiel 2)

Eine Lösung von 1-(4-(Aminomethyl)cyclohexyl)-*N,N*-dimethyl-1-phenylmethanamin **A12** (200 mg, 0,81 mmol) und Phenyl 3-(4-methoxy-*N*,2,3,6-tetramethylphenylsulfonamido)-propylcarbamat **V1** (366 mg, 0,81 mmol) in 1,4-Dioxan (10 ml) wurde 1 d bei 110°C gerührt. Anschließend wurde das Lösungsmittel i. Vak. entfernt und der Rückstand durch Flashchromatographie mit Chlorform / Methanol / Triethylamin (100:5:1) gereinigt.
Ausbeute: 351 mg (76 %)
¹H-NMR (DMSO-d₆): 0,78 (m, 4H); 1,17 (t, 2H); 1,36 (d, 1H); 1,56 (td, 3H); 1,70 (d, 1 H); 1,84 (d, 1 H); 1,99 (s, 6H); 2,08 (s, 3H); 2,41 (s, 3H); 2,56 (s, 3H); 2,59 (s, 3H); 2,78 (t, 2H); 2,90 (dd, 2H);.3,02 (m, 2H); 3,37 (d, 1H); 3,83 (s, 3H); 5,70.(t, 1H); 5,78 (d, 1H); 6,82 (s, 1H); 7,13 (t, 2H); 7,24 (m, 1H); 7,31 (m, 2H).

### 4-Methoxy-2,3,6,N-tetramethyl-N-(3-{3-[4-(phenylpyrrolidin-1-ylmethyl)cyclohexyl]-ureido}propyl)benzolsulfonamid (Beispiel 3)

Eine Lösung von 4-(Phenyl(pyrrolidin-1-yl)methyl)cyclohexanamin **A9** (268 mg, 1,03 mmol) und Phenyl 3-(4-methoxy-*N*,2,3,6-tetramethylphenylsulfonamido)-propylcarbamat **V1** (469 mg, 1,03 mmol) in 1,4-Dioxan (10 ml) wurde 1 d bei 110 °C gerührt. Anschließend wurde das Lösungsmittel i. Vak. entfernt und der Rückstand durch Flashchromatographie Chlorform / Methanol / Triethylamin (100:2:1 → 100:5:1) gereinigt.
Ausbeute: 210 mg (35 %)
¹H-NMR (DMSO-d₆): 0,61 (dd, 1H); 0,78 (m, 1H); 1,02 (m, 3H) 1,56 (m, 7H); 1,73 (d, 4H); 2,08 (s, 3H); 2,33 (m, 4H); 2,40 (s, 3H); 2,56 (s, 3H); 2,59 (s, 3H); 2,86 (dd, 2H,); 2,99 (m, 2H); 3,09 (d, 1H); 3,83 (s, 3H); 5,59 (t, 1 H); 5,64 (t, 1 H); 6,81 (s, 1H); 7,16-7,19 (m, 2H); 7,22-7,25 (m, 1 H); 7,29-7,31 (m, 2H).

### 4-(Dimethylaminophenylmethyl)piperidin-1-carbonsäure-{3-[(4-methoxy-2,3,6-trimethylbenzolsulfonyl)methylamino]propyl}amid (Beispiel 4)

Eine Lösung von *N,N*-Dimethyl-1-phenyl-1-(piperidin-4-yl)methanamin **A28** (300 mg, 1,37 mmol) und Phenyl 3-(4-methoxy-*N*,2,3,6-tetramethylphenylsulfonamido)-propylcarbamat **V1** (621 mg, 1,37 mmol) in 1,4-Dioxan (10 ml) wurde 1 d bei 110°C gerührt. Anschließend wurde das Lösungsmittel i. Vak. entfernt und der Rückstand durch Flashchromatographie mit Chlorform / Methanol / Triethylamin (100:5:1) gereinigt. Ausbeute: 425 mg (57 %)
¹H-NMR (DMSO-d₆): 0,77 (ddd, 2H); 0,91 (ddd, 2H); 1,20 (d, 1H); 1,58 (m, 2H); 1,88 (d, 1 H); 2,01 (s, 6H); 2,08 (s, 3H); 2,41 (s, 3H); 2,56 (s, 3H); 2,61 (s, 3H); 2,63 (d, 1 H); 2,90 (dd, 2H); 2,97 (m, 2H); 3,08 (d, 1 H); 3,75 (d, 1H); 3,83 (s, 3H); 3,89 (d, 1H,); 6,24 (t, 1 H); 6,82 (s, 1 H); 7,14 (d, 2H); 7,25 (t, 1 H); 7,33 (t, 2H).

### Amide

Es wurden die folgenden Beispiele in Einzelsynthesen hergestellt

### N-(4-((Dimethylamino)(phenyl)methyl)cyclohexyl)-5-(2,4,6-trichlor-N-methylphenylsulfonamido)pentanamid (Beispiel 5)

Eine Lösung von 5-(2,4,6-Trichlor-*N*-methylphenylsulfonamido)pentansäure **S10** (250 mg, 0.667 mmol), *N*-Methylmorpholin (201 mg, 218 µL, 2.0 mmol) und 1-Benzotriazolyloxy-tris(dimethylamino)phosphonium-hexafluorphosphat (381 mg, 0.867 mmol) in wasserfreiem *N,N*-Dimethylformamid (10 ml) wurde nach einstündigem Rühren mit einer Lösung von 4-((Dimethylamino)(phenyl)methyl)cyclohexanamin **A1** (174 mg, 1.0 mmol) versetzt und 18 h bei Raumtemperatur gerührt. Das Reaktionsgemisch wurde anschließend i. Vak. eingeengt, der Rückstand in Wasser aufgenommen und mit 5 % Natriumhydrogencarbonat-Lösung auf pH 8 eingestellt. Das Gemisch wurde mit Ethylacetat (3 × 40 ml) extrahiert, die vereinigten organischen Phasen mit Natriumsulfat getrocknet und i. Vak. eingeengt. Das Rohprodukt wurde durch Flashchromatographie mit Chlorform / Methanol (95:5) gereinigt.
Ausbeute: 122 mg (31 %), gelbliches Öl
¹H-NMR (CDCl₃): 1.40-2.18 (m, 14H); 2.08 und 2.10 (2 s, 6H); 2.87 und 2.88 (2 s, 3H); 3.02 (d, 0.35); 3.22-3.35 (m, 3.65H); 3.94-4.04 (m, 1H); 5.58 (d, 0.35H); 5.80 (d, 0.65H); 7.06-7.14 (m, 2H); 7.20-7.35 (m, 3H); 7.45 und 7.46 (2 s, 2H).

### N-(4-(Phenyl(piperidin-1-yl)methyl)cyclohexyl)-5-(2,4,6-trichlor-N-methylphenylsulfon- amido)pentanamid (Beispiel 6)

Eine Lösung von 5-(2,4,6-Trichlor-*N*-methylphenylsulfonamido)pentansäure S10 (374 mg, 1,0 mmol), *N*-Methylmorpholin (302 mg, 328 µL, 2,9 mmol) und 1-Benzotriazolyloxy-tris(dimethylamino)phosphonium-hexafluorphosphat (571 mg, 1,3 mmol) in wasserfreiem *N,N*-Dimethylformamid (10 ml) wurde nach einstündigem Rühren mit einer Lösung von 4-(Phenyl(piperidin-1-yl)methyl)cyclohexanamin **A10** (302 mg, 1,11 mmol) in wasserfreiem *N,N*-Dimethylformamid (5 ml) versetzt und 18h bei Raumtemperatur gerührt. Das Reaktionsgemisch wurde anschließend i. Vak. eingeengt, der Rückstand in Wasser aufgenommen und mit 5 % Natriumhydrogencarbonat-Lösung auf pH 8 eingestellt. Das Gemisch wurde mit Ethylacetat (3 × 40 ml) extrahiert, die vereinigten organischen Phasen mit Natriumsulfat getrocknet und i. Vak. eingeengt. Das Rohprodukt wurde durch Flashchromatographie mit Chlorform / Methanol (95:5) gereinigt. Ausbeute: 128 mg (21 %)
¹H-NMR (DMSO-d₆): 1,00-2-30 (m, 26H); 2,84 (m, 3H); 3,19-3,26 (m, 2H); 3,74 (br s, 1 H); 7,09-7,15 (m, 2H); 7,25 (d, 0,5H); 7,28-7,36 (m, 3H); 7,55 (d, 0,5H); 7,86 und 7,88 (2 s, 2H).

### N-((4-((Dimethylamino)(phenyl)methyl)cyclohexyl)methyl)-5-(2,4,6-trichlor-N-methylphenylsulfonamido)pentanamid (Beispiel 7)

Eine Lösung von 5-(2,4,6-Trichlor-*N*-methylphenylsulfonamido)pentansäure **S10** (495 mg, 1,32 mmol) in wasserfreiem THF (10 ml) wurde mit *N,N*'-Carbonyldiimidazol (248 mg, 1,45 mmol) versetzt und 1 h bei Raumtemperatur gerührt. Anschließend wurde eine Lösung von 1-(4-(Aminomethyl)cyclohexyl)-*N,N*-dimethyl-1-phenylmethanamin **A12** (357 mg, 1,45 mmol) in wasserfreiem THF (10 ml) zugegeben, Das Reaktionsgemisch wurde 18 h bei Raumtemperatur gerührt, danach i. Vak. eingeengt, der Rückstand mit 5 % Natriumhydrogencarbonat-Lösung (50 ml) versetzt und mit Ethylacetat (3 × 50 ml) extrahiert. Die vereinigten organischen Phasen wurden mit Natriumsulfat getrocknet und i. Vak. eingeengt. Das Rohprodukt wurde durch Flashchromatographie mit Chlorform / Methanol (9: 1) gereinigt.
Ausbeute: 638 mg (80 %)
¹H-NMR (DMSO-d₆): 0,60-1,95 (m, 13H); 1,99 (s, 6H); 2,05 (t, 2H); 2,82 (s, 3H), 2,85-2,99 (m, 2H); 3,00 (d, 1H); 3,21 (t, 2H); 7,15-7,40 (m, 5H); 7,68 (t, 1H), 7,85 (s, 2H).

### N-((4-(-(Dimethylamino)(phenyl)methyl)cyclohexyl)methyl)-2-(1-(3-(trifluormethyl)phenylsulfonyl)piperidin-2-yl)acetamid (Beispiel 8)

Eine Lösung von 2-(1-(3-(Trifluormethyl)phenylsulfonyl)piperidin-2-yl)essigsäure **S8** (351 mg, 1 mmo) in wasserfreiem THF (10 ml) wurde mit *N,N'*-Carbonyldiimidazol (188 mg, 1,1 mmol) versetzt und 1 h bei Raumtemperatur gerührt. Anschließend wurde das Gemisch mit einer Lösung von 1-(4-(Aminomethyl)cyclohexyl)-*N,N-*dimethyl-1-phenylmethanamin **A12** (271 mg, 1,1 mmol) in wasserfreiem THF (10 ml) versetzt und 18 h bei Raumtemperatur gerührt. Das Reaktionsgemisch wurde i. Vak. eingeengt, der Rückstand mit 5 % Natriumhydrogencarbonat-Lösung versetzt und mit Ethylacetat (3 × 30 ml) extrahiert. Die vereinigten organischen Phasen wurden mit Natriumsulfat getrocknet und i. Vak. eingeengt. Das Rohprodukt wurde durch Flashchromatographie mit Chlorform / Methanol (9:1) gereinigt.
Ausbeute: 298 mg (51 %)
¹H-NMR (DMSO-d₆): 0,50-1,90 (m, 16H); 1,98 (s, 6H); 2,19 (dd, 1 H), 2,43 (dd, 1H); 2,65-2,85 (m, 1,5H); 2,85-3,10 (m, 1,5H); 3,68 (br d, 1 H); 4,43 (m, 1H); 7,05-7,38 (m, 5H); 7,70-7,88 (m, 2H); 7,95-8,20 (m, 2H).

### Automatisierte Synthese

### Methode A

Es wurden auf einem Accelerator SLT106 von Chemspeed bei Raumtemperatur zunächst 105 µmol CDI-Lösung (0,105 M in DCM, 1 ml) in die Reaktoren gegeben, mit der entsprechenden Säure-Lösung (0,05 M in DCM, 2 ml) versetzt und 1 h bei Raumtemperatur geschüttelt. Anschließend wurde bei Raumtemperatur mit 100 µmol des entsprechenden Amins (0,1 M in DCM, 1 ml) versetzt und weitere 12 h bei RT geschüttelt.

Nach beendeter Reaktion wurde mit 3 ml Wasser versetzt, 15 min geschüttelt und anschließend die organische Phase abgetrennt.

Das Lösungsmittel wurde in einer Genevac Vakuumzentrifuge abgezogen und die Produkte mittels HPLC gereinigt.

### Methode B

Auf der Anlage von Zymark wurde in einem trockenen Gewindeglas bei RT 100 µmol Säure-Lösung (0,05 M in DCM, 2 ml) vorgelegt und mit 105µmol CDI-Lösung (0,105 M in DCM, 1 ml) versetzt. Nach 1 Stunde Rührzeit bei RT wurde zu der Reaktionslösung 100 µmol des entsprechenden Amins (0,1 M in DCM) hinzupipettiert. Die Reaktionslösung wurde 16 h bei RT im gerührt. Anschließend wurden 3 ml Wasser zugegeben und 30 min. durchmischt. Der Rührfisch wurde abfiltriert, und das Gefäß mit 1,5 ml DCM ausgespült.

Die wässrige Phase wurde abgenommen und verworfen. Die organische Phase wurde mit 3ml dest H₂O und 0,5 ml DCM versetzt, gevortext und 15 min. intensiv durchmischt. Nach dem Zentrifugieren wurde die wässrige Phase abgetrennt und verworfen. Die organische Phase wufdeanalog ein zweites Mal mit 3 ml ges. NaCl-Lösung extrahiert. Anschließend wurde die organische Phase abgenommen, in ein Reagenzglas gegeben und über eine MgSO₄-Kartusche getrocknet. Die Lösung wurde in einer Vakuumzentrifuge (GeneVac) eingeengt und die Produkte mittels HPLC gereinigt.

### Methode C

In paralleler Synthese wurde der Säurebaustein (50 mg, 1 Äq.) in DCM (3 ml/mmol) gelöst und mit dem Aminbaustein (1,2 Äq.), EDCI (1,5 Äq.), HOBt (1Äq.) und DIPEA (2Äq.) versetzt.

Die erhaltenen Rohprodukte wurden über ein Parallel-Aufreinigungssystem von Biotage aufgereinigt.

Die folgenden Verbindungen wurden nach diesen Methoden synthetisiert. Die Zielmasse wurde in allen Fällen als M+1 durch Massenspektroskopie bestätigt:

| Nr. | Amin | Säure | Methode | Masse+1 | Name |
|---|---|---|---|---|---|
| 9 | A1 | S15 | A | 576.3 | N-[4-(Dimethylamino-phenyl-methyl)-cyclohexyl]-2-[2-(4-methoxy-benzolsulfonyl)-1,2,3,4-tetrahydro-isochinolin-1-yl]-acetamid |
| 10 | A1 | S1 | A | 614.2 | 2-[2-(3,4-Dichlor-benzolsulfonyl)-1,2,3,4-tetrahydro-isochinolin-1-yl]-N-[4-(dimethylamino-phenyl-methyl)-cyclohexyl]-acetamid |
| 11 | A1 | S3 | A | 574.2 | 2-[(3,4-Dichlor-benzolsulfonyl)-methyl-amino]-N-[4-(dimethylamino-phenyl-methyl)-cyclohexyl]-benzamid |
| 12 | A1 | S6 | A | 634.2 | 2-[(3,4-Dichlor-benzolsulfonyl)-methyl-amino]-4,5,6,7-tetrahydro-benzo[b]thiophen-3-carbonsäure-[4-(dimethylamino-phenyl-methyl)-cyclohexyl]-amid |
| 13 | A3 | S15 | A | 594.3 | N-{4-[Dimethylamino-(3-fluor phenyl)-methyl]-cyclohexyl}-2-[2-(4-methoxy-benzolsulfonyl)-1,2,3,4-tetrahydro-isochinolin-1-y]-acetamid |
| 14 | A2 | S15 | A | 594.3 | N-{4-[Dimethylamino-(4-fluor-phenyl)-methyl]-cyclohexyl}-2-[2-(4-methoxy-benzolsulfonyl)-1,2,3,4-tetrahydro-isochinolin-1-yl]-acetamid |
| 15 | A4 | S15 | A | 582.2 | N-[4-(Dimethylamino-thiophen-2-yl-methyl)-cyclohexyl]-2-[2-(4-methoxy-benzolsulfonyl)-1,2,3,4-tetrahydro-isochinolin-1-yl]-acetamid |
| 16 | A14 | S15 | A | 608.3 | N-{4-[Dimethylamino-(3-fluor-phenyl)-methyl]-cyclohexylmethyl}-2-[2-(4-methoxy-benzolsulfonyl)-1,2,3,4-tetrahydro-isochinolin-1-yl]-acetamid |
| 17 | A12 | S15 | A | 590.3 | N-[4-(Dimethylamino-phenyl-methyl)-cyclohexylmethyl]-2-[2-(4-methoxy-benzolsulfonyl)-1,2,3,4-tetrahydro-isochinolin-1-yl]-acetamid |
| 18 | A17 | S15 | A | 618.3 | N-[4-(1-Dimethylamino-3-phenyl-propyl)-cyclohexylmethyl]-2-[2-(4-methoxy-benzolsulfonyl)-1,2,3,4-tetrahydro-isochinolin-1-yl]acetamid |
| 19 | A13 | S15 | A | 608.3 | N-{4-[Dimethylamino-(4-fluor-phenyl)-methyl]-cyclohexylmethyl}-2-[2-(4-methoxy-benzolsulfonyl)-1,2,3,4-tetrahydro-isochinolin-1-yl]-acetamid |
| 20 | A15 | S15 | A | 624.3 | N-{4-[(4-Chlor-phenyl)-dimethylamino-methyl]-cyclohexylmethyl}-2-[2-(4-methoxy-benzolsulfonyl)-1,2,3,4-tetrahydro-isochinolin-1-yl]-acetamid |
| 21 | A16 | S15 | A | 596.3 | N-[4-(Dimethylamino-thiophen-2-yl-methyl)-cyclohexylmethyl]-2-[2-(4-methoxy-benzolsulfonyl)-1,2,3,4-tetrahydro-isochinolin-1-yl]-acetamid |
| 22 | A25 | S15 | A | 638.3 | N-(2-{4-[(4-Chlor-phenyl)-dimethylamino-methyl]-cyclohexyl}-ethyl)-2-[2-(4-methoxy-benzolsulfonyl)-1,2,3,4-tetrahydro-isochinolin-1-yl]-acetamid |
| 23 | A24 | S15 | A | 622.3 | N-(2-{4-[Dimethylamino-(3-fluor-phenyl)-methyl]-cyclohexyl}ethyl)-2-[2-(4-methoxy-benzolsulfonyl)-1,2,3,4-tetrahydro-isochinolin-1-yl]-acetamid |
| 24 | A23 | S15 | A | 622.3 | N-(2-{4-[Dimethylamino-(4-fluor-phenyl)-methyl]-cyclohexyl}-ethyl)-2-[2-(4-methoxy-benzolsulfonyl)-1,2,3,4-tetrahydro-isochinolin-1-yl]-acetamid |
| 25 | A26 | S15 | A | 610.3 | N-{2-[4-(Dimethylamino-thiophen-2-yl-methyl)-cyclohexyl]-ethyl}-2-[2-(4-methoxy-benzolsulfonyl)-1,2,3,4-tetrahydro-isochinolin-1-yl]-acetamid |
| 26 | A3 | S1 | A | 632.2 | 2-(2-(3,4-Dichlorphenylsulfonyl)-1,2,3,4-tetrahydroisochinolin-1-yl)-N-(4-((dimethylamino)(3-fluorphenyl)methyl)cyclohexyl)acetamid |
| 27 | A2 | S1 | A | 632.2 | 2-(2-(3,4-Dichlorphenylsulfonyl)-1,2,3,4-tetrahydroisochinolin-1-yl)-N-(4-((dimethylamino)(4-fluorphenyl)methyl)cyclohexyl)acetamid |
| 28 | A14 | S1 | A | 646.2 | 2-[2-(3,4-Dichlor-benzolsulfonyl)-1,2,3,4-tetrahydro-isochinolin-1-yl]-N-{4-[dimethylamino-(3-fluor-phenyl)-methyl]-cyclohexylmethyl}-acetamid |
| 29 | A12 | S1 | A | 628.2 | 2-[2-(3,4-Dichlor-benzolsulfonyl)-1,2,3,4-tetrahydro-isochinolin-1-yl]-N-[4-(dimethylamino-phenyl-methyl)-cyclohexylmethyl]-acetamid |
| 30 | A17 | S1 | A | 656.2 | 2-[2-(3,4-Dichlor-benzolsulfonyl)-1,2,3,4-tetrahydro-isochinolin-1-yl]-N-[4-(1-dimethylamino-3-phenyl-propyl)-cyclohexylmethyl]-acetamid |
| 31 | A13 | S1 | A | 646.2 | 2-[2-(3,4-Dichlor-benzolsulfonyl)-1,2,3,4-tetrahydro-isochinolin-1-yl]-N-{4-[dimethylamino-(4-fluor-phenyl)-methyl]-cyclohexylmethyl}-acetamid |
| 32 | A15 | . S1 | A | 662.2 | N-{4-[(4-Chlor-phenyl)-dimethylamino-methyl]-cyclohexylmethyl}-2-[2-(3,4-dichlor-benzolsulfonyl)-1,2,3,4-tetrahydro-isochinolin-1-yl]-acetamid |
| 33 | A25 | S1 | A | 676.2 | N-(2-{4-[(4-Chlor-phenyl)-dimethylamino-methyl]-cyclohexyl}-ethyl)-2-[2-(3,4-dichlor-benzolsulfonyl)-1,2,3,4-tetrahydro-isochinolin-1-yl]-acetamid |
| 34 | A24 | S1 | A | 660.2 | 2-[2-(3,4-Dichlor-benzolsulfonyl)-1,2,3,4-tetrahydro-isochinolin-1-yl]-N-(2-{4-[dimethylamino-(3-fluor-phenyl)-methyl]-cyclohexyl}-ethyl)-acetamid |
| 35 | A23 | S1 | A | 660.2 | 2-[2-(3,4-Dichlor-benzolsulfonyl)-1,2,3,4-tetrahydro-isochinolin-1-yl]-N-(2-{4-[dimethylamino-(4-fluor-phenyl)-methyl]-cyclohexyl}-ethyl)-acetamid |
| 36 | A3 | S4 | A | 592.2 | 2-[(3,4-Dichlor-benzolsulfonyl)-methyl-amino]-N-{4-[dimethylamino-(3-fluor-phenyl)-methyl]-cyclohexyl}-benzamid |
| 37 | A2 | S4 | A | 592.2 | 2-[(3,4-Dichlor-benzolsulfonyl)-methyl-amino]-N-{4-[dimethylamino-(4-fluor-phenyl)-methyl]-cyclohexyl}-benzamid |
| 38 | A12 | S4 | A | 588.2 | 2-[(3,4-Dichlor-benzolsulfonyl)-methyl-amino]-N-[4-(dimethylamino-phenyl-methyl)-cyclohexylmethyl]-benzamid |
| 39 | A17 | S4 | A | 616.2 | 2-[(3,4-Dichlor-benzolsulfonyl)-methyl-amino]-N-[4-(1-dimethylamino-3-phenyl-propyl)-cyclohexylmethyl]-benzamid |
| 40 | A13 | S4 | A | 606.2 | 2-[(3,4-Dichlor-benzolsulfonyl)-methyl-amino]-N-{4-[dimethylamino-(4-fluor-phenyl)-methyl]-cyclohexylmethyl}-benzamid |
| 41 | A15 | S4 | A | 622.1 | N-{4-[(4-Chlor-phenyl)-dimethylamino-methyl]-cyclohexylmethyl}-2-[(3,4-dichlor-benzolsulfonyl)-methyl-amino]-benzamid |
| 42 | A16 | S4 | A | 594.1 | 2-[(3,4-Dichlor-benzolsulfonyl)-methyl-amino]-N-[4-(dimethylamino-thiophen-2-yl-methyl)-cyclohexylmethyl]-benzamid |
| 43 | A25 | S4 | A | 636.2 | N-(2-{4-[(4-Chlor-phenyl)-dimethylamino-methyl]-cyclohexyl}-ethyl)-2-[(3,4-dichlor-benzolsulfonyl)-methyl-amino]-benzamid |
| 44 | A24 | S4 | A | 620.2 | 2-[(3,4-Dichlor-benzolsulfonyl)-methyl-amino]-N-(2-{4-[dimethylamino-(3-fluor-phenyl)-methyl]-cyclohexyl}-ethyl)-benzamid |
| 45 | A23 | S4 | A | 620.2 | 2-[(3,4-Dichlor-benzolsulfonyl)-methyl-amino]-N-(2-{4-[dimethylamino-(4-fluor-phenyl)-methyl]-cyclohexyl}ethyl)-benzamid |
| 46 | A12 | S5 | A | 648.2 | 2-[(3,4-Dichlor-benzolsulfonyl)-methyl-amino]-4,5,6,7-tetrahydro-benzo[b]thiophen-3-carbonsäure-[4-(dimethylamino-phenyl-methyl)-cyclohexylmethyl]-amid |
| 47 | A13 | S5 | A | 666.2 | 2-[(3,4-Dichlor-benzolsulfonyl)-methyl-amino]-4,5,6,7-tetrahydro-benzo[b]thiophen-3-carbonsäure-(4-[dimethylamino-(4-fluor-phenyl)-methyl]-cyclohexylmethyl)-amid |
| 48 | A15 | S5 | A | 682.1 | 2-[(3,4-Dichlor-benzolsulfonyl)-methyl-amino]-4,5,6,7-tetrahydro-benzo[b]thiophen-3-carbonsäure-{4-[(4-chlor-phenyl)-dimethylamino-methyl]-cyclohexylmethyl}-amid |
| 49 | A16 | S5 | A | 654.1 | 2-[(3,4-Dichlor-benzolsulfonyl)-methyl-amino]-4,5,6,7-tetrahydro-benzo[b]thiophen-3-carbonsäure-[4-(dimethylamino-thiophen-2-yl-methyl)-cyclohexylmethyl]-amid |
| 50 | A25 | S5 | A | 696.2 | N-(2-(4-((4-chlorphenyl)(dimethylamino)methyl)cyclohexyl)ethyl)-2-(3,4-dichlor-N-methylphenylsulfonamido)-4,5,6,7-tetrahydrobenzo[b]thiophen-3-carboxamid |
| 51 | A24 | S5 | A | 680.2 | 2-(3,4-Dichlor-N-methylphenylsulfonamido)-N-(2-(4-((dimethylamino)(3-fluorphenyl)methyl)cyclohexyl)ethyl)-4,5,6,7-tetrahydrobenzo[b]thiophen-3-carboxamid |
| 52 | A23 | S5 | A | 680.2 | 2-(3,4-Dichlor-N-methylphenylsulfonamido)-N-(2-(4-((dimethylamino)(4-fluorphenyl)methyl)cyclohexyl)ethyl)-4,5,6,7-tetrahydrobenzo[b]thiophen-3-carboxamid |
| 53 | A26 | S5 | A | 668.2 | 2-(3,4-Dichlor-N-methylphenylsulfonamido)-N-(2-(4-((dimethylamino)(thiophen-2-yl)methyl)cyclohexyl)ethyl)-4,5,6,7-tetrahydrobenzo[b]thiophen-3-carboxamid |
| 54 | A11 | S12 | C | 671.2 | 2-[1-(3,4-Dichlor-benzolsulfonyl)-3-oxo-1,2,3,4-tetrahydro-chinoxalin-2-yl]-N-[4-(morpholin-4-yl-phenyl-methyl)-cyclohexyl]-acetamid |
| 55 | A18 | S11 | C | 637.2 | 2-[1-(2,4-Dichlor-benzolsulfonyl)-3-oxo-piperazin-2-yl]-N-[4-(morpholin-4-yl-phenyl-methyl)-cyclohexylmethyl]-acetamid |
| 56 | A19 | S11 | C | 665.2 | 2-[1-(2,4-Dichlor-benzolsulfonyl)-3-oxo-piperazin-2-yl]-N-[4-(1-morpholin-4-yl-3-phenyl-propyl)-cyclohexylmethyl]-acetamid |
| 57 | A21 | 511 | C | 649.2 | 2-[1-(2,4-Dichlor-benzolsulfonyl)-3-oxo-piperazin-2-yl]-N-[4-(3-phenyl-1-pyrrolidin-1-yl-propyl)-cyclohexylmethyl]acetamid |
| 58 | A20 | 511 | C | 621.2 | 2-[1-(2,4-Dichlor-benzolsulfonyl)-3-oxo-piperazin-2-yl]-N-[4-(phenyl-pyrrolidin-1-yl-methyl)-cyclohexylmethyl]-acetamid |
| 59 | A7 | S11 | C | 649.2 | 2-[1-(2,4-Dichlor-benzolsulfonyl)-3-oxo-piperazin-2-yl]-N-[4-(3-phenyl-1-piperidin-1-yl-propyl)-cyclohexyl]-acetamid |
| 60 | A6 | 511 | C | 651.2 | 2-[1-(2,4-Dichlor-benzolsulfonyl)-3-oxo-piperazin-2-yl]-N-[4-(1-morpholin-4-yl-3-phenyPpropyl)-cyclohexyl]-acetamid |
| 61 | A8 | S11 | C | 636.2 | 2-[1-(2,4-Dichtor-benzolsulionyl)-3-oxo-piperazin-2-yl]-N-{4-[(4-methyl-piperazin-1-yl)-phenyl-methyl]-cyclohexyl}-acetamid |
| 62 | A29 | S11 | C | 609.2 | 4-(2,4-Dichlor-benzolsulfonyl)-3-{2-[4-(morpholin-4-yl-phenyl-methyl)-piperidin-1-yl]-2-oxo-ethyl}-piperazin-2-on |
| 63 | A30 | 511 | C | 623.2 | 4-(2,4-dichlorphenylsulfonyl)-3-(2-(2-(1-morpholino-2-phenylethyl)piperidin-1-yl)-2-oxoethyl)piperazin-2-on |
| 64 | A18 | S12 | C | 685.2 | 2-[1-(3,4-Dichlor-benzolsulfonyl)-3-oxo-1,2,3,4-tetrahydro-chinoxalin-2-yl]-N-[4-(morpholin-4-yl-phenyl-methyl)-cyclohexylmethyl]-acetamid |
| 65 | A19 | S12 | C | 713.2 | 2-[1-(3,4-Dichlor-benzolsulfonyl)-3-oxo-1,2,3,4-tetrahydro-chinoxalin-2-yl]-N-[4-(1-morpholin-4-yl-3-phenyl-propyl)-cyclohexylmethyl]-acetamid |
| 66 | A21 | S12 | C | 697.2 | 2-[1-(3,4-Dichlor-benzolsulfonyl)-3-oxo-1,2,3,4-tetrahydro-chinoxalin-2-yl]-N-[4-(3-phenyl-1-pyrrolidin-1-yl-propyl)-cyclohexylmethyl]-acetamid |
| 67 | A20 | S12 | C | 669.2 | 2-[1-(3,4-Dichlor-benzolsulfonyl)-3-oxo-1,2,3,4-tetrahydro-chinoxalin-2-yl]-N-[4-(phenyl-pyrrolidin-1-yl-methyl)-cyclohexylmethyl]-acetamid |
| 68 | A7 | S12 | C | 697.2 | 2-[1-(3,4-Dichlor-benzolsulfonyl)-3-oxo-1,2,3,4-tetrahydro-chinoxalin-2-yl]-N-[4-(3-phenyl-1-piperidin-1-yl-propyl)-cyclohexyl]-acetamid |
| 69 | A6 | S12 | C | 699.2 | 2-[1-(3,4-Dichlor-benzolsulfonyl)-3-oxo-1,2,3,4-tetrahydro-chinoxalin-2-yl]-N-[4-(1-morpholin-4-yl-3-phenyl-propyl)-cyclohexyl]-acetamid |
| 70 | A8 | S12 | C | 684.2 | 2-[1-(3,4-Dichlor-benzolsulfonyl)-3-oxo-1,2,3,4-tetrahydro-chinoxalin-2-yl]-N-{4-[(4-methyl-piperazin-1-yl)-phenyl-methyl]-cyclohexyl}acetamid |
| 71 | A29 | S12 | C | 657.2 | 4-(3,4-Dichlor-benzolsulfonyl)-3-{2-[4-(morpholin-4-yl-phenyl-methyl)-piperidin-1-yl]-2-oxo-ethyl}-3,4-dihydro-1H-chinoxalin-2-on |
| 72 | A30 | S12 | C | 671.2 | 4-(3,4-dichlorphenylsulfonyl)-3-(2-(4-(1-morpholino-2-phenylethyl)piperidin-1-yl)-2-oxoethyl)-3,4-dihydrochinoxalin-2(1 H)-on |
| 73 | A18 | S13 | C | 608.2 | 2-[1-(3,4-Dichlor-benzolsulfonyl)-pyrrolidin-2-yl]-N-[4-(morpholin-4-yl-phenyl-methyl)-cyclohexylmethyl]-acetamid |
| 74 | A19 | S13 | C | 636.2 | 2-[1-(3,4-Dichlor-benzolsulfonyl)-pyrrolidin-2-yl]-N-[4-(1-morpholin-4-yl-3-phenyl-propyl)-cyclohexylmethyl]-acetamid |
| 75 | A21 | S13 | C | 620.2 | 2-[1-(3,4-Dichlor-benzolsulfonyl)-pyrrolidin-2-yl]-N-[4-(3-phenyl-1-pyrrolidin-1-yl-propyl)-cyclohexylmethyl]-acetamid |
| 76 | A20 | S13 | C | 592.2 | 2-[1-(3,4-Dichlor-benzolsulfonyl)-pyrrolidin-2-yl]-N-[4-(phenyl-pyrrolidin-1-yl-methyl)-cyclohexylmethyl]-acetamid |
| 77 | A7 | S13 | C | 620.2 | 2-(1-(3,4-dichlorphenylsulfonyl)pyrrolidin-2-yl)-N-(4-(3-phenyl-1-(piperidin-1-yl)propyl)cyclohexyl)acetamid |
| 78 | A6 | S13 | C | 622.2 | 2-(1-(3,4-dichlorphenylsulfonyl)pyrrolidin-2-yl)-N-(4-(1-morpholino-3-phenylpropyl)cyclohexyl)acetamid |
| 79 | A8 | S13 | C | 607.2 | 2-[1-(3,4-Dichlor-benzolsulfonyl)-pyrrolidin-2-yl]-N-{4-[(4-methyl-piperazin-1-yl)-phenyl-methyl]-cyclohexyl} acetamid |
| 80 | A29 | S13 | C | 580.2 | 2-[1-(3,4-Dichlor-benzolsulfonyl)-pyrrolidin-2-yl]-1-[4-(morpholin-4-yl-phenyl-methyl)-piperidin-1-yl]-ethanon |
| 81 | A30 | S13 | C | 594.2 | 2-(1-(3,4-dichlorphenylsulfonyl)pyrrolidin-2-yl)-1-(4-(1-morpholino-2-phenylethyl)piperidin-1-yl)ethanon |
| 82 | A18 | S14 | C | 622.2 | 2-[1-(3,4-Dichlor-benzolsulfonyl)-piperidin-2-yl]-N-[4-(morpholin-4-yl-phenyl-methyl)-cyclohexylmethyl]-acetamid |
| 83 | A19 | S14 | C | 650.3 | 2-[1-(3,4-Dichlor-benzolsulfonyl)-piperidin-2-yl]-N-[4-(1-morpholin-4-yl-3-phenyl-propyl)-cyclohexylmethyl]-acetamid |
| 84 | A21 | S14 | C | 634.3 | 2-[1-(3,4-Dichlor-benzolsulfonyl)-piperidin-2-yl]-N-[4-(3-phenyl-1-pyrrolidin-1-yl-propyl)-cyclohexylmethyl]-acetamid |
| 85 | A20 | S14 | C | 606.2 | 2-[1-(3,4-Dichlor-benzolsulfonyl)-piperidin-2-yl]-N-[4-(phenyl-pyrrolidin-1-yl-methyl)-cyclohexylmethyl]-acetamid |
| 86 | A7 | S14 | C | 634.3 | 2-[1-(3,4-Dichlor-benzolsulfonyl)piperidin-2-yl]-N-[4-(3-phenyl-1-piperidin-1-yl-propyl)-cyclohexyl]-acetamid |
| 87 | A6 | S14 | C | 636.2 | 2-[1-(3,4-Dichlor-benzolsulfonyl)-piperidin-2-yl]-N-[4-(1-morpholin-4-yl-3-phenyl-propyl)-cyclohexyl]-acetamid |
| 88 | A8 | S14 | C | 621.2 | 2-[1-(3,4-Dichlor-benzolsulfonyl)-piperidin-2-yl]-N-{4-[(4-methyl-piperazin-1-yl)-phenyl-methyl]-cyclohexyl}-acetamid |
| 89 | A29 | S14 | C | 594.2 | 2-[1-(3,4-Dichlor-benzolsulfonyl)-piperidin-2-yl]-1-[4-(morpholin-4-yl-phenyl-methyl)-piperidin-1-yl]-ethanon |
| 90 | A30 | S14 | C | 608.2 | 2-(1-(3,4-Dichlorphenylsulfonyl)piperidin-2-yl)-1-(4-(1-morpholino-2-phenylethyl)piperidin-1-yl)ethanon |
| 91 | A1 | S6 | B | 586.2 | 1-(3,4-Dichlorphenylsulfonyl)-N-(4-((dimethylamino)(phenyl)methyl)cyclohexyl)indolin-2-carboxamid |
| 92 | A3 | S6 | B | 604.2 | 1-(3,4-Dichlorphenylsulfonyl)-N-(4-((dimethylamino)(3-fluorphenyl)methyl)cyclohexyl)indolin-2-carboxamid |
| 93 | A5 | S6 | B | 614.2 | 1-(3,4-Dichlorphenylsulfonyl)-N-(4-(1-(dimethylamino)-3-phenylpropyl)cyclohexyl)indolin-2-carboxamid |
| 94 | A2 | S6 | B | 604.2 | 1-(3,4-Dichlorphenylsulfonyl)-N-(4-((dimethylamino)(4-fluorphenyl)methyl)cyclohexyl)indolin-2-carboxamid |
| 95 | A12 | S6 | B | 600.2 | 1-(3,4-Dichlorphenylsulfonyl)-N-((4-((dimethylamino)(phenyl)methyl)cyclohexyL)methyl)in dolin-2-carboxamid |
| 96 | A17 | S6 | B | 628.2 | 1-(3,4-Dichlorphenylsulfonyl)-N-((4-(1-(dimethylamino)-3-phenylpropyl)cyclohexyl)methyl)indolin-2-carboxamid |
| 97 | A27 | S6 | B | 642.2 | 1-(3,4-Dichlorphenylsulfonyl)-N-(2-(4-(1-(dimethylamino)-3-phenylpropyl)cyclohexyl)ethyl)indolin-2-carboxamid |
| 98 | A22 | S2 | B | 616.2 | 2-(2-(3,4-Dichlor-N-methylphenylsulionamido)phenyl)-N-(2-(4-((dimethylamino)(phenyl)methyl)cyclohexyl)ethyl)ace tamid |
| 99 | A27 | S2 | B | 644.2 | 2-(2-(3,4-Dichlor-N-methylphenylsulfonamido)phenyl)-N-(2-(4-(1-(dimethylamino)-3-phenylpropyl)cyclohexyl)ethyl)acetamid |
| 100 | A22 | S9 | B | 570.3 | N-(2-(4-((Dimethylamino)(phenyl)methyl)cyclohexyl)ethy)-1-(4-methoxy-N-methylphenylsulfonamido)cyclohexancarboxamid |
| 101 | A11 | S11 | C | 623.2 | 2-(1-(2,4-Dichlorphenylsulfonyl)-3-oxopiperazin-2-yl)-N-(4-(morpholino(phenyl)methyl)cyclohexyl)acetamid |
| 102 | A9 | S11 | C | 607.2 | 2-(1-(2,4-Dichlorphenylsulfonyl)-3-oxopiperazin-2-yl)-N-(4-(phenyl(pyrrolidin-1-yl)methyl)cyclohexyl)acetamid |
| 103 | A9 | S12 | C | 655.2 | 2-(1-(3,4-Dichlorphenylsulfonyl)-3-oxo-1,2,3,4-tetrahydrochinoxalin-2-yl)-N-(4-(phenyl(pyrrolidin-1-yl)methyl)cyclohexyl)acetamid |
| 104 | A11 | S13 | C | 594.2 | 2-(1-(3,4-Dichlorphenylsulfonyl)pyrrolidin-2-yl)-N-(4-(morpholino(phenyl)methyl)cyclohexyl)acetamid |
| 105 | A9 | S13 | C | 578.2 | 2-(1-(3,4-Dichlorphenylsulfonyl)pyrrolidin-2-yl)-N-(4-(phenyl(pyrrolidin-1-yl)methyl)cyclohexyl)acetamid |
| 106 | A11 | S14 | C | 608.2 | 2-(1-(3,4-Dichlorphenylsulfonyl)piperidin-2-yl)-N-(4-(morpholino(phenyl)methyl)cyclohexyl)acetamid |
| 107 | A9 | S14 | C | 592.2 | 2-(1-(3,4-Dichlorphenylsulfonyl)piperidin-2-yl)-N-(4-(phenyl(pyrrolidin-1-yl)methyl)cyclohexyl)acetamid |
| 108 | A33 | S8 | C | 607,3 | 1-(4-((4-Methylpiperazin-1-yl)(phenyl)methyl)-piperidin-1-yl)-2-(1-(3-(trifluormethyl)-phenyl-sulfonyl)piperidin-2-yl)ethanon |
| 109 | A32 | S8 | C | 625,3 | 1-(4-((4-Fluorphenyl)(4-methylpiperazin-1-yl)methyl)piperidin-1-yl)-2-(1-(3-(trifluormethyl)phenylsulfonyl)piperidin-2-yl)ethanon |
| 110 | A31 | S16 | C | 629,3 | N-(3-(4-((3-Fluorphenyl)(4-methylpiperazin-1-yl)methyl)piperidin-1-yl)-3-oxo-1-phenylpropyl)naphthalin-2-sulfonamid |
| 111 | A34 | S8 | C | 621,3 | 1-(4-(1-(4-Methylpiperazin-1-yl)-2-phenylethyl)piperidin-1-yl)-2-(1-(3-(trifluormethyl)phenylsulfonyl)piperidin-2-yl)ethanon |
| 112 | A35 | S8 | C | 621,3 | 1-(4-(1-(4-Methylpiperazin-1-yl)-3-phenylpropyl)piperidin-1-yl)-2-(1-(3-(trifluormethyl)phenylsulfonyl)piperidin-2-yl)ethanon |
| 113 | A35 | S16 | C | 635,3 | N-(3-(4-(1-(4-Methylpiperazin-1-yl)-3-phenylpropyl)piperidin-1-yl)-3-oxo-1-phenylpropyl)naphthalene-2-sulfonamid |
| 114 | A33 | S16 | C | 639,3 | N-(3-(4-((4-Methylpiperazin-1-yl)(phenyl)methyl)piperidin-1-yl)-3-oxo-1-phenylpropyl)naphthalin-2-sulfonamid |
| 115 | A31 | S8 | C | 611,3 | 1-(4-((3-Fluorphenyl)(4-methylpiperazin-1-yl)methyl)piperidin-1-yl)-2-(1-(3-(trifluormethyl)phenylsulfonyl)piperidin-2-yl)ethanon |
| 116 | A34 | S16 | C | 625,3 | N-(3-(4-(1-(4-Methylpiperazin-1-yl)-2-phenylethyl)piperidin-1-yl)-3-oxo-1-phenylpropyl)naphthalin-2-sulfonamid |
| 117 | A32 | S16 | C | 626,3 | N-(3-(4-((4-Fluorphenyl)(4-methylpiperazin-1-yl)methyl)piperidin-1-yl)-3-oxo-1-phenylpropyl)naphthalen-2-sulfonamid |
| 118 | A34 | S17 | C | 629,3 | 3-(1-(4-Chlor-2,5-dimethylphenylsulfonyl)piperidin-2-yl)-1-(4-(1-(4-methylpiperazin-1-yl)-2-phenylethyl)piperidin-1-yl)propan-1-on |
| 119 | A35 | S17 | C | 630,3 | 3-(1-(4-Chlor-2,5-dimethylphenylsulfonyl)piperidin-2-yl)-1-(4-(1-(4-methylpiperazin-1-yl)-3-phenylpropyl)piperidin-1-yl)propan-1-on |
| 120 | A31 | S17 | C | 643,3 | 3-(1-(4-Chlor-2,5-dimethylphenylsulfonyl)piperidin-2-yl)-1-(4-((3-fluorphenyl)(4-methylpiperazin-1-yl)methyl)piperidin-1-yl)propan-1-on |
| 121 | A32 | S17 | C | 633,3 | 3-(1-(4-Chlor-2,5-dimethylphenylsulfonyl)piperidin-2-yl)-1-(4-((4-fluorphenyl)(4-methylpiperazin-1-yl)methyl)piperidin-1-yl)propan-1-on |
| 122 | A33 | S17 | C | 633,3 | 3-(1-(4-Chlor-2,5-dimethylphenylsulfonyl)piperidin-2-yl)-1-(4-((4-methylpiperazin-1-yl)(phenyl)methyl)piperidin-1-yl)propan-1-on |

### Untersuchungen zur Wirksamkeit der erfindungsgemäßen Verbindungen

### Methode zur Bestimmung der Affinität zum humanen µ-Opiatrezeptor

Die Rezeptoraffinität zum humanen µ-Opiatrezeptor wird in einem homogenen Ansatz in Mikrotiterplatten bestimmt. Hierzu werden Verdünnungsreihen der zu prüfenden Substanzen mit einer Rezeptormembranpräparation (15 - 40 µg Protein /250 µl Inkubationsansatz) von CHO-K1-Zellen, welche den humanen µ-Opiatrezeptor exprimieren (RB-HOM-Rezeptormembran-Präparation von Fa PerkinElmer Life Sciences, Zaventem, Belgien) in Gegenwart von 1 nmol/l des radioaktiven Liganden [³H]-Naloxon (NET719, Fa. PerkinElmer Life Sciences, Zaventem, Belgien) sowie von 1 mg WGA-SPA-Beads (Wheat germ agglutinin SPA Beads der FA. Amersham/Pharmacia, Freiburg, Deutschland) in einem Gesamtvolumen von 250 µl für 90 Minuten bei Raumtemperatur inkubiert. Als Inkubationspuffer wird 50 mmol/l Tris-HCl supplementiert mit 0,06 % bovinem Serumalbumin verwendet. Zur Bestimmung der unspezifischen Bindung wird zusätzlich 100 µmol/l Naloxon zugegeben. Nach Beendigung der neunzigminütigen Inkubationszeit werden die Mikrotiterplatten für 20 Minuten bei 1000 g abzentrifugiert und die Radioaktivität in einem ß-Counter (Microbeta-Trilux, Fa. PerkinElmer Wallac, Freiburg, Deutschland) vermessen. Es wird die prozentuale Verdrängung des radioaktiven Liganden aus seiner Bindung zum humanen µ-Opiatrezeptor bei einer Konzentration der Prüfsubstanzen von 1 µmol/l bestimmt und als Prozent Hemmung der spezifischen Bindung angegeben. Ausgehend von der prozentualen Verdrängung durch unterschiedliche Konzentrationen der Prüfsubstanzen werden IC₅₀ Hemmkonzentrationen berechnet, die eine 50-prozentige Verdrängung des radioaktiven Liganden bewirken. Durch Umrechnung mittels der Cheng-Prusoff-Beziehung werden Kᵢ-Werte für die Prüfsubstanzen erhalten.

### Funktionelle Untersuchung am humanan Bradykinin Rezeptor 1 (B1R)

Die agonistische bzw. antagonistische Wirkung von Substanzen kann am Bradykinin Rezeptor 1 (B1R) der Spezies Mensch und Ratte mit folgendem Assay bestimmt werden. Gemäß diesem Assay wird der Ca²⁺-Einstrom durch den Kanal mit Hilfe eines Ca²⁺-sensitiven Farbstoffs (Typ Fluo-4, Molecular Probes Europe BV, Leiden Niederlande) im Fluorescent Imaging Plate Reader (FLIPR, Molecular Devices, Sunnyvale, USA) quantifiziert.

### Methode:

Es werden Chinese Hamster Ovary Zellen (CHO K1 Zellen) verwendet, die stabil mit dem humanen B1 R-Gen (hB1 R-Zellen) bzw. dem B1 R-Gen der Ratte (rB1 R-Zellen) transfiziert sind. Für funktionelle Untersuchungen werden diese Zellen auf schwarze 96-Loch-Platten mit klarem Boden (BD Biosciences, Heidelberg, Deutschland oder Greiner, Frickenhausen, Deutschland) in einer Dichte von 20.000 - 35.000 Zellen/Loch ausplattiert. Über Nacht werden die Zellen bei 37 °C und 5 % CO₂ in Kulturmedium (hB1R-Zellen: Nutrient Mixture Ham's F12, Gibco Invitrogen GmbH, Karlsruhe, Deutschland oder DMEM, Sigma-Aldrich, Taufkirchen, Deutschland; rB1R-Zellen: D-MEM/F12, Gibco Invitrogen GmbH, Karlsruhe, Deutschland) mit 10 Vol% FBS (Fetal bovine serum, Gibco Invitrogen GmbH, Karlsruhe, Deutschland oder PAN Biotech GmbH, Aidenbach, Deutschland) belassen.

Am folgenden Tag werden die Zellen mit 2,13 µM Fluo-4 (Molecular Probes Europe BV, Leiden Niederlande) in HBSS-Puffer (Hank's buffered saline solution, Gibco Invitrogen GmbH, Karlsruhe, Deutschland) mit 2,5 mM Probenecid (Sigma-Aldrich, Taufkirchen, Deutschland) und 10 mM HEPES (Sigma-Aldrich, Taufkirchen, Deutschland) für 60 min bei 37 °C beladen. Anschließend werden die Platten 2 x mit HBSS-Puffer gewaschen und mit HBSS-Puffer versetzt, der zusätzlich 0,1 % BSA (bovines Serumalbumin; Sigma-Aldrich, Taufkirchen, Deutschland), 5,6 mM Glucose und 0,05 % Gelatine (Merck KGaA, Darmstadt, Deutschland) enthält. Nach einer weiteren Inkubation von 20 Minuten bei Raumtemperatur werden die Platten zur Ca²⁺-Messung im FLIPR eingesetzt.

Alternativ wird mit Puffer A (15 mM HEPES, 80 mM NaCl, 5 mM KCI, 1,2 mM CaCl₂, 0,7 mM MgSO₄, 2g/L Glucose, 2,5 mM Probenecid) gewaschen und mit Puffer A versetzt mit 2,5 µM Fluo-4 und 0,025 % Pluronic F127 (Sigma-Aldrich, Taufkirchen, Deutschland) beladen. Danach werden die Zellen 2 x mit Puffer A gewaschen und für 30 Minuten mit Puffer A, der zusätzlich 0,05 % BSA und 0,05 % Gelatine enthält bei Raumtemperatur inkubiert und danach zur Ca²⁺-Messung im FLIPR eingesetzt.

Die Ca²⁺-abhängige Fluoreszenz wird dabei vor und nach Zugabe von Substanzen gemessen (λₑₓ = 488 nm, λₑₘ = 540 nm). Die Quantifizierung erfolgt durch die Messung der höchsten Fluoreszenzintensität (FC, Fluorescence Counts) über die Zeit.

### FLIPR-ASSAY:

Das FLIPR-Protokoll besteht aus 2 Substanzzugaben. Zunächst werden Testsubstanzen (10 µM) auf die Zellen pipettiert und der Ca²⁺-Einstrom mit der Kontrolle (hB1R: Lys-Des-Arg⁹-Bradykinin >= 50 nM; rB1R: Des-Arg⁹-Bradykinin 10 µM) verglichen. Daraus ergibt sich die Angabe in % Aktivierung bezogen auf das Ca²⁺-Signal nach Zugabe von Lys-Des-Arg⁹-Bradykinin (>= 50 nM), bzw Des-Arg⁹-Bradykinin (10 µM).

Nach 10-20 Minuten Inkubation werden Lys-Des-Arg⁹-Bradykinin (hB1 R) bzw. Des-Arg⁹-Bradykinin (rB1 R) in der Konzentration des EC₈₀ appliziert und ebenfalls der Einstrom von Ca²⁺ ermittelt.

Antagonisten führen zu einer Unterdrückung des Ca²⁺-Einstroms. Es werden % Inhibition im Vergleich zu der maximal erreichbaren Inhibition berechnet.

Zur Bestimmung des IC₅₀-Wertes werden die Substanzen in verschiedenen Konzentrationen zugegeben. Es werden Zweifach- oder Dreifach-Bestimmungen (n=2 oder n=3) durchgeführt und diese in mindestens einem weiteren unabhängigen Experiment wiederholt (N>=2).

| **Beispiel** | **B1R Antagonismus, human [10 µM] % Hemmung** | **B1R Antagonismus, Ratte [10 µM] % Hemmung** | **µ-Opioid-Rezeptor [1 µM] % Hemmung** |
|---|---|---|---|
| 1 | 71.5 | 56 | 94 |
| 2 | 55.6 | 43 | 97 |
| 3 | 80.8 | 58 | 71 |
| 4 | 54.8 | 95 | 100 |
| 5 | 100.8 | 60 | 89 |
| 6 | 75.1 | 41 | 29 |
| 7 | 101.5 | 59 | 96 |
| 8 | 71.4 | 9 | 94 |
| 9 | 105 | 0 | |
| 10 | 70.1 | 0 | |
| 11 | 85.45 | 107 | |
| 12 | - | 0 | |
| 13 | 64.4 | 76 | |
| 14 | 86.9 | 75 | |
| 15 | 44.6 | 57 | |
| 16 | 35.5 | 57 | |
| 17 | 36.6 | 41 | |
| 18 | 58.8 | 73 | |
| 19 | 42.7 | 41 | |
| 20 | 38.8 | 28 | |
| 21 | 52.9 | 41 | |
| 22 | 70.3 | 34 | |
| 23 | 64 | 57 | |
| 24 | 82.2 | 67 | |
| 25 | 79.5 | 68 | |
| 26 | 97.4 | 73 | |
| 27 | 102.6 | 78 | |
| 28 | 26.3 | 34 | |
| 29 | 64.5 | 45 | |
| 30 | 75.7 | 58 | |
| 31 | 54. | 48 | |
| 32 | 10.7 | 15 | |
| 33 | 42.2 | 18 | |
| 34 | 71.2 | 41 | |
| 35 | 79.5 | 49 | |
| 36 | 64.1 | 67 | |
| 37 | 82.2 | 64 | |
| 38 | 17.9 | 15 | |
| 39 | 79.3 | 42 | |
| 40 | 17.1 | 20 | |
| 41 | 22. | 25 | |
| 42 | | 3 | |
| 43 | 28.9 | 11 | |
| 44 | 46.5 | 18 | |
| 45 | 75.7 | 26 | |
| 46 | - | -9 | |
| 47 | - | 12 | |
| 48 | - | 19 | |
| 49 | - | 19 | |
| 50 | - | 11 | |
| 51 | - | 1 | |
| 52 | - | -22 | |
| 53 | - | 12 | |
| 54 | 105 | 44 | 12 |
| 55 | 102.9 | 26 | 24 |
| 56 | 103.6 | 20 | 11 |
| 57 | 103.9 | 27 | 25 |
| 58 | 103.8 | 11 | 70 |
| 59 | 104.1 | 27 | 61 |
| 60 | 103.7 | 8 | 54 |
| 61 | 103.8 | 39 | 34 |
| 62 | - | 27 | 4 |
| 63 | 28.6 | -3 | 47 |
| 64 | 104.2 | 29 | 10 |
| 65 | 103.3 | 24 | 9 |
| 66 | 102.1 | 96 | 11 |
| 67 | 103.8 | 104 | 64 |
| 68 | 103.9 | 37 | 30 |
| 69 | 104.8 | 19 | 7 |
| 70 | 104 | 98 | 11 |
| 71 | 104.5 | 32 | 12 |
| 72 | 101.6 | 30 | 26 |
| 73 | 76.3 | 36 | 17 |
| 74 | 54.7 | 19 | 7 |
| 75 | 91.7 | 2 | 65 |
| 76 | 80.4 | 9 | 84 |
| 77 | 101.7 | 1 | 35 |
| 78 | 78.3 | 10 | 18 |
| 79 | 96.8 | 33 | 47 |
| 80 | 16.7 | 15 | 7 |
| 81 | 65.3 | 56 | 16 |
| 82 | 23.5 | 7 | 23 |
| 83 | 37 | -3 | 22 |
| 84 | 95.4 | 19 | 90 |
| 85 | 92.7 | 22 | 95 |
| 86 | 100.7 | 30 | 54 |
| 87 | 96.8 | 25 | 38 |
| 88 | 101 | 61 | 58 |
| 89 | 16.4 | 26 | 8.5 |
| 90 | 14.4 | 17 | 12 |
| 91 | 17.8 | 8 | |
| 92 | 26 | -13 | |
| 93 | 15.5 | -24 | |
| 94 | - | -19 | |
| 95 | 18.6 | -15 | |
| 96 | - | 12 | |
| 97 | 16 | 11 | |
| 98 | 66.8 | -8 | |
| 99 | 83.3 | -7 | |
| 100 | - | -2 | |
| 101 | 103.6 | 31 | 4 |
| 102 | 104.3 | 12 | 35 |
| 103 | 103.4 | 37 | 71 |
| 104 | 32.8 | 13 | 11 |
| 105 | 99.5 | 24 | 76 |
| 106 | 48.3 | 19 | 21 |
| 107 | 102.3 | 26 | 78 |
| 108 | 97 | 78 | 6 |
| 109 | 102 | 99 | 5 |
| 110 | 102 | 100 | 3 |
| 111 | 94 | 61 | -2 |
| 112 | 90 | 61 | 28 |
| 113 | 97 | 39 | 21 |
| 114 | 93 | 71 | 40 |
| 115 | 87 | 52 | 11 |
| 116 | 102 | 93 | 7 |
| 117 | - | | 47 |
| 118 | 101 | 93 | 3 |
| 119 | 102 | 97 | 52 |
| 120 | 105 | 73 | 41 |
| 121 | 105 | 58 | 6 |
| 122 | 98 | 40 | 47 |

## Patentansprüche

1. Substituierte Sulfonamid-Derivate der allgemeinen Formel I, 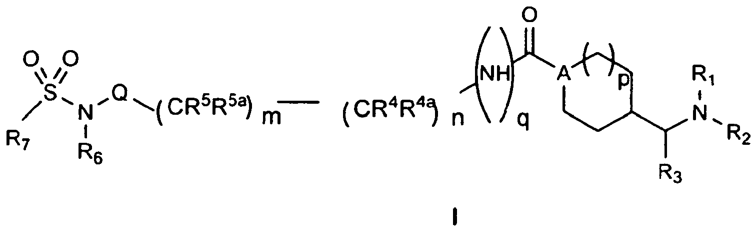 worin
m für 0, 1 oder 2 steht
n für 0, 1 oder 2 steht
p für 0, 1 oder 2 steht
q für 0 oder 1 steht
A für N, CH-NH-, CH-CH₂-NH-, CH-CH₂-CH₂NH oder CH-CH₂-CH₂-CH₂-NH steht, wobei einzelne H-Atome auch durch C₁₋₅-Alkyl ersetzt sein können,
R¹ und R² unabhängig voneinander H; C₁₋₆-Alkyl, jeweils gesättigt oder ungesättigt, verzweigt oder unverzweigt, unsubstituiert oder einfach oder mehrfach substituiert; oder über eine C₁₋₃-Alkylkette verknüpftes Aryl, unsubstituiert oder einfach oder mehrfach substituiert, bedeuten, wobei R¹ und R² nicht gleichzeitig H bedeuten,
oder die Reste R¹ und R² zusammen CH₂H₂OCH₂CH₂, CH₂CH₂NR⁸CH₂CH₂ oder (CH₂)₃₋₆ bedeuten,
wobei R⁸ H; C₁₋₆-Alkyl, jeweils gesättigt oder ungesättigt, verzweigt oder unverzweigt, unsubstituiert oder einfach oder mehrfach substituiert; Aryl, Hetereoaryl, jeweils unsubstituiert oder einfach oder mehrfach substituiert, oder über eine C₁₋₃-Alkylkette verknüpftes Aryl oder Heteroaryl, jeweils unsubstituiert oder einfach oder mehrfach substituiert, bedeutet;
R³ für C₁₋₈-Alkyl jeweils gesättigt oder ungesättigt, verzweigt oder unverzweigt, unsubstituiert oder einfach oder mehrfach substituiert; Aryl oder Heteroaryl, jeweils unsubstituiert oder einfach oder mehrfach substituiert; oder über eine C₁₋₃-Alkylkette verknüpftes Aryl oder Heteroaryl, jeweils unsubstituiert oder einfach oder mehrfach substituiert, steht;
R⁴ und R^{4a} unabhängig voneinander für H, C₁₋₆-Alkyl, jeweils gesättigt oder ungesättigt, verzweigt oder unverzweigt, unsubstituiert oder einfach oder mehrfach substituiert; F; Cl; Aryl jeweils unsubstituiert oder einfach oder mehrfach substituiert; oder über eine C₁₋₃-Alkylkette verknüpftes Aryl, jeweils unsubstituiert oder einfach oder mehrfach substituiert, steht;
R⁵ und R^{5a} unabhängig voneinander für H; oder C₁₋₆-Alkyl, jeweils gesättigt oder ungesättigt, verzweigt oder unverzweigt, unsubstituiert oder einfach oder mehrfach substituiert; OH, OC₁₋₆-Alkyl, F, Cl, Phenoxy oder Benzyloxy; stehen;
Q eine Einfachbindung, -CH₂-, -CH₂-CH₂-, oder 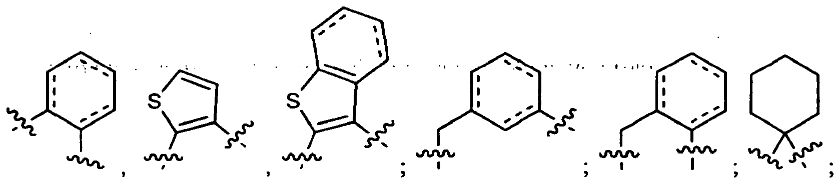 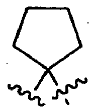 bedeutet,
wobei -̅ -̅ -̅ -̅ für eine Einfachbindung oder für eine Doppelbindung steht;
R⁶ für H; C₁₋₆-Alkyl, jeweils gesättigt oder ungesättigt, verzweigt oder unverzweigt, unsubstituiert oder einfach oder mehrfach substituiert; C₃₋₈-Cycloalkyl, gesättigt oder ungesättigt, unsubstituiert oder einfach oder mehrfach substituiert; Aryl oder Heteroaryl, unsubstituiert oder einfach oder mehrfach substituiert; über eine C₁₋₃-Alkylkette verknüpftes Aryl oder C₃₋₈-Cycloalkyl;
oder R⁶ mit Q zusammen unter Einschluss des benachbarten Stickstoffs einen vier-, fünf-, sechs- oder siebengliedrigen Carbocyclus bildet, der gesättigt oder ungesättigt sein und ein weiteres Heteroatom O, S oder N enthalten kann, an den ein weiterer fünf- oder sechsgliedriger Ring, gesättigt oder ungesättigt, ankondensiert sein kann; wobei im Fall des gemeinsamen Ringschlusses Q für 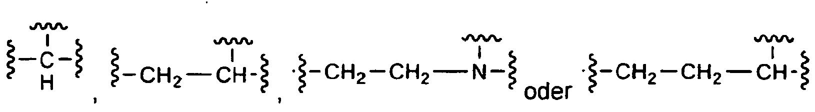 steht, und der Ring in jeder Position mit Phenyl, =O, OH, OC₁₋₆-Alkyl, F, Cl, CF₃, oder C₁₋₆-Alkyl substituiert sein kann;
und R⁷ für Aryl oder Heteroaryl, jeweils unsubstituiert oder einfach oder mehrfach substituiert; über eine C₁₋₃-Alkylgruppe verknüpftes Aryl oder Heteroaryl jeweils unsubstituiert oder einfach oder mehrfach substituiert;
in Form des Razemats; der Enantiomere, Diastereomere, Mischungen der Enantiomere oder Diastereomere oder eines einzelnen Enantiomers oder Diastereomers; der Basen und/oder Salze physiologisch verträglicher Säuren.

2. Substituierte Sulfonamid-Derivate gemäß Anspruch 1,
wobei
"Alkyl sustituiert" und "Cycloalkyl substituiert" die Substitution eines oder mehrerer Wasserstoffreste durch F; Cl, Br, I, -CN, NH₂, NH-C₁₋₆-Alkyl, NH-C₁₋₆-Alkyl-OH, C₁₋₆-Alkyl, N(C₁₋₆-Alkyl)₂, N(C₁₋₆-Alkyl-OH)₂, NO₂, SH, S-C₁₋₆Alkyl, S-Benzyl, O-C₁₋₆-Alkyl, OH, O-C₁₋₆-Alkyl-OH, =O, O-Benzyl, C(=O)C₁₋₆-Alkyl, CO₂H, CO₂-C₁₋₆-Alkyl oder Benzyl,
"Aryl substituiert" und "Heteroaryl substituiert" die ein- oder mehrfache, z.B. zwei-, drei- oder vierfache, Substitution eines oder mehrerer Wasserstoffatome des Ringsystems durch F, Cl, Br, I, CN, NH₂, NH-C₁₋₆-Alkyl, NH- C₁₋₆-Alkyl-OH, N(C₁-₆Alkyl)₂, N(C₁₋₆-Alkyl-OH)₂, NO₂, SH, S-C₁₋₆-Alkyl, OH, O-C₁₋₆-Alkyl, O-C₁₋₆Alkyl-OH, C(=O)C₁₋₆-Alkyl, CO₂H, CH₂SO₂-Phenyl, CO₂-C₁₋₆-Alkyl, OCF₃, CF₃, 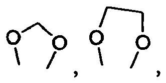 C₁₋₆-Alkyl, Phenyl, Pyridyl, Thienyl oder Furyl bedeutet,
in Form des Razemats; der Enantiomere, Diastereomere, Mischungen der Enantiomere oder Diastereomere oder eines einzelnen Enantiomers oder Diastereomers; der Basen und/oder Salze physiologisch verträglicher Säuren.

3. Substituierte Sulfonamid-Derivate gemäß Anspruch 1, worin A für CH-NH-, CH-CH₂-NH-, CH-CH₂-CH₂-NH oder CH-CH₂-CH₂-CH₂-NH steht, wobei einzelne H-Atome auch durch C₁₋₅-Alkyl ersetzt sein können.

4. Substituierte Sulfonamid-Derivate gemäß Anspruch 1, worin R¹ und R² unabhängig voneinander H; CH₃; C₂H₅, oder über eine C₁₋₃-Alkylkette verknüpftes Phenyl, bedeuten, wobei R¹ und R² nicht gleichzeitig H bedeuten,
oder die Reste R¹ und R² zusammen CH₂CH₂OCH₂CH₂, CH₂CH₂NR⁸CH₂CH₂ oder (CH₂)₃₋₅ bedeuten.

5. Substituierte Sulfonamid-Derivate gemäß Anspruch 1, worin R³ für Aryl oder Heteroaryl, jeweils unsubstituiert oder einfach oder mehrfach substituiert; oder über eine C₁₋₃-Alkylkette verknüpftes Aryl, jeweils unsubstituiert oder einfach oder mehrfach substituiert, steht.

6. Substituierte Sulfonamid-Derivate gemäß Anspruch 5, worin R³ für 2-Thienyl, 4-Fluorphenyl, 3-Fluorphenyl, 4-Chlorphenyl, Phenethyl, Phenyl oder Benzyl steht.

7. Substituierte Sulfonamid-Derivate gemäß Anspruch 1, worin R⁴ und R^{4a} für H stehen.

8. Substituierte Sulfonamid-Derivate gemäß Anspruch 1, worin R⁵ und R^{5a} für H stehen.

9. Substituierte Sulfonamid-Derivate gemäß Anspruch 1, worin R⁶ für Methyl, Ethyl oder Benzyl steht und Q eine Einfachbindung darstellt, wobei R⁶ insbesondere Methyl bedeutet.

10. Substituierte Sulfonamid-Derivate gemäß Anspruch 1, worin die Gruppe 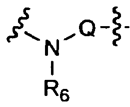 der allgemeinen Formel I für 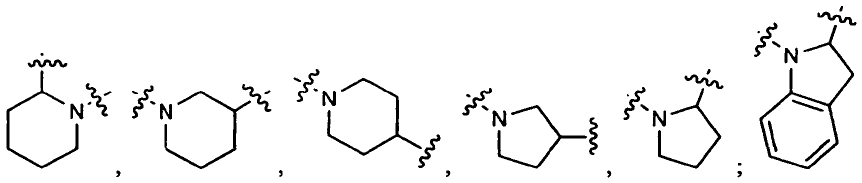 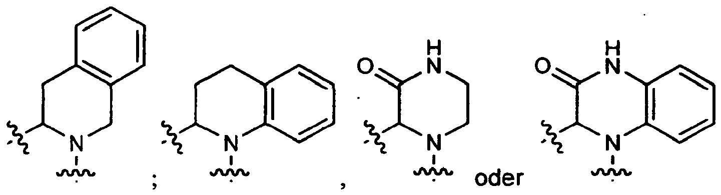 steht.

11. Substituierte Sulfonamid-Derivate gemäß Anspruch 1, worin Q 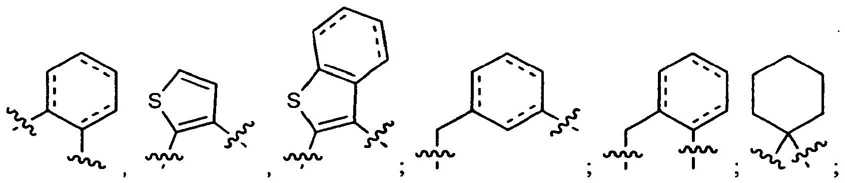 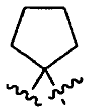 bedeutet.

12. Substituierte Sulfonamid-Derivate gemäß Anspruch 1, worin die Gruppe 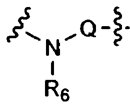 der allgemeinen Formel I für 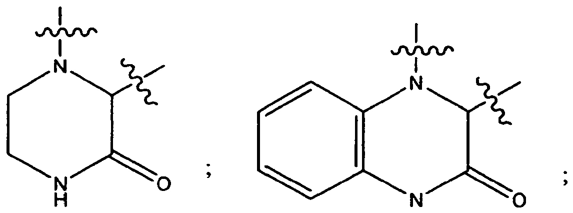 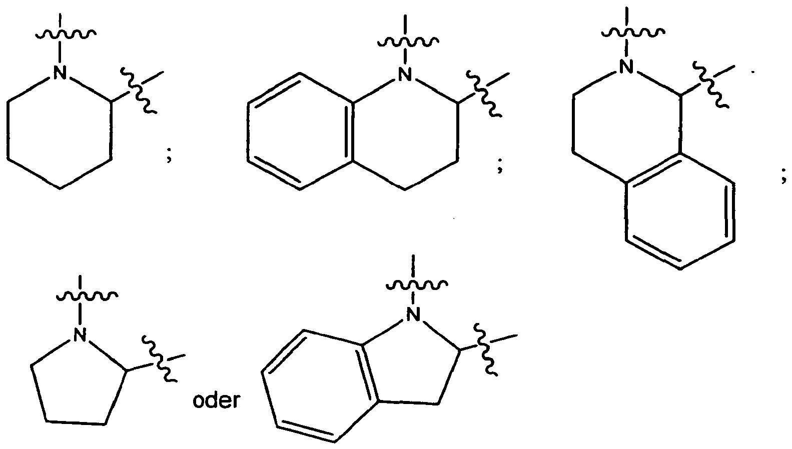 steht;
m für 0, 1 oder 2 steht;
n für 0 steht,
q für 0 steht; und
R⁵ und R^{5a} für H stehen;
oder
Q für eine Einfachbindung steht;
m für 0, 1 oder 2 steht;
n für 1 oder 2 steht;
q für 0 oder 1 steht;
R⁴ und R^{4a} jeweils unabhängig voneinander für H oder Aryl stehen;
R⁵ und R^{5a} für H stehen; und
R⁶ für H oder C₁₋₆-Alkyl steht;
oder
Q für 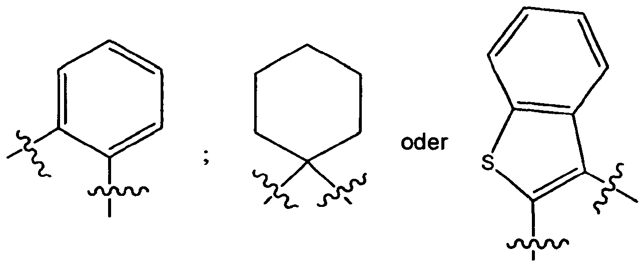 steht;
m für 0 oder 1, vorzugsweise 0, steht;
n und q für 0 stehen;
R⁵ und R^{5a} für H stehen; und
R⁶ für H oder C₁₋₆Alkyl steht.

13. Substituierte Sulfonamid-Derivate gemäß Anspruch 1, worin in der allgemeinen Formel I
A für N, NH-CH, NH-CH₂-CH oder NH-CH₂-CH₂-CH steht, wobei einzelne H-Atome auch durch C₁₋₅-Alkyl ersetzt sein können;
R¹ und R² unabhängig voneinander für C₁₋₆-Alkyl, insbesondere Methyl, Ethyl, n-Propyl, iso-Propyl, n-Butyl, sec-Butyl, iso-Butyl und t-Butyl, stehen, oder zusammen mit dem N-Atom, an das sie gebunden sind, eine Gruppe ausgesucht aus 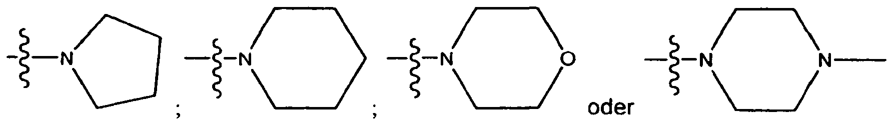 bilden; und
R³ für Aryl, insbesondere Phenyl oder Furanyl, das über eine C₁₋₃-Alkylgruppe verknüpft sein kann, steht, wobei das Aryl jeweils unsubstituiert oder einfach oder mehrfach, gleich oder verschieden mit Substituenten unabhängig ausgewählt aus der Gruppe bestehend aus Methyl, Ethyl, Methoxy, Ethoxy, F, Cl, Br, F, CN, CF₃, OCF₃ und OH substituiert ist;

14. Substituierte Sulfonamid-Derivate gemäß Anspruch 1,
worin die Gruppe 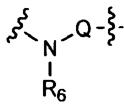 in der allgemeinen Formel I für 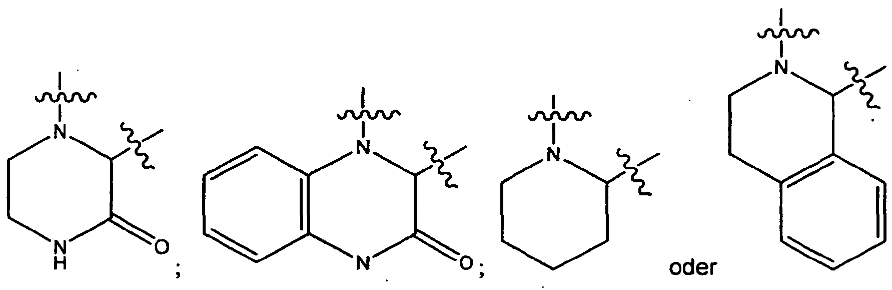 steht;
m für 1 oder 2, insbesondere 1, steht;
n und q für 0 stehen;
R⁵ und R^{5a} für H stehen;
A für N, NH-CH oder NH-CH₂-CH, insbesondere N oder NH-CH steht,
R¹ und R² unabhängig voneinander für C₁₋₆-Alkyl, insbesondere Methyl, Ethyl, n-Propyl, iso-Propyl, n-Butyl, sec-Butyl, iso-Butyl und t-Butyl, stehen, oder zusammen mit dem N-Atom, an das sie gebunden sind, eine Gruppe ausgesucht aus 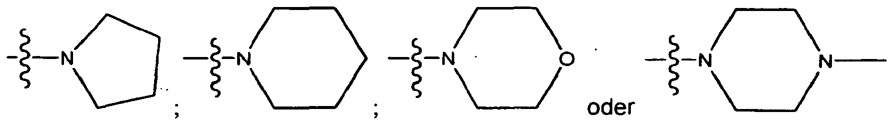 bilden; und
R₃ für Phenyl steht, das über eine C₁₋₃-Alkylgruppe verknüpft sein kann, wobei dass Phenyl jeweils unsubstituiert oder einfach oder mehrfach, gleich oder verschieden mit Substituenten unabhängig ausgewählt aus der Gruppe bestehend aus Methyl, Ethyl, Methoxy, Ethoxy, F, Cl, Br, F, CN, CF₃, OCF₃ und OH, insbesondere Methyl, Methosy, F, Cl, Br, CN, CF₃ und OH, substituiert ist.

15. Substituierte Sulfonamid-Derivate gemäß Anspruch 1, worin R⁷ 2,4-Dichlorphenyl, 2,4,6-Trichlorphenyl, 4-Methoxyphenyl, 3-Trifluormethylphenyl, 2,6-Dimethyl-4-methoxyphenyl oder 3,4-Dichlorphenyl steht.

16. Substituierte Sulfonamid-Derivate gemäß Anspruch 1 aus der Gruppe
(1) N-(3-{3-[4-(Dimethylaminophenylmethyl)cyclohexyl]ureido}propyl)-4-methoxy-2,3,6,N-tetramethylbenzolsulfonamid
(2) (*N*-(3-(3-((4-((dimethylamino)(phenyl)methyl)cyclohexyl)methyl)ureido)propyl)-4-methoxy-*N*,2,3,6-tetramethylbenzolsulfonamid
(3) 4-Methoxy-N,2,3,6-tetramethyl-N-(3-(3-(4-(phenyl(pyrrolidin-1-yl)methyl)cyclohexyl)ureido)propyl)benzolsulfonamid
(4) 4-(Dimethylamino-phenyl-methyl)-piperidin-1-carbonsäure-{3-[(4-methoxy-2,3,6-trimethyl-benzolsulfonyl)-methyl-amino]-propyl}-amid
(5) 5-[Methyl-(2,4,6-trichlor-benzolsulfonyl)-amino]-pentancarbonsäure-[4-(dimethylamino-phenyl-methyl)-cyclohexyl]-amid
(6) 5-[Methyl-(2,4,6-trichlor-benzolsulfonyl)-amino]-pentancarbonsäure-[4-(phenylpiperidin-1-yl-methyl)-cyclohexyl]-amid
(7) 5-[Methyl-(2,4,6-trichlor-benzolsulfonyl)-amino]-pentancarbonsäure-[4-(dimethylamino-phenyl-methyl)-cyclohexylmethyl]-amid
(8) N-[4-(Dimethylamino-phenyl-methyl)-cyclohexylmethyl]-2-[1-(3-trifluormethylbenzolsulfonyl)-piperidin-2-yl]-acetamid
(9) N-[4-(Dimethylamino-phenyl-methyl)-cyclohexyl]-2-[2-(4-methoxybenzolsulfonyl)-1,2,3,4-tetrahydro-isochinolin-1-yl]-acetamid
(10) 2-[2-(3,4-Dichlor-benzolsulfonyl)-1,2,3,4-tetrahydro-isochinolin-1-yl]-N-[4-(dimethylamino-phenyl-methyl)-cyclohexyl]-acetamid
(11) 2-[(3,4-Dichlor-benzolsulfonyl)-methyl-amino]-N-[4-(dimethylamino-phenylmethyl)-cyclohexyl]-benzamid
(12) 2-[(3,4-Dichlor-benzolsulfonyl)-methyl-amino]-4,5,6,7-tetrahydrobenzo[b]thiophen-3-carbonsäure-[4-(dimethylamino-phenyl-methyl)-cyclohexyl]-amid
(13) N-{4-[Dimethylamino-(3-fluor-phenyl)-methyl]-cyclohexyl}-2-[2-(4-methoxybenzolsulfonyl)-1,2,3,4-tetrahydro-isochinolin-1-yl]-acetamid
(14) N-{4-[Dimethylamino-(4-fluor-phenyl)-methyl]-cyclohexyl}-2-[2-(4-methoxybenzolsulfonyl)-1,2,3,4-tetrahydro-isochinolin-1-yl]-acetamid
(15) N-[4-(Dimethylamino-thiophen-2-yl-methyl)-cyclohexyl]-2-[2-(4-methoxybenzolsulfonyl)-1,2,3,4-tetrahydro-isochinolin-1-yl]-acetamid
(16) N-{4-[Dimethylamino-(3-fluor-phenyl)-methyl]-cyclohexylmethyl}-2-[2-(4-methoxy-benzolsulfonyl)-1,2,3,4-tetrahydro-isochinolin-1-yl]-acetamid
(17) N-[4-(Dimethylamino-phenyl-methyl)-cyclohexylmethyl]-2-[2-(4-methoxybenzolsulfonyl)-1,2,3,4-tetrahydro-isochinolin-1-yl]-acetamid
(18) N-[4-(1-Dimethylamino-3-phenyl-propyl)-cyclohexylmethyl]-2-[2-(4-methoxybenzolsulfonyl)-1,2,3,4-tetrahydro-isochinolin-1-yl]-acetamid
(19) N-{4-[Dimethylamino-(4-fluor-phenyl)-methyl]-cyclohexylmethyl}-2-[2-(4-methoxy-benzolsulfonyl)-1,2,3,4-tetrahydro-isochinolin-1-yl]-acetamid
(20) N-{4-[(4-Chlor-phenyl)-dimethylamino-methyl]-cyclohexylmethyl}-2-[2-(4-methoxy-benzolsulfonyl)-1,2,3,4-tetrahydro-isochinolin-1-yl]-acetamid
(21) N-[4-(Dimethylamino-thiophen-2-yl-methyl)-cyclohexylmethyl]-2-[2-(4-methoxybenzolsulfonyl)-1,2,3,4-tetrahydro-isochinolin-1-yl]-acetamid
(22) N-(2-{4-[(4-Chlor-phenyl)-dimethylamino-methyl]-cyclohexyl}-ethyl)-2-[2-(4-methoxy-benzolsulfonyl)-1,2,3,4-tetrahydro-isochinolin-1-yl]-acetamid
(23) N-(2-{4-[Dimethylamino-(3-fluor-phenyl)-methyl]-cyclohexyl}-ethyl)-2-[2-(4-methoxy-benzolsulfonyl)-1,2,3,4-tetrahydro-isochinolin-1-yl]-acetamid
(24) N-(2-{4-[Dimethylamino-(4-fluor-phenyl)-methyl]-cyclohexyl}-ethyl)-2-[2-(4-methoxy-benzolsulfonyl)-1,2, 3,4-tetrahydro-isochinolin-1-yl]-acetamid
(25) N-{2-[4-(Dimethylamino-thiophen-2-yl-methyl)-cyclohexyl]-ethyl}-2-[2-(4-methoxy-benzolsulfonyl)-1,2,3,4-tetrahydro-isochinolin-1-yl]-acetamid
(26) 2-(2-(3,4-Dichlorphenylsulfonyl)-1,2,3,4-tetrahydroisochinolin-1-yl)-N-(4-((dimethylamino)(3-fluorphenyl)methyl)cyclohexyl)acetamid
(27) 2-(2-(3,4-Dichlorphenylsulfonyl)-1,2,3,4-tetrahydroisochinolin-1-yl)-N-(4-((dimethylamino)(4-fluorphenyl)methyl)cyclohexyl)acetamid
(28) 2-[2-(3,4-Dichlor-benzolsulfonyl)-1,2,3,4-tetrahydro-isochinolin-1-yl]-N-{4-[dimethylamino-(3-fluor-phenyl)-methyl]-cyclohexylmethyl}-acetamid
(29) 2-[2-(3,4-Dichlor-benzolsulfonyl)-1,2,3,4-tetrahydro-isochinolin-1-yl]-N-[4-(dimethylamino-phenyl-methyl)-cyclohexylmethyl]-acetamid
(30) 2-[2-(3,4-Dichlor-benzolsulfonyl)-1,2,3,4-tetrahydro-isochinolin-1-yl]-N-[4-(1-dimethylamino-3-phenyl-propyl)-cyclohexylmethyl]-acetamid
(31) 2-[2-(3,4-Dichlor-benzolsulfonyl)-1,2,3,4-tetrahydro-isochinolin-1-yl]-N-{4-[dimethylamino-(4-fluor-phenyl)-methyl]-cyclohexylmethyl}-acetamid
(32) N-{4-[(4-Chlor-phenyl)-dimethylamino-methyl]-cyclohexylmethyl}-2-[2-(3,4-dichlor-benzolsulfonyl)-1,2,3,4-tetrahydro-isochinolin-1-yl]-acetamid
(33) N-(2-(4-[(4-Chlor-phenyl)-dimethylamino-methyl]-cydohexyl)-ethyl)-2-[2-(3,4-dichlor-benzolsulfonyl)-1,2,3,4-tetrahydro-isochinolin-1-yl]-acetamid
(34) 2-[2-(3,4-Dichlor-benzolsulfonyl)-1,2,3,4-tetrahydro-isochinolin-1-yl]-N-(2-{4-[dimethylamino-(3-fluor-phenyl)-methyl]-cyclohexyl}-ethyl)-acetamid
(35) 2-[2-(3,4-Dichlor-benzolsulfonyl)-1,2,3,4-tetrahydro-isochinolin-1-yl]-N-(2-{4-[dimethylamino-(4-fluor-phenyl)-methyl]-cyclohexyl}-ethyl)-acetamid
(36) 2-[(3,4-Dichlor-benzolsulfonyl)-methyl-amino]-N-{4-[dimethylamino-(3-fluorphenyl)-methyl]-cyclohexyl}-benzamid
(37) 2-[(3,4-Dichlor-benzolsulfonyl)-methyl-amino]-N-{4-[dimethylamino-(4-fluorphenyl)-methyl]-cyclohexyl}-benzamid
(38) 2-[(3,4-Dichlor-benzolsulfonyl)-methyl-amino]-N-[4-(dimethylamino-phenylmethyl)-cyclohexylmethyl]-benzamid
(39) 2-[(3,4-Dichlor-benzolsulfonyl)-methyl-amino]-N-[4-(1-dimethylamino-3-phenylpropyl)-cyclohexylmethyl]-benzamid
(40) 2-[(3,4-Dichlor-benzolsulfonyl)-methyl-amino]-N-{4-[dimethylamino-(4-fluorphenyl)-methyl]-cyclohexylmethyl}-benzamid
(41) N-{4-[(4-Chlor-phenyl)-dimethylamino-methyl]-cyclohexylmethyl}-2-[(3,4-dichlor-benzolsulfonyl)-methyl-amino]-benzamid
(42) 2-[(3,4-Dichlor-benzolsulfonyl)-methyl-amino]-N-[4-(dimethylamino-thiophen-2-yl-methyl)-cyclohexylmethyl]-benzamid
(43) N-(2-{4-[(4-Chlor-phenyl)-dimethylamino-methyl]-cyclohexyl}-ethyl)-2-[(3,4-dichlor-benzolsulfonyl)-methyl-amino]-benzamid
(44) 2-[(3,4-Dichlor-benzolsulfonyl)-methyl-amino]-N-(2-{4-[dimethylamino-(3-fluorphenyl)-methyl]-cyclohexyl}-ethyl)-benzamid
(45) 2-[(3,4-Dichlor-benzolsulfonyl)-methyl-amino]-N-(2-{4-[dimethylamino-(4-fluorphenyl)-methyl]-cyclohexyl}-ethyl)-benzamid
(46) 2-[(3,4-Dichlor-benzolsulfonyl)-methyl-amino]-4,5,6,7-tetrahydrobenzo[b]thiophen-3-carbonsäure-[4-(dimethylamino-phenyl-methyl)-cyclohexylmethyl]-amid
(47) 2-[(3,4-Dichlor-benzolsulfonyl)-methyl-amino]-4,5,6,7-tetrahydrobenzo[b]thiophen-3-carbonsäure-{4-[dimethylamino-(4-fluor-phenyl)-methyl]-cyclohexylmethyl}-amid
(48) 2-[(3,4-Dichlor-benzolsulfonyl)-methyl-amino]-4,5,6,7-tetrahydrobenzo[b]thiophen-3-carbonsäure-(4-[(4-chlor-phenyl)-dimethylamino-methyl]-cyclohexylmethyl}-amid
(49) 2-[(3,4-Dichlor-benzolsulfonyl)-methyl-amino]-4,5,6,7-tetrahydrobenzo[b]thiophen-3-carbonsäure-[4-(dimethylamino-thiophen-2-yl-methyl)-cyclohexylmethyl]-amid
(50) N-(2-(4-((4-chlorphenyl)(dimethylamino)methyl)cyclohexyl)ethyl)-2-(3,4-dichlor-N-methylphenylsulfonamido)-4,5,6,7-tetrahydrobenzo[b]thiophen-3-carboxamid
(51) 2-(3,4-Dichlor-N-methylphenylsulfonamido)-N-(2-(4-((dimethylamino)(3-fluorphenyl)methyl)cyclohexyl)ethyl)-4,5,6,7-tetrahydrobenzo[b]thiophen-3-carboxamid
(52) 2-(3,4-Dichlor-N-methylphenylsulfonamido)-N-(2-(4-((dimethylamino)(4-fluorphenyl)methyl)cyclohexyl)ethyl)-4,5,6,7-tetrahydrobenzo[b]thiophen-3-carboxamid
(53) 2-(3,4-Dichlor-N-methylphenylsulfonamido)-N-(2-(4-((dimethylamino)(thiophen-2-yl)methyl)cyclohexyl)ethyt)-4,5,6,7-tetrahydrobenzo[b]thiophen-3-carboxamid
(54) 2-[1-(3,4-Dichlor-benzolsulfonyl)-3-oxo-1,2,3,4-tetrahydro-chinoxalin-2-yl]-N-[4-(morpholin-4-yl-phenyl-methyl)-cyclohexyl]-acetamid
(55) 2-[1-(2,4-Dichlor-benzolsulfonyl)-3-oxo-piperazin-2-yl]-N-[4-(morpholin-4-yl-phenyl-methyl)-cyclohexylmethyl]-acetamid
(56) 2-[1-(2,4-Dichlor-benzolsulfonyl)-3-oxo-piperazin-2-yl]-N-[4-(1-morpholin-4-yl-3-phenyl-propyl)-cyclohexylmethyl]-acetamid
(57) 2-[1-(2,4-Dichlor-benzolsulfonyl)-3-oxo-piperazin-2-yl]-N-[4-(3-phenyl-1-pyrrolidin-1-yl-propyl)-cyclohexylmethyl]-acetamid
(58) 2-[1-(2,4-Dichlor-benzolsulfonyl)-3-oxo-piperazin-2-yl]-N-[4-(phenyl-pyrrolidin-1-yl-methyl)-cyclohexylmethyl]-acetamid
(59) 2-[1-(2,4-Dichlor-benzolsulfonyl)-3-oxo-piperazin-2-yl]-N-[4-(3-phenyl-1-piperidin-1-yl-propyl)-cyclohexyl]-acetamid
(60) 2-[1-(2,4-Dichlor-benzolsulfonyl)-3-oxo-piperazin-2-yl]-N-[4-(1-morpholin-4-yl-3-phenyl-propyl)-cyclohexyl]-acetamid
(61) 2-[1-(2,4-Dichlor-benzolsulfonyl)-3-oxo-piperazin-2-yl]-N-{4-[(4-methylpiperazin-1-yl)-phenyl-methyl]-cyclohexyl}-acetamid
(62) 4-(2,4-Dichlor-benzolsulfonyl)-3-{2-[4-(morpholin-4-yl-phenyl-methyl)-piperidin-1-yl]-2-oxo-ethyl}-piperazin-2-on
(63) 4-(2,4-dichlorphenylsulfonyl)-3-(2-(2-(1-morpholino-2-phenylethyl)piperidin-1-yl)-2-oxoethyl)piperazin-2-on
(64) 2-[1-(3,4-Dichlor-benzolsulfonyl)-3-oxo-1,2,3,4-tetrahydro-chinoxalin-2-yl]-N-[4-(morpholin-4-yl-phenyl-methyl)-cyclohexylmethyl]-acetamid
(65) 2-[1-(3,4-Dichlor-benzolsulfonyl)-3-oxo-1,2,3,4-tetrahydro-chinoxalin-2-yl]-N-[4-(1-morpholin-4-yl-3-phenyl-propyl)-cyclohexylmethyl]-acetamid
(66) 2-[1-(3,4-Dichlor-benzolsulfonyl)-3-oxo-1,2,3,4-tetrahydro-chinoxalin-2-yl]-N-[4-(3-phenyl-1-pyrrolidin-1-yl-propyl)-cyclohexylmethyl]-acetamid
(67) 2-[1-(3,4-Dichlor-benzolsulfonyl)-3-oxo-1,2,3,4-tetrahydro-chinoxalin-2-yl]-N-[4-(phenyl-pyrrolidin-1-yl-methyl)-cyclohexylmethyl]-acetamid
(68) 2-[1-(3,4-Dichlor-benzolsulfonyl)-3-oxo-1,2,3,4-tetrahydro-chinoxalin-2-yl]-N-[4-(3-phenyl-1-piperidin-1-yl-propyl)-cyclohexyl]-acetamid
(69) 2-[1-(3,4-Dichlor-benzolsulfonyl)-3-oxo-1,2,3,4-tetrahydro-chinoxalin-2-yl]-N-[4-(1-morpholin-4-yl-3-phenyl-propyl)-cyclohexyl]-acetamid
(70) 2-[1-(3,4-Dichlor-benzolsulfonyl)-3-oxo-1,2,3,4-tetrahydro-chinoxalin-2-yl)-N-{4-[(4-methyl-piperazin-1-yl)-phenyl-methyl]-cyclohexyl}-acetamid
(71) 4-(3,4-Dichlor-benzolsulfonyl)-3-{2-[4-(morpholin-4-yl-phenyl-methyl)-piperidin-1-yl]-2-oxo-ethyl}-3,4-dihydro-1H-chinoxalin-2-on
(72) 4-(3,4-dichlorphenylsulfonyl)-3-(2-(4-(1-morpholino-2-phenylethyl)piperidin-1-yl)-2-oxoethyl)-3,4-dihydrochinoxalin-2(1H)-on
(73) 2-[1-(3,4-Dichlor-benzolsulfonyl)-pyrrolidin-2-yl]-N-[4-(morpholin-4-yl-phenylmethyl)-cyclohexylmethyl]-acetamid
(74) 2-[1-(3,4-Dichlor-benzolsulfonyl)-pyrrolidin-2-yl]-N-[4-(1-morpholin-4-yl-3-phenyl-propyl)-cyclohexylmethyl]-acetamid
(75) 2-[1-(3,4-Dichlor-benzolsulfonyl)-pyrrolidin-2-yl]-N-[4-(3-phenyl-1-pyrrolidin-1-yl-propyl)-cyclohexylmethyl]-acetamid
(76) 2-[1-(3,4-Dichlor-benzolsulfonyl)-pyrrolidin-2-yl]-N-[4-(phenyl-pyrrolidin-1-yl-methyl)-cyclohexylmethyl]-acetamid
(77) 2-(1-(3,4-dichlorphenylsulfonyl)pyrrolidin-2-yl)-N-(4-(3-phenyl-1-(piperidin-1-yl)propyl)cyclohexyl)acetamid
(78) 2-(1-(3,4-dichlorphenylsulfonyl)pyrrolidin-2-yl)-N-(4-(1-morpholino-3-phenylpropyl)cyclohexyl)acetamid
(79) 2-[1-(3,4-Dichlor-benzolsulfonyl)-pyrrolidin-2-yl]-N-{4-((4-methyl-piperazin-1-yl)-phenyl-methyl]-cyclohexyl}-acetamid
(80) 2-[1-(3,4-Dichlor-benzolsulfonyl)-pyrrolidin-2-yl]-1-[4-(morpholin-4-yl-phenylmethyl)-piperidin-1-yl]-ethanon
(81) 2-(1-(3,4-dichlorphenylsulfonyl)pyrrolidin-2-yl)-1-(4-(1-morpholino-2-phenylethyl)piperidin-1-yl)ethanon
(82) 2-[1-(3,4-Dichlor-benzolsulfonyl)-piperidin-2-yl]-N-[4-(morpholin-4-yl-phenylmethyl)-cyclohexylmethyl]-acetamid
(83) 2-[1-(3,4-Dichlor-benzolsulfonyl)-piperidin-2-yl]-N-[4-(1-morpholin-4-yl-3-phenyl-propyl)-cyclohexylmethyl]-acetamid
(84) 2-[1-(3,4-Dichlor-benzolsulfonyl)-piperidin-2-yl]-N-[4-(3-phenyl-1-pyrrolidin-1-yl-propyl)-cyclohexylmethyl]-acetamid
(85) 2-[1-(3,4-Dichlor-benzolsulfonyl)-piperidin-2-yl]-N-[4-(phenyl-pyrrolidin-1-yl-methyl)-cyclohexylmethyl]-acetamid
(86) 2-[1-(3,4-Dichlor-benzolsulfonyl)-piperidin-2-yl]-N-[4-(3-phenyl-1-piperidin-1-yl-propyl)-cyclohexyl]-acetamid
(87) 2-[1-(3,4-Dichlor-benzolsulfonyl)-piperidin-2-yl]-N-[4-(1-morpholin-4-yl-3-phenyl-propyl)-cyclohexyl]-acetamid
(88) 2-[1-(3,4-Dichlor-benzolsulfonyl)-piperidin-2-yl]-N-{4-[(4-methyl-piperazin-1-yl)-phenyl-methyl]-cyclohexyl}-acetamid
(89) 2-[1-(3,4-Dichlor-benzolsulfonyl)-piperidin-2-yl]-1-[4-(morpholin-4-yl-phenylmethyl)-piperidin-1-yl]-ethanon
(90) 2-(1-(3,4-Dichlorphenylsulfonyl)piperidin-2-yl)-1-(4-(1-morpholino-2-phenylethyl)piperidin-1-yl)ethanon
(91) 1-(3,4-Dichlorphenylsulfonyl)-N-(4-((dimethylamino)(phenyl)methyl)cyclohexyl) indolin-2-carboxamid
(92) 1-(3,4-Dichlorphenylsulfonyl)-N-(4-((dimethylamino)(3-fluorphenyl)methyl) cyclohexyl)indolin-2-carboxamid
(93) 1-(3,4-Dichlorphenylsulfonyl)-N-(4-(1-(dimethylamino)-3-phenylpropyl) cyclohexyl)indolin-2-carboxamid
(94) 1-(3,4-Dichlorphenylsulfonyl)-N-(4-((dimethylamino)(4-fluorphenyl)methyl)cyclohexyl)indolin-2-carboxamid
(95) 1-(3,4-Dichlorphenylsulfonyl)-N-((4-((dimethylamino)(phenyl) methyl)cyclohexyl)methyl)indolin-2-carboxamid
(96) 1-(3,4-Dichlorphenylsulfonyl)-N-((4-(1-(dimethylamino)-3-phenylpropyl)cyclohexyl)methyl)indolin-2-carboxamid
(97) 1-(3,4-Dichlorphenylsulfonyl)-N-(2-(4-(1-(dimethylamino)-3-phenylpropyl)cyclohexyl)ethyl)indolin-2-carboxamid
(98) 2-(2-(3,4-Dichlor-N-methylphenylsulfonamido)phenyl)-N-(2-(4-((dimethylamino)(phenyl)methyl)cyclohexyl)ethyl)acetamid
(99) 2-(2-(3,4-Dichlor-N-methylphenylsulfonamido)phenyl)-N-(2-(4-(1-(dimethylamino)-3-phenylpropyl)cyclohexyl)ethyl)acetamid
(100) N-(2-(4-((Dimethylamino)(phenyl)methyl)cyclohexyl)ethyl)-1-(4-methoxy-N-methylphenylsulfonamido)cyclohexancarboxamid
(101) 2-(1-(2,4-Dichlorphenylsulfonyl)-3-oxopiperazin-2-yl)-N-(4-(morpholino(phenyl)methyl)cyclohexyl)acetamid
(102) 2-(1-(2,4-Dichlorphenylsulfonyl)-3-oxopiperazin-2-yl)-N-(4-(phenyl(pyrrolidin-1-yl)methyl)cyclohexyl)acetamid
(103) 2-(1-(3,4-Dichlorphenylsulfonyl)-3-oxo-1,2,3,4-tetrahydrochinoxalin-2-yl)-N-(4-(phenyl(pyrrolidin-1-yl)methyl)cyclohexyl)acetamid
(104) 2-(1-(3,4-Dichlorphenylsulfonyl)pyrrolidin-2-yl)-N-(4-(morpholino(phenyl)methyl)cyclohexyl)acetamid
(105) 2-(1-(3,4-Dichlorphenylsulfonyl)pyrrolidin-2-yl)-N-(4-(phenyl(pyrrolidin-1-yl)methyl)cyclohexyl)acetamid
(106) 2-(1-(3,4-Dichlorphenylsulfonyl)piperidin-2-yl)-N-(4-(morpholino(phenyl)methyl)cyclohexyl)acetamid
(107) 2-(1-(3,4-Dichlorphenylsulfonyl)piperidin-2-yl)-N-(4-(phenyl(pyrrolidin-1-yl)methyl)cyclohexyl)acetamid
(108) 1-(4-((4-Methylpiperazin-1-yl)(phenyl)methyl)piperidin-1-yl)-2-(1-(3-(trifluormethyl)phenylsulfonyl)piperidin-2-yl)ethanon
(109) 1-(4-((4-Fluorphenyl)(4-methylpiperazin-1-yl)methyl)piperidin-1-yl)-2-(1-(3-(trifluormethyl)phenylsulfonyl)piperidin-2-yl)ethanon
(110) N-(3-(4-((3-Fluorphenyl)(4-methylpiperazin-1-yl)methyl)piperidin-1-yl)-3-oxo-1-phenylpropyl)naphthalin-2-sulfonamid
(111) 1-(4-(1-(4-Methylpiperazin-1-yl)-2-phenylethyl)piperidin-1-yl)-2-(1-(3-(trifluormethyl)phenylsulfonyl)piperidin-2-yl)ethanon
(112) 1-(4-(1-(4-Methylpiperazin-1-yl)-3-phenylpropyl)piperidin-1-yl)-2-(1-(3-(trifluormethyl)phenylsulfonyl)piperidin-2-yl)ethanon
(113) N-(3-(4-(1-(4-Methylpiperazin-1-yl)-3-phenylpropyl)piperidin-1-yl)-3-oxo-1-phenylpropyl)naphthalene-2-sulfonamid
(114) N-(3-(4-((4-Methylpiperazin-1-yl)(phenyl)methyl)piperidin-1-yl)-3-oxo-1-phenylpropyl)naphthalin-2-sulfonamid
(115) 1-(4-((3-Fluorphenyl)(4-methylpiperazin-1-yl)methyl)piperidin-1-yl)-2-(1-(3-(trifluormethyl)phenylsulfonyl)piperidin-2-yl)ethanon
(116) N-(3-(4-(1-(4-Methylpiperazin-1-yl)-2-phenylethyl)piperidin-1-yl)-3-oxo-1-phenylpropyl)naphthalin-2-sulfonamid
(117) N-(3-(4-((4-Fluorphenyl)(4-methylpiperazin-1-yl)methyl)piperidin-1-yl)-3-oxo-1-phenylpropyl)naphthalen-2-sulfonamid
(118) 3-(1-(4-Chlor-2,5-dimethylphenylsulfonyl)piperidin-2-yl)-1-(4-(1-(4-methylpiperazin-1-yl)-2-phenylethyl)piperidin-1-yl)propan-1-on
(119) 3-(1-(4-Chlor-2,5-dimethylphenylsulfonyl)piperidin-2-yl)-1-(4-(1-(4-methylpiperazin-1-yl)-3-phenylpropyl)piperidin-1-yl)propan-1-on
(120) 3-(1-(4-Chlor-2,5-dimethylphenylsulfonyl)piperidin-2-yl)-1-(4-((3-fluorphenyl)(4-methylpiperazin-1-yl)methyl)piperidin-1-yl)propan-1-on
(121) 3-(1-(4-Chlor-2,5-dimethylphenylsulfonyl)piperidin-2-yl)-1-(4-((4-fluorphenyl)(4-methylpiperazin-1-yl)methyl)piperidin-1-yl)propan-1-on
(122) 3-(1-(4-Chlor-2,5-dimethylphenylsulfonyl)piperidin-2-yl)-1-(4-((4-methylpiperazin-1-yl)(phenyl)methyl)piperidin-1-yl)propan-1-on
in Form des Razemats; der Enantiomere, Diastereomere, Mischungen der Enantiomere oder Diastereomere oder eines einzelnen Enantiomers oder Diastereomers; der Basen und/oder Salze physiologisch verträglicher Säuren.

17. Verfahren zur Herstellung eines substituierten Sulfonamid-Derivats gemäß Formel **Ia**, worin q für 0 steht und die Reste oder Gruppen R¹-R⁸, R^{H}, R^{J}, A, Z und Q sowie m, n und p die in Anspruch 1 angegebene Bedeutung haben, wobei Carbonsäuren **III** unter Verwendung primärer oder sekundärer Amine der allgemeinen Formel **II** 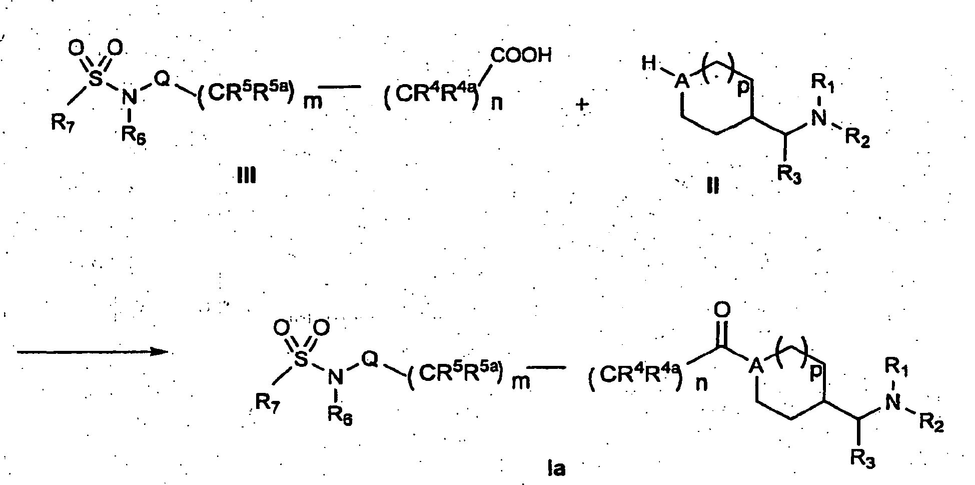 in Gegenwart wasserentziehender Mittel, beispielsweise Natrium- oder Magnesiumsulfat, Phosphorxid oder Reagenzien wie beispielsweise CDI, DCC (ggf. polyrriergebunden), TBTU, EDCl, PyBOP oder PFPTFA, gegebenenfalls in Gegenwart von HOAt oder HOBt und einer organischen Base beispielsweise DIPEA oder Pyridin in einem organischen Lösungsmittel wie THF, Dichlormethan, Diethylether, Dioxan, DMF oder Acetonitril umgesetzt werden.

18. Arzneimittel enthaltend wenigstens ein substituiertes Sulfonamid-Derivat gemäß Anspruch 1, gegebenenfalls in Form des Razemats; der Enantiomere, Diastereomere, Mischungen der Enantiomere oder Diastereomere oder eines einzelnen Enantiomers oder Diastereomers; der Basen und/oder Salze physiologisch verträglicher Säuren, sowie gegebenenfalls enthaltend geeignete Zusatz- und/oder Hilfsstofe und/oder gegebenenfalls weiterer Wirkstoffe.

19. Verwendung eines substituierten Sulfonamid-Derivats gemäß Anspruch 1, gegebenenfalls in Form des Razemats; der Enantiomere, Diastereomere, Mischungen der Enantiomere oder Diastereomere oder eines einzelnen Enantiomers oder Diastereomers; der Basen und/oder Salze physiologisch verträglicher Säuren, zur Herstellung eines Arzneimittels zur Behandlung von Schmerz, insbesondere von akutem, viszeralem, neuropathischem oder chronischem Schmerz und Entzündungsschmerz.

20. Verwendung eines substituierten Sulfonamid-Derivats gemäß Anspruch 1 zur Herstellung eines Arzneimittels zur Behandlung von Erkrankungen der Atemwege.

21. Verwendung eines substituierten Sulfonamid-Derivats gemäß Anspruch 1 zur Herstellung eines Arzneimittels zur Behandlung von von Depressionen, Harninkontinenz, Diarrhöe, Pruritus, Alkohol- und Drogenmissbrauch, Antriebslosigkeit, Migräne, Diabetes, entzündlichen Darmerkrankungen, neurologische Erkrankungen, Entzündungen der Haut, rheumatischen Erkrankungen, septischem Schock, Reperfusionssyndrom, Fettleibigkeit, als Angiognese-Inhibitor und zur Anxiolyse.

## Claims

1. Substituted sulfonamide derivatives of the general formula I 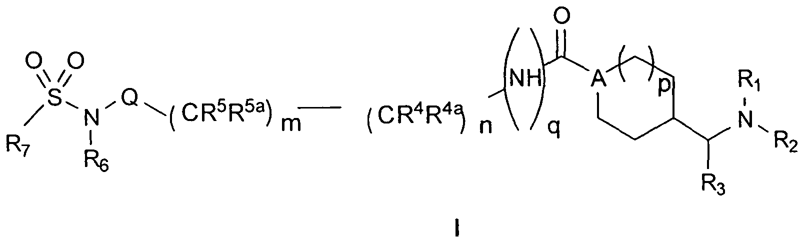 wherein
m represents 0, 1 or 2,
n represents 0, 1 or 2,
p represents 0, 1 or 2,
q represents 0 or 1,
A represents N, CH-NH-, CH-CH₂-NH-, CH-CH₂-CH₂-NH or CH-CH₂-CH₂-CH₂-NH, wherein individual hydrogen atoms can also be replaced by C₁₋₅-alkyl,
R¹ and R² independently of one another denote H; C₁₋₆-alkyl, in each case saturated or unsaturated, branched or unbranched, unsubstituted or mono- or poly-substituted; or aryl linked *via* a C₁₋₃-alkyl chain and unsubstituted or mono- or poly-substituted, wherein R¹ and R² do not simultaneously denote H,
or the radicals R¹ and R² together denote CH₂CH₂OCH₂CH₂, CH₂CH₂NR⁸CH₂CH₂ or (CH₂)₃₋₆,
wherein R⁸ denotes H; C₁₋₆-alkyl, in each case saturated or unsaturated, branched or unbranched, unsubstituted or mono- or poly-substituted; aryl, heteroaryl, in each case unsubstituted or mono- or poly-substituted, or aryl or heteroaryl linked *via* a C₁₋₃-alkyl chain and in each case unsubstituted or mono- or poly-substituted;
R³ represents C₁₋₈-alkyl, in each case saturated or unsaturated, branched or unbranched, unsubstituted or mono- or poly-substituted; aryl or heteroaryl, in each case unsubstituted or mono- or poly-substituted; or aryl or heteroaryl linked *via* a C₁₋₃-alkyl chain and in each case unsubstituted or mono- or poly-substituted;
R⁴ and R^{4a} independently of one another represent H, C₁₋₆-alkyl, in each case saturated or unsaturated, branched or unbranched, unsubstituted or mono- or poly-substituted; F; Cl; aryl, in each case unsubstituted or mono- or poly-substituted; or aryl linked *via* a C₁₋₃-alkyl chain and in each case unsubstituted or mono- or poly-substituted;
R⁵ and R^{5a} independently of one another represent H; or C₁₋₆-alkyl, in each case saturated or unsaturated, branched or unbranched, unsubstituted or mono- or poly-substituted; OH, OC₁₋₆-alkyl, F, Cl, phenoxy or benzyloxy;
Q denotes a single bond, -CH₂-, -CH₂-CH₂- or 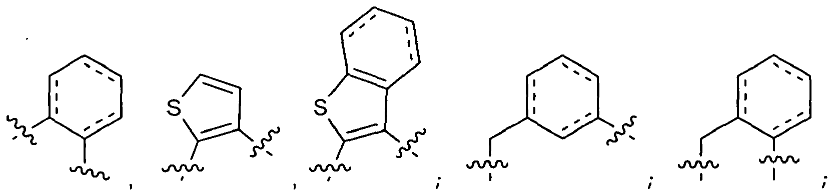 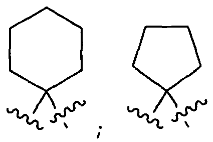
wherein -̅ -̅ -̅ -̅ represents a single bond or a double bond;
R⁶ represents H; C₁₋₆-alkyl, in each case saturated or unsaturated, branched or unbranched, unsubstituted or mono- or poly-substituted; C₃₋₈-cycloalkyl, saturated or unsaturated, unsubstituted or mono- or poly-substituted; aryl or heteroaryl, unsubstituted or mono- or poly-substituted; aryl or C₃₋₈-cycloalkyl linked *via* a C₁₋₃-alkyl chain;
or R⁶ together with Q and including the adjacent nitrogen forms a four-, five-, six- or seven-membered carbocyclic ring which can be saturated or unsaturated and can contain a further hetero atom O, S or N, to which a further five- or six- membered ring, saturated or unsaturated, can be fused; wherein in the case of the common ring closure Q represents 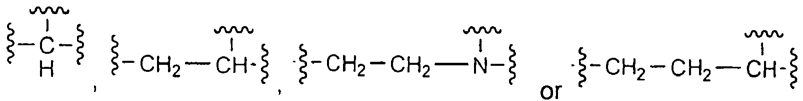 and the ring can be substituted in any position by phenyl, =O, OH, OC₁₋₆-alkyl, F, Cl, CF₃ or C₁₋₆-alkyl;
and R⁷ represents aryl or heteroaryl, in each case unsubstituted or mono- or poly-substituted; aryl or heteroaryl linked *via* a C₁₋₃-alkyl group and in each case unsubstituted or mono- or poly-substituted;
in the form of the racemate; of the enantiomers, diastereoisomers, mixtures of the enantiomers or diastereoisomers or of an individual enantiomer or diastereoisomer; of the bases and/or salts of physiologically acceptable acids.

2. Substituted sulfonamide derivatives according to claim 1,
wherein
"alkyl substituted" and "cycloalkyl substituted" mean the substitution of one or more hydrogen radicals by F, Cl, Br, I, -CN, NH₂, NH-C₁₋₆-alkyl, NH-C₁₋₆-alkyl-OH, C₁₋₆-alkyl, N(C₁₋₆-alkyl)₂, N(C₁₋₆-alkyl-OH)₂, NO₂, SH, S-C₁₋₆-alkyl, S-benzyl, O-C₁₋₆-alkyl, OH, O-C₁₋₆-alkyl-OH, =O, O-benzyl, C(=O)C₁₋₆-alkyl, CO₂H, CO₂-C₁₋₆-alkyl or benzyl,
"aryl substituted" and "heteroaryl substituted" mean the substitution of one or more hydrogen atoms of the ring system one or more times, for example two, three or four times, by F, Cl, Br, I, CN, NH₂, NH-C₁₋₆-alkyl, NH-C₁₋₆-alkyl-OH, N(C₁₋₆-alkyl)₂, N(C₁₋₆-alkyl-OH)₂, NO₂, SH, S-C₁₋₆-alkyl, OH, O-C₁₋₆-alkyl, O-C₁₋₆- alkyl-OH, C(=O)C₁₋₆-alkyl, CO₂H, CH₂SO₂-phenyl, CO₂-C₁₋₆-alkyl, OCF₃, CF₃, 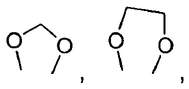 C₁₋₆-alkyl, phenyl, pyridyl, thienyl or furyl,
in the form of the racemate; of the enantiomers, diastereoisomers, mixtures of the enantiomers or diastereoisomers or of an individual enantiomer or diastereoisomer; of the bases and/or salts of physiologically acceptable acids.

3. Substituted sulfonamide derivatives according to claim 1, wherein A represents CH-NH-, CH-CH₂-NH-, CH-CH₂-CH₂-NH or CH-CH₂-CH₂-CH₂-NH, wherein individual hydrogen atoms can also be replaced by C₁₋₅-alkyl.

4. Substituted sulfonamide derivatives according to claim 1, wherein R¹ and R² independently of one another denote H; CH₃; C₂H₅; or phenyl linked *via* a C₁₋₃-alkyl chain, wherein R¹ and R² do not simultaneously denote H,
or the radicals R¹ and R² together denote CH₂CH₂OCH₂CH₂, CH₂CH₂NR⁸CH₂CH₂ or (CH₂)₃₋₅.

5. Substituted sulfonamide derivatives according to claim 1, wherein R³ represents aryl or heteroaryl, in each case unsubstituted or mono- or poly-substituted; or aryl linked *via* a C₁₋₃-alkyl chain and in each case unsubstituted or mono- or poly-substituted.

6. Substituted sulfonamide derivatives according to claim 5, wherein R³ represents 2-thienyl, 4-fluorophenyl, 3-fluorophenyl, 4-chlorophenyl, phenethyl, phenyl or benzyl.

7. Substituted sulfonamide derivatives according to claim 1, wherein R⁴ and R^{4a} represent H.

8. Substituted sulfonamide derivatives according to claim 1, wherein R⁵ and R^{5a} represent H.

9. Substituted sulfonamide derivatives according to claim 1, wherein R⁶ represents methyl, ethyl or benzyl and Q represents a single bond, wherein R⁶ in particular denotes methyl.

10. Substituted sulfonamide derivatives according to claim 1, wherein the group 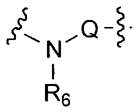 of the general formula I represents

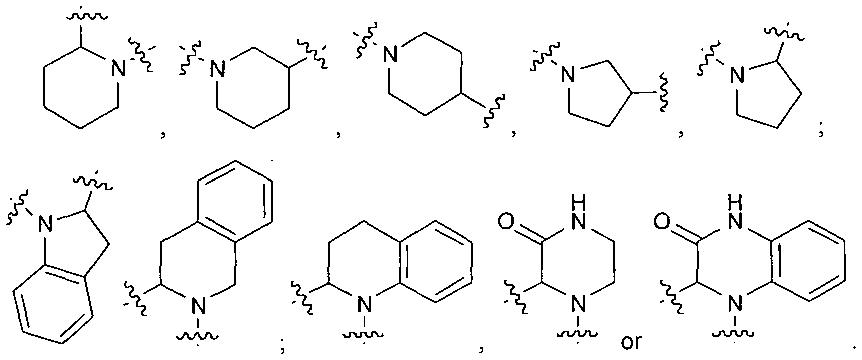

11. Substituted sulfonamide derivatives according to claim 1, wherein Q denotes

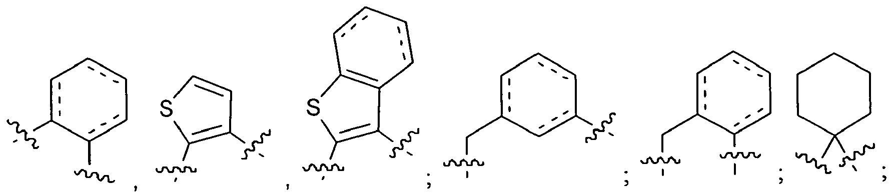

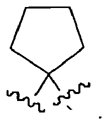

12. Substituted sulfonamide derivatives according to claim 1, wherein the group 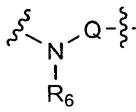 of the general formula I represents 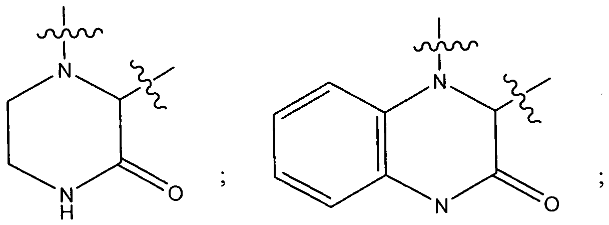 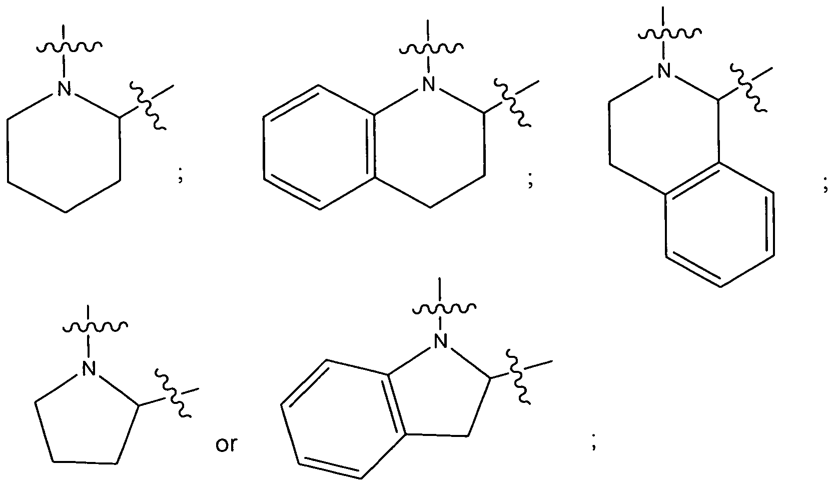
m represents 0, 1 or 2;
n represents 0;
q represents 0; and
R⁵ and R^{5a} represent H;
or
Q represents a single bond;
m represents 0, 1 or 2;
n represents 1 or 2;
q represents 0 or 1;
R⁴ and R^{4a} each independently of the other represents H or aryl;
R⁵ and R^{5a} represent H; and
R⁶ represents H or C₁₋₆-alkyl;
or
Q represents 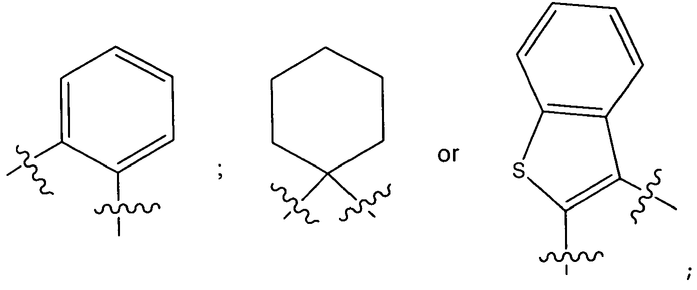
m represents 0 or 1, preferably 0;
n and q represent 0;
R⁵ and R^{5a} represent H; and
R⁶ represents H or C₁₋₆-alkyl.

13. Substituted sulfonamide derivatives according to claim 1, wherein in the general formula I
A represents N, NH-CH, NH-CH₂-CH or NH-CH₂-CH₂-CH, wherein individual hydrogen atoms can also be replaced by C₁₋₅-alkyl;
R¹ and R² independently of one another represent C₁₋₆-alkyl, in particular methyl, ethyl, n-propyl, isopropyl, n-butyl, sec-butyl, isobutyl and tert-butyl, or together with the nitrogen atom to which they are bonded form a group selected from 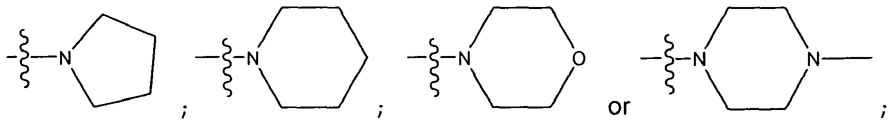 and
R³ represents aryl, in particular phenyl or furanyl, which can be linked *via* a C₁₋₃-alkyl group, wherein the aryl is in each case unsubstituted or mono- or poly-substituted by identical or different substituents selected independently from the group consisting of methyl, ethyl, methoxy, ethoxy, F, Cl, Br, F, CN, CF₃, OCF₃ and OH.

14. Substituted sulfonamide derivatives according to claim 1,
wherein the group 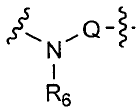 in the general formula I represents 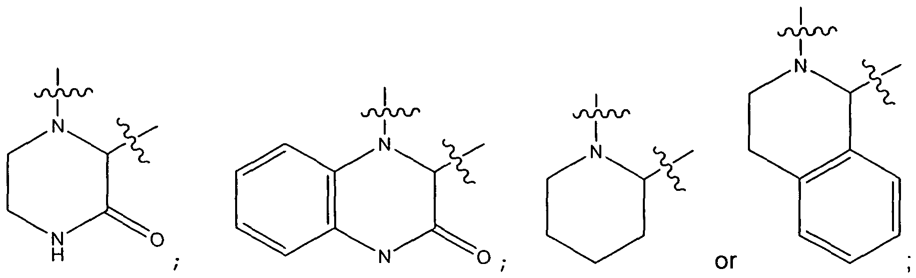
m represents 1 or 2, in particular 1;
n and q represent 0;
R⁵ and R^{5a} represent H;
A represents N, NH-CH or NH-CH₂-CH, in particular N or NH-CH;
R¹ and R² independently of one another represent C₁₋₆-alkyl, in particular methyl, ethyl, n-propyl, isopropyl, n-butyl, sec-butyl, isobutyl and tert-butyl, or together with the nitrogen atom to which they are bonded form a group selected from 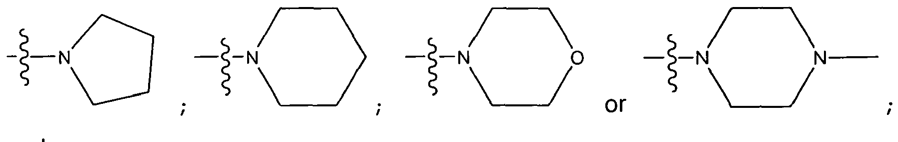 and
R₃ represents phenyl which can be linked *via* a C₁₋₃-alkyl group, wherein the phenyl is in each case unsubstituted or mono- or poly-substituted by identical or different substituents selected independently from the group consisting of methyl, ethyl, methoxy, ethoxy, F, Cl, Br, F, CN, CF₃, OCF₃ and OH, in particular methyl, methoxy, F, Cl, Br, CN, CF₃ and OH.

15. Substituted sulfonamide derivatives according to claim 1, wherein R⁷ represents 2,4-dichlorophenyl, 2,4,6-trichlorophenyl, 4-methoxyphenyl, 3-trifluoromethylphenyl, 2,6-dimethyl-4-methoxyphenyl or 3,4-dichlorophenyl.

16. Substituted sulfonamide derivatives according to claim 1 from the group
(1) N-(3-{3-[4-(dimethylaminophenylmethyl)cyclohexyl]ureido}propyl)-4-methoxy-2,3,6,N-tetramethylbenzenesulfonamide
(2) (*N*-(3-(3-((4-((dimethylamino)(phenyl)methyl)cyclohexyl)methyl)ureido)-propyl)-4-methoxy-*N*,2,3,6-tetramethylbenzenesulfonamide
(3) 4-methoxy-N,2,3,6-tetramethyl-N-(3-(3-(4-(phenyl(pyrrolidin-1-yl)methyl)-cyclohexyl)ureido)propyl)benzenesulfonamide
(4) 4-(dimethylamino-phenyl-methyl)-piperidine-1-carboxylic acid {3-[(4-methoxy-2,3,6-trimethyl-benzenesulfonyl)-methyl-amino]-propyl}-amide
(5) 5-[methyl-(2,4,6-trichloro-benzenesulfonyl)-amino]-pentanecarboxylic acid [4-(dimethylamino-phenyl-methyl)-cyclohexyl]-amide
(6) 5-[methyl-(2,4,6-trichloro-benzenesulfonyl)-amino]-pentanecarboxylic acid [4-(phenyl-piperidin-1-yl-methyl)-cyclohexyl]-amide
(7) 5-[methyl-(2,4,6-trichloro-benzenesulfonyl)-amino]-pentanecarboxylic acid [4-(dimethylamino-phenyl-methyl)-cyclohexylmethyl]-amide
(8) N-[4-(dimethylamino-phenyl-methyl)-cyclohexylmethyl]-2-[1-(3-trifluoromethyl-benzenesulfonyl)-piperidin-2-yl]-acetamide
(9) N-[4-(dimethylamino-phenyl-methyl)-cyclohexyl]-2-[2-(4-methoxy-benzenesulfonyl)-1,2,3,4-tetrahydro-isoquinolin-1-yl]-acetamide
(10) 2-[2-(3,4-dichloro-benzenesulfonyl)-1,2,3,4-tetrahydro-isoquinolin-1-yl]-N-[4-(dimethylamino-phenyl-methyl)-cyclohexyl]-acetamide
(11) 2-[(3,4-dichloro-benzenesulfonyl)-methyl-amino]-N-[4-(dimethylaminophenyl-methyl)-cyclohexyl]-benzamide
(12) 2-[(3,4-dichloro-benzenesulfonyl)-methyl-amino]-4,5,6,7-tetrahydrobenzo[b]thiophene-3-carboxylic acid [4-(dimethylamino-phenyl-methyl)-cyclohexyl]-amide
(13) N-{4-[dimethylamino-(3-fluoro-phenyl)-methyl]-cyclohexyl}-2-[2-(4-methoxy-benzenesulfonyl)-1,2,3,4-tetrahydro-isoquinolin-1-yl]-acetamide
(14) N-{4-[dimethylamino-(4-fluoro-phenyl)-methyl]-cyclohexyl}-2-[2-(4-methoxy-benzenesulfonyl)-1,2,3,4-tetrahydro-isoquinolin-1-yl]-acetamide
(15) N-[4-(dimethylamino-thiophen-2-yl-methyl)-cyclohexyl]-2-[2-(4-methoxybenzenesulfonyl)-1,2,3,4-tetrahydro-isoquinolin-1-yl]-acetamide
(16) N-{4-[dimethylamino-(3-fluoro-phenyl)-methyl]-cyclohexylmethyl}-2-[2-(4-methoxy-benzenesulfonyl)-1,2,3,4-tetrahydro-isoquinolin-1-yl]-acetamide
(17) N-[4-(dimethylamino-phenyl-methyl)-cyclohexylmethyl]-2-[2-(4-methoxybenzenesulfonyl)-1,2,3,4-tetrahydro-isoquinolin-1-yl]-acetamide
(18) N-[4-(1-dimethylamino-3-phenyl-propyl)-cyclohexylmethyl]-2-[2-(4-methoxy-benzenesulfonyl)-1,2,3,4-tetrahydro-isoquinolin-1-yl]-acetamide
(19) N-{4-[dimethylamino-(4-fluoro-phenyl)-methyl]-cyclohexylmethyl}-2-[2-(4-methoxy-benzenesulfonyl)-1,2,3,4-tetrahydro-isoquinolin-1-yl]-acetamide
(20) N-{4-[(4-chloro-phenyl)-dimethylamino-methyl]-cyclohexylmethyl}-2-[2-(4-methoxy-benzenesulfonyl)-1,2,3,4-tetrahydro-isoquinolin-1-yl]-acetamide
(21) N-[4-(dimethylamino-thiophen-2-yl-methyl)-cyclohexylmethyl]-2-[2-(4-methoxy-benzenesulfonyl)-1,2,3,4-tetrahydro-isoquinolin-1-yl]-acetamide
(22) N-(2-{4-[(4-chloro-phenyl)-dimethylamino-methyl]-cyclohexyl}-ethyl)-2-[2-(4-methoxy-benzenesulfonyl)-1,2,3,4-tetrahydro-isoquinolin-1-yl]-acetamide
(23) N-(2-{4-[dimethylamino-(3-fluoro-phenyl)-methyl]-cyclohexyl}-ethyl)-2-[2-(4-methoxy-benzenesulfonyl)-1,2,3,4-tetrahydro-isoquinolin-1-yl]-acetamide
(24) N-(2-{4-[dimethylamino-(4-fluoro-phenyl)-methyl]-cyclohexyl}-ethyl)-2-[2-(4-methoxy-benzenesulfonyl)-1,2,3,4-tetrahydro-isoquinolin-1-yl]-acetamide
(25) N-{2-[4-(dimethylamino-thiophen-2-yl-methyl)-cyclohexyl]-ethyl}-2-[2-(4-methoxy-benzenesulfonyl)-1,2,3,4-tetrahydro-isoquinolin-1-yl]-acetamide
(26) 2-(2-(3,4-dichlorophenylsulfonyl)-1,2,3,4-tetrahydroisoquinolin-1-yl)-N-(4-((dimethylamino)(3-fluorophenyl)methyl)cyclohexyl)acetamide
(27) 2-(2-(3,4-dichlorophenylsulfonyl)-1,2,3,4-tetrahydroisoquinolin-1-yl)-N-(4-((dimethylamino)(4-fluorophenyl)methyl)cyclohexyl)acetamide
(28) 2-[2-(3,4-dichloro-benzenesulfonyl)-1,2,3,4-tetrahydro-isoquinolin-1-yl]-N-{4-[dimethylamino-(3-fluoro-phenyl)-methyl]-cyclohexylmethyl}-acetamide
(29) 2-[2-(3,4-dichloro-benzenesulfonyl)-1,2,3,4-tetrahydro-isoquinolin-1-yl]-N-[4-(dimethylamino-phenyl-methyl)-cyclohexylmethyl]-acetamide
(30) 2-[2-(3,4-dichloro-benzenesulfonyl)-1,2,3,4-tetrahydro-isoquinolin-1-yl]-N-[4-(1-dimethylamino-3-phenyl-propyl)-cyclohexylmethyl]-acetamide
(31) 2-[2-(3,4-dichloro-benzenesulfonyl)-1,2,3,4-tetrahydro-isoquinolin-1-yl]-N-{4-[dimethylamino-(4-fluoro-phenyl)-methyl]-cyclohexylmethyl}-acetamide
(32) N-{4-[(4-chloro-phenyl)-dimethylamino-methyl]-cyclohexylmethyl}-2-[2-(3,4-dichloro-benzenesulfonyl)-1,2,3,4-tetrahydro-isoquinolin-1-yl]-acetamide
(33) N-(2-{4-[(4-chloro-phenyl)-dimethylamino-methyl]-cyclohexyl}-ethyl)-2-[2-(3,4-dichloro-benzenesulfonyl)-1,2,3,4-tetrahydro-isoquinolin-1-yl]-acetamide
(34) 2-[2-(3,4-dichloro-benzenesulfonyl)-1,2,3,4-tetrahydro-isoquinolin-1-yl]-N-(2-{4-[dimethylamino-(3-fluoro-phenyl)-methyl]-cyclohexyl}-ethyl)-acetamide
(35) 2-[2-(3,4-dichloro-benzenesulfonyl)-1,2,3,4-tetrahydro-isoquinolin-1-yl]-N-(2-{4-[dimethylamino-(4-fluoro-phenyl)-methyl]-cyclohexyl}-ethyl)-acetamide
(36) 2-[(3,4-dichloro-benzenesulfonyl)-methyl-amino]-N-{4-[dimethylamino-(3-fluoro-phenyl)-methyl]-cyclohexyl}-benzamide
(37) 2-[(3,4-dichloro-benzenesulfonyl)-methyl-amino]-N-{4-[dimethylamino-(4-fluoro-phenyl)-methyl]-cyclohexyl}-benzamide
(38) 2-[(3,4-dichloro-benzenesulfonyl)-methyl-amino]-N-[4-(dimethylaminophenyl-methyl)-cyclohexylmethyl]-benzamide
(39) 2-[(3,4-dichloro-benzenesulfonyl)-methyl-amino]-N-[4-(1-dimethylamino-3-phenyl-propyl)-cyclohexylmethyl]-benzamide
(40) 2-[(3,4-dichloro-benzenesulfonyl)-methyl-amino]-N-{4-[dimethylamino-(4-fluoro-phenyl)-methyl]-cyclohexylmethyl}-benzamide
(41) N-{4-[(4-chloro-phenyl)-dimethylamino-methyl]-cyclohexylmethyl}-2-[(3,4-dichloro-benzenesulfonyl)-methyl-amino]-benzamide
(42) 2-[(3,4-dichloro-benzenesulfonyl)-methyl-amino]-N-[4-(dimethylaminothiophen-2-yl-methyl)-cyclohexylmethyl]-benzamide
(43) N-(2-{4-[(4-chloro-phenyl)-dimethylamino-methyl]-cyclohexyl}-ethyl)-2-[(3,4-dichloro-benzenesulfonyl)-methyl-amino]-benzamide
(44) 2-[(3,4-dichloro-benzenesulfonyl)-methyl-amino]-N-(2-{4-[dimethylamino-(3-fluoro-phenyl)-methyl]-cyclohexyl}-ethyl)-benzamide
(45) 2-[(3,4-dichloro-benzenesulfonyl)-methyl-amino]-N-(2-{4-[dimethylamino-(4-fluoro-phenyl)-methyl]-cyclohexyl}-ethyl)-benzamide
(46) 2-[(3,4-dichloro-benzenesulfonyl)-methyl-amino]-4,5,6,7-tetrahydrobenzo[b]thiophene-3-carboxylic acid [4-(dimethylamino-phenyl-methyl)-cyclohexylmethyl]-amide
(47) 2-[(3,4-dichloro-benzenesulfonyl)-methyl-amino]-4,5,6,7-tetrahydrobenzo[b]thiophene-3-carboxylic acid {4-[dimethylamino-(4-fluoro-phenyl)-methyl]-cyclohexylmethyl}-amide
(48) 2-[(3,4-dichloro-benzenesulfonyl)-methyl-amino]-4,5,6,7-tetrahydrobenzo[b]thiophene-3-carboxylic acid {4-[(4-chloro-phenyl)-dimethylaminomethyl]-cyclohexylmethyl}-amide
(49) 2-[(3,4-dichloro-benzenesulfonyl)-methyl-amino]-4,5,6,7-tetrahydrobenzo[b]thiophene-3-carboxylic acid [4-(dimethylamino-thiophen-2-yl-methyl)-cyclohexylmethyl]-amide
(50) N-(2-(4-((4-chlorophenyl)(dimethylamino)methyl)cyclohexyl)ethyl)-2-(3,4-dichloro-N-methylphenylsulfonamido)-4,5,6,7-tetrahydrobenzo[b]-thiophene-3-carboxamide
(51) 2-(3,4-dichloro-N-methylphenylsulfonamido)-N-(2-(4-((dimethylamino)(3-fluorophenyl)methyl)cyclohexyl)ethyl)-4,5,6,7-tetrahydrobenzo[b]-thiophene-3-carboxamide
(52) 2-(3,4-dichloro-N-methylphenylsulfonamido)-N-(2-(4-((dimethylamino)(4-fluorophenyl)methyl)cyclohexyl)ethyl)-4,5,6,7-tetrahydrobenzo[b]-thiophene-3-carboxamide
(53) 2-(3,4-dichloro-N-methylphenylsulfonamido)-N-(2-(4-((dimethylamino)-(thiophen-2-yl)methyl)cyclohexyl)ethyl)-4,5,6,7-tetrahydrobenzo[b]-thiophene-3-carboxamide
(54) 2-[1-(3,4-dichloro-benzenesulfonyl)-3-oxo-1,2,3,4-tetrahydro-quinoxalin-2-yl]-N-[4-(morpholin-4-yl-phenyl-methyl)-cyclohexyl]-acetamide
(55) 2-[1-(2,4-dichloro-benzenesulfonyl)-3-oxo-piperazin-2-yl]-N-[4-(morpholin-4-yl-phenyl-methyl)-cyclohexylmethyl]-acetamide
(56) 2-[1-(2,4-dichloro-benzenesulfonyl)-3-oxo-piperazin-2-yl]-N-[4-(1-morpholin-4-yl-3-phenyl-propyl)-cyclohexylmethyl]-acetamide
(57) 2-[1-(2,4-dichloro-benzenesulfonyl)-3-oxo-piperazin-2-yl]-N-[4-(3-phenyl-1-pyrrolidin-1-yl-propyl)-cyclohexylmethyl]-acetamide
(58) 2-[1-(2,4-dichloro-benzenesulfonyl)-3-oxo-piperazin-2-yl]-N-[4-(phenylpyrrolidin-1-yl-methyl)-cyclohexylmethyl]-acetamide
(59) 2-[1-(2,4-dichloro-benzenesulfonyl)-3-oxo-piperazin-2-yl]-N-[4-(3-phenyl-1-piperidin-1-yl-propyl)-cyclohexyl]-acetamide
(60) 2-[1-(2,4-dichloro-benzenesulfonyl)-3-oxo-piperazin-2-yl]-N-[4-(1-morpholin-4-yl-3-phenyl-propyl)-cyclohexyl]-acetamide
(61) 2-[1-(2,4-dichloro-benzenesulfonyl)-3-oxo-piperazin-2-yl]-N-{4-[(4-methylpiperazin-1-yl)-phenyl-methyl]-cyclohexyl}-acetamide
(62) 4-(2,4-dichloro-benzenesulfonyl)-3-{2-[4-(morpholin-4-yl-phenyl-methyl)-piperidin-1-yl]-2-oxo-ethyl}-piperazin-2-one
(63) 4-(2,4-dichlorophenylsulfonyl)-3-(2-(2-(1-morpholino-2-phenylethyl)-piperidin-1-yl)-2-oxoethyl)piperazin-2-one
(64) 2-[1-(3,4-dichloro-benzenesulfonyl)-3-oxo-1,2,3,4-tetrahydro-quinoxalin-2-yl]-N-[4-(morpholin-4-yl-phenyl-methyl)-cyclohexylmethyl]-acetamide
(65) 2-[1-(3,4-dichloro-benzenesulfonyl)-3-oxo-1,2,3,4-tetrahydro-quinoxalin-2-yl]-N-[4-(1-morpholin-4-yl-3-phenyl-propyl)-cyclohexylmethyl]-acetamide
(66) 2-[1-(3,4-dichloro-benzenesulfonyl)-3-oxo-1,2,3,4-tetrahydro-quinoxalin-2-yl]-N-[4-(3-phenyl-1-pyrrolidin-1-yl-propyl)-cyclohexylmethyl]-acetamide
(67) 2-[1-(3,4-dichloro-benzenesulfonyl)-3-oxo-1,2,3,4-tetrahydro-quinoxalin-2-yl]-N-[4-(phenyl-pyrrolidin-1-yl-methyl)-cyclohexylmethyl]-acetamide
(68) 2-[1-(3,4-dichloro-benzenesulfonyl)-3-oxo-1,2,3,4-tetrahydro-quinoxalin-2-yl]-N-[4-(3-phenyl-1-piperid in-1-yl-propyl)-cyclohexyl]-acetamide
(69) 2-[1-(3,4-dichloro-benzenesulfonyl)-3-oxo-1,2,3,4-tetrahydro-quinoxalin-2-yl]-N-[4-(1-morpholin-4-yl-3-phenyl-propyl)-cyclohexyl]-acetamide
(70) 2-[1-(3,4-dichloro-benzenesulfonyl)-3-oxo-1,2,3,4-tetrahydro-quinoxalin-2-yl]-N-{4-[(4-methyl-piperazin-1-yl)-phenyl-methyl]-cyclohexyl}-acetamide
(71) 4-(3,4-dichloro-benzenesulfonyl)-3-{2-[4-(morpholin-4-yl-phenyl-methyl)-piperidin-1-yl]-2-oxo-ethyl}-3,4-dihydro-1H-quinoxalin-2-one
(72) 4-(3,4-dichlorophenylsulfonyl)-3-(2-(4-(1-morpholino-2-phenylethyl)-piperidin-1-yl)-2-oxoethyl)-3,4-dihydroquinoxalin-2(1H)-one
(73) 2-[1-(3,4-dichloro-benzenesulfonyl)-pyrrolidin-2-yl]-N-[4-(morpholin-4-yl-phenyl-methyl)-cyclohexylmethyl]-acetamide
(74) 2-[1-(3,4-dichloro-benzenesulfonyl)-pyrrolidin-2-yl]-N-[4-(1-morpholin-4-yl-3-phenyl-propyl)-cyclohexylmethyl]-acetamide
(75) 2-[1-(3,4-dichloro-benzenesulfonyl)-pyrrolidin-2-yl]-N-[4-(3-phenyl-1-pyrrolidin-1-yl-propyl)-cyclohexylmethyl]-acetamide
(76) 2-[1-(3,4-dichloro-benzenesulfonyl)-pyrrolidin-2-yl]-N-[4-(phenyl-pyrrolidin-1-yl-methyl)-cyclohexylmethyl]-acetamide
(77) 2-(1-(3,4-dichlorophenylsulfonyl)pyrrolidin-2-yl)-N-(4-(3-phenyl-1-(piperidin-1-yl)propyl)cyclohexyl)acetamide
(78) 2-(1-(3,4-dichlorophenylsulfonyl)pyrrolidin-2-yl)-N-(4-(1-morpholino-3-phenylpropyl)cyclohexyl)acetamide
(79) 2-[1-(3,4-dichloro-benzenesulfonyl)-pyrrolidin-2-yl]-N-{4-[(4-methylpiperazin-1-yl)-phenyl-methyl]-cyclohexyl}-acetamide
(80) 2-[1-(3,4-dichloro-benzenesulfonyl)-pyrrolidin-2-yl]-1-[4-(morpholin-4-yl-phenyl-methyl)-piperidin-1-yl]-ethanone
(81) 2-(1-(3,4-dichlorophenylsulfonyl)pyrrolidin-2-yl)-1-(4-(1-morphotino-2-phenylethyl)piperidin-1-yl)ethanone
(82) 2-[1-(3,4-dichloro-benzenesulfonyl)-piperidin-2-yl]-N-[4-(morpholin-4-yl-phenyl-methyl)-cyclohexylmethyl]-acetamide
(83) 2-[1-(3,4-dichloro-benzenesulfonyl)-piperidin-2-yl]-N-[4-(1-morpholin-4-yl-3-phenyl-propyl)-cyclohexylmethyl]-acetamide
(84) 2-[1-(3,4-dichloro-benzenesulfonyl)-piperidin-2-yl]-N-[4-(3-phenyl-1-pyrrolidin-1-yl-propyl)-cyclohexylmethyl]-acetamide
(85) 2-[1-(3,4-dichloro-benzenesulfonyl)-piperidin-2-yl]-N-[4-(phenyl-pyrrolidin-1-yl-methyl)-cyclohexylmethyl]-acetamide
(86) 2-[1-(3,4-dichloro-benzenesulfonyl)-piperidin-2-yl]-N-[4-(3-phenyl-1-piperidin-1-yl-propyl)-cyclohexyl]-acetamide
(87) 2-[1-(3,4-dichloro-benzenesulfonyl)-piperidin-2-yl]-N-[4-(1-morpholin-4-yl-3-phenyl-propyl)-cyclohexyl]-acetamide
(88) 2-[1-(3,4-dichloro-benzenesulfonyl)-piperidin-2-yl]-N-{4-[(4-methylpiperazin-1-yl)-phenyl-methyl]-cyclohexyl}-acetamide
(89) 2-[1-(3,4-dichloro-benzenesulfonyl)-piperidin-2-yl]-1-[4-(morpholin-4-yl-phenyl-methyl)-piperidin-1-yl]-ethanone
(90) 2-(1-(3,4-dichlorophenylsulfonyl)piperidin-2-yl)-1-(4-(1-morpholino-2-phenylethyl)piperidin-1-yl)ethanone
(91) 1-(3,4-dichlorophenylsulfonyl)-N-(4-((dimethylamino)(phenyl)methyl)-cyclohexyl)indoline-2-carboxamide
(92) 1-(3,4-dichlorophenylsulfonyl)-N-(4-((dimethylamino)(3-fluorophenyl)-methyl)cyclohexyl)indoline-2-carboxamide
(93) 1-(3,4-dichlorophenylsulfonyl)-N-(4-(1-(dimethylamino)-3-phenylpropyl) cyclohexyl)indoline-2-carboxamide
(94) 1-(3,4-dichlorophenylsulfonyl)-N-(4-((dimethylamino)(4-fluorophenyl)-methyl)cyclohexyl)indoline-2-carboxamide
(95) 1-(3,4-dichlorophenylsulfonyl)-N-((4-((dimethylamino)(phenyl)methyl)-cyclohexyl)methyl)indoline-2-carboxamide
(96) 1-(3,4-dichlorophenylsulfonyl)-N-((4-(1-(dimethylamino)-3-phenylpropyl)-cyclohexyl)methyl)indoline-2-carboxamide
(97) 1-(3,4-dichlorophenylsulfonyl)-N-(2-(4-(1-(dimethylamino)-3-phenylpropyl)-cyclohexyl)ethyl)indoline-2-carboxamide
(98) 2-(2-(3,4-dichloro-N-methylphenylsulfonamido)phenyl)-N-(2-(4-((dimethylamino)(phenyl)methyl)cyclohexyl)ethyl)acetamide
(99) 2-(2-(3,4-dichloro-N-methylphenylsulfonamido)phenyl)-N-(2-(4-(1-(dimethylamino)-3-phenylpropyl)cyclohexyl)ethyl)acetamide
(100) N-(2-(4-((dimethylamino)(phenyl)methyl)cyclohexyl)ethyl)-1-(4-methoxy-N-methylphenylsulfonamido)cyclohexanecarboxamide
(101) 2-(1-(2,4-dichlorophenylsulfonyl)-3-oxopiperazin-2-yl)-N-(4-(morpholino-(phenyl)methyl)cyclohexyl)acetamide
(102) 2-(1-(2,4-dichlorophenylsulfonyl)-3-oxopiperazin-2-yl)-N-(4-(phenyl-(pyrrolidin-1-yl)methyl)cyclohexyl)acetamide
(103) 2-(1-(3,4-dichlorophenylsulfonyl)-3-oxo-1,2,3,4-tetrahydroquinoxalin-2-yl)-N-(4-(phenyl(pyrrolidin-1-yl)methyl)cyclohexyl)acetamide
(104) 2-(1-(3,4-dichlorophenylsulfonyl)pyrrolidin-2-yl)-N-(4-(morpholino(phenyl)-methyl)cyclohexyl)acetamide
(105) 2-(1-(3,4-dichlorophenylsulfonyl)pyrrolidin-2-yl)-N-(4-(phenyl(pyrrolidin-1-yl)methyl)cyclohexyl)acetamide
(106) 2-(1-(3,4-dichlorophenylsulfonyl)piperidin-2-yl)-N-(4-(morpholino(phenyl)-methyl)cyclohexyl)acetamide
(107) 2-(1-(3,4-dichlorophenylsulfonyl)piperidin-2-yl)-N-(4-(phenyl(pyrrolidin-1-yl)methyl)cyclohexyl)acetamide
(108) 1-(4-((4-methylpiperazin-1-yl)(phenyl)methyl)piperidin-1-yl)-2-(1-(3-(trifluoromethyl)phenylsulfonyl)piperidin-2-yl)ethanone
(109) 1-(4-((4-fluorophenyl)(4-methylpiperazin-1-yl)methyl)piperidin-1-yl)-2-(1-(3-(trifluoromethyl)phenylsulfonyl)piperidin-2-yl)ethanone
(110) N-(3-(4-((3-fluorophenyl)(4-methylpiperazin-1-yl)methyl)piperidin-1-yl)-3-oxo-1-phenylpropyl)naphthalene-2-sulfonamide
(111) 1-(4-(1-(4-methylpiperazin-1-yl)-2-phenylethyl)piperidin-1-yl)-2-(1-(3-(trifluoromethyl)phenylsulfonyl)piperidin-2-yl)ethanone
(112) 1-(4-(1-(4-methylpiperazin-1-yl)-3-phenylpropyl)piperidin-1-yl)-2-(1-(3-(trifluoromethyl)phenylsulfonyl)piperidin-2-yl)ethanone
(113) N-(3-(4-(1-(4-methylpiperazin-1-yl)-3-phenylpropyl)piperidin-1-yl)-3-oxo-1-phenylpropyl)naphthalene-2-sulfonamide
(114) N-(3-(4-((4-methylpiperazin-1-yl)(phenyl)methyl)piperidin-1-yl)-3-oxo-1-phenylpropyl)naphthalene-2-sulfonamide
(115) 1-(4-((3-fluorophenyl)(4-methylpiperazin-1-yl)methyl)piperidin-1-yl)-2-(1-(3-(trifluoromethyl)phenylsulfonyl)piperidin-2-yl)ethanone
(116) N-(3-(4-(1-(4-methylpiperazin-1-yl)-2-phenylethyl)piperidin-1-yl)-3-oxo-1-phenylpropyl)naphthalene-2-sulfonamide
(117) N-(3-(4-((4-fluorophenyl)(4-methylpiperazin-1-yl)methyl)piperidin-1-yl)-3-oxo-1-phenylpropyl)naphthalene-2-sulfonamide
(118) 3-(1-(4-chloro-2,5-dimethylphenylsulfonyl)piperidin-2-yl)-1-(4-(1-(4-methylpiperazin-1-yl)-2-phenylethyl)piperidin-1-yl)propan-1-one
(119) 3-(1-(4-chloro-2,5-dimethylphenylsulfonyl)piperidin-2-yl)-1-(4-(1-(4-methylpiperazin-1-yl)-3-phenylpropyl)piperidin-1-yl)propan-1-one
(120) 3-(1-(4-chloro-2,5-dimethylphenylsulfonyl)piperidin-2-yl)-1-(4-((3-fluorophenyl)(4-methylpiperazin-1-yl)methyl)piperidin-1-yl)propan-1-one
(121) 3-(1-(4-chloro-2,5-dimethylphenylsulfonyl)piperidin-2-yl)-1-(4-((4-fluorophenyl)(4-methylpiperazin-1-yl)methyl)piperid in-1-yl)propan-1-one
(122) 3-(1-(4-chloro-2,5-dimethylphenylsulfonyl)piperidin-2-yl)-1-(4-((4-methylpiperazin-1-yl)(phenyl)methyl)piperidin-1-yl)propan-1-one
in the form of the racemate; of the enantiomers, diastereoisomers, mixtures of the enantiomers or diastereoisomers or of an individual enantiomer or diastereoisomer; of the bases and/or salts of physiologically acceptable acids.

17. Process for the preparation of a substituted sulfonamide derivative according to formula **Ia** wherein q represents 0 and the radicals or groups R¹-R⁸, R^{H}, R^{J}, A, Z and Q and also m, n and p have the meaning given in claim 1, wherein carboxylic acids **III** are reacted using primary or secondary amines of the general formula **II** 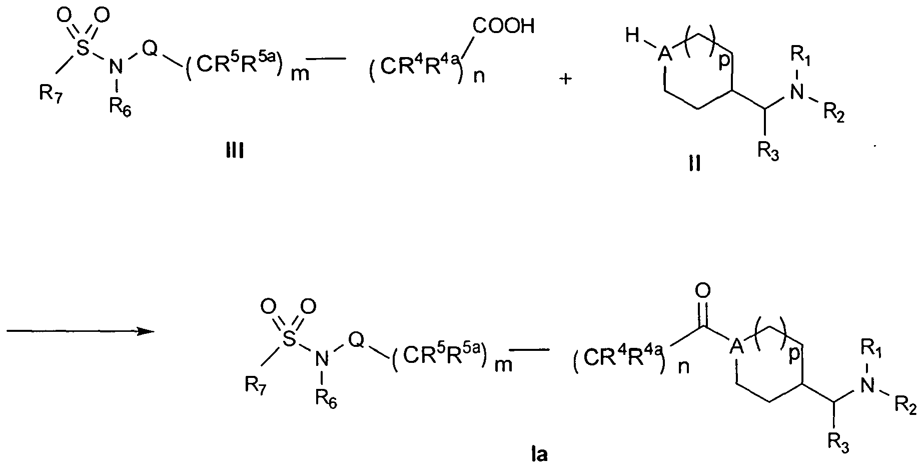 in the presence of water-removing agents, for example sodium or magnesium sulfate, phosphorus oxide or reagents such as, for example, CDI, DCC (optionally polymer-bonded), TBTU, EDCI, PyBOP or PFPTFA, optionally in the presence of HOAt or HOBt and of an organic base, for example DIPEA or pyridine, in an organic solvent such as THF, dichloromethane, diethyl ether, dioxane, DMF or acetonitrile.

18. Medicament comprising at least one substituted sulfonamide derivative according to claim 1, optionally in the form of the racemate; of the enantiomers, diastereoisomers, mixtures of the enantiomers or diastereoisomers or of an individual enantiomer or diastereoisomer; of the bases and/or salts of physiologically acceptable acids, and also optionally comprising suitable additives and/or auxiliary substances and/or optionally further active ingredients.

19. Use of a substituted sulfonamide derivative according to claim 1, optionally in the form of the racemate; of the enantiomers, diastereoisomers, mixtures of the enantiomers or diastereoisomers or of an individual enantiomer or diastereoisomer; of the bases and/or salts of physiologically acceptable acids, in the preparation of a medicament for the treatment of pain, in particular acute, visceral, neuropathic or chronic pain and inflammatory pain.

20. Use of a substituted sulfonamide derivative according to claim 1 in the preparation of a medicament for the treatment of respiratory diseases.

21. Use of a substituted sulfonamide derivative according to claim 1 in the preparation of a medicament for the treatment of depression, urinary incontinence, diarrhoea, pruritus, alcohol and drug abuse, lack of drive, migraine, diabetes, inflammatory intestinal diseases, neurological diseases, inflammations of the skin, rheumatic diseases, septic shock, reperfusion syndrome, obesity, as an angiogenesis inhibitor and for anxiolysis.

## Revendications

1. Dérivé de sulfonamide substitué de formule
générale I, 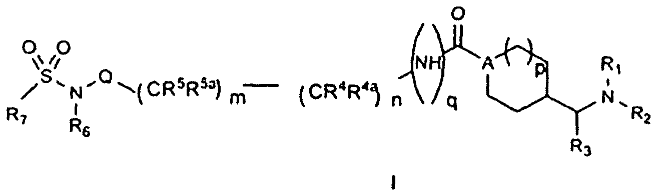 dans laquelle :
m représente 0, 1 ou 2,
n représente 0, 1 ou 2,
p représente 0, 1 ou 2,
q représente 0 ou 1,
A représente N, un groupe CH-NH-, CH-CH₂-NH-, CH-CH₂-CH₂-NH ou CH-CH₂-CH₂-CH₂-NH, dans lesquels les atomes de H individuels peuvent également être remplacés par un groupe alkyle en C₁ à C₅,
R¹ et R² désignent indépendamment l'un de l'autre H ; un alkyle en C₁ à C₆, dans chaque cas saturé ou insaturé, ramifié ou non ramifié, non substitué ou mono- ou polysubstitué ; ou un groupe aryle lié par une chaîne alkyle en C₁ à C₃, non substitué ou mono- ou polysubstitué, où R¹ et R² ne désignent pas simultanément H,
ou les radicaux R¹ et R² désignent ensemble un groupe CH₂CH₂OCH₂CH₂, CH₂CH₂NR⁸CH₂CH₂ ou (CH₂) ₃₋₆,
où R⁸ désigne H ; un groupe alkyle en C₁ à C₆, dans chaque cas saturé ou insaturé, ramifié ou non ramifié, non substitué ou mono- ou polysubstitué ; un groupe aryle, un groupe hétéroaryle, dans chaque cas non substitué ou mono- ou polysubstitué, ou un groupe aryle ou un groupe hétéroaryle lié par une chaîne alkyle en C₁ à C₃, dans chaque cas non substitué ou mono- ou polysubstitué ;
R³ représente un groupe alkyle en C₁ à C₈, dans chaque cas saturé ou insaturé, ramifié ou non ramifié, non substitué ou mono- ou polysubstitué ; un groupe aryle ou groupe un hétéroaryle, dans chaque cas non substitué ou mono- ou polysubstitué ; ou un groupe aryle ou un groupe hétéroaryle lié par une chaîne alkyle en C₁ à C₃, dans chaque cas non substitué ou mono- ou polysubstitué ;
R⁴ et R^{4a} représentent indépendamment l'un de l'autre H, un groupe alkyle en C₁ à C₆, dans chaque cas saturé ou insaturé, ramifié ou non ramifié, non substitué ou mono- ou polysubstitué ; F ; Cl ; un groupe aryle dans chaque cas non substitué ou mono- ou polysubstitué ; ou un groupe aryle lié par une chaîne alkyle en C₁ à C₃, dans chaque cas non substitué ou mono- ou polysubstitué ;
R⁵ et R^{5a} représentent indépendamment l'un de l'autre H, un groupe alkyle en C₁ à C₆, dans chaque cas saturé ou insaturé, ramifié ou non ramifié, non substitué ou mono- ou polysubstitué ; OH, O (alkyle en C₁ à C₆), F, Cl, un groupe phénoxy ou un groupe benzyloxy ;
Q désigne une simple liaison ou un groupe -CH₂-, -CH₂-CH₂-, ou 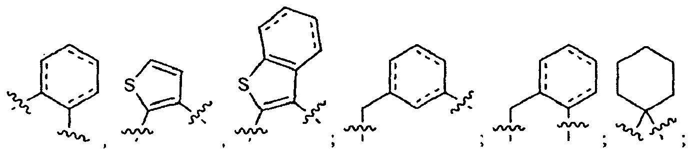 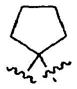
où ---- représente une simple liaison ou une double liaison ;
R⁶ représente H ; un groupe alkyle en C₁ à C₆, dans chaque cas saturé ou insaturé, ramifié ou non ramifié, non substitué ou mono- ou polysubstitué ; un groupe cycloalkyle en C₃ à C₈, saturé ou insaturé, non substitué ou mono- ou polysubstitué ; un groupe aryle ou un groupe hétéroaryle, non substitué ou mono- ou polysubstitué ; un groupe aryle ou un groupe cycloalkyle en C₃ à C₈ lié par une chaîne alkyle en C₁ à C₃ ;
ou R⁶ forme conjointement avec Q, avec la participation de l'atome d'azote voisin, un noyau carbocyclique tétra-, penta-, hexa- ou heptagonal, qui peut être saturé ou insaturé et peut contenir un autre hétéroatome O, S ou N, sur lequel un noyau penta- ou hexagonal, saturé ou insaturé, peut être condensé ; dans lequel, dans le cas de la fermeture de cycle commune, Q représente 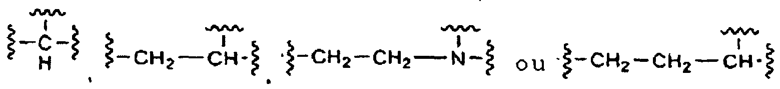
et le cycle peut être substitué en chaque position par un groupe phényle, =O, OH, O (alkyle en C₁ à C₆), F, Cl, CF₃, ou un groupe alkyle en C₁ à C₆ ;
et R⁷ représente un groupe aryle ou un groupe hétéroaryle, dans chaque cas non substitué ou mono- ou polysubstitué ; un groupe aryle ou un groupe hétéroaryle lié par un groupe alkyle en C₁ à C₃, dans chaque cas non substitué ou mono- ou polysubstitué ;
sous la forme du racémate ; des énantiomères, des diastéréoisomères, de mélanges des énantiomères ou des diastéréoisomères ou d'un énantiomère ou d'un diastéréoisomère individuel ; des bases et/ou des sels d'acides physiologiquement acceptables.

2. Dérivé de sulfonamide substitué selon la revendication 1,
dans lequel
les expressions « alkyle substitué » et « cycloalkyle substitué » désignent la substitution d'un ou plusieurs radicaux hydrogène par F, Cl, Br, I, un groupe -CN, NH₂, NH-(alkyle en C₁ à C₆), NH-(alkyle en C₁ à C₆)-OH, alkyle en C₁ à C₆, N- (alkyle en C₁ à C₆)₂, N- ((alkyle en C₁ à C₆) -OH) ₂, NO₂, SH, S- (alkyle en C₁ à C₆), S-benzyle, O- (alkyle en C₁ à C₆), OH, O- (alkyle en C₁ à C₆)-OH, =O, O-benzyle, C(=O) - (alkyle en C₁ à C₆), CO₂H, CO₂-(alkyle en C₁ à C₆) ou benzyle,
les expressions « aryle substitué » et « hétéroaryle substitué » désignent la mono- ou la polysubstitution, par exemple la substitution double, triple ou quadruple, d'un ou plusieurs atomes d'hydrogène du système cyclique par F, Cl, Br, I, un groupe CN, NH₂, NH-(alkyle en C₁ à C₆), NH- (alkyle en C₁ à C₆) -OH, N- (alkyle en C₁ à C₆) 2, N- ( (alkyle en C₁ à C₆)-OH) ₂, NO₂, SH, S- (alkyle en C₁ à C₆), OH, O- (alkyle en C₁ à C₆), O- (alkyle en C₁ à C₆)-OH, C (=O) - (alkyle en C₁ à C₆), CO₂H, CH₂SO₂ -phényle, CO₂-(alkyle en C₁ à C₆),
OCF₃ , CF₃ , 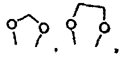 alkyle en C₁ à C₆, phényle, pyridyle, thiényle ou furyle,
sous la forme du racémate ; des énantiomères, des diastéréoisomères, de mélanges des énantiomères ou des diastéréoisomères ou d'un énantiomère ou d'un diastéréoisomère individuel ; des bases et/ou des sels d'acides physiologiquement acceptables.

3. Dérivé de sulfonamide substitué selon la revendication 1, dans lequel A représente un groupe CH-NH-, CH-CH₂-NH-, CH-CH₂-CH₂-NH ou CH-CH₂-CH₂-CH₂-NH, dans lequel les atomes de H individuels peuvent également être remplacés par un groupe alkyle en C₁ à C₅.

4. Dérivé de sulfonamide substitué selon la revendication 1, dans lequel R¹ et R² désignent indépendamment l'un de l'autre H ; un groupe CH₃ ; C₂H₅ ; ou phényle lié par une chaîne alkyle en C₁ à C₃, où R¹ et R² ne désignent pas simultanément H,
ou les radicaux R¹ et R² désignent ensemble un groupe CH₂CH₂OCH₂CH₂, CH₂CH₂NR⁸CH₂CH₂ ou (CH₂)₃₋₅.

5. Dérivé de sulfonamide substitué selon la revendication 1, dans lequel R³ représente un groupe aryle ou un groupe hétéroaryle, dans chaque cas non substitué ou mono- ou polysubstitué ; ou un groupe aryle lié par une chaîne alkyle en C₁ à C₃, dans chaque cas non substitué ou mono- ou polysubstitué.

6. Dérivé de sulfonamide substitué selon la revendication 5, dans lequel R³ représente un groupe 2-thiényle, 4-fluorophényle, 3-fluorophényle, 4-chlorophényle, phénéthyle, phényle ou benzyle.

7. Dérivé de sulfonamide substitué selon la revendication 1, dans lequel R⁴ et R^{4a} représentent H.

8. Dérivé de sulfonamide substitué selon la revendication 1, dans lequel R⁵ et R^{5a} représentent H.

9. Dérivé de sulfonamide substitué selon la revendication 1, dans lequel R⁶ représente un groupe méthyle, éthyle ou benzyle et Q représente une simple liaison, où R⁶ désigne en particulier un groupe méthyle.

10. Dérivé de sulfonamide substitué selon la revendication 1, dans lequel le groupe 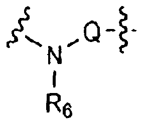 de la formule générale I représente un groupe

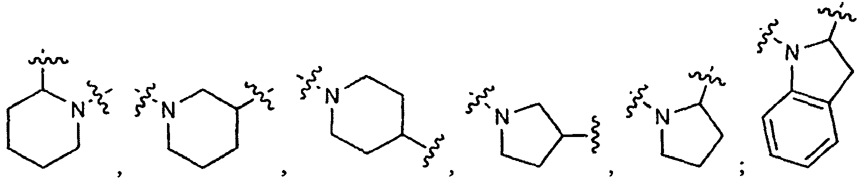

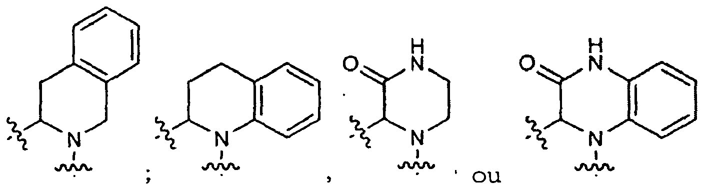

11. Dérivé de sulfonamide substitué selon la revendication 1, dans lequel Q désigne un groupe

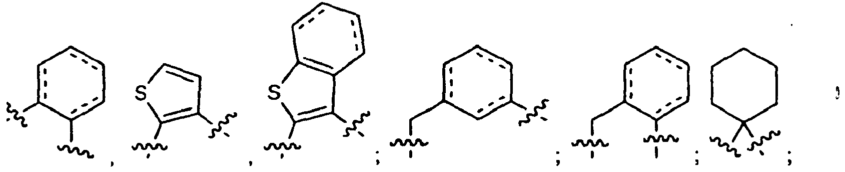

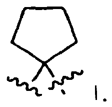

12. Dérivé de sulfonamide substitué selon la revendication 1, dans lequel le groupe 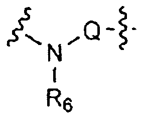 de la formule générale I représente un groupe 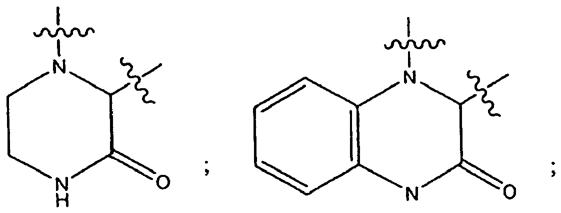 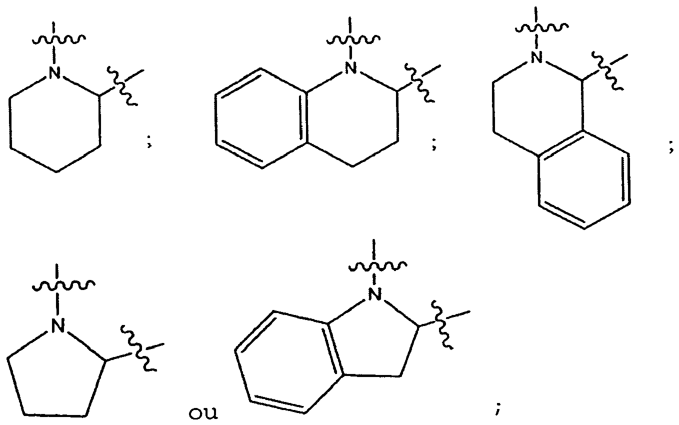
m représente 0, 1 ou 2 ;
n représente 0,
q représente 0 ; et
R⁵ et R^{5a} représentent H ;
ou
représente une simple liaison ;
m représente 0, 1 ou 2 ;
n représente 1 ou 2 ;
q représente 0 ou 1 ;
R⁴ et R^{4a} représentent chacun indépendamment l'un de l'autre H ou un groupe aryle ;
R⁵ et R^{5a} représentent H ; et
R⁶ représente H ou un groupe alkyle en C₁ à C₆ ; ou
Q représente un groupe 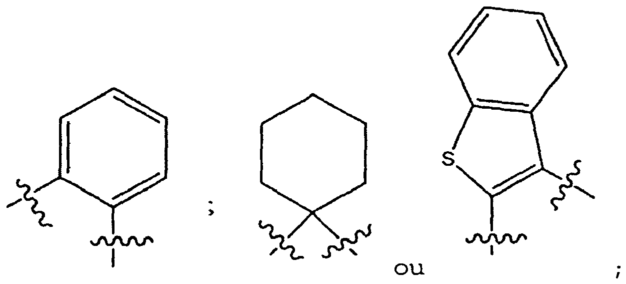
m représente 0 ou 1, de préférence 0 ;
n et q représentent 0 ;
R⁵ et R^{5a} représentent H ; et
R⁶ représente H ou un groupe alkyle en C₁ à C₆.

13. Dérivé de sulfonamide substitué selon la revendication 1, dans lequel dans la formule générale I
A représente N, un groupe NH-CH, NH-CH₂-CH ou NH-CH₂-CH₂-CH, dans lequel les atomes de H individuels peuvent également être remplacés par un groupe alkyle en C₁ à C₅ ;
R¹ et R² représentent indépendamment l'un de l'autre un groupe alkyle en C₁ à C₆, en particulier un groupe méthyle, éthyle, n-propyle, isopropyle, n-butyle, sec.-butyle, isobutyle et tertiobutyle, ou bien forment conjointement avec l'atome de N auquel ils sont liés un groupe choisi parmi : 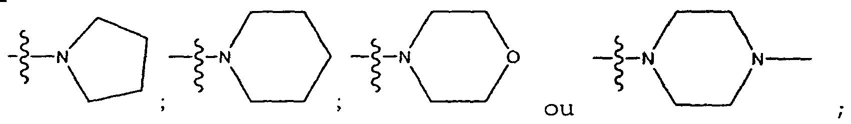 et
R³ représente un groupe aryle, en particulier un groupe phényle ou furanyle, qui peut être lié par un groupe alkyle en C₁ à C₃, groupe aryle qui est dans chaque cas non substitué ou mono- ou polysubstitué, de manière identique ou différente, par des substituants indépendamment choisis dans le groupe comprenant des groupes méthyle, éthyle, méthoxy, éthoxy, F, Cl, Br, F, CN, CF₃, OCF₃ et OH.

14. Dérivé de sulfonamide substitué selon la revendication 1, dans lequel le groupe 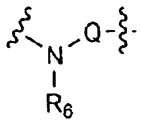 dans la formule générale I représente un groupe 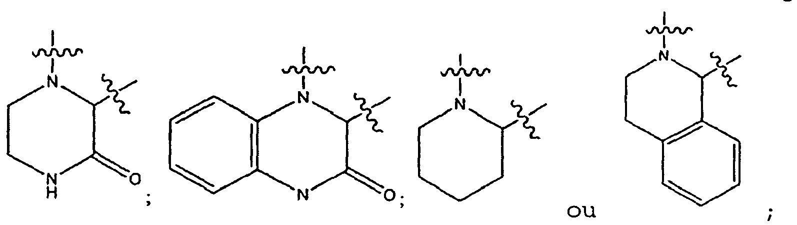
m représente 1 ou 2, en particulier 1 ;
n et q représentent 0 ;
R⁵ et R^{5a} représentent H ;
A représente N, un groupe NH-CH ou NH-CH₂-CH, en particulier N ou NH-CH,
R¹ et R² représentent indépendamment l'un de l'autre un groupe alkyle en C₁ à C₆, en particulier un groupe méthyle, éthyle, n-propyle, isopropyle, n-butyle, sec.-butyle, isobutyle et tertiobutyle, ou bien forment conjointement avec l'atome de N auquel ils sont liés un groupe choisi parme : 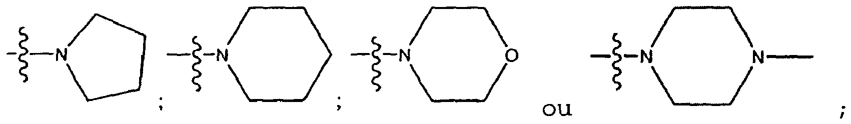 et
R³ représente un groupe phényle, qui peut être lié par un groupe alkyle en C₁ à C₃, groupe phényle qui est dans chaque cas non substitué ou mono- ou polysubstitué, de manière identique ou différente, par des substituants indépendamment choisis dans le groupe comprenant des groupes méthyle, éthyle, méthoxy, éthoxy, F, Cl, Br, F, CN, CF₃, OCF₃ et OH, en particulier un groupe méthyle, méthoxy, F, Cl, Br, CN, CF₃ et OH.

15. Dérivé de sulfonamide substitué selon la revendication 1, dans lequel R⁷ représente un groupe 2,4-dichlorophényle, 2,4,6-trichlorophényle, 4-méthoxyphényle, 3-trifluorométhylphényle, 2,6-diméthyl-4-méthoxyphényle ou 3,4-dichlorophényle.

16. Dérivé de sulfonamide substitué selon la revendication 1 choisi dans le groupe comprenant :
(1) le N-(3-{3-[4-(diméthylaminophénylméthyl)-cyclohexyl]-uréido}-propyl)-4-méthoxy-2,3,6,N-tétraméthylbenzènesulfonamide ;
(2) le (N-(3-(3-((4-((diméthylamino)-(phényl)-méthyl)-cyclohexyl)-méthyl)-uréido)-propyl)-4-méthoxy-N,2,3,6-tétraméthylbenzènesulfonamide ;
(3) le 4-méthoxy-N,2,3,6-tétraméthyl-N-(3-(3-(4-(phényl-(pyrrolidine-1-yl)-méthyl)-cyclohexyl)-uréido)-propyl)-benzènesulfonamide ;
(4) le {3-[(4-méthoxy-2,3,6-triméthyl-benzènesulfonyl)-méthylamino]-propyl}-amide d'acide 4-(diméthylamino-phénylméthyl)-pipéridine-1-carboxylique ;
(5) le [4-(diméthylamino-phényl-méthyl)-cyclohexyl]-amide d'acide 5-[méthyl-(2,4,6-trichloro-benzènesulfonyl)-amino]-pentanecarboxylique ;
(6) le [4-(phényl-pipéridine-1-yl-méthyl)-cyclohexyl]-amide d'acide 5-[méthyl-(2,4,6-trichloro-benzènesulfonyl)-amino]-pentanecarboxylique ;
(7) le [4-(diméthylamino-phényl-méthyl)-cyclohexylméthyl]-amide d'acide 5-[méthyl-(2,4,6-trichloro-benzènesulfonyl)-amino]-pentanecarboxylique ;
(8) le N-[4-(diméthylamino-phényl-méthyl)-cyclohexylméthyl]-2-[1-(3-trifluorométhylbenzènesulfonyl)-pipéridine-2-yl]-acétamide ;
(9) le N-[4-(diméthylamino-phényl-méthyl)-cyclohexyl]-2-[2-(4-méthoxy-benzènesulfonyl)-1,2,3,4-tétrahydro-isoquinoléine-1-yl]-acétamide ;
(10) le 2-[2-(3,4-dichloro-benzènesulfonyl)-1,2,3,4-tétrahydroisoquinoléine-1-yl]-N-[4-(diméthylamino-phényl-méthyl)-cyclohexyl]-acétamide ;
(11) le 2-[(3,4-dichlorobenzènesulfonyl)-méthyl-amino]-N-[4-(diméthylamino-phényl-méthyl)-cyclohexyl]-benzamide ;
(12) le [4-(diméthylamino-phényl-méthyl)-cyclohexyl]-amide d'acide 2-[(3,4-dichloro-benzènesulfonyl)-méthyl-amino]-4,5,6,7-tétrahydro-benzo[b]thiophène-3-carboxylique ;
(13) le N-{4-[diméthylamino-(3-fluorophényl)-méthyl]-cyclohexyl}-2-[2-(4-méthoxy-benzènesulfonyl)-1,2,3,4-tétrahydroisoquinoléine-1-yl]-acétamide ;
(14) le N-{4-[diméthylamino-(4-fluorophényl)-méthyl]-cyclohexyl}-2-[2-(4-méthoxy-benzènesulfonyl)-1,2,3,4-tétrahydroisoquinoléine-1-ylie]-acétamide ;
(15) le N-[4-(diméthylamino-thiophène-2-yl-méthyl)-cyclohexyl]-2-[2-(4-méthoxy-benzènesulfonyl)-1,2,3,4-tétrahydroisoquinoléine-1-yl]-acétamide ;
(16) le N-{4-[diméthylamino-(3-fluorophényl)-méthyl]-cyclohexylméthyl}-2-[2-(4-méthoxy-benzènesulfonyl)-1,2,3,4tétrahydro-isoquinoléine-1-yl]-acétamide ;
(17) le N-[4-(diméthylamino-phényl-méthyl)-cyclohexylméthyl]-2-[2-(4-méthoxy-benzènesulfonyl)-1,2,3,4-tétrahydroisoquinoléine-1-yl]-acétamide ;
(18) le N-[4-(1-diméthylamino-3-phényl-propyl)-cyclohexylméthyl]-2-[2-(4-méthoxy-benzènesulfonyl)-1,2,3,4-tétrahydro-isoquinoléine-1-yl]-acétamide ;
(19) le N-{4-[diméthylamino-(4-fluorophényl)-méthyl]-cyclohexylméthyl}-2-[2-(4-méthoxy-benzènesulfonyl)-1,2,3,4-tétrahydroisoquinoléine-1-yl]-acétamide ;
(20) le N-{4-[(4-chlorophényl)-diméthylamino-méthyl]-cyclohexylméthyl}-2-[2-(4-méthoxy-benzènesulfonyl)-1,2,3,4-tétrahydroisoquinoléine-1-yl]-acétamide ;
(21) le N-[4-(diméthylamino-thiophène-2-yl-méthyl)-cyclohexylméthyl]-2-[2-(4-méthoxy-benzènesulfonyl)-1,2,3,4-tétrahydroisoquinoléine-1-yl]-acétamide ;
(22) le N-(2-{4-[(4-chlorophényl)-diméthylamino-méthyl]-cyclohexyl}-éthyl)-2-[2-(4-méthoxy-benzènesulfonyl)-1,2,3,4-tétrahydro-isoquinoléine-1-yl]-acétamide ;
(23) le N-(2-{4-[diméthylamino-(3-fluorophényl)-méthyl]-cyclohexyl}-éthyl)-2-[2-(4-méthoxy-benzènesulfonyl)-1,2,3,4-tétrahydro-isoquinoléine-1-yl]-acétamide ;
(24) le N-(2-{4-[diméthylamino-(4-fluorophényl)-méthyl]-cyclohexyl}-éthyl)-2-[2-(4-méthoxy-benzènesulfonyl)-1,2,3,4-tétrahydro-isoquinoléine-1-yl]-acétamide ;
(25) le N-{2-[4-(diméthylamino-thiophène-2-yl-méthyl)-cyclohexyl]-éthyl}-2-[2-(4-méthoxy-benzènesulfonyl)-1,2,3,4-tétrahydroisoquinoléine-1-yl]-acétamide ;
(26) le 2-(2-(3,4-dichlorophénylsulfonyl)-1,2,3,4-tétrahydroisoquinoléine-1-yl)-N-(4-((diméthylamino)-(3-fluorophényl)-méthyl)-cyclohexyl)-acétamide ;
(27) le 2-(2-(3,4-dichlorophénylsulfonyl)-1,2,3,4-tétrahydroisoquinoléine-1-yl)-N-(4-((diméthylamino)-(4-fluorophényl)-méthyl)-cyclohexyl)-acétamide ;
(28) le 2-[2-(3,4-dichloro-benzènesulfonyl)-1,2,3,4-tétrahydroisoquinoléiné-1-yl]-N-{4-[diméthylamino-(3-fluorophényl)-méthyl]-cyclohexylméthyl}-acétamide ;
(29) le 2-[2-(3,4-dichloro-benzènesulfonyl)-1,2,3,4-tétrahydroisoquinoléine-1-yl]-N-[4-(diméthylamino-phényl-méthyl)-cyclohexylméthyl]-acétamide ;
(30) le 2-[2-(3,4-dichloro-benzènesulfonyl)-1,2,3,4-tétrahydroisoquinoléine-1-yl]-N-[4-(1-diméthylamino-3-phényl-propyl)-cyclohexylméthyl]-acétamide ;
(31) le 2-[2-(3,4-dichloro-benzènesulfonyl)-1,2,3,4-tétrahydroisoquinoléine-1-yl]-N-{4-[diméthylamino-(4-fluorophényl)-méthyl]-cyclohexylméthyl}-acétamide ;
(32) le N-{4-[(4-chlorophényl)-diméthylamino-méthyl]-cyclohexylméthyl}-2-[2-(3,4-dichloro-benzènesulfonyl)-1,2,3,4-tétrahydro-isoquinoléine-1-yl]-acétamide ;
(33) le N-(2-{4-[(4-chlorophényl)-diméthylamino-méthyl]-cyclohexyl}-éthyl)-2-[2-(3,4-dichloro-benzènesulfonyl)-1,2,3,4-tétrahydro-isoquinoléine-1-yle]-acétamide ;
(34) le 2-[2-(3,4-dichloro-benzènesulfonyl)-1,2,3,4-tétrahydroisoquinoléine-1-yl]-N-(2-{4-[diméthylamino-(3-fluorophényl)-méthyl]-cyclohexyl}-éthyl)-acétamide ;
(35) le 2-[2-(3,4-dichloro-benzènesulfonyl)-1,2,3,4-tétrahydroisoquinoléine-1-yl]-N-(2-{4-[diméthylamino-(4-fluorophényl)-méthyl]-cyclohexyl}-éthyl)-acétamide ;
(36) le 2-[(3,4-dichloro-benzènesulfonyl)-méthyl-amino]-N-{4-[diméthylamino-(3-fluorophényl)-méthyl]-cyclohexyl}-benzamide ;
(37) le 2-[(3,4-dichloro-benzènesulfonyl)-méthyl-amino]-N-{4-[diméthylamino-(4-fluorophényl)-méthyl]-cyclohexyl}-benzamide ;
(38) le 2-[(3,4-dichloro-benzènesulfonyl)-méthyl-amino]-N-[4-(diméthylamino-phénylméthyl)-cyclohexylméthyl]-benzamide ;
(39) le 2-[(3,4-dichloro-benzènesulfonyl)-méthyl-amino]-N-[4-(1-diméthylamino-3-phényl-propyl)-cyclohexylméthyl]-benzamide ;
(40) le 2-[(3,4-dichloro-benzènesulfonyl)-méthyl-amino]-N-{4-[diméthylamino-(4-fluorophényl)-méthyl]-cyclohexylméthyl}-benzamide ;
(41) le N-{4-[(4-chlorophényl)-diméthylamino-méthyl]-cyclohexylméthyl}-2-[(3,4-dichloro-benzènesulfonyl)-méthyl-amino]-benzamide ;
(42) le 2-[(3,4-dichloro-benzènesulfonyl)-méthyl-amino]-N-[4-(diméthylamino-thiophène-2-yl-méthyl)-cyclohexylméthyl]-benzamide ;
(43) le N-(2-{4-[(4-chlorophényl)-diméthylamino-méthyl]-cyclohexyl}-éthyl)-2-[(3,4-dichloro-benzènesulfonyl)-méthyl-amino]-benzamide ;
(44) le 2-[(3,4-dichloro-benzènesulfonyl)-méthyl-amino]-N-(2-{4-[diméthylamino-(3-fluoro-phényl)-méthyl]-cyclohexyl}éthyl)-benzamide ;
(45) le 2-[(3,4-dichloro-benzènesulfonyl)-méthyl-amino]-N-(2-{4-[diméthylamino-(4-fluorophényl)-méthyl]-cyclohexyl}éthyl)-benzamide ;
(46) le [4-(diméthylamino-phényl-méthyl)-cyclohexylméthyl]-amide d'acide 2-[(3,4-dichloro-benzènesulfonyl)-méthylamino]-4,5,6,7-tétrahydro-benzo[b]thiophène-3-carboxylique ;
(47) le {4-[diméthylamino-(4-fluorophényl)-méthyl]-cyclohexylméthyl}-amide d'acide 2-[(3,4-dichloro-benzènesulfonyl)-méthyl-amino]-4,5,6,7-tétrahydro-benzo[b]thiophène-3-carboxylique ;
(48) le {4-[(4-chlorophényl)-diméthylamino-méthyl]-cyclohexylméthyl}-amide d'acide 2-[(3,4-dichloro-benzènesulfonyl)-méthyl-amino]-4,5,6,7-tétrahydro-benzo[b]thiophène-3-carboxylique ;
(49) le [4-(diméthylamino-thiophène-2-yl-méthyl)-cyclohexylméthyl]-amide d'acide 2-[(3,4-dichloro-benzènesulfonyl)-méthyl-amino]-4,5,6,7-tétrahydro-benzo[b]thiophène-3-carboxylique ;
(50) le N-(2-(4-((4-chlorophényl)-(diméthylamino)-méthyl)-cyclohexyl)-éthyl)-2-(3,4-dichloro-N-méthylphénylsulfonamido)-4,5,6,7-tétrahydrobenzo[b]thiophène-3-carboxamide ;
(51) le 2-(3,4-dichloro-N-méthylphénylsulfonamido)-N-(2-(4-((diméthylamino)-(3-fluorophényl)-méthyl)-cyclohexyl)-éthyl)-4,5,6,7-tétrahydrobenzo[b]thiophène-3-carboxamide ;
(52) le 2-(3,4-dichloro-N-méthylphénylsulfonamido)-N-(2-(4-((diméthylamino)-(4-fluorophényl)-méthyl)-cyclohexyl)-éthyl)-4,5,6,7-tétrahydrobenzo[b]thiophène-3-carboxamide ;
(53) le 2-(3,4-dichloro-N-méthylphénylsulfonamido)-N-(2-(4-((diméthylamino)-(thiophène-2-yl)-méthyl)-cyclohexyl)-éthyl)-4,5,6,7-tétrahydrobenzo[b]thiophène-3-carboxamide ;
(54) le 2-[1-(3,4-dichloro-benzènesulfonyl)-3-oxo-1,2,3,4-tétrahydro-quinoxaline-2-yl]-N-[4-(morpholine-4-yl-phénylméthyl)-cyclohexyl]-acétamide ;
(55) le 2-[1-(2,4-dichloro-benzènesulfonyl)-3-oxo-pipérazine-2-yl]-N-[4-(morpholine-4-yl-phényl-méthyl)-cyclohexylméthyl]-acétamide ;
(56) le 2-[1-(2,4-dichloro-benzènesulfonyl)-3-oxo-pipérazine-2-yl]-N-[4-(1-morpholine-4-yl-3-phényl-propyl)-cyclohexylméthyl]-acétamide ;
(57) le 2-[1-(2,4-dichloro-benzènesulfonyl)-3-oxo-pipérazine-2-yl]-N- [4-(3-phényl-1-pyrrolidine-1-yl-propyl)-cyclohexylméthyl]-acétamide ;
(58) le 2-[1-(2,4-dichloro-benzènesulfonyl)-3-oxo-pipérazine-2-yl]-N-[4-(phényl-pyrrolidine-1-yl-méthyl)-cyclohexylméthyl]-acétamide ;
(59) le 2-[1-(2,4-dichloro-benzènesulfonyl)-3-oxo-pipérazine-2-yl]-N-[4-(3-phényl-1-pipéridine-1-yl-propyl)-cyclohexyl]-acétamide ;
(60) le 2-[1-(2,4-dichloro-benzènesulfonyl)-3-oxo-pipérazine-2-yl]-N-[4-(1-morpholine-4-yl-3-phényl-propyl)-cyclohexyl]-acétamide ;
(61) le 2-[1-(2,4-dichloro-benzènesulfonyl)-3-oxo-pipérazine-2-yl]-N-{4-[(4-méthyl-pipérazine-1-yl)-phényl-méthyl]-cyclohexyl}-acétamide ;
(62) la 4-(2,4-dichloro-benzènesulfonyl)-3-{2-[4-(morpholine-4-yl-phényl-méthyl)-pipéridine-1-yl]-2-oxo-éthyl}-pipérazine-2-one ;
(63) la 4-(2,4-dichlorophénylsulfonyl)-3-(2-(2-(1-morpholino-2-phényléthyl)-pipéridine-1-yl)-2-oxoéthyl)-pipérazine-2-one ;
(64) le 2-[1-(3,4-dichloro-benzènesulfonyl)-3-oxo-1,2,3,4-tétrahydro-quinoxaline-2-yl]-N-[4-(morpholine-4-ylphényl-méthyl)-cyclohexylméthyl]-acétamide ;
(65) le 2-[1-(3,4-dichloro-benzènesulfonyl)-3-oxo-1,2,3,4-tétrahydro-quinoxaline-2-yl]-N-[4-(1-morpholine-4-yl-3-phényl-propyl)-cyclohexylméthyl]-acétamide ;
(66) le 2-[1-(3,4-dichloro-benzènesulfonyl)-3-oxo-1,2,3,4-tétrahydro-quinoxaline-2-yl]-N-[4-(3-phényl-1-pyrrolidine-1-yl-propyl)-cyclohexylméthyl]-acétamide ;
(67) le 2-[1-(3,4-dichloro-benzènesulfonyl)-3-oxo-1,2,3,4-tétrahydro-quinoxaline-2-yl]-N-[4-(phényl-pyrrolidine-1-yl-méthyl)-cyclohexylméthyl]-acétamide ;
(68) le 2-[1-(3,4-dichloro-benzènesulfonyl)-3-oxo-1,2,3,4-tétrahydro-quinoxaline-2-yle]-N-[4-(3-phényl-1-pipéridine-1-yl-propyl)-cyclohexyl]-acétamide ;
(69) le 2-[1-(3,4-dichloro-benzènesulfonyl)-3-oxo-1,2,3,4-tétrahydro-quinoxaline-2-yl]-N-[4-(1-morpholine-4-yl-3-phényl-propyl)-cyclohexyl]-acétamide ;
(70) le 2-[1-(3,4-dichloro-benzènesulfonyl)-3-oxo-1,2,3,4-tétrahydro-quinoxaline-2-yl]-N-{4-[(4-méthyl-pipérazine-1-yl)-phényl-méthyl]-cyclohexyl}-acétamide ;
(71) la 4-(3,4-dichloro-benzènesulfonyl)-3-{2-[4-(morpholine-4-yl-phényl-méthyl)-pipéridine-1-yle]-2-oxo-éthyl}-3,4-dihydro-1H-quinoxaline-2-one ;
(72) la 4-(3,4-dichlorophénylsulfonyl)-3-(2-(4-(1-morpholino-2-phényléthyl)-pipéridine-1-yl)-2-oxoéthyl)-3,4-dihydroquinoxaline-2-(1H)-one ;
(73) le 2-[1-(3,4-dichloro-benzènesulfonyl)-pyrrolidine-2-yl]-N-[4-(morpholine-4-yl-phényl-méthyl)-cyclohexylméthyl]-acétamide ;
(74) le 2-[1-(3,4-dichloro-benzènesulfonyl)-pyrrolidine-2-yl]-N-[4-(1-morpholine-4-yl-3-phényl-propyl)-cyclohexylméthyl]-acétamide ;
(75) le 2-[1-(3,4-dichloro-benzènesulfonyl)-pyrrolidine-2-yl]-N-[4-(3-phényl-1-pyrrolidine-1-yl-propyl)-cyclohexylméthyl]-acétamide ;
(76) le 2-[1-(3,4-dichloro-benzènesulfonyl)-pyrrolidine-2-yle]-N-[4-(phényl-pyrrolidine-1-yl-méthyl)-cyclohexylméthyl]-acétamide ;
(77) le 2-(1-(3,4-dichlorophénylsulfonyl)-pyrrolidine-2-yl)-N-(4-(3-phényl-1-(pipéridine-1-yl)-propyl)-cyclohexyl)-acétamide ;
(78) le 2-(1-(3,4-dichlorophénylsulfonyl)-pyrrolidine-2-yl)-N-(4-(1-morpholino-3-phénylpropyl)-cyclohexyl)-acétamide ;
(79) le 2-[1-(3,4-dichloro-benzènesulfonyl)-pyrrolidine-2-yl]-N-{4-[(4-méthyl-pipérazine-1-yl)-phényl-méthyl]-cyclohexyl}-acétamide ;
(80) la 2-[1-(3,4-dichloro-benzènesulfonyl)-pyrrolidine-2-yl]-1-[4-(morpholine-4-yl-phénylméthyl)-pipéridine-1-yl]-éthanone ;
(81) la 2-(1-(3,4-dichlorophénylsulfonyl)-pyrrolidine-2-yl)-1-(4-(1-morpholino-2-phényléthyl)-pipéridine-1-yl)-éthanone ;
(82) le 2-[1-(3,4-dichloro-benzènesulfonyl)-pipéridine-2-yl]-N-[4-(morpholine-4-yl-phénylméthyl)-cyclohexylméthyl]-acétamide ;
(83) le 2-[1-(3,4-dichloro-benzènesulfonyl)-pipéridine-2-yl]-N-[4-(1-morpholine-4-yl-3-phényl-propyl)-cyclohexylméthyl]-acétamide ;
(84) le 2-[1-(3,4-dichloro-benzènesulfonyl)-pipéridine-2-yl]-N-[4-(3-phényl-1-pyrrolidine-1-yl-propyl)-cyclohexylméthyl]-acétamide ;
(85) le 2-[1-(3,4-dichloro-benzènesulfonyl)-pipéridine-2-yl]-N-[4-(phényl-pyrrolidine-1-yl-méthyl)-cyclohexylméthyl]-acétamide ;
(86) le 2-[1-(3,4-dichloro-benzènesulfonyl)-pipéridine-2-yl]-N-[4-(3-phényl-1-pipéridine-1-yl-propyl)-cyclohexyl]-acétamide ;
(87) le 2-[1-(3,4-dichloro-benzènesulfonyl)-pipéridine-2-yl]-N-[4-(1-morpholine-4-yl-3-phényl-propyl)-cyclohexyl]-acétamide ;
(88) le 2-[1-(3,4-dichloro-benzènesulfonyl)-pipéridine-2-yl]-N-{4-[(4-méthyl-pipérazine-1-yl)-phényl-méthyl]-cyclohexyl}-acétamide ;
(89) la 2-[1-(3,4-dichloro-benzènesulfonyl)-pipéridine-2-yl]-1-[4-(morpholine-4-yl-phényl-méthyl)-pipéridine-1-yl]-éthanone ;
(90) la 2-(1-(3,4-dichlorophénylsulfonyl)-pipéridine-2-yl)-1-(4-(1-morpholino-2-phényléthyl)-pipéridine-1-yl)-éthanone ;
(91) le 1-(3,4-dichlorophénylsulfonyl)-N-(4-((diméthylamino)-(phényl)-méthyl)-cyclohexyl-indoline-2-carboxamide ;
(92) le 1-(3,4-dichlorophénylsulfonyl)-N-(4-((diméthylamino)-(3-fluorophényl)-méthyl)-cyclohexyl)-indoline-2-carboxamide ;
(93) le 1-(3,4-dichlorophénylsulfonyl)-N-(4-(1-(diméthylamino)-3-phénylpropyl)-cyclohexyl)-indoline-2-carboxamide ;
(94) le 1-(3,4-dichlorophénylsulfonyl)-N-(4-((diméthylamino)-(4-fluorophényl)-méthyl)-cyclohexyl)-indoline-2-carboxamide ;
(95) le 1-(3,4-dichlorophénylsulfonyl)-N-((4-((diméthylamino)-(phényl)-méthyl)-cyclohexyl)-méthyl)-indoline-2-carboxamide ;
(96) le 1-(3,4-dichlorophénylsulfonyl)-N-((4-(1-(diméthylamino)-3-phénylpropyl)-cyclohexyl)-méthyl)-indoline-2-carboxamide ;
(97) le 1-(3,4-dichlorophénylsulfonyl)-N-(2-(4-(1-(diméthylamino)-3-phénylpropyl)-cyclohexyl)-éthyl)-indoline-2-carboxamide ;
(98) le 2-(2-(3,4-dichloro-N-méthylphénylsulfonamido)-phényl)-N-(2-(4-((diméthylamino)-(phényl)-méthyl)-cyclohexyl)-éthyl)-acétamide,
(99) le 2-(2-(3,4-dichloro-N-méthylphénylsulfonamido)-phényl)-N-(2-(4-(1-(diméthylamino)-3-phénylpropyl)-cyclohexyl)-éthyl-acétamide ;
(100) le N-(2-(4-((diméthylamino)-(phényl)-méthyl)-cyclohexyl)-éthyl)-1-(4-méthoxy-N-méthylphénylsulfonamido)-cyclohexanecarboxamide ;
(101) le 2-(1-(2,4-dichlorophénylsulfonyl)-3-oxopipérazine-2-yl)-N-(4-(morpholino-(phényl)-méthyl)-cyclohexyl)-acétamide ;
(102) le 2-(1-(2,4-dichlorophénylsulfonyl)-3-oxopipérazine-2-yl)-N-(4-(phényl-(pyrrolidine-1-yl)-méthyl)-cyclohexyl)-acétamide ;
(103) le 2-(1-(3,4-dichlorophénylsulfonyl)-3-oxo-1,2,3,4-tétrahydroquinoxaline-2-yl)-N-(4-(phényl-(pyrrolidine-1-yl)-méthyl)-cyclohexyl)-acétamide ;
(104) le 2-(1-(3,4-dichlorophénylsulfonyl)-pyrrolidine-2-yl)-N-(4-(morpholino-(phényl)-méthyl)-cyclohexyl)-acétamide ;
(105) le 2-(1-(3,4-dichlorophénylsulfonyl)-pyrrolidine-2-yl)-N-(4-(phényl-(pyrrolidine-1-yl)-méthyl)-cyclohexyl)-acétamide ;
(106) le 2-(1-(3,4-dichlorophénylsulfonyl)-pipéridine-2-yl)-N-(4-(morpholino-(phényl)-méthyl)-cyclohexyl)-acétamide ;
(107) le 2-(1-(3,4-dichlorophénylsulfonyl)-pipéridine-2-yl)-N-(4-(phényl-(pyrrolidine-1-yl)-méthyl)-cyclohexyl)-acétamide ;
(108) la 1-(4-((4-méthylpipérazine-1-yl)-(phényl)-méthyl)-pipéridine-1-yl)-2-(1-(3-(trifluorométhyl)-phénylsulfonyl)-pipéridine-2-yl)-éthanone ;
(109) la 1-(4-((4-fluorophényl)-(4-méthylpipérazine-1-yl)-méthyl)-pipéridine-1-yl)-2-(1-(3-(trifluorométhyl)-phénylsulfonyl)-pipéridine-2-yl)-éthanone ;
(110) le N-(3-(4-((3-fluorophényl)-(4-méthylpipérazine-1-yl)-méthyl)-pipéridine-1-yl)-3-oxo-1-phénylpropyl)-naphtaline-2-sulfonamide ;
(111) la 1-(4-(1-(4-méthylpipérazine-1-yl)-2-phényléthyl)-pipéridine-1-yl)-2-(1-(3-(trifluorométhyl)-phénylsulfonyl)-pipéridine-2-yl)-éthanone ;
(112) la 1-(4-(1-(4-méthylpipérazine-1-yl)-3-phénylpropyl)-pipéridine-1-yl)-2-(1-(3-(trifluorométhyl)-phénylsulfonyl)-pipéridine-2-yl)-éthanone ;
(113) le N-(3-(4 (1-(4-méthylpipérazine-1-yl)-3-phénylpropyl)-pipéridine-1-yl)-3-oxo-1-phénylpropyl)-naphtalène-2-sulfonamide ;
(114) le N-(3-(4-((4-méthylpipérazine-1-yl)-(phényl)-méthyl)-pipéridine-1-yl)-3-oxo-1-phénylpropyl)-naphtaline-2-sulfonamide ;
(115) la 1-(4-((3-fluorophényl)-(4-méthylpipérazine-1-yl)-méthyl)-pipéridine-1-yl)-2-(1-(3-(trifluorométhyl)-phénylsulfonyl)-pipéridine-2-yl)-éthanone ;
(116) le N-(3-(4-(1-(4-méthylpipérazine-1-yl)-2-phényléthyl)-pipéridine-1-yl)-3-oxo-1-phénylpropyl)-naphtaline-2-sulfonamide ;
(117) le N-(3-(4-((4-fluorophényl) (4-méthylpipérazine-1-yl)-méthyl)-pipéridine-1-yl)-3-oxo-1-phénylpropyl)-naphtalène-2-sulfonamide ;
(118) la 3-(1-(4-chloro-2,5-diméthylphénylsulfonyl)-pipéridine-2-yl)-1-(4-(1-(4-méthyl-pipérazine-1-yl)-2-phényléthyl)-pipéridine-1-yl)-propane-1-one ;
(119) la 3-(1-(4-chloro-2,5-diméthylphénylsulfonyl)-pipéridine-2-yl)-1-(4-(1-(4-méthyl-pipérazine-1-yl)-3-phénylpropyl)-pipéridine-1-yl)-propane-1-one ;
(120) la 3-(1-(4-chloro-2,5-diméthylphénylsulfonyl)-pipéridine-2-yl)-1-(4-((3-fluorophényl)-(4-méthylpipérazine-1-yl)-méthyl)-pipéridine-1-yl)-propane-1-one ;
(121) la 3-(1-(4-chloro-2,5-diméthylphénylsulfonyl)-pipéridine-2-yl)-1-(4-((4-fluorophényl)-(4-méthylpipérazine-1-yl)-méthyl)-pipéridine-1-yl)-propane-1-one ;
(122) la 3-(1-(4-chloro-2,5-diméthylphénylsulfonyl)-pipéridine-2-yl)-1-(4-((4-méthylpipérazine-1-yl)-(phényl)-méthyl)-pipéridine-1-yl)-propane-1-one ;
sous la forme du racémate ; des énantiomères, des diastéréoisomères, de mélanges des énantiomères ou des diastéréoisomères ou d'un énantiomère ou d'un diastéréoisomère individuel ; des bases et/ou des sels d'acides physiologiquement acceptables.

17. Procédé de préparation d'un dérivé de sulfonamide substitué selon la formule Ia, dans laquelle q représente 0 et les radicaux ou les groupes R¹ à R⁸, R^{H}, R^{J}, A, Z et Q ainsi que m, n et p ont la signification donnée dans la revendication 1, dans lequel des acides carboxyliques III sont amenés à réagir avec des amines primaires ou secondaires de formule générale II 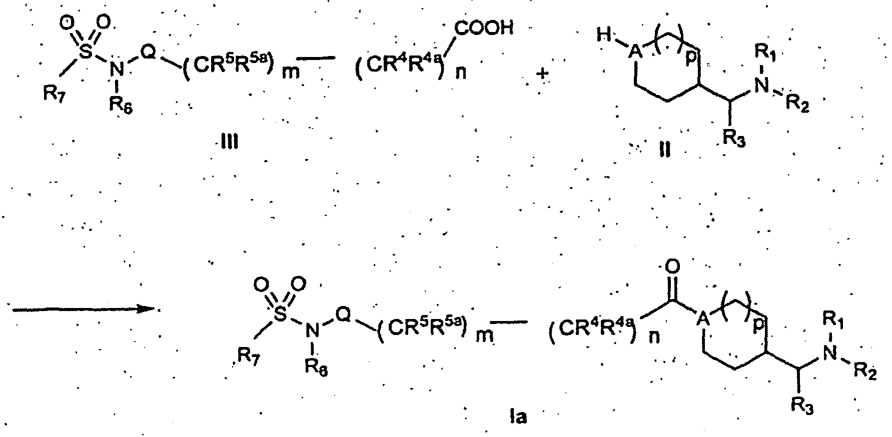 en présence d'un agent déshydratant, par exemple du sulfate de sodium ou de magnésium, de l'oxyde de phosphore ou des réactifs comme par exemple les CDI, DCC (éventuellement lié à un polymère), TBTU, EDCI, PyBOP ou PFPTFA, éventuellement en présence de HOAt ou de HOBt et d'une base organique, par exemple la DIPEA ou la pyridine, dans un solvant organique comme le THF, le dichlorométhane, l'éther diéthylique, le dioxanne, le DMF ou l'acétonitrile.

18. Médicament contenant au moins un dérivé de sulfonamide substitué selon la revendication 1, éventuellement sous la forme du racémate ; des énantiomères, des diastéréoisomères, de mélanges des énantiomères ou des diastéréoisomères ou d'un énantiomère ou d'un diastéréoisomère individuel ; des bases et/ou des sels d'acides physiologiquement acceptables, et contenant éventuellement des additifs et/ou des adjuvants appropriés et/ou éventuellement d'autres substances actives.

19. Utilisation d'un dérivé de sulfonamide substitué selon la revendication 1, éventuellement sous la forme du racémate ; des énantiomères, des diastéréoisomères, de mélanges des énantiomères ou des diastéréoisomères ou d'un énantiomère ou d'un diastéréoisomère individuel ; des bases et/ou des sels d'acides physiologiquement acceptables, pour la préparation d'un médicament pour le traitement de la douleur, en particulier de la douleur aiguë, viscérale, neuropathique ou chronique et de la douleur inflammatoire.

20. Utilisation d'un dérivé de sulfonamide substitué selon la revendication 1, pour la préparation d'un médicament pour le traitement des maladies des voies respiratoires.

21. Utilisation d'un dérivé de sulfonamide substitué selon la revendication 1, pour la préparation d'un médicament pour le traitement de la dépression, de l'incontinence urinaire, de la diarrhée, du prurit, de l'abus d'alcool et de drogues, de la somnolence, de la migraine, du diabète, des maladies intestinales inflammatoires, des maladies neurologiques, des inflammations de la peau, des maladies rhumatismales, du choc septique, du syndrome de reperfusion, de l'obésité, en tant qu'inhibiteur de l'angiogenèse et pour l'anxiolyse.
